# EUROPEAN PATENT SPECIFICATION

(11) **EP 3 057 614 B1**
(45) Date of publication and mention of the grant of the patent: **30.09.2020**
(21) Application number: 14734570.6
(22) Date of filing: 14.05.2014
(51) Int. Cl.: A61K 39/395, A61K 31/277, C07K 16/30, A61P 35/00

(54) **COMBINATION THERAPIES WITH PSMA LIGAND CONJUGATES**
KOMBINATIONSTHERAPIEN MIT PSMA-LIGANDEN-KONJUGATEN
POLYTHÉRAPIES AVEC CONJUGUÉS PSMA LIGAND

(30) Priority: 18.10.2013 US 201361893145 P; 13.11.2013 JP 2013235506; 13.11.2013 US 201361903589 P
(43) Date of publication of application: 24.08.2016
(73) Proprietor: Psma Development Company, L.L.C., Tarrytown, New York 10591 (US)
(72) Inventor: OLSON, William C., Yorktown Heights, NY 10598 (US); DIPIPPO, Vincent, Mahopac, NY 10541 (US)
(74) Representative: Kehoe, Laura Ellen
(86) International application number: PCT/US2014/000124
(87) International publication number: WO 2015/057250

(56) References cited:
- WO-A2-2007/002222
- WO-A2-2010/027513
- DIPIPPO: "Antiandrogen modulation of prostate-specific membrane antigen (PSMA): Dynamics and synergy with PSMA-targeted therapy.", JOURNAL OF CLINICAL ONCOLOGY, 2013 ASCO ANNUAL MEETING ABSTRACTS., vol. 31, no. 15, E16007, 20 May 2013 (2013-05-20), page 1, XP002729554,
- Weill Medical College Of Cornell University: "Docetaxel/Prednisone Plus Fractionated 177Lu- J591 Antibody for Metastatic, Castrate-resistant Prostate Cancer - Full Text View - ClinicalTrials.gov", , 5 June 2009 (2009-06-05), pages 1-6, XP055139381, Retrieved from the Internet: URL:http://clinicaltrials.gov/show/NCT0091 6123 [retrieved on 2014-09-10]
- Weill Medical College Of Cornell University: "177Lu Radiolabeled Monoclonal Antibody HuJ591 (177Lu-J591) and Ketoconazole in Patients With Prostate Cancer - Full Text View - ClinicalTrials.gov", , 10 March 2009 (2009-03-10), XP055139382, Retrieved from the Internet: URL:http://clinicaltrials.gov/ct2/show/NCT 00859781?term=00859781&rank=1 [retrieved on 2014-09-10]
- GAN LU ET AL: "Inhibition of the Androgen Receptor as a Novel Mechanism of Taxol Chemotherapy in Prostate Cancer", CANCER RESEARCH, AMERICAN ASSOCIATION FOR CANCER RESEARCH, US, vol. 69, no. 21, 1 November 2009 (2009-11-01), pages 8386-8394, XP009175194, ISSN: 0008-5472, DOI: 10.1158/0008-5472.CAN-09-1504
- West Lafayette ET AL: "Endocyte to Advance Development of EC1069, a PSMA-Targeted Small Molecule Drug Conjugate Therapy for Prostate Cancer Company Expects to File IND for Therapeutic Agent in 2013 Human Study of Companion Imaging Agent EC0652 Demonstrates Safety and Binding Specificity", , 29 January 2013 (2013-01-29), XP055359848, Retrieved from the Internet: URL:http://files.shareholder.com/downloads /ABEA-5OM1NY/4169323026x0x631497/7C4FDCF6- 95E9-44F1-90F5-7D8254E0A40B/ECYT_News_2013 _1_29_General_Releases.pdf

### SEQUENCE LISTING

<110> PSMA Development Company, LLC
   Olson, William C.
<120> COMBINATION THERAPIES WITH PSMA LIGAND CONJUIGATES
<130> P0741.70014WO00
<140> TBD
   <141> 2014-05-14
<150> US 61/893,145
   <151> 2013-10-18
<150> US 61/903,589
   <151> 2013-11-13
<150> JP2013-235506
   <151> 2013-11-13
<160> 7
<170> PatentIn version 3.5
<210> 1
   <211> 750
   <212> PRT
   <213> Homo sapiens
<400> 1
   Met Trp Asn Leu Leu His Glu Thr Asp Ser Ala Val Ala Thr Ala Arg
   1 5 10 15
   Arg Pro Arg Trp Leu Cys Ala Gly Ala Leu Val Leu Ala Gly Gly Phe
   20 25 30
   Phe Leu Leu Gly Phe Leu Phe Gly Trp Phe Ile Lys Ser Ser Asn Glu
   35 40 45
   Ala Thr Asn Ile Thr Pro Lys His Asn Met Lys Ala Phe Leu Asp Glu
   50 55 60
   Leu Lys Ala Glu Asn Ile Lys Lys Phe Leu Tyr Asn Phe Thr Gln Ile
   65 70 75 80
   Pro His Leu Ala Gly Thr Glu Gln Asn Phe Gln Leu Ala Lys Gln Ile
   85 90 95
   Gln Ser Gln Trp Lys Glu Phe Gly Leu Asp Ser Val Glu Leu Ala His
   100 105 110
   Tyr Asp Val Leu Leu Ser Tyr Pro Asn Lys Thr His Pro Asn Tyr Ile
   115 120 125
   Ser Ile Ile Asn Glu Asp Gly Asn Glu Ile Phe Asn Thr Ser Leu Phe
       130                 135                 140
   Glu Pro Pro Pro Pro Gly Tyr Glu Asn Val Ser Asp Ile Val Pro Pro
   145                 150                 155                 160
   Phe Ser Ala Phe Ser Pro Gln Gly Met Pro Glu Gly Asp Leu Val Tyr
                   165                 170                 175
   Val Asn Tyr Ala Arg Thr Glu Asp Phe Phe Lys Leu Glu Arg Asp Met
               180                 185                 190
   Lys Ile Asn Cys Ser Gly Lys Ile Val Ile Ala Arg Tyr Gly Lys Val
           195                 200                 205
   Phe Arg Gly Asn Lys Val Lys Asn Ala Gln Leu Ala Gly Ala Lys Gly
       210                 215                 220
   Val Ile Leu Tyr Ser Asp Pro Ala Asp Tyr Phe Ala Pro Gly Val Lys
   225                 230                 235                 240
   Ser Tyr Pro Asp Gly Trp Asn Leu Pro Gly Gly Gly Val Gln Arg Gly
                   245                 250                 255
   Asn Ile Leu Asn Leu Asn Gly Ala Gly Asp Pro Leu Thr Pro Gly Tyr
               260                 265                 270
   Pro Ala Asn Glu Tyr Ala Tyr Arg Arg Gly Ile Ala Glu Ala Val Gly
           275                 280                 285
   Leu Pro Ser Ile Pro Val His Pro Ile Gly Tyr Tyr Asp Ala Gln Lys
       290                 295                 300
   Leu Leu Glu Lys Met Gly Gly Ser Ala Pro Pro Asp Ser Ser Trp Arg
   305                 310                 315                 320
   Gly Ser Leu Lys Val Pro Tyr Asn Val Gly Pro Gly Phe Thr Gly Asn
                   325                 330                 335
   Phe Ser Thr Gln Lys Val Lys Met His Ile His Ser Thr Asn Glu Val
               340                 345                 350
   Thr Arg Ile Tyr Asn Val Ile Gly Thr Leu Arg Gly Ala Val Glu Pro
           355                 360                 365
   Asp Arg Tyr Val Ile Leu Gly Gly His Arg Asp Ser Trp Val Phe Gly
       370                 375                 380
   Gly Ile Asp Pro Gln Ser Gly Ala Ala Val Val His Glu Ile Val Arg
   385 390 395 400
   Ser Phe Gly Thr Leu Lys Lys Glu Gly Trp Arg Pro Arg Arg Thr Ile
   405 410 415
   Leu Phe Ala Ser Trp Asp Ala Glu Glu Phe Gly Leu Leu Gly Ser Thr
   420 425 430
   Glu Trp Ala Glu Glu Asn Ser Arg Leu Leu Gln Glu Arg Gly Val Ala
   435 440 445
   Tyr Ile Asn Ala Asp Ser Ser Ile Glu Gly Asn Tyr Thr Leu Arg Val
   450 455 460
   Asp Cys Thr Pro Leu Met Tyr Ser Leu Val His Asn Leu Thr Lys Glu
   465 470 475 480
   Leu Lys Ser Pro Asp Glu Gly Phe Glu Gly Lys Ser Leu Tyr Glu Ser
   485 490 495
   Trp Thr Lys Lys Ser Pro Ser Pro Glu Phe Ser Gly Met Pro Arg Ile
   500 505 510
   Ser Lys Leu Gly Ser Gly Asn Asp Phe Glu Val Phe Phe Gln Arg Leu
   515 520 525
   Gly Ile Ala Ser Gly Arg Ala Arg Tyr Thr Lys Asn Trp Glu Thr Asn
   530 535 540
   Lys Phe Ser Gly Tyr Pro Leu Tyr His Ser Val Tyr Glu Thr Tyr Glu
   545 550 555 560
   Leu Val Glu Lys Phe Tyr Asp Pro Met Phe Lys Tyr His Leu Thr Val
   565 570 575
   Ala Gln Val Arg Gly Gly Met Val Phe Glu Leu Ala Asn Ser Ile Val
   580 585 590
   Leu Pro Phe Asp Cys Arg Asp Tyr Ala Val Val Leu Arg Lys Tyr Ala
   595 600 605
   Asp Lys Ile Tyr Ser Ile Ser Met Lys His Pro Gln Glu Met Lys Thr
   610 615 620
   Tyr Ser Val Ser Phe Asp Ser Leu Phe Ser Ala Val Lys Asn Phe Thr
   625 630 635 640
   Glu Ile Ala Ser Lys Phe Ser Glu Arg Leu Gln Asp Phe Asp Lys Ser
                   645                 650                 655
   Asn Pro Ile Val Leu Arg Met Met Asn Asp Gln Leu Met Phe Leu Glu
               660                 665                 670
   Arg Ala Phe Ile Asp Pro Leu Gly Leu Pro Asp Arg Pro Phe Tyr Arg
           675                 680                 685
   His Val Ile Tyr Ala Pro Ser Ser His Asn Lys Tyr Ala Gly Glu Ser
       690                 695                 700
   Phe Pro Gly Ile Tyr Asp Ala Leu Phe Asp Ile Glu Ser Lys Val Asp
   705                 710                 715                 720
   Pro Ser Lys Ala Trp Gly Glu Val Lys Arg Gln Ile Tyr Val Ala Ala
                   725                 730                 735
   Phe Thr Val Gln Ala Ala Ala Glu Thr Leu Ser Glu Val Ala
               740                 745                 750
<210> 2
   <211> 7570
   <212> DNA
   <213> Artificial Sequence
<220>
   <223> Plasmid
<400> 2
   gacggatcgg gagatctccc gatcccctat ggtcgactct cagtacaatc tgctctgatg      60
   ccgcatagtt aagccagtat ctgctccctg cttgtgtgtt ggaggtcgct gagtagtgcg     120
   cgagcaaaat ttaagctaca acaaggcaag gcttgaccga caattgcatg aagaatctgc     180
   ttagggttag gcgttttgcg ctgcttcgcg atgtacgggc cagatatacg cgttgacatt     240
   gattattgac tagttattaa tagtaatcaa ttacggggtc attagttcat agcccatata     300
   tggagttccg cgttacataa cttacggtaa atggcccgcc tggctgaccg cccaacgacc     360
   cccgcccatt gacgtcaata atgacgtatg ttcccatagt aacgccaata gggactttcc     420
   attgacgtca atgggtggac tatttacggt aaactgccca cttggcagta catcaagtgt     480
   atcatatgcc aagtacgccc cctattgacg tcaatgacgg taaatggccc gcctggcatt     540
   atgcccagta catgacctta tgggactttc ctacttggca gtacatctac gtattagtca     600
   tcgctattac catggtgatg cggttttggc agtacatcaa tgggcgtgga tagcggtttg 660
   actcacgggg atttccaagt ctccacccca ttgacgtcaa tgggagtttg ttttggcacc 720
   aaaatcaacg ggactttcca aaatgtcgta acaactccgc cccattgacg caaatgggcg 780
   gtaggcgtgt acggtgggag gtctatataa gcagagctct ctggctaact agagaaccca 840
   ctgcttactg gcttatcgaa attaatacga ctcactatag ggagacccaa gctggctaga 900
   ggtaccaagc ttggatctca ccatggagtt gggactgcgc tggggcttcc tcgttgctct 960
   tttaagaggt gtccagtgtc aggtgcaatt ggtggagtct gggggaggcg tggtccagcc 1020
   tgggaggtcc ctgagactct cctgtgcagc gtctggattc gccttcagta gatatggcat 1080
   gcactgggtc cgccaggctc caggcaaggg gctggagtgg gtggcagtta tatggtatga 1140
   tggaagtaat aaatactatg cagactccgt gaagggccga ttcaccatct ccagagacaa 1200
   ttccaagaac acgcagtatc tgcaaatgaa cagcctgaga gccgaggaca cggctgtgta 1260
   ttactgtgcg agaggcggtg acttcctcta ctactactat tacggtatgg acgtctgggg 1320
   ccaagggacc acggtcaccg tctcctcagc ctccaccaag ggcccatcgg tcttccccct 1380
   ggcaccctct agcaagagca cctctggggg cacagcggcc ctgggctgcc tggtcaagga 1440
   ctacttcccc gaaccggtga cggtgtcgtg gaactcaggc gccctgacca gcggcgtgca 1500
   caccttcccg gctgtcctac agtcctcagg actctactcc ctcagcagcg tggtgaccgt 1560
   gccctccagc agcttgggca cccagaccta catctgcaac gtgaatcaca agcccagcaa 1620
   caccaaggtg gacaagagag ttggtgagag gccagcacag ggagggaggg tgtctgctgg 1680
   aagccaggct cagcgctcct gcctggacgc atcccggcta tgcagtccca gtccagggca 1740
   gcaaggcagg ccccgtctgc ctcttcaccc ggaggcctct gcccgcccca ctcatgctca 1800
   gggagagggt cttctggctt tttccccagg ctctgggcag gcacaggcta ggtgccccta 1860
   acccaggccc tgcacacaaa ggggcaggtg ctgggctcag acctgccaag agccatatcc 1920
   gggaggaccc tgcccctgac ctaagcccac cccaaaggcc aaactctcca ctccctcagc 1980
   tcggacacct tctctcctcc cagattccag taactcccaa tcttctctct gcagagccca 2040
   aatcttgtga caaaactcac acatgcccac cgtgcccagg taagccagcc caggcctcgc 2100
   cctccagctc aaggcgggac aggtgcccta gagtagcctg catccaggga caggccccag 2160
   ccgggtgctg acacgtccac ctccatctct tcctcagcac ctgaactcct ggggggaccg 2220
   tcagtcttcc tcttcccccc aaaacccaag gacaccctca tgatctcccg gacccctgag 2280
   gtcacatgcg tggtggtgga cgtgagccac gaagaccctg aggtcaagtt caactggtac 2340
   gtggacggcg tggaggtgca taatgccaag acaaagccgc gggaggagca gtacaacagc 2400
   acgtaccgtg tggtcagcgt cctcaccgtc ctgcaccagg actggctgaa tggcaaggag 2460
   tacaagtgca aggtctccaa caaagccctc ccagccccca tcgagaaaac catctccaaa 2520
   gccaaaggtg ggacccgtgg ggtgcgaggg ccacatggac agaggccggc tcggcccacc 2580
   ctctgccctg agagtgaccg ctgtaccaac ctctgtccct acagggcagc cccgagaacc 2640
   acaggtgtac accctgcccc catcccggga ggagatgacc aagaaccagg tcagcctgac 2700
   ctgcctggtc aaaggcttct atcccagcga catcgccgtg gagtgggaga gcaatgggca 2760
   gccggagaac aactacaaga ccacgcctcc cgtgctggac tccgacggct ccttcttcct 2820
   ctatagcaag ctcaccgtgg acaagagcag gtggcagcag gggaacgtct tctcatgctc 2880
   cgtgatgcat gaggctctgc acaaccacta cacgcagaag agcctctccc tgtctccggg 2940
   taaatgagaa ttcctcgagt ctagagggcc cgtttaaacc cgctgatcag cctcgactgt 3000
   gccttctagt tgccagccat ctgttgtttg cccctccccc gtgccttcct tgaccctgga 3060
   aggtgccact cccactgtcc tttcctaata aaatgaggaa attgcatcgc attgtctgag 3120
   taggtgtcat tctattctgg ggggtggggt ggggcaggac agcaaggggg aggattggga 3180
   agacaatagc aggcatgctg gggatgcggt gggctctatg gcttctgagg cggaaagaac 3240
   cagctggggc tctagggggt atccccacgc gccctgtagc ggcgcattaa gcgcggcggg 3300
   tgtggtggtt acgcgcagcg tgaccgctac acttgccagc gccctagcgc ccgctccttt 3360
   cgctttcttc ccttcctttc tcgccacgtt cgccggcttt ccccgtcaag ctctaaatcg 3420
   ggggctccct ttagggttcc gatttagtgc tttacggcac ctcgacccca aaaaacttga 3480
   ttagggtgat ggttcacgta gtgggccatc gccctgatag acggtttttc gccctttgac 3540
   gttggagtcc acgttcttta atagtggact cttgttccaa actggaacaa cactcaaccc 3600
   tatctcggtc tattcttttg atttataagg gattttgggg atttcggcct attggttaaa 3660
   aaatgagctg atttaacaaa aatttaacgc gaattaattc tgtggaatgt gtgtcagtta 3720
   gggtgtggaa agtccccagg ctccccaggc aggcagaagt atgcaaagca tgcatctcaa 3780
   ttagtcagca accaggtgtg gaaagtcccc aggctcccca gcaggcagaa gtatgcaaag 3840
   catgcatctc aattagtcag caaccatagt cccgccccta actccgccca tcccgcccct 3900
   aactccgccc agttccgccc attctccgcc ccatggctga ctaatttttt ttatttatgc 3960
   agaggccgag gccgcctctg cctctgagct attccagaag tagtgaggag gcttttttgg 4020
   aggcctaggc ttttgcaaaa agctcccggg agcttgtata tccattttcg gatctgatca 4080
   gcacgtgatg aaaaagcctg aactcaccgc gacgtctgtc gagaagtttc tgatcgaaaa 4140
   gttcgacagc gtctccgacc tgatgcagct ctcggagggc gaagaatctc gtgctttcag 4200
   cttcgatgta ggagggcgtg gatatgtcct gcgggtaaat agctgcgccg atggtttcta 4260
   caaagatcgt tatgtttatc ggcactttgc atcggccgcg ctcccgattc cggaagtgct 4320
   tgacattggg gaattcagcg agagcctgac ctattgcatc tcccgccgtg cacagggtgt 4380
   cacgttgcaa gacctgcctg aaaccgaact gcccgctgtt ctgcagccgg tcgcggaggc 4440
   catggatgcg atcgctgcgg ccgatcttag ccagacgagc gggttcggcc cattcggacc 4500
   gcaaggaatc ggtcaataca ctacatggcg tgatttcata tgcgcgattg ctgatcccca 4560
   tgtgtatcac tggcaaactg tgatggacga caccgtcagt gcgtccgtcg cgcaggctct 4620
   cgatgagctg atgctttggg ccgaggactg ccccgaagtc cggcacctcg tgcacgcgga 4680
   tttcggctcc aacaatgtcc tgacggacaa tggccgcata acagcggtca ttgactggag 4740
   cgaggcgatg ttcggggatt cccaatacga ggtcgccaac atcttcttct ggaggccgtg 4800
   gttggcttgt atggagcagc agacgcgcta cttcgagcgg aggcatccgg agcttgcagg 4860
   atcgccgcgg ctccgggcgt atatgctccg cattggtctt gaccaactct atcagagctt 4920
   ggttgacggc aatttcgatg atgcagcttg ggcgcagggt cgatgcgacg caatcgtccg 4980
   atccggagcc gggactgtcg ggcgtacaca aatcgcccgc agaagcgcgg ccgtctggac 5040
   cgatggctgt gtagaagtac tcgccgatag tggaaaccga cgccccagca ctcgtccgag 5100
   ggcaaaggaa tagcacgtgc tacgagattt cgattccacc gccgccttct atgaaaggtt 5160
   gggcttcgga atcgttttcc gggacgccgg ctggatgatc ctccagcgcg gggatctcat 5220
   gctggagttc ttcgcccacc ccaacttgtt tattgcagct tataatggtt acaaataaag 5280
   caatagcatc acaaatttca caaataaagc atttttttca ctgcattcta gttgtggttt 5340
   gtccaaactc atcaatgtat cttatcatgt ctgtataccg tcgacctcta gctagagctt 5400
   ggcgtaatca tggtcatagc tgtttcctgt gtgaaattgt tatccgctca caattccaca 5460
   caacatacga gccggaagca taaagtgtaa agcctggggt gcctaatgag tgagctaact 5520
   cacattaatt gcgttgcgct cactgcccgc tttccagtcg ggaaacctgt cgtgccagct 5580
   gcattaatga atcggccaac gcgcggggag aggcggtttg cgtattgggc gctcttccgc 5640
   ttcctcgctc actgactcgc tgcgctcggt cgttcggctg cggcgagcgg tatcagctca 5700
   ctcaaaggcg gtaatacggt tatccacaga atcaggggat aacgcaggaa agaacatgtg 5760
   agcaaaaggc cagcaaaagg ccaggaaccg taaaaaggcc gcgttgctgg cgtttttcca 5820
   taggctccgc ccccctgacg agcatcacaa aaatcgacgc tcaagtcaga ggtggcgaaa 5880
   cccgacagga ctataaagat accaggcgtt tccccctgga agctccctcg tgcgctctcc 5940
   tgttccgacc ctgccgctta ccggatacct gtccgccttt ctcccttcgg gaagcgtggc 6000
   gctttctcaa tgctcacgct gtaggtatct cagttcggtg taggtcgttc gctccaagct 6060
   gggctgtgtg cacgaacccc ccgttcagcc cgaccgctgc gccttatccg gtaactatcg 6120
   tcttgagtcc aacccggtaa gacacgactt atcgccactg gcagcagcca ctggtaacag 6180
   gattagcaga gcgaggtatg taggcggtgc tacagagttc ttgaagtggt ggcctaacta 6240
   cggctacact agaaggacag tatttggtat ctgcgctctg ctgaagccag ttaccttcgg 6300
   aaaaagagtt ggtagctctt gatccggcaa acaaaccacc gctggtagcg gtggtttttt 6360
   tgtttgcaag cagcagatta cgcgcagaaa aaaaggatct caagaagatc ctttgatctt 6420
   ttctacgggg tctgacgctc agtggaacga aaactcacgt taagggattt tggtcatgag 6480
   attatcaaaa aggatcttca cctagatcct tttaaattaa aaatgaagtt ttaaatcaat 6540
   ctaaagtata tatgagtaaa cttggtctga cagttaccaa tgcttaatca gtgaggcacc 6600
   tatctcagcg atctgtctat ttcgttcatc catagttgcc tgactccccg tcgtgtagat 6660
   aactacgata cgggagggct taccatctgg ccccagtgct gcaatgatac cgcgagaccc 6720
   acgctcaccg gctccagatt tatcagcaat aaaccagcca gccggaaggg ccgagcgcag 6780
   aagtggtcct gcaactttat ccgcctccat ccagtctatt aattgttgcc gggaagctag 6840
   agtaagtagt tcgccagtta atagtttgcg caacgttgtt gccattgcta caggcatcgt 6900
   ggtgtcacgc tcgtcgtttg gtatggcttc attcagctcc ggttcccaac gatcaaggcg 6960
   agttacatga tcccccatgt tgtgcaaaaa agcggttagc tccttcggtc ctccgatcgt 7020
   tgtcagaagt aagttggccg cagtgttatc actcatggtt atggcagcac tgcataattc 7080
   tcttactgtc atgccatccg taagatgctt ttctgtgact ggtgagtact caaccaagtc 7140
   attctgagaa tagtgtatgc ggcgaccgag ttgctcttgc ccggcgtcaa tacgggataa 7200
   taccgcgcca catagcagaa ctttaaaagt gctcatcatt ggaaaacgtt cttcggggcg 7260
   aaaactctca aggatcttac cgctgttgag atccagttcg atgtaaccca ctcgtgcacc 7320
   caactgatct tcagcatctt ttactttcac cagcgtttct gggtgagcaa aaacaggaag 7380
   gcaaaatgcc gcaaaaaagg gaataagggc gacacggaaa tgttgaatac tcatactctt 7440
   cctttttcaa tattattgaa gcatttatca gggttattgt ctcatgagcg gatacatatt 7500
   tgaatgtatt tagaaaaata aacaaatagg ggttccgcgc acatttcccc gaaaagtgcc 7560
   acctgacgtc 7570
<210> 3
   <211> 7597
   <212> DNA
   <213> Artificial Sequence
<220>
   <223> Plasmid
<400> 3
   gacggatcgg gagatctccc gatcccctat ggtcgactct cagtacaatc tgctctgatg 60
   ccgcatagtt aagccagtat ctgctccctg cttgtgtgtt ggaggtcgct gagtagtgcg 120
   cgagcaaaat ttaagctaca acaaggcaag gcttgaccga caattgcatg aagaatctgc 180
   ttagggttag gcgttttgcg ctgcttcgcg atgtacgggc cagatatacg cgttgacatt 240
   gattattgac tagttattaa tagtaatcaa ttacggggtc attagttcat agcccatata 300
   tggagttccg cgttacataa cttacggtaa atggcccgcc tggctgaccg cccaacgacc 360
   cccgcccatt gacgtcaata atgacgtatg ttcccatagt aacgccaata gggactttcc 420
   attgacgtca atgggtggac tatttacggt aaactgccca cttggcagta catcaagtgt 480
   atcatatgcc aagtacgccc cctattgacg tcaatgacgg taaatggccc gcctggcatt 540
   atgcccagta catgacctta tgggactttc ctacttggca gtacatctac gtattagtca 600
   tcgctattac catggtgatg cggttttggc agtacatcaa tgggcgtgga tagcggtttg 660
   actcacgggg atttccaagt ctccacccca ttgacgtcaa tgggagtttg ttttggcacc 720
   aaaatcaacg ggactttcca aaatgtcgta acaactccgc cccattgacg caaatgggcg 780
   gtaggcgtgt acggtgggag gtctatataa gcagagctct ctggctaact agagaaccca 840
   ctgcttactg gcttatcgaa attaatacga ctcactatag ggagacccaa gctggctaga 900
   ggtaccaagc ttggatctca ccatggggtc aaccgccatc ctcaccatgg agttggggct 960
   gcgctgggtt ctcctcgttg ctcttttaag aggtgtccag tgtcaggtgc agctggtgga 1020
   gtctggggga ggcgtggtcc agcctgggag gtccctgaga ctctcctgtg cagcgtctgg 1080
   attcaccttc agtaactatg tcatgcactg ggtccgccag gctccaggca aggggctgga 1140
   gtgggtggca attatatggt atgatggaag taataaatac tatgcagact ccgtgaaggg 1200
   ccgattcacc atctccagag acaattccaa gaacacgctg tatctgcaaa tgaacagcct 1260
   gagagccgag gacacggctg tgtattactg tgcgggtgga tataactgga actacgagta 1320
   ccactactac ggtatggacg tctggggcca agggaccacg gtcaccgtct cctcagcctc 1380
   caccaagggc ccatcggtct tccccctggc accctctagc aagagcacct ctgggggcac 1440
   agcggccctg ggctgcctgg tcaaggacta cttccccgaa ccggtgacgg tgtcgtggaa 1500
   ctcaggcgcc ctgaccagcg gcgtgcacac cttcccggct gtcctacagt cctcaggact 1560
   ctactccctc agcagcgtgg tgaccgtgcc ctccagcagc ttgggcaccc agacctacat 1620
   ctgcaacgtg aatcacaagc ccagcaacac caaggtggac aagagagttg gtgagaggcc 1680
   agcacaggga gggagggtgt ctgctggaag ccaggctcag cgctcctgcc tggacgcatc 1740
   ccggctatgc agtcccagtc cagggcagca aggcaggccc cgtctgcctc ttcacccgga 1800
   ggcctctgcc cgccccactc atgctcaggg agagggtctt ctggcttttt ccccaggctc 1860
   tgggcaggca caggctaggt gcccctaacc caggccctgc acacaaaggg gcaggtgctg 1920
   ggctcagacc tgccaagagc catatccggg aggaccctgc ccctgaccta agcccacccc 1980
   aaaggccaaa ctctccactc cctcagctcg gacaccttct ctcctcccag attccagtaa 2040
   ctcccaatct tctctctgca gagcccaaat cttgtgacaa aactcacaca tgcccaccgt 2100
   gcccaggtaa gccagcccag gcctcgccct ccagctcaag gcgggacagg tgccctagag 2160
   tagcctgcat ccagggacag gccccagccg ggtgctgaca cgtccacctc catctcttcc 2220
   tcagcacctg aactcctggg gggaccgtca gtcttcctct tccccccaaa acccaaggac 2280
   accctcatga tctcccggac ccctgaggtc acatgcgtgg tggtggacgt gagccacgaa 2340
   gaccctgagg tcaagttcaa ctggtacgtg gacggcgtgg aggtgcataa tgccaagaca 2400
   aagccgcggg aggagcagta caacagcacg taccgtgtgg tcagcgtcct caccgtcctg 2460
   caccaggact ggctgaatgg caaggagtac aagtgcaagg tctccaacaa agccctccca 2520
   gcccccatcg agaaaaccat ctccaaagcc aaaggtggga cccgtggggt gcgagggcca 2580
   catggacaga ggccggctcg gcccaccctc tgccctgaga gtgaccgctg taccaacctc 2640
   tgtccctaca gggcagcccc gagaaccaca ggtgtacacc ctgcccccat cccgggagga 2700
   gatgaccaag aaccaggtca gcctgacctg cctggtcaaa ggcttctatc ccagcgacat 2760
   cgccgtggag tgggagagca atgggcagcc ggagaacaac tacaagacca cgcctcccgt 2820
   gctggactcc gacggctcct tcttcctcta tagcaagctc accgtggaca agagcaggtg 2880
   gcagcagggg aacgtcttct catgctccgt gatgcatgag gctctgcaca accactacac 2940
   gcagaagagc ctctccctgt ctccgggtaa atgagaattc ctcgagtcta gagggcccgt 3000
   ttaaacccgc tgatcagcct cgactgtgcc ttctagttgc cagccatctg ttgtttgccc 3060
   ctcccccgtg ccttccttga ccctggaagg tgccactccc actgtccttt cctaataaaa 3120
   tgaggaaatt gcatcgcatt gtctgagtag gtgtcattct attctggggg gtggggtggg 3180
   gcaggacagc aagggggagg attgggaaga caatagcagg catgctgggg atgcggtggg 3240
   ctctatggct tctgaggcgg aaagaaccag ctggggctct agggggtatc cccacgcgcc 3300
   ctgtagcggc gcattaagcg cggcgggtgt ggtggttacg cgcagcgtga ccgctacact 3360
   tgccagcgcc ctagcgcccg ctcctttcgc tttcttccct tcctttctcg ccacgttcgc 3420
   cggctttccc cgtcaagctc taaatcgggg catcccttta gggttccgat ttagtgcttt 3480
   acggcacctc gaccccaaaa aacttgatta gggtgatggt tcacgtagtg ggccatcgcc 3540
   ctgatagacg gtttttcgcc ctttgacgtt ggagtccacg ttctttaata gtggactctt 3600
   gttccaaact ggaacaacac tcaaccctat ctcggtctat tcttttgatt tataagggat 3660
   tttggggatt tcggcctatt ggttaaaaaa tgagctgatt taacaaaaat ttaacgcgaa 3720
   ttaattctgt ggaatgtgtg tcagttaggg tgtggaaagt ccccaggctc cccaggcagg 3780
   cagaagtatg caaagcatgc atctcaatta gtcagcaacc aggtgtggaa agtccccagg 3840
   ctccccagca ggcagaagta tgcaaagcat gcatctcaat tagtcagcaa ccatagtccc 3900
   gcccctaact ccgcccatcc cgcccctaac tccgcccagt tccgcccatt ctccgcccca 3960
   tggctgacta atttttttta tttatgcaga ggccgaggcc gcctctgcct ctgagctatt 4020
   ccagaagtag tgaggaggct tttttggagg cctaggcttt tgcaaaaagc tcccgggagc 4080
   ttgtatatcc attttcggat ctgatcagca cgtgatgaaa aagcctgaac tcaccgcgac 4140
   gtctgtcgag aagtttctga tcgaaaagtt cgacagcgtc tccgacctga tgcagctctc 4200
   ggagggcgaa gaatctcgtg ctttcagctt cgatgtagga gggcgtggat atgtcctgcg 4260
   ggtaaatagc tgcgccgatg gtttctacaa agatcgttat gtttatcggc actttgcatc 4320
   ggccgcgctc ccgattccgg aagtgcttga cattggggaa ttcagcgaga gcctgaccta 4380
   ttgcatctcc cgccgtgcac agggtgtcac gttgcaagac ctgcctgaaa ccgaactgcc 4440
   cgctgttctg cagccggtcg cggaggccat ggatgcgatc gctgcggccg atcttagcca 4500
   gacgagcggg ttcggcccat tcggaccgca aggaatcggt caatacacta catggcgtga 4560
   tttcatatgc gcgattgctg atccccatgt gtatcactgg caaactgtga tggacgacac 4620
   cgtcagtgcg tccgtcgcgc aggctctcga tgagctgatg ctttgggccg aggactgccc 4680
   cgaagtccgg cacctcgtgc acgcggattt cggctccaac aatgtcctga cggacaatgg 4740
   ccgcataaca gcggtcattg actggagcga ggcgatgttc ggggattccc aatacgaggt 4800
   cgccaacatc ttcttctgga ggccgtggtt ggcttgtatg gagcagcaga cgcgctactt 4860
   cgagcggagg catccggagc ttgcaggatc gccgcggctc cgggcgtata tgctccgcat 4920
   tggtcttgac caactctatc agagcttggt tgacggcaat ttcgatgatg cagcttgggc 4980
   gcagggtcga tgcgacgcaa tcgtccgatc cggagccggg actgtcgggc gtacacaaat 5040
   cgcccgcaga agcgcggccg tctggaccga tggctgtgta gaagtactcg ccgatagtgg 5100
   aaaccgacgc cccagcactc gtccgagggc aaaggaatag cacgtgctac gagatttcga 5160
   ttccaccgcc gccttctatg aaaggttggg cttcggaatc gttttccggg acgccggctg 5220
   gatgatcctc cagcgcgggg atctcatgct ggagttcttc gcccacccca acttgtttat 5280
   tgcagcttat aatggttaca aataaagcaa tagcatcaca aatttcacaa ataaagcatt 5340
   tttttcactg cattctagtt gtggtttgtc caaactcatc aatgtatctt atcatgtctg 5400
   tataccgtcg acctctagct agagcttggc gtaatcatgg tcatagctgt ttcctgtgtg 5460
   aaattgttat ccgctcacaa ttccacacaa catacgagcc ggaagcataa agtgtaaagc 5520
   ctggggtgcc taatgagtga gctaactcac attaattgcg ttgcgctcac tgcccgcttt 5580
   ccagtcggga aacctgtcgt gccagctgca ttaatgaatc ggccaacgcg cggggagagg 5640
   cggtttgcgt attgggcgct cttccgcttc ctcgctcact gactcgctgc gctcggtcgt 5700
   tcggctgcgg cgagcggtat cagctcactc aaaggcggta atacggttat ccacagaatc 5760
   aggggataac gcaggaaaga acatgtgagc aaaaggccag caaaaggcca ggaaccgtaa 5820
   aaaggccgcg ttgctggcgt ttttccatag gctccgcccc cctgacgagc atcacaaaaa 5880
   tcgacgctca agtcagaggt ggcgaaaccc gacaggacta taaagatacc aggcgtttcc 5940
   ccctggaagc tccctcgtgc gctctcctgt tccgaccctg ccgcttaccg gatacctgtc 6000
   cgcctttctc ccttcgggaa gcgtggcgct ttctcaatgc tcacgctgta ggtatctcag 6060
   ttcggtgtag gtcgttcgct ccaagctggg ctgtgtgcac gaaccccccg ttcagcccga 6120
   ccgctgcgcc ttatccggta actatcgtct tgagtccaac ccggtaagac acgacttatc 6180
   gccactggca gcagccactg gtaacaggat tagcagagcg aggtatgtag gcggtgctac 6240
   agagttcttg aagtggtggc ctaactacgg ctacactaga aggacagtat ttggtatctg 6300
   cgctctgctg aagccagtta ccttcggaaa aagagttggt agctcttgat ccggcaaaca 6360
   aaccaccgct ggtagcggtg gtttttttgt ttgcaagcag cagattacgc gcagaaaaaa 6420
   aggatctcaa gaagatcctt tgatcttttc tacggggtct gacgctcagt ggaacgaaaa 6480
   ctcacgttaa gggattttgg tcatgagatt atcaaaaagg atcttcacct agatcctttt 6540
   aaattaaaaa tgaagtttta aatcaatcta aagtatatat gagtaaactt ggtctgacag 6600
   ttaccaatgc ttaatcagtg aggcacctat ctcagcgatc tgtctatttc gttcatccat 6660
   agttgcctga ctccccgtcg tgtagataac tacgatacgg gagggcttac catctggccc 6720
   cagtgctgca atgataccgc gagacccacg ctcaccggct ccagatttat cagcaataaa 6780
   ccagccagcc ggaagggccg agcgcagaag tggtcctgca actttatccg cctccatcca 6840
   gtctattaat tgttgccggg aagctagagt aagtagttcg ccagttaata gtttgcgcaa 6900
   cgttgttgcc attgctacag gcatcgtggt gtcacgctcg tcgtttggta tggcttcatt 6960
   cagctccggt tcccaacgat caaggcgagt tacatgatcc cccatgttgt gcaaaaaagc 7020
   ggttagctcc ttcggtcctc cgatcgttgt cagaagtaag ttggccgcag tgttatcact 7080
   catggttatg gcagcactgc ataattctct tactgtcatg ccatccgtaa gatgcttttc 7140
   tgtgactggt gagtactcaa ccaagtcatt ctgagaatag tgtatgcggc gaccgagttg 7200
   ctcttgcccg gcgtcaatac gggataatac cgcgccacat agcagaactt taaaagtgct 7260
   catcattgga aaacgttctt cggggcgaaa actctcaagg atcttaccgc tgttgagatc 7320
   cagttcgatg taacccactc gtgcacccaa ctgatcttca gcatctttta ctttcaccag 7380
   cgtttctggg tgagcaaaaa caggaaggca aaatgccgca aaaaagggaa taagggcgac 7440
   acggaaatgt tgaatactca tactcttcct ttttcaatat tattgaagca tttatcaggg 7500
   ttattgtctc atgagcggat acatatttga atgtatttag aaaaataaac aaataggggt 7560
   tccgcgcaca tttccccgaa aagtgccacc tgacgtc 7597
<210> 4
   <211> 7579
   <212> DNA
   <213> Artificial sequence
<220>
   <223> Plasmid
<400> 4
   gacggatcgg gagatctccc gatcccctat ggtcgactct cagtacaatc tgctctgatg     60
   ccgcatagtt aagccagtat ctgctccctg cttgtgtgtt ggaggtcgct gagtagtgcg    120
   cgagcaaaat ttaagctaca acaaggcaag gcttgaccga caattgcatg aagaatctgc    180
   ttagggttag gcgttttgcg ctgcttcgcg atgtacgggc cagatatacg cgttgacatt    240
   gattattgac tagttattaa tagtaatcaa ttacggggtc attagttcat agcccatata    300
   tggagttccg cgttacataa cttacggtaa atggcccgcc tggctgaccg cccaacgacc    360
   cccgcccatt gacgtcaata atgacgtatg ttcccatagt aacgccaata gggactttcc    420
   attgacgtca atgggtggac tatttacggt aaactgccca cttggcagta catcaagtgt    480
   atcatatgcc aagtacgccc cctattgacg tcaatgacgg taaatggccc gcctggcatt    540
   atgcccagta catgacctta tgggactttc ctacttggca gtacatctac gtattagtca    600
   tcgctattac catggtgatg cggttttggc agtacatcaa tgggcgtgga tagcggtttg    660
   actcacgggg atttccaagt ctccacccca ttgacgtcaa tgggagtttg ttttggcacc    720
   aaaatcaacg ggactttcca aaatgtcgta acaactccgc cccattgacg caaatgggcg    780
   gtaggcgtgt acggtgggag gtctatataa gcagagctct ctggctaact agagaaccca    840
   ctgcttactg gcttatcgaa attaatacga ctcactatag ggagacccaa gctggctaga    900
   ggtaccaagc ttggatctca ccatggagtt gggacttagc tgggttttcc tcgttgctct    960
   tttaagaggt gtccagtgtc aggtccagct ggtggagtct gggggaggcg tggtccagcc   1020
   tgggaggtcc ctgagactct cctgtgcagc gtctggattc accttcagta gctatggcat   1080
   gcactgggtc cgccaggctc caggcaaggg gctggactgg gtggcaatta tttggcatga   1140
   tggaagtaat aaatactatg cagactccgt gaagggccga ttcaccatct ccagagacaa   1200
   ttccaagaag acgctgtacc tgcaaatgaa cagtttgaga gccgaggaca cggctgtgta   1260
   ttactgtgcg agagcttggg cctatgacta cggtgactat gaatactact tcggtatgga   1320
   cgtctggggc caagggacca cggtcaccgt ctcctcagcc tccaccaagg gcccatcggt   1380
   cttccccctg gcaccctcta gcaagagcac ctctgggggc acagcggccc tgggctgcct   1440
   ggtcaaggac tacttccccg aaccggtgac ggtgtcgtgg aactcaggcg ccctgaccag   1500
   cggcgtgcac accttcccgg ctgtcctaca gtcctcagga ctctactccc tcagcagcgt   1560
   ggtgaccgtg ccctccagca gcttgggcac ccagacctac atctgcaacg tgaatcacaa 1620
   gcccagcaac accaaggtgg acaagagagt tggtgagagg ccagcacagg gagggagggt 1680
   gtctgctgga agccaggctc agcgctcctg cctggacgca tcccggctat gcagtcccag 1740
   tccagggcag caaggcaggc cccgtctgcc tcttcacccg gaggcctctg cccgccccac 1800
   tcatgctcag ggagagggtc ttctggcttt ttccccaggc tctgggcagg cacaggctag 1860
   gtgcccctaa cccaggccct gcacacaaag gggcaggtgc tgggctcaga cctgccaaga 1920
   gccatatccg ggaggaccct gcccctgacc taagcccacc ccaaaggcca aactctccac 1980
   tccctcagct cggacacctt ctctcctccc agattccagt aactcccaat cttctctctg 2040
   cagagcccaa atcttgtgac aaaactcaca catgcccacc gtgcccaggt aagccagccc 2100
   aggcctcgcc ctccagctca aggcgggaca ggtgccctag agtagcctgc atccagggac 2160
   aggccccagc cgggtgctga cacgtccacc tccatctctt cctcagcacc tgaactcctg 2220
   gggggaccgt cagtcttcct cttcccccca aaacccaagg acaccctcat gatctcccgg 2280
   acccctgagg tcacatgcgt ggtggtggac gtgagccacg aagaccctga ggtcaagttc 2340
   aactggtacg tggacggcgt ggaggtgcat aatgccaaga caaagccgcg ggaggagcag 2400
   tacaacagca cgtaccgtgt ggtcagcgtc ctcaccgtcc tgcaccagga ctggctgaat 2460
   ggcaaggagt acaagtgcaa ggtctccaac aaagccctcc cagcccccat cgagaaaacc 2520
   atctccaaag ccaaaggtgg gacccgtggg gtgcgagggc cacatggaca gaggccggct 2580
   cggcccaccc tctgccctga gagtgaccgc tgtaccaacc tctgtcccta cagggcagcc 2640
   ccgagaacca caggtgtaca ccctgccccc atcccgggag gagatgacca agaaccaggt 2700
   cagcctgacc tgcctggtca aaggcttcta tcccagcgac atcgccgtgg agtgggagag 2760
   caatgggcag ccggagaaca actacaagac cacgcctccc gtgctggact ccgacggctc 2820
   cttcttcctc tatagcaagc tcaccgtgga caagagcagg tggcagcagg ggaacgtctt 2880
   ctcatgctcc gtgatgcatg aggctctgca caaccactac acgcagaaga gcctctccct 2940
   gtctccgggt aaatgagaat tcctcgagtc tagagggccc gtttaaaccc gctgatcagc 3000
   ctcgactgtg ccttctagtt gccagccatc tgttgtttgc ccctcccccg tgccttcctt 3060
   gaccctggaa ggtgccactc ccactgtcct ttcctaataa aatgaggaaa ttgcatcgca 3120
   ttgtctgagt aggtgtcatt ctattctggg gggtggggtg gggcaggaca gcaaggggga 3180
   ggattgggaa gacaatagca ggcatgctgg ggatgcggtg ggctctatgg cttctgaggc 3240
   ggaaagaacc agctggggct ctagggggta tccccacgcg ccctgtagcg gcgcattaag 3300
   cgcggcgggt gtggtggtta cgcgcagcgt gaccgctaca cttgccagcg ccctagcgcc 3360
   cgctcctttc gctttcttcc cttcctttct cgccacgttc gccggctttc cccgtcaagc 3420
   tctaaatcgg ggcatccctt tagggttccg atttagtgct ttacggcacc tcgaccccaa 3480
   aaaacttgat tagggtgatg gttcacgtag tgggccatcg ccctgataga cggtttttcg 3540
   ccctttgacg ttggagtcca cgttctttaa tagtggactc ttgttccaaa ctggaacaac 3600
   actcaaccct atctcggtct attcttttga tttataaggg attttgggga tttcggccta 3660
   ttggttaaaa aatgagctga tttaacaaaa atttaacgcg aattaattct gtggaatgtg 3720
   tgtcagttag ggtgtggaaa gtccccaggc tccccaggca ggcagaagta tgcaaagcat 3780
   gcatctcaat tagtcagcaa ccaggtgtgg aaagtcccca ggctccccag caggcagaag 3840
   tatgcaaagc atgcatctca attagtcagc aaccatagtc ccgcccctaa ctccgcccat 3900
   cccgccccta actccgccca gttccgccca ttctccgccc catggctgac taattttttt 3960
   tatttatgca gaggccgagg ccgcctctgc ctctgagcta ttccagaagt agtgaggagg 4020
   cttttttgga ggcctaggct tttgcaaaaa gctcccggga gcttgtatat ccattttcgg 4080
   atctgatcag cacgtgatga aaaagcctga actcaccgcg acgtctgtcg agaagtttct 4140
   gatcgaaaag ttcgacagcg tctccgacct gatgcagctc tcggagggcg aagaatctcg 4200
   tgctttcagc ttcgatgtag gagggcgtgg atatgtcctg cgggtaaata gctgcgccga 4260
   tggtttctac aaagatcgtt atgtttatcg gcactttgca tcggccgcgc tcccgattcc 4320
   ggaagtgctt gacattgggg aattcagcga gagcctgacc tattgcatct cccgccgtgc 4380
   acagggtgtc acgttgcaag acctgcctga aaccgaactg cccgctgttc tgcagccggt 4440
   cgcggaggcc atggatgcga tcgctgcggc cgatcttagc cagacgagcg ggttcggccc 4500
   attcggaccg caaggaatcg gtcaatacac tacatggcgt gatttcatat gcgcgattgc 4560
   tgatccccat gtgtatcact ggcaaactgt gatggacgac accgtcagtg cgtccgtcgc 4620
   gcaggctctc gatgagctga tgctttgggc cgaggactgc cccgaagtcc ggcacctcgt 4680
   gcacgcggat ttcggctcca acaatgtcct gacggacaat ggccgcataa cagcggtcat 4740
   tgactggagc gaggcgatgt tcggggattc ccaatacgag gtcgccaaca tcttcttctg 4800
   gaggccgtgg ttggcttgta tggagcagca gacgcgctac ttcgagcgga ggcatccgga 4860
   gcttgcagga tcgccgcggc tccgggcgta tatgctccgc attggtcttg accaactcta 4920
   tcagagcttg gttgacggca atttcgatga tgcagcttgg gcgcagggtc gatgcgacgc 4980
   aatcgtccga tccggagccg ggactgtcgg gcgtacacaa atcgcccgca gaagcgcggc 5040
   cgtctggacc gatggctgtg tagaagtact cgccgatagt ggaaaccgac gccccagcac 5100
   tcgtccgagg gcaaaggaat agcacgtgct acgagatttc gattccaccg ccgccttcta 5160
   tgaaaggttg ggcttcggaa tcgttttccg ggacgccggc tggatgatcc tccagcgcgg 5220
   ggatctcatg ctggagttct tcgcccaccc caacttgttt attgcagctt ataatggtta 5280
   caaataaagc aatagcatca caaatttcac aaataaagca tttttttcac tgcattctag 5340
   ttgtggtttg tccaaactca tcaatgtatc ttatcatgtc tgtataccgt cgacctctag 5400
   ctagagcttg gcgtaatcat ggtcatagct gtttcctgtg tgaaattgtt atccgctcac 5460
   aattccacac aacatacgag ccggaagcat aaagtgtaaa gcctggggtg cctaatgagt 5520
   gagctaactc acattaattg cgttgcgctc actgcccgct ttccagtcgg gaaacctgtc 5580
   gtgccagctg cattaatgaa tcggccaacg cgcggggaga ggcggtttgc gtattgggcg 5640
   ctcttccgct tcctcgctca ctgactcgct gcgctcggtc gttcggctgc ggcgagcggt 5700
   atcagctcac tcaaaggcgg taatacggtt atccacagaa tcaggggata acgcaggaaa 5760
   gaacatgtga gcaaaaggcc agcaaaaggc caggaaccgt aaaaaggccg cgttgctggc 5820
   gtttttccat aggctccgcc cccctgacga gcatcacaaa aatcgacgct caagtcagag 5880
   gtggcgaaac ccgacaggac tataaagata ccaggcgttt ccccctggaa gctccctcgt 5940
   gcgctctcct gttccgaccc tgccgcttac cggatacctg tccgcctttc tcccttcggg 6000
   aagcgtggcg ctttctcaat gctcacgctg taggtatctc agttcggtgt aggtcgttcg 6060
   ctccaagctg ggctgtgtgc acgaaccccc cgttcagccc gaccgctgcg ccttatccgg 6120
   taactatcgt cttgagtcca acccggtaag acacgactta tcgccactgg cagcagccac 6180
   tggtaacagg attagcagag cgaggtatgt aggcggtgct acagagttct tgaagtggtg 6240
   gcctaactac ggctacacta gaaggacagt atttggtatc tgcgctctgc tgaagccagt 6300
   taccttcgga aaaagagttg gtagctcttg atccggcaaa caaaccaccg ctggtagcgg 6360
   tggttttttt gtttgcaagc agcagattac gcgcagaaaa aaaggatctc aagaagatcc 6420
   tttgatcttt tctacggggt ctgacgctca gtggaacgaa aactcacgtt aagggatttt 6480
   ggtcatgaga ttatcaaaaa ggatcttcac ctagatcctt ttaaattaaa aatgaagttt 6540
   taaatcaatc taaagtatat atgagtaaac ttggtctgac agttaccaat gcttaatcag 6600
   tgaggcacct atctcagcga tctgtctatt tcgttcatcc atagttgcct gactccccgt 6660
   cgtgtagata actacgatac gggagggctt accatctggc cccagtgctg caatgatacc 6720
   gcgagaccca cgctcaccgg ctccagattt atcagcaata aaccagccag ccggaagggc 6780
   cgagcgcaga agtggtcctg caactttatc cgcctccatc cagtctatta attgttgccg 6840
   ggaagctaga gtaagtagtt cgccagttaa tagtttgcgc aacgttgttg ccattgctac 6900
   aggcatcgtg gtgtcacgct cgtcgtttgg tatggcttca ttcagctccg gttcccaacg 6960
   atcaaggcga gttacatgat cccccatgtt gtgcaaaaaa gcggttagct ccttcggtcc 7020
   tccgatcgtt gtcagaagta agttggccgc agtgttatca ctcatggtta tggcagcact 7080
   gcataattct cttactgtca tgccatccgt aagatgcttt tctgtgactg gtgagtactc 7140
   aaccaagtca ttctgagaat agtgtatgcg gcgaccgagt tgctcttgcc cggcgtcaat 7200
   acgggataat accgcgccac atagcagaac tttaaaagtg ctcatcattg gaaaacgttc 7260
   ttcggggcga aaactctcaa ggatcttacc gctgttgaga tccagttcga tgtaacccac 7320
   tcgtgcaccc aactgatctt cagcatcttt tactttcacc agcgtttctg ggtgagcaaa 7380
   aacaggaagg caaaatgccg caaaaaaggg aataagggcg acacggaaat gttgaatact 7440
   catactcttc ctttttcaat attattgaag catttatcag ggttattgtc tcatgagcgg 7500
   atacatattt gaatgtattt agaaaaataa acaaataggg gttccgcgca catttccccg 7560
   aaaagtgcca cctgacgtc 7579
<210> 5
   <211> 7558
   <212> DNA
   <213> Artificial sequence
<220>
   <223> Plasmid
<400> 5
   gacggatcgg gagatctccc gatcccctat ggtcgactct cagtacaatc tgctctgatg 60
   ccgcatagtt aagccagtat ctgctccctg cttgtgtgtt ggaggtcgct gagtagtgcg 120
   cgagcaaaat ttaagctaca acaaggcaag gcttgaccga caattgcatg aagaatctgc 180
   ttagggttag gcgttttgcg ctgcttcgcg atgtacgggc cagatatacg cgttgacatt 240
   gattattgac tagttattaa tagtaatcaa ttacggggtc attagttcat agcccatata 300
   tggagttccg cgttacataa cttacggtaa atggcccgcc tggctgaccg cccaacgacc 360
   cccgcccatt gacgtcaata atgacgtatg ttcccatagt aacgccaata gggactttcc 420
   attgacgtca atgggtggac tatttacggt aaactgccca cttggcagta catcaagtgt 480
   atcatatgcc aagtacgccc cctattgacg tcaatgacgg taaatggccc gcctggcatt 540
   atgcccagta catgacctta tgggactttc ctacttggca gtacatctac gtattagtca 600
   tcgctattac catggtgatg cggttttggc agtacatcaa tgggcgtgga tagcggtttg 660
   actcacgggg atttccaagt ctccacccca ttgacgtcaa tgggagtttg ttttggcacc 720
   aaaatcaacg ggactttcca aaatgtcgta acaactccgc cccattgacg caaatgggcg 780
   gtaggcgtgt acggtgggag gtctatataa gcagagctct ctggctaact agagaaccca 840
   ctgcttactg gcttatcgaa attaatacga ctcactatag ggagacccaa gctggctaga 900
   ggtaccaagc ttggatccca ccatggggtc aaccgtcatc ctcgccctcc tcctggctgt 960
   tctccaagga gtctgtgccg aggtgcagct ggtgcagtct ggagcagagg tgaaaaagcc 1020
   cggggagtct ctgaagatct cctgtaaggg ttctggatac agctttacca gttactggat 1080
   cggctgggtg cgccagatgc ccgggaaagg cctggagtgg atggggatca tctatcctgg 1140
   tgactctgat accagataca gcccgtcctt ccaaggccag gtcaccatct cagccgacaa 1200
   gtccatcagc accgcctacc tgcagtggag cagcctgaag gcctcggaca ccgccatgta 1260
   ttactgtgcg agacggatgg cagcagctgg cccctttgac tactggggcc agggaaccct 1320
   ggtcaccgtc tcctcagcct ccaccaaggg cccatcggtc ttccccctgg caccctctag 1380
   caagagcacc tctgggggca cagcggccct gggctgcctg gtcaaggact acttccccga 1440
   accggtgacg gtgtcgtgga actcaggcgc cctgaccagc ggcgtgcaca ccttcccggc 1500
   tgtcctacag tcctcaggac tctactccct cagcagcgtg gtgaccgtgc cctccagcag 1560
   cttgggcacc cagacctaca tctgcaacgt gaatcacaag cccagcaaca ccaaggtgga 1620
   caagagagtt ggtgagaggc cagcacaggg agggagggtg tctgctggaa gccaggctca 1680
   gcgctcctgc ctggacgcat cccggctatg cagtcccagt ccagggcagc aaggcaggcc 1740
   ccgtctgcct cttcacccgg aggcctctgc ccgccccact catgctcagg gagagggtct 1800
   tctggctttt tccccaggct ctgggcaggc acaggctagg tgcccctaac ccaggccctg 1860
   cacacaaagg ggcaggtgct gggctcagac ctgccaagag ccatatccgg gaggaccctg 1920
   cccctgacct aagcccaccc caaaggccaa actctccact ccctcagctc ggacaccttc 1980
   tctcctccca gattccagta actcccaatc ttctctctgc agagcccaaa tcttgtgaca 2040
   aaactcacac atgcccaccg tgcccaggta agccagccca ggcctcgccc tccagctcaa 2100
   ggcgggacag gtgccctaga gtagcctgca tccagggaca ggccccagcc gggtgctgac 2160
   acgtccacct ccatctcttc ctcagcacct gaactcctgg ggggaccgtc agtcttcctc 2220
   ttccccccaa aacccaagga caccctcatg atctcccgga cccctgaggt cacatgcgtg 2280
   gtggtggacg tgagccacga agaccctgag gtcaagttca actggtacgt ggacggcgtg 2340
   gaggtgcata atgccaagac aaagccgcgg gaggagcagt acaacagcac gtaccgtgtg 2400
   gtcagcgtcc tcaccgtcct gcaccaggac tggctgaatg gcaaggagta caagtgcaag 2460
   gtctccaaca aagccctccc agcccccatc gagaaaacca tctccaaagc caaaggtggg 2520
   acccgtgggg tgcgagggcc acatggacag aggccggctc ggcccaccct ctgccctgag 2580
   agtgaccgct gtaccaacct ctgtccctac agggcagccc cgagaaccac aggtgtacac 2640
   cctgccccca tcccgggagg agatgaccaa gaaccaggtc agcctgacct gcctggtcaa 2700
   aggcttctat cccagcgaca tcgccgtgga gtgggagagc aatgggcagc cggagaacaa 2760
   ctacaagacc acgcctcccg tgctggactc cgacggctcc ttcttcctct atagcaagct 2820
   caccgtggac aagagcaggt ggcagcaggg gaacgtcttc tcatgctccg tgatgcatga 2880
   ggctctgcac aaccactaca cgcagaagag cctctccctg tctccgggta aatgagaatt 2940
   cctcgagtct agagggcccg tttaaacccg ctgatcagcc tcgactgtgc cttctagttg 3000
   ccagccatct gttgtttgcc cctcccccgt gccttccttg accctggaag gtgccactcc 3060
   cactgtcctt tcctaataaa atgaggaaat tgcatcgcat tgtctgagta ggtgtcattc 3120
   tattctgggg ggtggggtgg ggcaggacag caagggggag gattgggaag acaatagcag 3180
   gcatgctggg gatgcggtgg gctctatggc ttctgaggcg gaaagaacca gctggggctc 3240
   tagggggtat ccccacgcgc cctgtagcgg cgcattaagc gcggcgggtg tggtggttac 3300
   gcgcagcgtg accgctacac ttgccagcgc cctagcgccc gctcctttcg ctttcttccc 3360
   ttcctttctc gccacgttcg ccggctttcc ccgtcaagct ctaaatcggg gcatcccttt 3420
   agggttccga tttagtgctt tacggcacct cgaccccaaa aaacttgatt agggtgatgg 3480
   ttcacgtagt gggccatcgc cctgatagac ggtttttcgc cctttgacgt tggagtccac 3540
   gttctttaat agtggactct tgttccaaac tggaacaaca ctcaacccta tctcggtcta 3600
   ttcttttgat ttataaggga ttttggggat ttcggcctat tggttaaaaa atgagctgat 3660
   ttaacaaaaa tttaacgcga attaattctg tggaatgtgt gtcagttagg gtgtggaaag 3720
   tccccaggct ccccaggcag gcagaagtat gcaaagcatg catctcaatt agtcagcaac 3780
   caggtgtgga aagtccccag gctccccagc aggcagaagt atgcaaagca tgcatctcaa 3840
   ttagtcagca accatagtcc cgcccctaac tccgcccatc ccgcccctaa ctccgcccag 3900
   ttccgcccat tctccgcccc atggctgact aatttttttt atttatgcag aggccgaggc 3960
   cgcctctgcc tctgagctat tccagaagta gtgaggaggc ttttttggag gcctaggctt 4020
   ttgcaaaaag ctcccgggag cttgtatatc cattttcgga tctgatcagc acgtgatgaa 4080
   aaagcctgaa ctcaccgcga cgtctgtcga gaagtttctg atcgaaaagt tcgacagcgt 4140
   ctccgacctg atgcagctct cggagggcga agaatctcgt gctttcagct tcgatgtagg 4200
   agggcgtgga tatgtcctgc gggtaaatag ctgcgccgat ggtttctaca aagatcgtta 4260
   tgtttatcgg cactttgcat cggccgcgct cccgattccg gaagtgcttg acattgggga 4320
   attcagcgag agcctgacct attgcatctc ccgccgtgca cagggtgtca cgttgcaaga 4380
   cctgcctgaa accgaactgc ccgctgttct gcagccggtc gcggaggcca tggatgcgat 4440
   cgctgcggcc gatcttagcc agacgagcgg gttcggccca ttcggaccgc aaggaatcgg 4500
   tcaatacact acatggcgtg atttcatatg cgcgattgct gatccccatg tgtatcactg 4560
   gcaaactgtg atggacgaca ccgtcagtgc gtccgtcgcg caggctctcg atgagctgat 4620
   gctttgggcc gaggactgcc ccgaagtccg gcacctcgtg cacgcggatt tcggctccaa 4680
   caatgtcctg acggacaatg gccgcataac agcggtcatt gactggagcg aggcgatgtt 4740
   cggggattcc caatacgagg tcgccaacat cttcttctgg aggccgtggt tggcttgtat 4800
   ggagcagcag acgcgctact tcgagcggag gcatccggag cttgcaggat cgccgcggct 4860
   ccgggcgtat atgctccgca ttggtcttga ccaactctat cagagcttgg ttgacggcaa 4920
   tttcgatgat gcagcttggg cgcagggtcg atgcgacgca atcgtccgat ccggagccgg 4980
   gactgtcggg cgtacacaaa tcgcccgcag aagcgcggcc gtctggaccg atggctgtgt 5040
   agaagtactc gccgatagtg gaaaccgacg ccccagcact cgtccgaggg caaaggaata 5100
   gcacgtgcta cgagatttcg attccaccgc cgccttctat gaaaggttgg gcttcggaat 5160
   cgttttccgg gacgccggct ggatgatcct ccagcgcggg gatctcatgc tggagttctt 5220
   cgcccacccc aacttgttta ttgcagctta taatggttac aaataaagca atagcatcac 5280
   aaatttcaca aataaagcat ttttttcact gcattctagt tgtggtttgt ccaaactcat 5340
   caatgtatct tatcatgtct gtataccgtc gacctctagc tagagcttgg cgtaatcatg 5400
   gtcatagctg tttcctgtgt gaaattgtta tccgctcaca attccacaca acatacgagc 5460
   cggaagcata aagtgtaaag cctggggtgc ctaatgagtg agctaactca cattaattgc 5520
   gttgcgctca ctgcccgctt tccagtcggg aaacctgtcg tgccagctgc attaatgaat 5580
   cggccaacgc gcggggagag gcggtttgcg tattgggcgc tcttccgctt cctcgctcac 5640
   tgactcgctg cgctcggtcg ttcggctgcg gcgagcggta tcagctcact caaaggcggt 5700
   aatacggtta tccacagaat caggggataa cgcaggaaag aacatgtgag caaaaggcca 5760
   gcaaaaggcc aggaaccgta aaaaggccgc gttgctggcg tttttccata ggctccgccc 5820
   ccctgacgag catcacaaaa atcgacgctc aagtcagagg tggcgaaacc cgacaggact 5880
   ataaagatac caggcgtttc cccctggaag ctccctcgtg cgctctcctg ttccgaccct 5940
   gccgcttacc ggatacctgt ccgcctttct cccttcggga agcgtggcgc tttctcaatg 6000
   ctcacgctgt aggtatctca gttcggtgta ggtcgttcgc tccaagctgg gctgtgtgca 6060
   cgaacccccc gttcagcccg accgctgcgc cttatccggt aactatcgtc ttgagtccaa 6120
   cccggtaaga cacgacttat cgccactggc agcagccact ggtaacagga ttagcagagc 6180
   gaggtatgta ggcggtgcta cagagttctt gaagtggtgg cctaactacg gctacactag 6240
   aaggacagta tttggtatct gcgctctgct gaagccagtt accttcggaa aaagagttgg 6300
   tagctcttga tccggcaaac aaaccaccgc tggtagcggt ggtttttttg tttgcaagca 6360
   gcagattacg cgcagaaaaa aaggatctca agaagatcct ttgatctttt ctacggggtc 6420
   tgacgctcag tggaacgaaa actcacgtta agggattttg gtcatgagat tatcaaaaag 6480
   gatcttcacc tagatccttt taaattaaaa atgaagtttt aaatcaatct aaagtatata 6540
   tgagtaaact tggtctgaca gttaccaatg cttaatcagt gaggcaccta tctcagcgat 6600
   ctgtctattt cgttcatcca tagttgcctg actccccgtc gtgtagataa ctacgatacg 6660
   ggagggctta ccatctggcc ccagtgctgc aatgataccg cgagacccac gctcaccggc 6720
   tccagattta tcagcaataa accagccagc cggaagggcc gagcgcagaa gtggtcctgc 6780
   aactttatcc gcctccatcc agtctattaa ttgttgccgg gaagctagag taagtagttc 6840
   gccagttaat agtttgcgca acgttgttgc cattgctaca ggcatcgtgg tgtcacgctc 6900
   gtcgtttggt atggcttcat tcagctccgg ttcccaacga tcaaggcgag ttacatgatc 6960
   ccccatgttg tgcaaaaaag cggttagctc cttcggtcct ccgatcgttg tcagaagtaa 7020
   gttggccgca gtgttatcac tcatggttat ggcagcactg cataattctc ttactgtcat 7080
   gccatccgta agatgctttt ctgtgactgg tgagtactca accaagtcat tctgagaata 7140
   gtgtatgcgg cgaccgagtt gctcttgccc ggcgtcaata cgggataata ccgcgccaca 7200
   tagcagaact ttaaaagtgc tcatcattgg aaaacgttct tcggggcgaa aactctcaag 7260
   gatcttaccg ctgttgagat ccagttcgat gtaacccact cgtgcaccca actgatcttc 7320
   agcatctttt actttcacca gcgtttctgg gtgagcaaaa acaggaaggc aaaatgccgc 7380
   aaaaaaggga ataagggcga cacggaaatg ttgaatactc atactcttcc tttttcaata 7440
   ttattgaagc atttatcagg gttattgtct catgagcgga tacatatttg aatgtattta 7500
   gaaaaataaa caaatagggg ttccgcgcac atttccccga aaagtgccac ctgacgtc 7558
<210> 6
   <211> 7576
   <212> DNA
   <213> Artificial Sequence
<220>
   <223> Plasmid
<400> 6
   gacggatcgg gagatctccc gatcccctat ggtcgactct cagtacaatc tgctctgatg 60
   ccgcatagtt aagccagtat ctgctccctg cttgtgtgtt ggaggtcgct gagtagtgcg 120
   cgagcaaaat ttaagctaca acaaggcaag gcttgaccga caattgcatg aagaatctgc 180
   ttagggttag gcgttttgcg ctgcttcgcg atgtacgggc cagatatacg cgttgacatt 240
   gattattgac tagttattaa tagtaatcaa ttacggggtc attagttcat agcccatata 300
   tggagttccg cgttacataa cttacggtaa atggcccgcc tggctgaccg cccaacgacc 360
   cccgcccatt gacgtcaata atgacgtatg ttcccatagt aacgccaata gggactttcc 420
   attgacgtca atgggtggac tatttacggt aaactgccca cttggcagta catcaagtgt 480
   atcatatgcc aagtacgccc cctattgacg tcaatgacgg taaatggccc gcctggcatt 540
   atgcccagta catgacctta tgggactttc ctacttggca gtacatctac gtattagtca 600
   tcgctattac catggtgatg cggttttggc agtacatcaa tgggcgtgga tagcggtttg 660
   actcacgggg atttccaagt ctccacccca ttgacgtcaa tgggagtttg ttttggcacc     720
   aaaatcaacg ggactttcca aaatgtcgta acaactccgc cccattgacg caaatgggcg     780
   gtaggcgtgt acggtgggag gtctatataa gcagagctct ctggctaact agagaaccca     840
   ctgcttactg gcttatcgaa attaatacga ctcactatag ggagacccaa gctggctaga     900
   ggtaccaagc ttggatctca ccatggagtt tgggctgtgc tggattttcc tcgttgctct     960
   tttaagaggt gtccagtgtc aggtgcagct ggtggagtct gggggaggcg tggtccagcc    1020
   tgggaggtcc ctgagactct cctgtgcagc ctctggattc accttcatta gctatggcat    1080
   gcactgggtc cgccaggctc caggcaaggg gctggagtgg gtggcagtta tatcatatga    1140
   tggaagtaat aaatactatg cagactccgt gaagggccga ttcaccatct ccagagacaa    1200
   ttccaagaac acgctgtatc tgcaaatgaa cagcctgaga gctgaggaca cggctgtgta    1260
   ttactgtgcg agagtattag tgggagcttt atattattat aactactacg ggatggacgt    1320
   ctggggccaa gggaccacgg tcaccgtctc ctcagcctcc accaagggcc catcggtctt    1380
   ccccctggca ccctctagca agagcacctc tgggggcaca gcggccctgg gctgcctggt    1440
   caaggactac ttccccgaac cggtgacggt gtcgtggaac tcaggcgccc tgaccagcgg    1500
   cgtgcacacc ttcccggctg tcctacagtc ctcaggactc tactccctca gcagcgtggt    1560
   gaccgtgccc tccagcagct tgggcaccca gacctacatc tgcaacgtga atcacaagcc    1620
   cagcaacacc aaggtggaca agagagttgg tgagaggcca gcacagggag ggagggtgtc    1680
   tgctggaagc caggctcagc gctcctgcct ggacgcatcc cggctatgca gtcccagtcc    1740
   agggcagcaa ggcaggcccc gtctgcctct tcacccggag gcctctgccc gccccactca    1800
   tgctcaggga gagggtcttc tggctttttc cccaggctct gggcaggcac aggctaggtg    1860
   cccctaaccc aggccctgca cacaaagggg caggtgctgg gctcagacct gccaagagcc    1920
   atatccggga ggaccctgcc cctgacctaa gcccacccca aaggccaaac tctccactcc    1980
   ctcagctcgg acaccttctc tcctcccaga ttccagtaac tcccaatctt ctctctgcag    2040
   agcccaaatc ttgtgacaaa actcacacat gcccaccgtg cccaggtaag ccagcccagg    2100
   cctcgccctc cagctcaagg cgggacaggt gccctagagt agcctgcatc cagggacagg    2160
   ccccagccgg gtgctgacac gtccacctcc atctcttcct cagcacctga actcctgggg    2220
   ggaccgtcag tcttcctctt ccccccaaaa cccaaggaca ccctcatgat ctcccggacc    2280
   cctgaggtca catgcgtggt ggtggacgtg agccacgaag accctgaggt caagttcaac    2340
   tggtacgtgg acggcgtgga ggtgcataat gccaagacaa agccgcggga ggagcagtac    2400
   aacagcacgt accgtgtggt cagcgtcctc accgtcctgc accaggactg gctgaatggc    2460
   aaggagtaca agtgcaaggt ctccaacaaa gccctcccag cccccatcga gaaaaccatc    2520
   tccaaagcca aaggtgggac ccgtggggtg cgagggccac atggacagag gccggctcgg 2580
   cccaccctct gccctgagag tgaccgctgt accaacctct gtccctacag ggcagccccg 2640
   agaaccacag gtgtacaccc tgcccccatc ccgggaggag atgaccaaga accaggtcag 2700
   cctgacctgc ctggtcaaag gcttctatcc cagcgacatc gccgtggagt gggagagcaa 2760
   tgggcagccg gagaacaact acaagaccac gcctcccgtg ctggactccg acggctcctt 2820
   cttcctctat agcaagctca ccgtggacaa gagcaggtgg cagcagggga acgtcttctc 2880
   atgctccgtg atgcatgagg ctctgcacaa ccactacacg cagaagagcc tctccctgtc 2940
   tccgggtaaa tgagaattcc tcgagtctag agggcccgtt taaacccgct gatcagcctc 3000
   gactgtgcct tctagttgcc agccatctgt tgtttgcccc tcccccgtgc cttccttgac 3060
   cctggaaggt gccactccca ctgtcctttc ctaataaaat gaggaaattg catcgcattg 3120
   tctgagtagg tgtcattcta ttctgggggg tggggtgggg caggacagca agggggagga 3180
   ttgggaagac aatagcaggc atgctgggga tgcggtgggc tctatggctt ctgaggcgga 3240
   aagaaccagc tggggctcta gggggtatcc ccacgcgccc tgtagcggcg cattaagcgc 3300
   ggcgggtgtg gtggttacgc gcagcgtgac cgctacactt gccagcgccc tagcgcccgc 3360
   tcctttcgct ttcttccctt cctttctcgc cacgttcgcc ggctttcccc gtcaagctct 3420
   aaatcggggc atccctttag ggttccgatt tagtgcttta cggcacctcg accccaaaaa 3480
   acttgattag ggtgatggtt cacgtagtgg gccatcgccc tgatagacgg tttttcgccc 3540
   tttgacgttg gagtccacgt tctttaatag tggactcttg ttccaaactg gaacaacact 3600
   caaccctatc tcggtctatt cttttgattt ataagggatt ttggggattt cggcctattg 3660
   gttaaaaaat gagctgattt aacaaaaatt taacgcgaat taattctgtg gaatgtgtgt 3720
   cagttagggt gtggaaagtc cccaggctcc ccaggcaggc agaagtatgc aaagcatgca 3780
   tctcaattag tcagcaacca ggtgtggaaa gtccccaggc tccccagcag gcagaagtat 3840
   gcaaagcatg catctcaatt agtcagcaac catagtcccg cccctaactc cgcccatccc 3900
   gcccctaact ccgcccagtt ccgcccattc tccgccccat ggctgactaa ttttttttat 3960
   ttatgcagag gccgaggccg cctctgcctc tgagctattc cagaagtagt gaggaggctt 4020
   ttttggaggc ctaggctttt gcaaaaagct cccgggagct tgtatatcca ttttcggatc 4080
   tgatcagcac gtgatgaaaa agcctgaact caccgcgacg tctgtcgaga agtttctgat 4140
   cgaaaagttc gacagcgtct ccgacctgat gcagctctcg gagggcgaag aatctcgtgc 4200
   tttcagcttc gatgtaggag ggcgtggata tgtcctgcgg gtaaatagct gcgccgatgg 4260
   tttctacaaa gatcgttatg tttatcggca ctttgcatcg gccgcgctcc cgattccgga 4320
   agtgcttgac attggggaat tcagcgagag cctgacctat tgcatctccc gccgtgcaca 4380
   gggtgtcacg ttgcaagacc tgcctgaaac cgaactgccc gctgttctgc agccggtcgc 4440
   ggaggccatg gatgcgatcg ctgcggccga tcttagccag acgagcgggt tcggcccatt 4500
   cggaccgcaa ggaatcggtc aatacactac atggcgtgat ttcatatgcg cgattgctga 4560
   tccccatgtg tatcactggc aaactgtgat ggacgacacc gtcagtgcgt ccgtcgcgca 4620
   ggctctcgat gagctgatgc tttgggccga ggactgcccc gaagtccggc acctcgtgca 4680
   cgcggatttc ggctccaaca atgtcctgac ggacaatggc cgcataacag cggtcattga 4740
   ctggagcgag gcgatgttcg gggattccca atacgaggtc gccaacatct tcttctggag 4800
   gccgtggttg gcttgtatgg agcagcagac gcgctacttc gagcggaggc atccggagct 4860
   tgcaggatcg ccgcggctcc gggcgtatat gctccgcatt ggtcttgacc aactctatca 4920
   gagcttggtt gacggcaatt tcgatgatgc agcttgggcg cagggtcgat gcgacgcaat 4980
   cgtccgatcc ggagccggga ctgtcgggcg tacacaaatc gcccgcagaa gcgcggccgt 5040
   ctggaccgat ggctgtgtag aagtactcgc cgatagtgga aaccgacgcc ccagcactcg 5100
   tccgagggca aaggaatagc acgtgctacg agatttcgat tccaccgccg ccttctatga 5160
   aaggttgggc ttcggaatcg ttttccggga cgccggctgg atgatcctcc agcgcgggga 5220
   tctcatgctg gagttcttcg cccaccccaa cttgtttatt gcagcttata atggttacaa 5280
   ataaagcaat agcatcacaa atttcacaaa taaagcattt ttttcactgc attctagttg 5340
   tggtttgtcc aaactcatca atgtatctta tcatgtctgt ataccgtcga cctctagcta 5400
   gagcttggcg taatcatggt catagctgtt tcctgtgtga aattgttatc cgctcacaat 5460
   tccacacaac atacgagccg gaagcataaa gtgtaaagcc tggggtgcct aatgagtgag 5520
   ctaactcaca ttaattgcgt tgcgctcact gcccgctttc cagtcgggaa acctgtcgtg 5580
   ccagctgcat taatgaatcg gccaacgcgc ggggagaggc ggtttgcgta ttgggcgctc 5640
   ttccgcttcc tcgctcactg actcgctgcg ctcggtcgtt cggctgcggc gagcggtatc 5700
   agctcactca aaggcggtaa tacggttatc cacagaatca ggggataacg caggaaagaa 5760
   catgtgagca aaaggccagc aaaaggccag gaaccgtaaa aaggccgcgt tgctggcgtt 5820
   tttccatagg ctccgccccc ctgacgagca tcacaaaaat cgacgctcaa gtcagaggtg 5880
   gcgaaacccg acaggactat aaagatacca ggcgtttccc cctggaagct ccctcgtgcg 5940
   ctctcctgtt ccgaccctgc cgcttaccgg atacctgtcc gcctttctcc cttcgggaag 6000
   cgtggcgctt tctcaatgct cacgctgtag gtatctcagt tcggtgtagg tcgttcgctc 6060
   caagctgggc tgtgtgcacg aaccccccgt tcagcccgac cgctgcgcct tatccggtaa 6120
   ctatcgtctt gagtccaacc cggtaagaca cgacttatcg ccactggcag cagccactgg 6180
   taacaggatt agcagagcga ggtatgtagg cggtgctaca gagttcttga agtggtggcc 6240
   taactacggc tacactagaa ggacagtatt tggtatctgc gctctgctga agccagttac 6300
   cttcggaaaa agagttggta gctcttgatc cggcaaacaa accaccgctg gtagcggtgg 6360
   tttttttgtt tgcaagcagc agattacgcg cagaaaaaaa ggatctcaag aagatccttt 6420
   gatcttttct acggggtctg acgctcagtg gaacgaaaac tcacgttaag ggattttggt 6480
   catgagatta tcaaaaagga tcttcaccta gatcctttta aattaaaaat gaagttttaa 6540
   atcaatctaa agtatatatg agtaaacttg gtctgacagt taccaatgct taatcagtga 6600
   ggcacctatc tcagcgatct gtctatttcg ttcatccata gttgcctgac tccccgtcgt 6660
   gtagataact acgatacggg agggcttacc atctggcccc agtgctgcaa tgataccgcg 6720
   agacccacgc tcaccggctc cagatttatc agcaataaac cagccagccg gaagggccga 6780
   gcgcagaagt ggtcctgcaa ctttatccgc ctccatccag tctattaatt gttgccggga 6840
   agctagagta agtagttcgc cagttaatag tttgcgcaac gttgttgcca ttgctacagg 6900
   catcgtggtg tcacgctcgt cgtttggtat ggcttcattc agctccggtt cccaacgatc 6960
   aaggcgagtt acatgatccc ccatgttgtg caaaaaagcg gttagctcct tcggtcctcc 7020
   gatcgttgtc agaagtaagt tggccgcagt gttatcactc atggttatgg cagcactgca 7080
   taattctctt actgtcatgc catccgtaag atgcttttct gtgactggtg agtactcaac 7140
   caagtcattc tgagaatagt gtatgcggcg accgagttgc tcttgcccgg cgtcaatacg 7200
   ggataatacc gcgccacata gcagaacttt aaaagtgctc atcattggaa aacgttcttc 7260
   ggggcgaaaa ctctcaagga tcttaccgct gttgagatcc agttcgatgt aacccactcg 7320
   tgcacccaac tgatcttcag catcttttac tttcaccagc gtttctgggt gagcaaaaac 7380
   aggaaggcaa aatgccgcaa aaaagggaat aagggcgaca cggaaatgtt gaatactcat 7440
   actcttcctt tttcaatatt attgaagcat ttatcagggt tattgtctca tgagcggata 7500
   catatttgaa tgtatttaga aaaataaaca aataggggtt ccgcgcacat ttccccgaaa 7560
   agtgccacct gacgtc 7576
<210> 7
   <211> 7561
   <212> DNA
   <213> Artificial Sequence
<220>
   <223> Plasmid
<400> 7
   gacggatcgg gagatctccc gatcccctat ggtcgactct cagtacaatc tgctctgatg 60
   ccgcatagtt aagccagtat ctgctccctg cttgtgtgtt ggaggtcgct gagtagtgcg 120
   cgagcaaaat ttaagctaca acaaggcaag gcttgaccga caattgcatg aagaatctgc 180
   ttagggttag gcgttttgcg ctgcttcgcg atgtacgggc cagatatacg cgttgacatt 240
   gattattgac tagttattaa tagtaatcaa ttacggggtc attagttcat agcccatata 300
   tggagttccg cgttacataa cttacggtaa atggcccgcc tggctgaccg cccaacgacc 360
   cccgcccatt gacgtcaata atgacgtatg ttcccatagt aacgccaata gggactttcc 420
   attgacgtca atgggtggac tatttacggt aaactgccca cttggcagta catcaagtgt 480
   atcatatgcc aagtacgccc cctattgacg tcaatgacgg taaatggccc gcctggcatt 540
   atgcccagta catgacctta tgggactttc ctacttggca gtacatctac gtattagtca 600
   tcgctattac catggtgatg cggttttggc agtacatcaa tgggcgtgga tagcggtttg 660
   actcacgggg atttccaagt ctccacccca ttgacgtcaa tgggagtttg ttttggcacc 720
   aaaatcaacg ggactttcca aaatgtcgta acaactccgc cccattgacg caaatgggcg 780
   gtaggcgtgt acggtgggag gtctatataa gcagagctct ctggctaact agagaaccca 840
   ctgcttactg gcttatcgaa attaatacga ctcactatag ggagacccaa gctggctaga 900
   ggtaccggat ctcaccatgg agttggggct gagctgggtt ttcctcgttg ctcttttaag 960
   aggtgtccag tgtcaggagc agctggtgga gtctggggga ggcgtggtcc agcctgggag 1020
   gtccctgaga ctctcctgtg cagcgtctgg attcaccttc agtacctatg gcatgcactg 1080
   ggtccgccag gctccaggca aggggctgga gtgggtggca gttacatggc atgatggaag 1140
   taataaatac tatgcagact ccgtgaaggg ccgattcacc atctccagag acaactccaa 1200
   gaacacgctg tatctgcaaa tgaacagcct gagagccgag gacacggctg tgtattactg 1260
   tgcgagagga ggagtgggag caacttacta ctactactac ggtatggacg tctggggcca 1320
   agggaccacg gtcaccgtct cctcagcctc caccaagggc ccatcggtct tccccctggc 1380
   accctctagc aagagcacct ctgggggcac agcggccctg ggctgcctgg tcaaggacta 1440
   cttccccgaa ccggtgacgg tgtcgtggaa ctcaggcgcc ctgaccagcg gcgtgcacac 1500
   cttcccggct gtcctacagt cctcaggact ctactccctc agcagcgtgg tgaccgtgcc 1560
   ctccagcagc ttgggcaccc agacctacat ctgcaacgtg aatcacaagc ccagcaacac 1620
   caaggtggac aagagagttg gtgagaggcc agcacaggga gggagggtgt ctgctggaag 1680
   ccaggctcag cgctcctgcc tggacgcatc ccggctatgc agtcccagtc cagggcagca 1740
   aggcaggccc cgtctgcctc ttcacccgga ggcctctgcc cgccccactc atgctcaggg 1800
   agagggtctt ctggcttttt ccccaggctc tgggcaggca caggctaggt gcccctaacc 1860
   caggccctgc acacaaaggg gcaggtgctg ggctcagacc tgccaagagc catatccggg 1920
   aggaccctgc ccctgaccta agcccacccc aaaggccaaa ctctccactc cctcagctcg 1980
   gacaccttct ctcctcccag attccagtaa ctcccaatct tctctctgca gagcccaaat 2040
   cttgtgacaa aactcacaca tgcccaccgt gcccaggtaa gccagcccag gcctcgccct 2100
   ccagctcaag gcgggacagg tgccctagag tagcctgcat ccagggacag gccccagccg 2160
   ggtgctgaca cgtccacctc catctcttcc tcagcacctg aactcctggg gggaccgtca 2220
   gtcttcctct tccccccaaa acccaaggac accctcatga tctcccggac ccctgaggtc 2280
   acatgcgtgg tggtggacgt gagccacgaa gaccctgagg tcaagttcaa ctggtacgtg 2340
   gacggcgtgg aggtgcataa tgccaagaca aagccgcggg aggagcagta caacagcacg 2400
   taccgtgtgg tcagcgtcct caccgtcctg caccaggact ggctgaatgg caaggagtac 2460
   aagtgcaagg tctccaacaa agccctccca gcccccatcg agaaaaccat ctccaaagcc 2520
   aaaggtggga cccgtggggt gcgagggcca catggacaga ggccggctcg gcccaccctc 2580
   tgccctgaga gtgaccgctg taccaacctc tgtccctaca gggcagcccc gagaaccaca 2640
   ggtgtacacc ctgcccccat cccgggagga gatgaccaag aaccaggtca gcctgacctg 2700
   cctggtcaaa ggcttctatc ccagcgacat cgccgtggag tgggagagca atgggcagcc 2760
   ggagaacaac tacaagacca cgcctcccgt gctggactcc gacggctcct tcttcctcta 2820
   tagcaagctc accgtggaca agagcaggtg gcagcagggg aacgtcttct catgctccgt 2880
   gatgcatgag gctctgcaca accactacac gcagaagagc ctctccctgt ctccgggtaa 2940
   atgactcgag tctagagggc ccgtttaaac ccgctgatca gcctcgactg tgccttctag 3000
   ttgccagcca tctgttgttt gcccctcccc cgtgccttcc ttgaccctgg aaggtgccac 3060
   tcccactgtc ctttcctaat aaaatgagga aattgcatcg cattgtctga gtaggtgtca 3120
   ttctattctg gggggtgggg tggggcagga cagcaagggg gaggattggg aagacaatag 3180
   caggcatgct ggggatgcgg tgggctctat ggcttctgag gcggaaagaa ccagctgggg 3240
   ctctaggggg tatccccacg cgccctgtag cggcgcatta agcgcggcgg gtgtggtggt 3300
   tacgcgcagc gtgaccgcta cacttgccag cgccctagcg cccgctcctt tcgctttctt 3360
   cccttccttt ctcgccacgt tcgccggctt tccccgtcaa gctctaaatc ggggcatccc 3420
   tttagggttc cgatttagtg ctttacggca cctcgacccc aaaaaacttg attagggtga 3480
   tggttcacgt agtgggccat cgccctgata gacggttttt cgccctttga cgttggagtc 3540
   cacgttcttt aatagtggac tcttgttcca aactggaaca acactcaacc ctatctcggt 3600
   ctattctttt gatttataag ggattttggg gatttcggcc tattggttaa aaaatgagct 3660
   gatttaacaa aaatttaacg cgaattaatt ctgtggaatg tgtgtcagtt agggtgtgga 3720
   aagtccccag gctccccagg caggcagaag tatgcaaagc atgcatctca attagtcagc 3780
   aaccaggtgt ggaaagtccc caggctcccc agcaggcaga agtatgcaaa gcatgcatct 3840
   caattagtca gcaaccatag tcccgcccct aactccgccc atcccgcccc taactccgcc 3900
   cagttccgcc cattctccgc cccatggctg actaattttt tttatttatg cagaggccga 3960
   ggccgcctct gcctctgagc tattccagaa gtagtgagga ggcttttttg gaggcctagg 4020
   cttttgcaaa aagctcccgg gagcttgtat atccattttc ggatctgatc agcacgtgat 4080
   gaaaaagcct gaactcaccg cgacgtctgt cgagaagttt ctgatcgaaa agttcgacag 4140
   cgtctccgac ctgatgcagc tctcggaggg cgaagaatct cgtgctttca gcttcgatgt 4200
   aggagggcgt ggatatgtcc tgcgggtaaa tagctgcgcc gatggtttct acaaagatcg 4260
   ttatgtttat cggcactttg catcggccgc gctcccgatt ccggaagtgc ttgacattgg 4320
   ggaattcagc gagagcctga cctattgcat ctcccgccgt gcacagggtg tcacgttgca 4380
   agacctgcct gaaaccgaac tgcccgctgt tctgcagccg gtcgcggagg ccatggatgc 4440
   gatcgctgcg gccgatctta gccagacgag cgggttcggc ccattcggac cgcaaggaat 4500
   cggtcaatac actacatggc gtgatttcat atgcgcgatt gctgatcccc atgtgtatca 4560
   ctggcaaact gtgatggacg acaccgtcag tgcgtccgtc gcgcaggctc tcgatgagct 4620
   gatgctttgg gccgaggact gccccgaagt ccggcacctc gtgcacgcgg atttcggctc 4680
   caacaatgtc ctgacggaca atggccgcat aacagcggtc attgactgga gcgaggcgat 4740
   gttcggggat tcccaatacg aggtcgccaa catcttcttc tggaggccgt ggttggcttg 4800
   tatggagcag cagacgcgct acttcgagcg gaggcatccg gagcttgcag gatcgccgcg 4860
   gctccgggcg tatatgctcc gcattggtct tgaccaactc tatcagagct tggttgacgg 4920
   caatttcgat gatgcagctt gggcgcaggg tcgatgcgac gcaatcgtcc gatccggagc 4980
   cgggactgtc gggcgtacac aaatcgcccg cagaagcgcg gccgtctgga ccgatggctg 5040
   tgtagaagta ctcgccgata gtggaaaccg acgccccagc actcgtccga gggcaaagga 5100
   atagcacgtg ctacgagatt tcgattccac cgccgccttc tatgaaaggt tgggcttcgg 5160
   aatcgttttc cgggacgccg gctggatgat cctccagcgc ggggatctca tgctggagtt 5220
   cttcgcccac cccaacttgt ttattgcagc ttataatggt tacaaataaa gcaatagcat 5280
   cacaaatttc acaaataaag catttttttc actgcattct agttgtggtt tgtccaaact 5340
   catcaatgta tcttatcatg tctgtatacc gtcgacctct agctagagct tggcgtaatc 5400
   atggtcatag ctgtttcctg tgtgaaattg ttatccgctc acaattccac acaacatacg 5460
   agccggaagc ataaagtgta aagcctgggg tgcctaatga gtgagctaac tcacattaat 5520
   tgcgttgcgc tcactgcccg ctttccagtc gggaaacctg tcgtgccagc tgcattaatg 5580
   aatcggccaa cgcgcgggga gaggcggttt gcgtattggg cgctcttccg cttcctcgct 5640
   cactgactcg ctgcgctcgg tcgttcggct gcggcgagcg gtatcagctc actcaaaggc 5700
   ggtaatacgg ttatccacag aatcagggga taacgcagga aagaacatgt gagcaaaagg 5760
   ccagcaaaag gccaggaacc gtaaaaaggc cgcgttgctg gcgtttttcc ataggctccg 5820
   cccccctgac gagcatcaca aaaatcgacg ctcaagtcag aggtggcgaa acccgacagg 5880
   actataaaga taccaggcgt ttccccctgg aagctccctc gtgcgctctc ctgttccgac 5940
   cctgccgctt accggatacc tgtccgcctt tctcccttcg ggaagcgtgg cgctttctca 6000
   atgctcacgc tgtaggtatc tcagttcggt gtaggtcgtt cgctccaagc tgggctgtgt 6060
   gcacgaaccc cccgttcagc ccgaccgctg cgccttatcc ggtaactatc gtcttgagtc 6120
   caacccggta agacacgact tatcgccact ggcagcagcc actggtaaca ggattagcag 6180
   agcgaggtat gtaggcggtg ctacagagtt cttgaagtgg tggcctaact acggctacac 6240
   tagaaggaca gtatttggta tctgcgctct gctgaagcca gttaccttcg gaaaaagagt 6300
   tggtagctct tgatccggca aacaaaccac cgctggtagc ggtggttttt ttgtttgcaa 6360
   gcagcagatt acgcgcagaa aaaaaggatc tcaagaagat cctttgatct tttctacggg 6420
   gtctgacgct cagtggaacg aaaactcacg ttaagggatt ttggtcatga gattatcaaa 6480
   aaggatcttc acctagatcc ttttaaatta aaaatgaagt tttaaatcaa tctaaagtat 6540
   atatgagtaa acttggtctg acagttacca atgcttaatc agtgaggcac ctatctcagc 6600
   gatctgtcta tttcgttcat ccatagttgc ctgactcccc gtcgtgtaga taactacgat 6660
   acgggagggc ttaccatctg gccccagtgc tgcaatgata ccgcgagacc cacgctcacc 6720
   ggctccagat ttatcagcaa taaaccagcc agccggaagg gccgagcgca gaagtggtcc 6780
   tgcaacttta tccgcctcca tccagtctat taattgttgc cgggaagcta gagtaagtag 6840
   ttcgccagtt aatagtttgc gcaacgttgt tgccattgct acaggcatcg tggtgtcacg 6900
   ctcgtcgttt ggtatggctt cattcagctc cggttcccaa cgatcaaggc gagttacatg 6960
   atcccccatg ttgtgcaaaa aagcggttag ctccttcggt cctccgatcg ttgtcagaag 7020
   taagttggcc gcagtgttat cactcatggt tatggcagca ctgcataatt ctcttactgt 7080
   catgccatcc gtaagatgct tttctgtgac tggtgagtac tcaaccaagt cattctgaga 7140
   atagtgtatg cggcgaccga gttgctcttg cccggcgtca atacgggata ataccgcgcc 7200
   acatagcaga actttaaaag tgctcatcat tggaaaacgt tcttcggggc gaaaactctc 7260
   aaggatctta ccgctgttga gatccagttc gatgtaaccc actcgtgcac ccaactgatc 7320
   ttcagcatct tttactttca ccagcgtttc tgggtgagca aaaacaggaa ggcaaaatgc 7380
   cgcaaaaaag ggaataaggg cgacacggaa atgttgaata ctcatactct tcctttttca 7440
   atattattga agcatttatc agggttattg tctcatgagc ggatacatat ttgaatgtat 7500
   ttagaaaaat aaacaaatag gggttccgcg cacatttccc cgaaaagtgc cacctgacgt 7560
   c 7561
<210> 8
   <211> 6082
   <212> DNA
   <213> Artificial sequence
<220>
   <223> Plasmid
<400> 8
   gacggatcgg gagatctccc gatcccctat ggtcgactct cagtacaatc tgctctgatg 60
   ccgcatagtt aagccagtat ctgctccctg cttgtgtgtt ggaggtcgct gagtagtgcg 120
   cgagcaaaat ttaagctaca acaaggcaag gcttgaccga caattgcatg aagaatctgc 180
   ttagggttag gcgttttgcg ctgcttcgcg atgtacgggc cagatatacg cgttgacatt 240
   gattattgac tagttattaa tagtaatcaa ttacggggtc attagttcat agcccatata 300
   tggagttccg cgttacataa cttacggtaa atggcccgcc tggctgaccg cccaacgacc 360
   cccgcccatt gacgtcaata atgacgtatg ttcccatagt aacgccaata gggactttcc 420
   attgacgtca atgggtggac tatttacggt aaactgccca cttggcagta catcaagtgt 480
   atcatatgcc aagtacgccc cctattgacg tcaatgacgg taaatggccc gcctggcatt 540
   atgcccagta catgacctta tgggactttc ctacttggca gtacatctac gtattagtca 600
   tcgctattac catggtgatg cggttttggc agtacatcaa tgggcgtgga tagcggtttg 660
   actcacgggg atttccaagt ctccacccca ttgacgtcaa tgggagtttg ttttggcacc 720
   aaaatcaacg ggactttcca aaatgtcgta acaactccgc cccattgacg caaatgggcg 780
   gtaggcgtgt acggtgggag gtctatataa gcagagctct ctggctaact agagaaccca 840
   ctgcttactg gcttatcgaa attaatacga ctcactatag ggagacccaa gctggctaga 900
   aagcttggat ctcaccatga gggtccctgc tcagctcctg ggactcctgc tgctctggct 960
   cccagatacc agatgtgaca tccagatgac ccagtctcca tcctccctgt ctgcatctgt 1020
   aggagacaga gtcaccatca cttgccgggc gagtcagggc attagcaatt atttagcctg 1080
   gtatcagcag aaaacaggga aagttcctaa gttcctgatc tatgaagcat ccactttgca 1140
   atcaggggtc ccatctcggt tcagtggcgg tggatctggg acagatttca ctctcaccat 1200
   cagcagcctg cagcctgaag atgttgcaac ttattactgt caaaattata acagtgcccc 1260
   attcactttc ggccctggga ccaaagtgga tatcaaacga actgtggctg caccctctgt 1320
   cttcatcttc ccgccatctg atgagcagtt gaaatctgga actgctagcg ttgtgtgcct 1380
   gctgaataac ttctatccca gagaggccaa agtacagtgg aaggtggata acgccctcca 1440
   atcgggtaac tcccaggaga gtgtcacaga gcaggacagc aaggacagca cctacagcct 1500
   cagcagcacc ctgacgctga gcaaagcaga ctacgagaaa cacaaagtct acgcctgcga 1560
   agtcacccat cagggcctga gctcgcccgt cacaaagagc ttcaacaggg gagagtgtta 1620
   ggaattcgcg gccgctcgag tctagagggc ccgtttaaac ccgctgatca gcctcgactg 1680
   tgccttctag ttgccagcca tctgttgttt gcccctcccc cgtgccttcc ttgaccctgg 1740
   aaggtgccac tcccactgtc ctttcctaat aaaatgagga aattgcatcg cattgtctga 1800
   gtaggtgtca ttctattctg gggggtgggg tggggcagga cagcaagggg gaggattggg 1860
   aagacaatag caggcatgct ggggatgcgg tgggctctat ggcttctgag gcggaaagaa 1920
   ccagctgggg ctctaggggg tatccccacg cgccctgtag cggcgcatta agcgcggcgg 1980
   gtgtggtggt tacgcgcagc gtgaccgcta cacttgccag cgccctagcg cccgctcctt 2040
   tcgctttctt cccttccttt ctcgccacgt tcgccggctt tccccgtcaa gctctaaatc 2100
   ggggcatccc tttagggttc cgatttagtg ctttacggca cctcgacccc aaaaaacttg 2160
   attagggtga tggttcacgt agtgggccat cgccctgata gacggttttt cgccctttga 2220
   cgttggagtc cacgttcttt aatagtggac tcttgttcca aactggaaca acactcaacc 2280
   ctatctcggt ctattctttt gatttataag ggattttggg gatttcggcc tattggttaa 2340
   aaaatgagct gatttaacaa aaatttaacg cgaattaatt ctgtggaatg tgtgtcagtt 2400
   agggtgtgga aagtccccag gctccccagg caggcagaag tatgcaaagc atgcatctca 2460
   attagtcagc aaccaggtgt ggaaagtccc caggctcccc agcaggcaga agtatgcaaa 2520
   gcatgcatct caattagtca gcaaccatag tcccgcccct aactccgccc atcccgcccc 2580
   taactccgcc cagttccgcc cattctccgc cccatggctg actaattttt tttatttatg 2640
   cagaggccga ggccgcctct gcctctgagc tattccagaa gtagtgagga ggcttttttg 2700
   gaggcctagg cttttgcaaa aagctcccgg gagcttgtat atccattttc ggatctgatc 2760
   aagagacagg atgaggatcg tttcgcatga ttgaacaaga tggattgcac gcaggttctc 2820
   cggccgcttg ggtggagagg ctattcggct atgactgggc acaacagaca atcggctgct 2880
   ctgatgccgc cgtgttccgg ctgtcagcgc aggggcgccc ggttcttttt gtcaagaccg 2940
   acctgtccgg tgccctgaat gaactgcagg acgaggcagc gcggctatcg tggctggcca 3000
   cgacgggcgt tccttgcgca gctgtgctcg acgttgtcac tgaagcggga agggactggc 3060
   tgctattggg cgaagtgccg gggcaggatc tcctgtcatc tcaccttgct cctgccgaga 3120
   aagtatccat catggctgat gcaatgcggc ggctgcatac gcttgatccg gctacctgcc 3180
   cattcgacca ccaagcgaaa catcgcatcg agcgagcacg tactcggatg gaagccggtc 3240
   ttgtcgatca ggatgatctg gacgaagagc atcaggggct cgcgccagcc gaactgttcg 3300
   ccaggctcaa ggcgcgcatg cccgacggcg aggatctcgt cgtgacccat ggcgatgcct 3360
   gcttgccgaa tatcatggtg gaaaatggcc gcttttctgg attcatcgac tgtggccggc 3420
   tgggtgtggc ggaccgctat caggacatag cgttggctac ccgtgatatt gctgaagagc 3480
   ttggcggcga atgggctgac cgcttcctcg tgctttacgg tatcgccgct cccgattcgc 3540
   agcgcatcgc cttctatcgc cttcttgacg agttcttctg agcgggactc tggggttcga 3600
   aatgaccgac caagcgacgc ccaacctgcc atcacgagat ttcgattcca ccgccgcctt 3660
   ctatgaaagg ttgggcttcg gaatcgtttt ccgggacgcc ggctggatga tcctccagcg 3720
   cggggatctc atgctggagt tcttcgccca ccccaacttg tttattgcag cttataatgg 3780
   ttacaaataa agcaatagca tcacaaattt cacaaataaa gcattttttt cactgcattc 3840
   tagttgtggt ttgtccaaac tcatcaatgt atcttatcat gtctgtatac cgtcgacctc 3900
   tagctagagc ttggcgtaat catggtcata gctgtttcct gtgtgaaatt gttatccgct 3960
   cacaattcca cacaacatac gagccggaag cataaagtgt aaagcctggg gtgcctaatg 4020
   agtgagctaa ctcacattaa ttgcgttgcg ctcactgccc gctttccagt cgggaaacct 4080
   gtcgtgccag ctgcattaat gaatcggcca acgcgcgggg agaggcggtt tgcgtattgg 4140
   gcgctcttcc gcttcctcgc tcactgactc gctgcgctcg gtcgttcggc tgcggcgagc 4200
   ggtatcagct cactcaaagg cggtaatacg gttatccaca gaatcagggg ataacgcagg 4260
   aaagaacatg tgagcaaaag gccagcaaaa ggccaggaac cgtaaaaagg ccgcgttgct 4320
   ggcgtttttc cataggctcc gcccccctga cgagcatcac aaaaatcgac gctcaagtca 4380
   gaggtggcga aacccgacag gactataaag ataccaggcg tttccccctg gaagctccct 4440
   cgtgcgctct cctgttccga ccctgccgct taccggatac ctgtccgcct ttctcccttc 4500
   gggaagcgtg gcgctttctc aatgctcacg ctgtaggtat ctcagttcgg tgtaggtcgt 4560
   tcgctccaag ctgggctgtg tgcacgaacc ccccgttcag cccgaccgct gcgccttatc 4620
   cggtaactat cgtcttgagt ccaacccggt aagacacgac ttatcgccac tggcagcagc 4680
   cactggtaac aggattagca gagcgaggta tgtaggcggt gctacagagt tcttgaagtg 4740
   gtggcctaac tacggctaca ctagaaggac agtatttggt atctgcgctc tgctgaagcc 4800
   agttaccttc ggaaaaagag ttggtagctc ttgatccggc aaacaaacca ccgctggtag 4860
   cggtggtttt tttgtttgca agcagcagat tacgcgcaga aaaaaaggat ctcaagaaga 4920
   tcctttgatc ttttctacgg ggtctgacgc tcagtggaac gaaaactcac gttaagggat 4980
   tttggtcatg agattatcaa aaaggatctt cacctagatc cttttaaatt aaaaatgaag 5040
   ttttaaatca atctaaagta tatatgagta aacttggtct gacagttacc aatgcttaat 5100
   cagtgaggca cctatctcag cgatctgtct atttcgttca tccatagttg cctgactccc 5160
   cgtcgtgtag ataactacga tacgggaggg cttaccatct ggccccagtg ctgcaatgat 5220
   accgcgagac ccacgctcac cggctccaga tttatcagca ataaaccagc cagccggaag 5280
   ggccgagcgc agaagtggtc ctgcaacttt atccgcctcc atccagtcta ttaattgttg 5340
   ccgggaagct agagtaagta gttcgccagt taatagtttg cgcaacgttg ttgccattgc 5400
   tacaggcatc gtggtgtcac gctcgtcgtt tggtatggct tcattcagct ccggttccca 5460
   acgatcaagg cgagttacat gatcccccat gttgtgcaaa aaagcggtta gctccttcgg 5520
   tcctccgatc gttgtcagaa gtaagttggc cgcagtgtta tcactcatgg ttatggcagc 5580
   actgcataat tctcttactg tcatgccatc cgtaagatgc ttttctgtga ctggtgagta 5640
   ctcaaccaag tcattctgag aatagtgtat gcggcgaccg agttgctctt gcccggcgtc 5700
   aatacgggat aataccgcgc cacatagcag aactttaaaa gtgctcatca ttggaaaacg 5760
   ttcttcgggg cgaaaactct caaggatctt accgctgttg agatccagtt cgatgtaacc 5820
   cactcgtgca cccaactgat cttcagcatc ttttactttc accagcgttt ctgggtgagc 5880
   aaaaacagga aggcaaaatg ccgcaaaaaa gggaataagg gcgacacgga aatgttgaat 5940
   actcatactc ttcctttttc aatattattg aagcatttat cagggttatt gtctcatgag 6000
   cggatacata tttgaatgta tttagaaaaa taaacaaata ggggttccgc gcacatttcc 6060
   ccgaaaagtg ccacctgacg tc 6082
<210> 9
   <211> 6082
   <212> DNA
   <213> Artificial Sequence
<220>
   <223> Plasmid
<400> 9
   gacggatcgg gagatctccc gatcccctat ggtcgactct cagtacaatc tgctctgatg 60
   ccgcatagtt aagccagtat ctgctccctg cttgtgtgtt ggaggtcgct gagtagtgcg 120
   cgagcaaaat ttaagctaca acaaggcaag gcttgaccga caattgcatg aagaatctgc 180
   ttagggttag gcgttttgcg ctgcttcgcg atgtacgggc cagatatacg cgttgacatt 240
   gattattgac tagttattaa tagtaatcaa ttacggggtc attagttcat agcccatata 300
   tggagttccg cgttacataa cttacggtaa atggcccgcc tggctgaccg cccaacgacc 360
   cccgcccatt gacgtcaata atgacgtatg ttcccatagt aacgccaata gggactttcc 420
   attgacgtca atgggtggac tatttacggt aaactgccca cttggcagta catcaagtgt 480
   atcatatgcc aagtacgccc cctattgacg tcaatgacgg taaatggccc gcctggcatt 540
   atgcccagta catgacctta tgggactttc ctacttggca gtacatctac gtattagtca 600
   tcgctattac catggtgatg cggttttggc agtacatcaa tgggcgtgga tagcggtttg 660
   actcacgggg atttccaagt ctccacccca ttgacgtcaa tgggagtttg ttttggcacc 720
   aaaatcaacg ggactttcca aaatgtcgta acaactccgc cccattgacg caaatgggcg    780
   gtaggcgtgt acggtgggag gtctatataa gcagagctct ctggctaact agagaaccca    840
   ctgcttactg gcttatcgaa attaatacga ctcactatag ggagacccaa gctggctaga    900
   aagcttggat ctcaccatga gggtccccgc tcagctcctg gggctcctgc tgctctgttt    960
   cccaggtgcc agatgtgaca tccagatgac ccagtctcca tcctcactgt ctgcatctgt   1020
   aggagacaga gtcaccatca cttgtcgggc gagtcagggc attaccaatt atttagcctg   1080
   gtttcagcag aaaccaggga aagcccctaa gtcccttatc tatgctgcat ccagtttgca   1140
   aagtggggtc ccatcaaagt tcagcggcag tggatctggg acagatttca gtctcaccat   1200
   cagcagcctg cagcctgaag attttgcaac ttattactgc caacagtata atagttaccc   1260
   gatcaccttc ggccaaggga cacgactgga gattaaacga actgtggctg caccatctgt   1320
   cttcatcttc ccgccatctg atgagcagtt gaaatctgga actgctagcg ttgtgtgcct   1380
   gctgaataac ttctatccca gagaggccaa agtacagtgg aaggtggata acgccctcca   1440
   atcgggtaac tcccaggaga gtgtcacaga gcaggacagc aaggacagca cctacagcct   1500
   cagcagcacc ctgacgctga gcaaagcaga ctacgagaaa cacaaagtct acgcctgcga   1560
   agtcacccat cagggcctga gctcgcccgt cacaaagagc ttcaacaggg gagagtgtta   1620
   ggaattcgcg gccgctcgag tctagagggc ccgtttaaac ccgctgatca gcctcgactg   1680
   tgccttctag ttgccagcca tctgttgttt gcccctcccc cgtgccttcc ttgaccctgg   1740
   aaggtgccac tcccactgtc ctttcctaat aaaatgagga aattgcatcg cattgtctga   1800
   gtaggtgtca ttctattctg gggggtgggg tggggcagga cagcaagggg gaggattggg   1860
   aagacaatag caggcatgct ggggatgcgg tgggctctat ggcttctgag gcggaaagaa   1920
   ccagctgggg ctctaggggg tatccccacg cgccctgtag cggcgcatta agcgcggcgg   1980
   gtgtggtggt tacgcgcagc gtgaccgcta cacttgccag cgccctagcg cccgctcctt   2040
   tcgctttctt cccttccttt ctcgccacgt tcgccggctt tccccgtcaa gctctaaatc   2100
   ggggcatccc tttagggttc cgatttagtg ctttacggca cctcgacccc aaaaaacttg   2160
   attagggtga tggttcacgt agtgggccat cgccctgata gacggttttt cgccctttga   2220
   cgttggagtc cacgttcttt aatagtggac tcttgttcca aactggaaca acactcaacc   2280
   ctatctcggt ctattctttt gatttataag ggattttggg gatttcggcc tattggttaa   2340
   aaaatgagct gatttaacaa aaatttaacg cgaattaatt ctgtggaatg tgtgtcagtt   2400
   agggtgtgga aagtccccag gctccccagg caggcagaag tatgcaaagc atgcatctca   2460
   attagtcagc aaccaggtgt ggaaagtccc caggctcccc agcaggcaga agtatgcaaa   2520
   gcatgcatct caattagtca gcaaccatag tcccgcccct aactccgccc atcccgcccc   2580
   taactccgcc cagttccgcc cattctccgc cccatggctg actaattttt tttatttatg 2640
   cagaggccga ggccgcctct gcctctgagc tattccagaa gtagtgagga ggcttttttg 2700
   gaggcctagg cttttgcaaa aagctcccgg gagcttgtat atccattttc ggatctgatc 2760
   aagagacagg atgaggatcg tttcgcatga ttgaacaaga tggattgcac gcaggttctc 2820
   cggccgcttg ggtggagagg ctattcggct atgactgggc acaacagaca atcggctgct 2880
   ctgatgccgc cgtgttccgg ctgtcagcgc aggggcgccc ggttcttttt gtcaagaccg 2940
   acctgtccgg tgccctgaat gaactgcagg acgaggcagc gcggctatcg tggctggcca 3000
   cgacgggcgt tccttgcgca gctgtgctcg acgttgtcac tgaagcggga agggactggc 3060
   tgctattggg cgaagtgccg gggcaggatc tcctgtcatc tcaccttgct cctgccgaga 3120
   aagtatccat catggctgat gcaatgcggc ggctgcatac gcttgatccg gctacctgcc 3180
   cattcgacca ccaagcgaaa catcgcatcg agcgagcacg tactcggatg gaagccggtc 3240
   ttgtcgatca ggatgatctg gacgaagagc atcaggggct cgcgccagcc gaactgttcg 3300
   ccaggctcaa ggcgcgcatg cccgacggcg aggatctcgt cgtgacccat ggcgatgcct 3360
   gcttgccgaa tatcatggtg gaaaatggcc gcttttctgg attcatcgac tgtggccggc 3420
   tgggtgtggc ggaccgctat caggacatag cgttggctac ccgtgatatt gctgaagagc 3480
   ttggcggcga atgggctgac cgcttcctcg tgctttacgg tatcgccgct cccgattcgc 3540
   agcgcatcgc cttctatcgc cttcttgacg agttcttctg agcgggactc tggggttcga 3600
   aatgaccgac caagcgacgc ccaacctgcc atcacgagat ttcgattcca ccgccgcctt 3660
   ctatgaaagg ttgggcttcg gaatcgtttt ccgggacgcc ggctggatga tcctccagcg 3720
   cggggatctc atgctggagt tcttcgccca ccccaacttg tttattgcag cttataatgg 3780
   ttacaaataa agcaatagca tcacaaattt cacaaataaa gcattttttt cactgcattc 3840
   tagttgtggt ttgtccaaac tcatcaatgt atcttatcat gtctgtatac cgtcgacctc 3900
   tagctagagc ttggcgtaat catggtcata gctgtttcct gtgtgaaatt gttatccgct 3960
   cacaattcca cacaacatac gagccggaag cataaagtgt aaagcctggg gtgcctaatg 4020
   agtgagctaa ctcacattaa ttgcgttgcg ctcactgccc gctttccagt cgggaaacct 4080
   gtcgtgccag ctgcattaat gaatcggcca acgcgcgggg agaggcggtt tgcgtattgg 4140
   gcgctcttcc gcttcctcgc tcactgactc gctgcgctcg gtcgttcggc tgcggcgagc 4200
   ggtatcagct cactcaaagg cggtaatacg gttatccaca gaatcagggg ataacgcagg 4260
   aaagaacatg tgagcaaaag gccagcaaaa ggccaggaac cgtaaaaagg ccgcgttgct 4320
   ggcgtttttc cataggctcc gcccccctga cgagcatcac aaaaatcgac gctcaagtca 4380
   gaggtggcga aacccgacag gactataaag ataccaggcg tttccccctg gaagctccct 4440
   cgtgcgctct cctgttccga ccctgccgct taccggatac ctgtccgcct ttctcccttc 4500
   gggaagcgtg gcgctttctc aatgctcacg ctgtaggtat ctcagttcgg tgtaggtcgt 4560
   tcgctccaag ctgggctgtg tgcacgaacc ccccgttcag cccgaccgct gcgccttatc 4620
   cggtaactat cgtcttgagt ccaacccggt aagacacgac ttatcgccac tggcagcagc 4680
   cactggtaac aggattagca gagcgaggta tgtaggcggt gctacagagt tcttgaagtg 4740
   gtggcctaac tacggctaca ctagaaggac agtatttggt atctgcgctc tgctgaagcc 4800
   agttaccttc ggaaaaagag ttggtagctc ttgatccggc aaacaaacca ccgctggtag 4860
   cggtggtttt tttgtttgca agcagcagat tacgcgcaga aaaaaaggat ctcaagaaga 4920
   tcctttgatc ttttctacgg ggtctgacgc tcagtggaac gaaaactcac gttaagggat 4980
   tttggtcatg agattatcaa aaaggatctt cacctagatc cttttaaatt aaaaatgaag 5040
   ttttaaatca atctaaagta tatatgagta aacttggtct gacagttacc aatgcttaat 5100
   cagtgaggca cctatctcag cgatctgtct atttcgttca tccatagttg cctgactccc 5160
   cgtcgtgtag ataactacga tacgggaggg cttaccatct ggccccagtg ctgcaatgat 5220
   accgcgagac ccacgctcac cggctccaga tttatcagca ataaaccagc cagccggaag 5280
   ggccgagcgc agaagtggtc ctgcaacttt atccgcctcc atccagtcta ttaattgttg 5340
   ccgggaagct agagtaagta gttcgccagt taatagtttg cgcaacgttg ttgccattgc 5400
   tacaggcatc gtggtgtcac gctcgtcgtt tggtatggct tcattcagct ccggttccca 5460
   acgatcaagg cgagttacat gatcccccat gttgtgcaaa aaagcggtta gctccttcgg 5520
   tcctccgatc gttgtcagaa gtaagttggc cgcagtgtta tcactcatgg ttatggcagc 5580
   actgcataat tctcttactg tcatgccatc cgtaagatgc ttttctgtga ctggtgagta 5640
   ctcaaccaag tcattctgag aatagtgtat gcggcgaccg agttgctctt gcccggcgtc 5700
   aatacgggat aataccgcgc cacatagcag aactttaaaa gtgctcatca ttggaaaacg 5760
   ttcttcgggg cgaaaactct caaggatctt accgctgttg agatccagtt cgatgtaacc 5820
   cactcgtgca cccaactgat cttcagcatc ttttactttc accagcgttt ctgggtgagc 5880
   aaaaacagga aggcaaaatg ccgcaaaaaa gggaataagg gcgacacgga aatgttgaat 5940
   actcatactc ttcctttttc aatattattg aagcatttat cagggttatt gtctcatgag 6000
   cggatacata tttgaatgta tttagaaaaa taaacaaata ggggttccgc gcacatttcc 6060
   ccgaaaagtg ccacctgacg tc 6082
<210> 10
   <211> 6082
   <212> DNA
   <213> Artificial Sequence
<220>
   <223> Plasmid
<400> 10
   gacggatcgg gagatctccc gatcccctat ggtcgactct cagtacaatc tgctctgatg 60
   ccgcatagtt aagccagtat ctgctccctg cttgtgtgtt ggaggtcgct gagtagtgcg 120
   cgagcaaaat ttaagctaca acaaggcaag gcttgaccga caattgcatg aagaatctgc 180
   ttagggttag gcgttttgcg ctgcttcgcg atgtacgggc cagatatacg cgttgacatt 240
   gattattgac tagttattaa tagtaatcaa ttacggggtc attagttcat agcccatata 300
   tggagttccg cgttacataa cttacggtaa atggcccgcc tggctgaccg cccaacgacc 360
   cccgcccatt gacgtcaata atgacgtatg ttcccatagt aacgccaata gggactttcc 420
   attgacgtca atgggtggac tatttacggt aaactgccca cttggcagta catcaagtgt 480
   atcatatgcc aagtacgccc cctattgacg tcaatgacgg taaatggccc gcctggcatt 540
   atgcccagta catgacctta tgggactttc ctacttggca gtacatctac gtattagtca 600
   tcgctattac catggtgatg cggttttggc agtacatcaa tgggcgtgga tagcggtttg 660
   actcacgggg atttccaagt ctccacccca ttgacgtcaa tgggagtttg ttttggcacc 720
   aaaatcaacg ggactttcca aaatgtcgta acaactccgc cccattgacg caaatgggcg 780
   gtaggcgtgt acggtgggag gtctatataa gcagagctct ctggctaact agagaaccca 840
   ctgcttactg gcttatcgaa attaatacga ctcactatag ggagacccaa gctggctaga 900
   aagcttggat ctcaccatga gggtccctgc tcagctcctg gggctcctgc tgctctgttt 960
   cccaggtgcc agatgtgaca tccagatgac ccagtctcca tcctcactgt ctgcatctgt 1020
   aggagacaga gtcaccatca cttgtcgggc gagtcagggc attagccatt atttagcctg 1080
   gtttcagcag aaaccaggga aagcccctaa gtccctgatc tatgctgcat ccagtttgca 1140
   aagtggggtc ccatcaaagt tcagcggcag tggatctggg acagatttca ctctcaccat 1200
   cagcagccta cagcctgaag attttgcaac ttattactgc caacagtata atagtttccc 1260
   gctcactttc ggcggaggga ccaaggtgga gatcaaacga actgtggctg caccatctgt 1320
   cttcatcttc ccgccatctg atgagcagtt gaaatctgga actgctagcg ttgtgtgcct 1380
   gctgaataac ttctatccca gagaggccaa agtacagtgg aaggtggata acgccctcca 1440
   atcgggtaac tcccaggaga gtgtcacaga gcaggacagc aaggacagca cctacagcct 1500
   cagcagcacc ctgacgctga gcaaagcaga ctacgagaaa cacaaagtct acgcctgcga 1560
   agtcacccat cagggcctga gctcgcccgt cacaaagagc ttcaacaggg gagagtgtta 1620
   ggaattcgcg gccgctcgag tctagagggc ccgtttaaac ccgctgatca gcctcgactg 1680
   tgccttctag ttgccagcca tctgttgttt gcccctcccc cgtgccttcc ttgaccctgg 1740
   aaggtgccac tcccactgtc ctttcctaat aaaatgagga aattgcatcg cattgtctga 1800
   gtaggtgtca ttctattctg gggggtgggg tggggcagga cagcaagggg gaggattggg 1860
   aagacaatag caggcatgct ggggatgcgg tgggctctat ggcttctgag gcggaaagaa 1920
   ccagctgggg ctctaggggg tatccccacg cgccctgtag cggcgcatta agcgcggcgg 1980
   gtgtggtggt tacgcgcagc gtgaccgcta cacttgccag cgccctagcg cccgctcctt 2040
   tcgctttctt cccttccttt ctcgccacgt tcgccggctt tccccgtcaa gctctaaatc 2100
   ggggcatccc tttagggttc cgatttagtg ctttacggca cctcgacccc aaaaaacttg 2160
   attagggtga tggttcacgt agtgggccat cgccctgata gacggttttt cgccctttga 2220
   cgttggagtc cacgttcttt aatagtggac tcttgttcca aactggaaca acactcaacc 2280
   ctatctcggt ctattctttt gatttataag ggattttggg gatttcggcc tattggttaa 2340
   aaaatgagct gatttaacaa aaatttaacg cgaattaatt ctgtggaatg tgtgtcagtt 2400
   agggtgtgga aagtccccag gctccccagg caggcagaag tatgcaaagc atgcatctca 2460
   attagtcagc aaccaggtgt ggaaagtccc caggctcccc agcaggcaga agtatgcaaa 2520
   gcatgcatct caattagtca gcaaccatag tcccgcccct aactccgccc atcccgcccc 2580
   taactccgcc cagttccgcc cattctccgc cccatggctg actaattttt tttatttatg 2640
   cagaggccga ggccgcctct gcctctgagc tattccagaa gtagtgagga ggcttttttg 2700
   gaggcctagg cttttgcaaa aagctcccgg gagcttgtat atccattttc ggatctgatc 2760
   aagagacagg atgaggatcg tttcgcatga ttgaacaaga tggattgcac gcaggttctc 2820
   cggccgcttg ggtggagagg ctattcggct atgactgggc acaacagaca atcggctgct 2880
   ctgatgccgc cgtgttccgg ctgtcagcgc aggggcgccc ggttcttttt gtcaagaccg 2940
   acctgtccgg tgccctgaat gaactgcagg acgaggcagc gcggctatcg tggctggcca 3000
   cgacgggcgt tccttgcgca gctgtgctcg acgttgtcac tgaagcggga agggactggc 3060
   tgctattggg cgaagtgccg gggcaggatc tcctgtcatc tcaccttgct cctgccgaga 3120
   aagtatccat catggctgat gcaatgcggc ggctgcatac gcttgatccg gctacctgcc 3180
   cattcgacca ccaagcgaaa catcgcatcg agcgagcacg tactcggatg gaagccggtc 3240
   ttgtcgatca ggatgatctg gacgaagagc atcaggggct cgcgccagcc gaactgttcg 3300
   ccaggctcaa ggcgcgcatg cccgacggcg aggatctcgt cgtgacccat ggcgatgcct 3360
   gcttgccgaa tatcatggtg gaaaatggcc gcttttctgg attcatcgac tgtggccggc 3420
   tgggtgtggc ggaccgctat caggacatag cgttggctac ccgtgatatt gctgaagagc 3480
   ttggcggcga atgggctgac cgcttcctcg tgctttacgg tatcgccgct cccgattcgc 3540
   agcgcatcgc cttctatcgc cttcttgacg agttcttctg agcgggactc tggggttcga 3600
   aatgaccgac caagcgacgc ccaacctgcc atcacgagat ttcgattcca ccgccgcctt 3660
   ctatgaaagg ttgggcttcg gaatcgtttt ccgggacgcc ggctggatga tcctccagcg 3720
   cggggatctc atgctggagt tcttcgccca ccccaacttg tttattgcag cttataatgg 3780
   ttacaaataa agcaatagca tcacaaattt cacaaataaa gcattttttt cactgcattc 3840
   tagttgtggt ttgtccaaac tcatcaatgt atcttatcat gtctgtatac cgtcgacctc 3900
   tagctagagc ttggcgtaat catggtcata gctgtttcct gtgtgaaatt gttatccgct 3960
   cacaattcca cacaacatac gagccggaag cataaagtgt aaagcctggg gtgcctaatg 4020
   agtgagctaa ctcacattaa ttgcgttgcg ctcactgccc gctttccagt cgggaaacct 4080
   gtcgtgccag ctgcattaat gaatcggcca acgcgcgggg agaggcggtt tgcgtattgg 4140
   gcgctcttcc gcttcctcgc tcactgactc gctgcgctcg gtcgttcggc tgcggcgagc 4200
   ggtatcagct cactcaaagg cggtaatacg gttatccaca gaatcagggg ataacgcagg 4260
   aaagaacatg tgagcaaaag gccagcaaaa ggccaggaac cgtaaaaagg ccgcgttgct 4320
   ggcgtttttc cataggctcc gcccccctga cgagcatcac aaaaatcgac gctcaagtca 4380
   gaggtggcga aacccgacag gactataaag ataccaggcg tttccccctg gaagctccct 4440
   cgtgcgctct cctgttccga ccctgccgct taccggatac ctgtccgcct ttctcccttc 4500
   gggaagcgtg gcgctttctc aatgctcacg ctgtaggtat ctcagttcgg tgtaggtcgt 4560
   tcgctccaag ctgggctgtg tgcacgaacc ccccgttcag cccgaccgct gcgccttatc 4620
   cggtaactat cgtcttgagt ccaacccggt aagacacgac ttatcgccac tggcagcagc 4680
   cactggtaac aggattagca gagcgaggta tgtaggcggt gctacagagt tcttgaagtg 4740
   gtggcctaac tacggctaca ctagaaggac agtatttggt atctgcgctc tgctgaagcc 4800
   agttaccttc ggaaaaagag ttggtagctc ttgatccggc aaacaaacca ccgctggtag 4860
   cggtggtttt tttgtttgca agcagcagat tacgcgcaga aaaaaaggat ctcaagaaga 4920
   tcctttgatc ttttctacgg ggtctgacgc tcagtggaac gaaaactcac gttaagggat 4980
   tttggtcatg agattatcaa aaaggatctt cacctagatc cttttaaatt aaaaatgaag 5040
   ttttaaatca atctaaagta tatatgagta aacttggtct gacagttacc aatgcttaat 5100
   cagtgaggca cctatctcag cgatctgtct atttcgttca tccatagttg cctgactccc 5160
   cgtcgtgtag ataactacga tacgggaggg cttaccatct ggccccagtg ctgcaatgat 5220
   accgcgagac ccacgctcac cggctccaga tttatcagca ataaaccagc cagccggaag 5280
   ggccgagcgc agaagtggtc ctgcaacttt atccgcctcc atccagtcta ttaattgttg 5340
   ccgggaagct agagtaagta gttcgccagt taatagtttg cgcaacgttg ttgccattgc 5400
   tacaggcatc gtggtgtcac gctcgtcgtt tggtatggct tcattcagct ccggttccca 5460
   acgatcaagg cgagttacat gatcccccat gttgtgcaaa aaagcggtta gctccttcgg 5520
   tcctccgatc gttgtcagaa gtaagttggc cgcagtgtta tcactcatgg ttatggcagc 5580
   actgcataat tctcttactg tcatgccatc cgtaagatgc ttttctgtga ctggtgagta 5640
   ctcaaccaag tcattctgag aatagtgtat gcggcgaccg agttgctctt gcccggcgtc 5700
   aatacgggat aataccgcgc cacatagcag aactttaaaa gtgctcatca ttggaaaacg 5760
   ttcttcgggg cgaaaactct caaggatctt accgctgttg agatccagtt cgatgtaacc 5820
   cactcgtgca cccaactgat cttcagcatc ttttactttc accagcgttt ctgggtgagc 5880
   aaaaacagga aggcaaaatg ccgcaaaaaa gggaataagg gcgacacgga aatgttgaat 5940
   actcatactc ttcctttttc aatattattg aagcatttat cagggttatt gtctcatgag 6000
   cggatacata tttgaatgta tttagaaaaa taaacaaata ggggttccgc gcacatttcc 6060
   ccgaaaagtg ccacctgacg tc 6082
<210> 11
   <211> 6085
   <212> DNA
   <213> Artificial Sequence
<220>
   <223> Plasmid
<400> 11
   gacggatcgg gagatctccc gatcccctat ggtcgactct cagtacaatc tgctctgatg 60
   ccgcatagtt aagccagtat ctgctccctg cttgtgtgtt ggaggtcgct gagtagtgcg 120
   cgagcaaaat ttaagctaca acaaggcaag gcttgaccga caattgcatg aagaatctgc 180
   ttagggttag gcgttttgcg ctgcttcgcg atgtacgggc cagatatacg cgttgacatt 240
   gattattgac tagttattaa tagtaatcaa ttacggggtc attagttcat agcccatata 300
   tggagttccg cgttacataa cttacggtaa atggcccgcc tggctgaccg cccaacgacc 360
   cccgcccatt gacgtcaata atgacgtatg ttcccatagt aacgccaata gggactttcc 420
   attgacgtca atgggtggac tatttacggt aaactgccca cttggcagta catcaagtgt 480
   atcatatgcc aagtacgccc cctattgacg tcaatgacgg taaatggccc gcctggcatt 540
   atgcccagta catgacctta tgggactttc ctacttggca gtacatctac gtattagtca 600
   tcgctattac catggtgatg cggttttggc agtacatcaa tgggcgtgga tagcggtttg 660
   actcacgggg atttccaagt ctccacccca ttgacgtcaa tgggagtttg ttttggcacc 720
   aaaatcaacg ggactttcca aaatgtcgta acaactccgc cccattgacg caaatgggcg 780
   gtaggcgtgt acggtgggag gtctatataa gcagagctct ctggctaact agagaaccca 840
   ctgcttactg gcttatcgaa attaatacga ctcactatag ggagacccaa gctggctaga    900
   aagcttggat ctcaccatga gggtccccgc tcagcttctc ttccttctgc tactctggct    960
   cccagatacc actggaggaa tagtgatgac gcagtctcca gccaccctgt ctgtgtctcc   1020
   aggggaaaga gccaccctct cctgcaggac cagtcagagt attggctgga acttagcctg   1080
   gtaccaacag aaacctggcc aggctcccag gctcctcatc tatggtgcat cttccaggac   1140
   cactggtatc ccagccaggt tcagtggcag tgggtctggg acagagttca ctctcaccat   1200
   cagcagcctg cagtctgaag attctgcagt ttattactgt cagcattatg ataactggcc   1260
   catgtgcagt tttggccagg ggaccgagct ggagatcaaa cgaactgtgg ctgcaccatc   1320
   tgtcttcatc ttcccgccat ctgatgagca gttgaaatct ggaactgcta gcgttgtgtg   1380
   cctgctgaat aacttctatc ccagagaggc caaagtacag tggaaggtgg ataacgccct   1440
   ccaatcgggt aactcccagg agagtgtcac agagcaggac agcaaggaca gcacctacag   1500
   cctcagcagc accctgacgc tgagcaaagc agactacgag aaacacaaag tctacgcctg   1560
   cgaagtcacc catcagggcc tgagctcgcc cgtcacaaag agcttcaaca ggggagagtg   1620
   ttaggaattc gcggccgctc gagtctagag ggcccgttta aacccgctga tcagcctcga   1680
   ctgtgccttc tagttgccag ccatctgttg tttgcccctc ccccgtgcct tccttgaccc   1740
   tggaaggtgc cactcccact gtcctttcct aataaaatga ggaaattgca tcgcattgtc   1800
   tgagtaggtg tcattctatt ctggggggtg gggtggggca ggacagcaag ggggaggatt   1860
   gggaagacaa tagcaggcat gctggggatg cggtgggctc tatggcttct gaggcggaaa   1920
   gaaccagctg gggctctagg gggtatcccc acgcgccctg tagcggcgca ttaagcgcgg   1980
   cgggtgtggt ggttacgcgc agcgtgaccg ctacacttgc cagcgcccta gcgcccgctc   2040
   ctttcgcttt cttcccttcc tttctcgcca cgttcgccgg ctttccccgt caagctctaa   2100
   atcggggcat ccctttaggg ttccgattta gtgctttacg gcacctcgac cccaaaaaac   2160
   ttgattaggg tgatggttca cgtagtgggc catcgccctg atagacggtt tttcgccctt   2220
   tgacgttgga gtccacgttc tttaatagtg gactcttgtt ccaaactgga acaacactca   2280
   accctatctc ggtctattct tttgatttat aagggatttt ggggatttcg gcctattggt   2340
   taaaaaatga gctgatttaa caaaaattta acgcgaatta attctgtgga atgtgtgtca   2400
   gttagggtgt ggaaagtccc caggctcccc aggcaggcag aagtatgcaa agcatgcatc   2460
   tcaattagtc agcaaccagg tgtggaaagt ccccaggctc cccagcaggc agaagtatgc   2520
   aaagcatgca tctcaattag tcagcaacca tagtcccgcc cctaactccg cccatcccgc   2580
   ccctaactcc gcccagttcc gcccattctc cgccccatgg ctgactaatt ttttttattt   2640
   atgcagaggc cgaggccgcc tctgcctctg agctattcca gaagtagtga ggaggctttt   2700
   ttggaggcct aggcttttgc aaaaagctcc cgggagcttg tatatccatt ttcggatctg   2760
   atcaagagac aggatgagga tcgtttcgca tgattgaaca agatggattg cacgcaggtt 2820
   ctccggccgc ttgggtggag aggctattcg gctatgactg ggcacaacag acaatcggct 2880
   gctctgatgc cgccgtgttc cggctgtcag cgcaggggcg cccggttctt tttgtcaaga 2940
   ccgacctgtc cggtgccctg aatgaactgc aggacgaggc agcgcggcta tcgtggctgg 3000
   ccacgacggg cgttccttgc gcagctgtgc tcgacgttgt cactgaagcg ggaagggact 3060
   ggctgctatt gggcgaagtg ccggggcagg atctcctgtc atctcacctt gctcctgccg 3120
   agaaagtatc catcatggct gatgcaatgc ggcggctgca tacgcttgat ccggctacct 3180
   gcccattcga ccaccaagcg aaacatcgca tcgagcgagc acgtactcgg atggaagccg 3240
   gtcttgtcga tcaggatgat ctggacgaag agcatcaggg gctcgcgcca gccgaactgt 3300
   tcgccaggct caaggcgcgc atgcccgacg gcgaggatct cgtcgtgacc catggcgatg 3360
   cctgcttgcc gaatatcatg gtggaaaatg gccgcttttc tggattcatc gactgtggcc 3420
   ggctgggtgt ggcggaccgc tatcaggaca tagcgttggc tacccgtgat attgctgaag 3480
   agcttggcgg cgaatgggct gaccgcttcc tcgtgcttta cggtatcgcc gctcccgatt 3540
   cgcagcgcat cgccttctat cgccttcttg acgagttctt ctgagcggga ctctggggtt 3600
   cgaaatgacc gaccaagcga cgcccaacct gccatcacga gatttcgatt ccaccgccgc 3660
   cttctatgaa aggttgggct tcggaatcgt tttccgggac gccggctgga tgatcctcca 3720
   gcgcggggat ctcatgctgg agttcttcgc ccaccccaac ttgtttattg cagcttataa 3780
   tggttacaaa taaagcaata gcatcacaaa tttcacaaat aaagcatttt tttcactgca 3840
   ttctagttgt ggtttgtcca aactcatcaa tgtatcttat catgtctgta taccgtcgac 3900
   ctctagctag agcttggcgt aatcatggtc atagctgttt cctgtgtgaa attgttatcc 3960
   gctcacaatt ccacacaaca tacgagccgg aagcataaag tgtaaagcct ggggtgccta 4020
   atgagtgagc taactcacat taattgcgtt gcgctcactg cccgctttcc agtcgggaaa 4080
   cctgtcgtgc cagctgcatt aatgaatcgg ccaacgcgcg gggagaggcg gtttgcgtat 4140
   tgggcgctct tccgcttcct cgctcactga ctcgctgcgc tcggtcgttc ggctgcggcg 4200
   agcggtatca gctcactcaa aggcggtaat acggttatcc acagaatcag gggataacgc 4260
   aggaaagaac atgtgagcaa aaggccagca aaaggccagg aaccgtaaaa aggccgcgtt 4320
   gctggcgttt ttccataggc tccgcccccc tgacgagcat cacaaaaatc gacgctcaag 4380
   tcagaggtgg cgaaacccga caggactata aagataccag gcgtttcccc ctggaagctc 4440
   cctcgtgcgc tctcctgttc cgaccctgcc gcttaccgga tacctgtccg cctttctccc 4500
   ttcgggaagc gtggcgcttt ctcaatgctc acgctgtagg tatctcagtt cggtgtaggt 4560
   cgttcgctcc aagctgggct gtgtgcacga accccccgtt cagcccgacc gctgcgcctt 4620
   atccggtaac tatcgtcttg agtccaaccc ggtaagacac gacttatcgc cactggcagc 4680
   agccactggt aacaggatta gcagagcgag gtatgtaggc ggtgctacag agttcttgaa 4740
   gtggtggcct aactacggct acactagaag gacagtattt ggtatctgcg ctctgctgaa 4800
   gccagttacc ttcggaaaaa gagttggtag ctcttgatcc ggcaaacaaa ccaccgctgg 4860
   tagcggtggt ttttttgttt gcaagcagca gattacgcgc agaaaaaaag gatctcaaga 4920
   agatcctttg atcttttcta cggggtctga cgctcagtgg aacgaaaact cacgttaagg 4980
   gattttggtc atgagattat caaaaaggat cttcacctag atccttttaa attaaaaatg 5040
   aagttttaaa tcaatctaaa gtatatatga gtaaacttgg tctgacagtt accaatgctt 5100
   aatcagtgag gcacctatct cagcgatctg tctatttcgt tcatccatag ttgcctgact 5160
   ccccgtcgtg tagataacta cgatacggga gggcttacca tctggcccca gtgctgcaat 5220
   gataccgcga gacccacgct caccggctcc agatttatca gcaataaacc agccagccgg 5280
   aagggccgag cgcagaagtg gtcctgcaac tttatccgcc tccatccagt ctattaattg 5340
   ttgccgggaa gctagagtaa gtagttcgcc agttaatagt ttgcgcaacg ttgttgccat 5400
   tgctacaggc atcgtggtgt cacgctcgtc gtttggtatg gcttcattca gctccggttc 5460
   ccaacgatca aggcgagtta catgatcccc catgttgtgc aaaaaagcgg ttagctcctt 5520
   cggtcctccg atcgttgtca gaagtaagtt ggccgcagtg ttatcactca tggttatggc 5580
   agcactgcat aattctctta ctgtcatgcc atccgtaaga tgcttttctg tgactggtga 5640
   gtactcaacc aagtcattct gagaatagtg tatgcggcga ccgagttgct cttgcccggc 5700
   gtcaatacgg gataataccg cgccacatag cagaacttta aaagtgctca tcattggaaa 5760
   acgttcttcg gggcgaaaac tctcaaggat cttaccgctg ttgagatcca gttcgatgta 5820
   acccactcgt gcacccaact gatcttcagc atcttttact ttcaccagcg tttctgggtg 5880
   agcaaaaaca ggaaggcaaa atgccgcaaa aaagggaata agggcgacac ggaaatgttg 5940
   aatactcata ctcttccttt ttcaatatta ttgaagcatt tatcagggtt attgtctcat 6000
   gagcggatac atatttgaat gtatttagaa aaataaacaa ataggggttc cgcgcacatt 6060
   tccccgaaaa gtgccacctg acgtc 6085
<210> 12
   <211> 6097
   <212> DNA
   <213> Artificial Sequence
<220>
   <223> Plasmid
<400> 12
   gacggatcgg gagatctccc gatcccctat ggtcgactct cagtacaatc tgctctgatg 60
   ccgcatagtt aagccagtat ctgctccctg cttgtgtgtt ggaggtcgct gagtagtgcg 120
   cgagcaaaat ttaagctaca acaaggcaag gcttgaccga caattgcatg aagaatctgc 180
   ttagggttag gcgttttgcg ctgcttcgcg atgtacgggc cagatatacg cgttgacatt 240
   gattattgac tagttattaa tagtaatcaa ttacggggtc attagttcat agcccatata 300
   tggagttccg cgttacataa cttacggtaa atggcccgcc tggctgaccg cccaacgacc 360
   cccgcccatt gacgtcaata atgacgtatg ttcccatagt aacgccaata gggactttcc 420
   attgacgtca atgggtggac tatttacggt aaactgccca cttggcagta catcaagtgt 480
   atcatatgcc aagtacgccc cctattgacg tcaatgacgg taaatggccc gcctggcatt 540
   atgcccagta catgacctta tgggactttc ctacttggca gtacatctac gtattagtca 600
   tcgctattac catggtgatg cggttttggc agtacatcaa tgggcgtgga tagcggtttg 660
   actcacgggg atttccaagt ctccacccca ttgacgtcaa tgggagtttg ttttggcacc 720
   aaaatcaacg ggactttcca aaatgtcgta acaactccgc cccattgacg caaatgggcg 780
   gtaggcgtgt acggtgggag gtctatataa gcagagctct ctggctaact agagaaccca 840
   ctgcttactg gcttatcgaa attaatacga ctcactatag ggagacccaa gctggctaga 900
   aagcttggat ctcaccatga gggtccctgc tcagctcctg gggctgctaa tgctctggat 960
   acctggatcc agtgcagata ttgtgatgac ccagactcca ctctctctgt ccgtcacccc 1020
   tggacagccg gcctccatct cctgcaagtc tagtcagagc ctcctgcata gtgatggaaa 1080
   gacctttttg tattggtatc tgcagaagcc aggccagcct ccacagctcc tgatctatga 1140
   ggtttccaac cggttctctg gagtgccaga taggttcagt ggcagcgggt cagggacaga 1200
   tttcacactg aaaatcagcc gggtggaggc tgaggatgtt gggctttatt actgcatgca 1260
   aagtatacag cttccgctca ctttcggcgg agggaccaag gtggagatca aacgaactgt 1320
   ggctgcacca tctgtcttca tcttcccgcc atctgatgag cagttgaaat ctggaactgc 1380
   tagcgttgtg tgcctgctga ataacttcta tcccagagag gccaaagtac agtggaaggt 1440
   ggataacgcc ctccaatcgg gtaactccca ggagagtgtc acagagcagg acagcaagga 1500
   cagcacctac agcctcagca gcaccctgac gctgagcaaa gcagactacg agaaacacaa 1560
   agtctacgcc tgcgaagtca cccatcaggg cctgagctcg cccgtcacaa agagcttcaa 1620
   caggggagag tgttaggaat tcgcggccgc tcgagtctag agggcccgtt taaacccgct 1680
   gatcagcctc gactgtgcct tctagttgcc agccatctgt tgtttgcccc tcccccgtgc 1740
   cttccttgac cctggaaggt gccactccca ctgtcctttc ctaataaaat gaggaaattg 1800
   catcgcattg tctgagtagg tgtcattcta ttctgggggg tggggtgggg caggacagca 1860
   agggggagga ttgggaagac aatagcaggc atgctgggga tgcggtgggc tctatggctt 1920
   ctgaggcgga aagaaccagc tggggctcta gggggtatcc ccacgcgccc tgtagcggcg 1980
   cattaagcgc ggcgggtgtg gtggttacgc gcagcgtgac cgctacactt gccagcgccc 2040
   tagcgcccgc tcctttcgct ttcttccctt cctttctcgc cacgttcgcc ggctttcccc 2100
   gtcaagctct aaatcggggc atccctttag ggttccgatt tagtgcttta cggcacctcg 2160
   accccaaaaa acttgattag ggtgatggtt cacgtagtgg gccatcgccc tgatagacgg 2220
   tttttcgccc tttgacgttg gagtccacgt tctttaatag tggactcttg ttccaaactg 2280
   gaacaacact caaccctatc tcggtctatt cttttgattt ataagggatt ttggggattt 2340
   cggcctattg gttaaaaaat gagctgattt aacaaaaatt taacgcgaat taattctgtg 2400
   gaatgtgtgt cagttagggt gtggaaagtc cccaggctcc ccaggcaggc agaagtatgc 2460
   aaagcatgca tctcaattag tcagcaacca ggtgtggaaa gtccccaggc tccccagcag 2520
   gcagaagtat gcaaagcatg catctcaatt agtcagcaac catagtcccg cccctaactc 2580
   cgcccatccc gcccctaact ccgcccagtt ccgcccattc tccgccccat ggctgactaa 2640
   ttttttttat ttatgcagag gccgaggccg cctctgcctc tgagctattc cagaagtagt 2700
   gaggaggctt ttttggaggc ctaggctttt gcaaaaagct cccgggagct tgtatatcca 2760
   ttttcggatc tgatcaagag acaggatgag gatcgtttcg catgattgaa caagatggat 2820
   tgcacgcagg ttctccggcc gcttgggtgg agaggctatt cggctatgac tgggcacaac 2880
   agacaatcgg ctgctctgat gccgccgtgt tccggctgtc agcgcagggg cgcccggttc 2940
   tttttgtcaa gaccgacctg tccggtgccc tgaatgaact gcaggacgag gcagcgcggc 3000
   tatcgtggct ggccacgacg ggcgttcctt gcgcagctgt gctcgacgtt gtcactgaag 3060
   cgggaaggga ctggctgcta ttgggcgaag tgccggggca ggatctcctg tcatctcacc 3120
   ttgctcctgc cgagaaagta tccatcatgg ctgatgcaat gcggcggctg catacgcttg 3180
   atccggctac ctgcccattc gaccaccaag cgaaacatcg catcgagcga gcacgtactc 3240
   ggatggaagc cggtcttgtc gatcaggatg atctggacga agagcatcag gggctcgcgc 3300
   cagccgaact gttcgccagg ctcaaggcgc gcatgcccga cggcgaggat ctcgtcgtga 3360
   cccatggcga tgcctgcttg ccgaatatca tggtggaaaa tggccgcttt tctggattca 3420
   tcgactgtgg ccggctgggt gtggcggacc gctatcagga catagcgttg gctacccgtg 3480
   atattgctga agagcttggc ggcgaatggg ctgaccgctt cctcgtgctt tacggtatcg 3540
   ccgctcccga ttcgcagcgc atcgccttct atcgccttct tgacgagttc ttctgagcgg 3600
   gactctgggg ttcgaaatga ccgaccaagc gacgcccaac ctgccatcac gagatttcga 3660
   ttccaccgcc gccttctatg aaaggttggg cttcggaatc gttttccggg acgccggctg 3720
   gatgatcctc cagcgcgggg atctcatgct ggagttcttc gcccacccca acttgtttat 3780
   tgcagcttat aatggttaca aataaagcaa tagcatcaca aatttcacaa ataaagcatt 3840
   tttttcactg cattctagtt gtggtttgtc caaactcatc aatgtatctt atcatgtctg 3900
   tataccgtcg acctctagct agagcttggc gtaatcatgg tcatagctgt ttcctgtgtg 3960
   aaattgttat ccgctcacaa ttccacacaa catacgagcc ggaagcataa agtgtaaagc 4020
   ctggggtgcc taatgagtga gctaactcac attaattgcg ttgcgctcac tgcccgcttt 4080
   ccagtcggga aacctgtcgt gccagctgca ttaatgaatc ggccaacgcg cggggagagg 4140
   cggtttgcgt attgggcgct cttccgcttc ctcgctcact gactcgctgc gctcggtcgt 4200
   tcggctgcgg cgagcggtat cagctcactc aaaggcggta atacggttat ccacagaatc 4260
   aggggataac gcaggaaaga acatgtgagc aaaaggccag caaaaggcca ggaaccgtaa 4320
   aaaggccgcg ttgctggcgt ttttccatag gctccgcccc cctgacgagc atcacaaaaa 4380
   tcgacgctca agtcagaggt ggcgaaaccc gacaggacta taaagatacc aggcgtttcc 4440
   ccctggaagc tccctcgtgc gctctcctgt tccgaccctg ccgcttaccg gatacctgtc 4500
   cgcctttctc ccttcgggaa gcgtggcgct ttctcaatgc tcacgctgta ggtatctcag 4560
   ttcggtgtag gtcgttcgct ccaagctggg ctgtgtgcac gaaccccccg ttcagcccga 4620
   ccgctgcgcc ttatccggta actatcgtct tgagtccaac ccggtaagac acgacttatc 4680
   gccactggca gcagccactg gtaacaggat tagcagagcg aggtatgtag gcggtgctac 4740
   agagttcttg aagtggtggc ctaactacgg ctacactaga aggacagtat ttggtatctg 4800
   cgctctgctg aagccagtta ccttcggaaa aagagttggt agctcttgat ccggcaaaca 4860
   aaccaccgct ggtagcggtg gtttttttgt ttgcaagcag cagattacgc gcagaaaaaa 4920
   aggatctcaa gaagatcctt tgatcttttc tacggggtct gacgctcagt ggaacgaaaa 4980
   ctcacgttaa gggattttgg tcatgagatt atcaaaaagg atcttcacct agatcctttt 5040
   aaattaaaaa tgaagtttta aatcaatcta aagtatatat gagtaaactt ggtctgacag 5100
   ttaccaatgc ttaatcagtg aggcacctat ctcagcgatc tgtctatttc gttcatccat 5160
   agttgcctga ctccccgtcg tgtagataac tacgatacgg gagggcttac catctggccc 5220
   cagtgctgca atgataccgc gagacccacg ctcaccggct ccagatttat cagcaataaa 5280
   ccagccagcc ggaagggccg agcgcagaag tggtcctgca actttatccg cctccatcca 5340
   gtctattaat tgttgccggg aagctagagt aagtagttcg ccagttaata gtttgcgcaa 5400
   cgttgttgcc attgctacag gcatcgtggt gtcacgctcg tcgtttggta tggcttcatt 5460
   cagctccggt tcccaacgat caaggcgagt tacatgatcc cccatgttgt gcaaaaaagc 5520
   ggttagctcc ttcggtcctc cgatcgttgt cagaagtaag ttggccgcag tgttatcact 5580
   catggttatg gcagcactgc ataattctct tactgtcatg ccatccgtaa gatgcttttc 5640
   tgtgactggt gagtactcaa ccaagtcatt ctgagaatag tgtatgcggc gaccgagttg 5700
   ctcttgcccg gcgtcaatac gggataatac cgcgccacat agcagaactt taaaagtgct 5760
   catcattgga aaacgttctt cggggcgaaa actctcaagg atcttaccgc tgttgagatc 5820
   cagttcgatg taacccactc gtgcacccaa ctgatcttca gcatctttta ctttcaccag 5880
   cgtttctggg tgagcaaaaa caggaaggca aaatgccgca aaaaagggaa taagggcgac 5940
   acggaaatgt tgaatactca tactcttcct ttttcaatat tattgaagca tttatcaggg 6000
   ttattgtctc atgagcggat acatatttga atgtatttag aaaaataaac aaataggggt 6060
   tccgcgcaca tttccccgaa aagtgccacc tgacgtc 6097
<210> 13
   <211> 6094
   <212> DNA
   <213> Artificial Sequence
<220>
   <223> Plasmid
<400> 13
   gacggatcgg gagatctccc gatcccctat ggtcgactct cagtacaatc tgctctgatg 60
   ccgcatagtt aagccagtat ctgctccctg cttgtgtgtt ggaggtcgct gagtagtgcg 120
   cgagcaaaat ttaagctaca acaaggcaag gcttgaccga caattgcatg aagaatctgc 180
   ttagggttag gcgttttgcg ctgcttcgcg atgtacgggc cagatatacg cgttgacatt 240
   gattattgac tagttattaa tagtaatcaa ttacggggtc attagttcat agcccatata 300
   tggagttccg cgttacataa cttacggtaa atggcccgcc tggctgaccg cccaacgacc 360
   cccgcccatt gacgtcaata atgacgtatg ttcccatagt aacgccaata gggactttcc 420
   attgacgtca atgggtggac tatttacggt aaactgccca cttggcagta catcaagtgt 480
   atcatatgcc aagtacgccc cctattgacg tcaatgacgg taaatggccc gcctggcatt 540
   atgcccagta catgacctta tgggactttc ctacttggca gtacatctac gtattagtca 600
   tcgctattac catggtgatg cggttttggc agtacatcaa tgggcgtgga tagcggtttg 660
   actcacgggg atttccaagt ctccacccca ttgacgtcaa tgggagtttg ttttggcacc 720
   aaaatcaacg ggactttcca aaatgtcgta acaactccgc cccattgacg caaatgggcg 780
   gtaggcgtgt acggtgggag gtctatataa gcagagctct ctggctaact agagaaccca 840
   ctgcttactg gcttatcgaa attaatacga ctcactatag ggagacccaa gctggctaga 900
   aagcttggat ctcaccatgg tgttgcagac ccaggtcttc atttctctgt tactctggat 960
   ctctggtgcc tacggggaca tcgtgatgac ccagtctcca gactccctgg ctgtgtctct 1020
   gggcgagagg gccaccatca actgcaagtc caaccagagt gtcttacaca gctccaacaa 1080
   taagaactat ttagcttggt accagcagaa accaggacag cctcctaaat tgctcattta 1140
   ttgggcattc ctccgggaat ccggggtccc tgaccgcttc agtggcagcg ggtctgggac 1200
   agatttcact ctcaccatca gcagcctgca ggctgaagat gtggcagttt attactgtca 1260
   ccaatattat tctactttat atactttcgg cggagggacc aaggtagaga tcaaacgaac 1320
   ygtggctgca ccatctgtct tcatcttccc gccatctgat gagcagttga aatctggaac 1380
   tgctagcgtt gtgtgcctgc tgaataactt ctatcccaga gaggccaaag tacagtggaa 1440
   ggtggataac gccctccaat cgggtaactc ccaggagagt gtcacagagc aggacagcaa 1500
   ggacagcacc tacagcctca gcagcaccct gacgctgagc aaagcagact acgagaaaca 1560
   caaagtctac gcctgcgaag tcacccatca gggcctgagc tcgcccgtca caaagagctt 1620
   caacagggga gagtgttagg cggccgctcg agtctagagg gcccgtttaa acccgctgat 1680
   cagcctcgac tgtgccttct agttgccagc catctgttgt ttgcccctcc cccgtgcctt 1740
   ccttgaccct ggaaggtgcc actcccactg tcctttccta ataaaatgag gaaattgcat 1800
   cgcattgtct gagtaggtgt cattctattc tggggggtgg ggtggggcag gacagcaagg 1860
   gggaggattg ggaagacaat agcaggcatg ctggggatgc ggtgggctct atggcttctg 1920
   aggcggaaag aaccagctgg ggctctaggg ggtatcccca cgcgccctgt agcggcgcat 1980
   taagcgcggc gggtgtggtg gttacgcgca gcgtgaccgc tacacttgcc agcgccctag 2040
   cgcccgctcc tttcgctttc ttcccttcct ttctcgccac gttcgccggc tttccccgtc 2100
   aagctctaaa tcggggcatc cctttagggt tccgatttag tgctttacgg cacctcgacc 2160
   ccaaaaaact tgattagggt gatggttcac gtagtgggcc atcgccctga tagacggttt 2220
   ttcgcccttt gacgttggag tccacgttct ttaatagtgg actcttgttc caaactggaa 2280
   caacactcaa ccctatctcg gtctattctt ttgatttata agggattttg gggatttcgg 2340
   cctattggtt aaaaaatgag ctgatttaac aaaaatttaa cgcgaattaa ttctgtggaa 2400
   tgtgtgtcag ttagggtgtg gaaagtcccc aggctcccca ggcaggcaga agtatgcaaa 2460
   gcatgcatct caattagtca gcaaccaggt gtggaaagtc cccaggctcc ccagcaggca 2520
   gaagtatgca aagcatgcat ctcaattagt cagcaaccat agtcccgccc ctaactccgc 2580
   ccatcccgcc cctaactccg cccagttccg cccattctcc gccccatggc tgactaattt 2640
   tttttattta tgcagaggcc gaggccgcct ctgcctctga gctattccag aagtagtgag 2700
   gaggcttttt tggaggccta ggcttttgca aaaagctccc gggagcttgt atatccattt 2760
   tcggatctga tcaagagaca ggatgaggat cgtttcgcat gattgaacaa gatggattgc 2820
   acgcaggttc tccggccgct tgggtggaga ggctattcgg ctatgactgg gcacaacaga 2880
   caatcggctg ctctgatgcc gccgtgttcc ggctgtcagc gcaggggcgc ccggttcttt 2940
   ttgtcaagac cgacctgtcc ggtgccctga atgaactgca ggacgaggca gcgcggctat 3000
   cgtggctggc cacgacgggc gttccttgcg cagctgtgct cgacgttgtc actgaagcgg 3060
   gaagggactg gctgctattg ggcgaagtgc cggggcagga tctcctgtca tctcaccttg 3120
   ctcctgccga gaaagtatcc atcatggctg atgcaatgcg gcggctgcat acgcttgatc 3180
   cggctacctg cccattcgac caccaagcga aacatcgcat cgagcgagca cgtactcgga 3240
   tggaagccgg tcttgtcgat caggatgatc tggacgaaga gcatcagggg ctcgcgccag 3300
   ccgaactgtt cgccaggctc aaggcgcgca tgcccgacgg cgaggatctc gtcgtgaccc 3360
   atggcgatgc ctgcttgccg aatatcatgg tggaaaatgg ccgcttttct ggattcatcg 3420
   actgtggccg gctgggtgtg gcggaccgct atcaggacat agcgttggct acccgtgata 3480
   ttgctgaaga gcttggcggc gaatgggctg accgcttcct cgtgctttac ggtatcgccg 3540
   ctcccgattc gcagcgcatc gccttctatc gccttcttga cgagttcttc tgagcgggac 3600
   tctggggttc gaaatgaccg accaagcgac gcccaacctg ccatcacgag atttcgattc 3660
   caccgccgcc ttctatgaaa ggttgggctt cggaatcgtt ttccgggacg ccggctggat 3720
   gatcctccag cgcggggatc tcatgctgga gttcttcgcc caccccaact tgtttattgc 3780
   agcttataat ggttacaaat aaagcaatag catcacaaat ttcacaaata aagcattttt 3840
   ttcactgcat tctagttgtg gtttgtccaa actcatcaat gtatcttatc atgtctgtat 3900
   accgtcgacc tctagctaga gcttggcgta atcatggtca tagctgtttc ctgtgtgaaa 3960
   ttgttatccg ctcacaattc cacacaacat acgagccgga agcataaagt gtaaagcctg 4020
   gggtgcctaa tgagtgagct aactcacatt aattgcgttg cgctcactgc ccgctttcca 4080
   gtcgggaaac ctgtcgtgcc agctgcatta atgaatcggc caacgcgcgg ggagaggcgg 4140
   tttgcgtatt gggcgctctt ccgcttcctc gctcactgac tcgctgcgct cggtcgttcg 4200
   gctgcggcga gcggtatcag ctcactcaaa ggcggtaata cggttatcca cagaatcagg 4260
   ggataacgca ggaaagaaca tgtgagcaaa aggccagcaa aaggccagga accgtaaaaa 4320
   ggccgcgttg ctggcgtttt tccataggct ccgcccccct gacgagcatc acaaaaatcg 4380
   acgctcaagt cagaggtggc gaaacccgac aggactataa agataccagg cgtttccccc 4440
   tggaagctcc ctcgtgcgct ctcctgttcc gaccctgccg cttaccggat acctgtccgc 4500
   ctttctccct tcgggaagcg tggcgctttc tcaatgctca cgctgtaggt atctcagttc 4560
   ggtgtaggtc gttcgctcca agctgggctg tgtgcacgaa ccccccgttc agcccgaccg 4620
   ctgcgcctta tccggtaact atcgtcttga gtccaacccg gtaagacacg acttatcgcc 4680
   actggcagca gccactggta acaggattag cagagcgagg tatgtaggcg gtgctacaga 4740
   gttcttgaag tggtggccta actacggcta cactagaagg acagtatttg gtatctgcgc 4800
   tctgctgaag ccagttacct tcggaaaaag agttggtagc tcttgatccg gcaaacaaac 4860
   caccgctggt agcggtggtt tttttgtttg caagcagcag attacgcgca gaaaaaaagg 4920
   atctcaagaa gatcctttga tcttttctac ggggtctgac gctcagtgga acgaaaactc 4980
   acgttaaggg attttggtca tgagattatc aaaaaggatc ttcacctaga tccttttaaa 5040
   ttaaaaatga agttttaaat caatctaaag tatatatgag taaacttggt ctgacagtta 5100
   ccaatgctta atcagtgagg cacctatctc agcgatctgt ctatttcgtt catccatagt 5160
   tgcctgactc cccgtcgtgt agataactac gatacgggag ggcttaccat ctggccccag 5220
   tgctgcaatg ataccgcgag acccacgctc accggctcca gatttatcag caataaacca 5280
   gccagccgga agggccgagc gcagaagtgg tcctgcaact ttatccgcct ccatccagtc 5340
   tattaattgt tgccgggaag ctagagtaag tagttcgcca gttaatagtt tgcgcaacgt 5400
   tgttgccatt gctacaggca tcgtggtgtc acgctcgtcg tttggtatgg cttcattcag 5460
   ctccggttcc caacgatcaa ggcgagttac atgatccccc atgttgtgca aaaaagcggt 5520
   tagctccttc ggtcctccga tcgttgtcag aagtaagttg gccgcagtgt tatcactcat 5580
   ggttatggca gcactgcata attctcttac tgtcatgcca tccgtaagat gcttttctgt 5640
   gactggtgag tactcaacca agtcattctg agaatagtgt atgcggcgac cgagttgctc 5700
   ttgcccggcg tcaatacggg ataataccgc gccacatagc agaactttaa aagtgctcat 5760
   cattggaaaa cgttcttcgg ggcgaaaact ctcaaggatc ttaccgctgt tgagatccag 5820
   ttcgatgtaa cccactcgtg cacccaactg atcttcagca tcttttactt tcaccagcgt 5880
   ttctgggtga gcaaaaacag gaaggcaaaa tgccgcaaaa aagggaataa gggcgacacg 5940
   gaaatgttga atactcatac tcttcctttt tcaatattat tgaagcattt atcagggtta 6000
   ttgtctcatg agcggataca tatttgaatg tatttagaaa aataaacaaa taggggttcc 6060
   gcgcacattt ccccgaaaag tgccacctga cgtc 6094
<210> 14
   <211> 481
   <212> DNA
   <213> Artificial Sequence
<220>
   <223> Includes BamHI/Bg1II cloning junction, signal peptide, v region, portion of C
   region and 3'XbaI/NheI (heavy) or NheI (light) cloning junction
<400> 14
   ggatctcacc atggagttgg gactgcgctg gggcttcctc gttgctcttt taagaggtgt 60
   ccagtgtcag gtgcaattgg tggagtctgg gggaggcgtg gtccagcctg ggaggtccct 120
   gagactctcc tgtgcagcgt ctggattcgc cttcagtaga tatggcatgc actgggtccg 180
   ccaggctcca ggcaaggggc tggagtgggt ggcagttata tggtatgatg gaagtaataa 240
   atactatgca gactccgtga agggccgatt caccatctcc agagacaatt ccaagaacac 300
   gcagtatctg caaatgaaca gcctgagagc cgaggacacg gctgtgtatt actgtgcgag 360
   aggcggtgac ttcctctact actactatta cggtatggac gtctggggcc aagggaccac 420
   ggtcaccgtc tcctcagcct ccaccaaggg cccatcggtc ttccccctgg caccctctag 480
   c 481
<210> 15
   <211> 142
   <212> PRT
   <213> Homo sapiens
<400> 15
   Met Glu Leu Gly Leu Arg Trp Gly Phe Leu Val Ala Leu Leu Arg Gly
   1               5                   10                  15
   Val Gln Cys Gln Val Gln Leu Val Glu Ser Gly Gly Gly Val Val Gln
               20                  25                  30
   Pro Gly Arg Ser Leu Arg Leu Ser Cys Ala Ala Ser Gly Phe Ala Phe
           35                  40                  45
   Ser Arg Tyr Gly Met His Trp Val Arg Gln Ala Pro Gly Lys Gly Leu
       50                  55                  60
   Glu Trp Val Ala Val Ile Trp Tyr Asp Gly Ser Asn Lys Tyr Tyr Ala
   65                  70                  75                  80
   Asp Ser Val Lys Gly Arg Phe Thr Ile Ser Arg Asp Asn Ser Lys Asn
                   85                  90                  95
   Thr Gln Tyr Leu Gln Met Asn Ser Leu Arg Ala Glu Asp Thr Ala Val
               100                 105                 110
   Tyr Tyr Cys Ala Arg Gly Gly Asp Phe Leu Tyr Tyr Tyr Tyr Tyr Gly
           115                 120                 125
   Met Asp Val Trp Gly Gln Gly Thr Thr Val Thr Val Ser Ser
       130                 135                 140
<210> 16
   <211> 463
   <212> DNA
   <213> Artificial Sequence
<220>
   <223> Includes BamHI/Bg1II cloning junction, signal peptide, v region, portion of C
   region and 3'XbaI/NheI (heavy) or NheI (light) cloning junction
<400> 16
   ggatctcacc atgagggtcc ctgctcagct cctgggactc ctgctgctct ggctcccaga     60
   taccagatgt gacatccaga tgacccagtc tccatcctcc ctgtctgcat ctgtaggaga    120
   cagagtcacc atcacttgcc gggcgagtca gggcattagc aattatttag cctggtatca    180
   gcagaaaaca gggaaagttc ctaagttcct gatctatgaa gcatccactt tgcaatcagg    240
   ggtcccatct cggttcagtg gcggtggatc tgggacagat ttcactctca ccatcagcag    300
   cctgcagcct gaagatgttg caacttatta ctgtcaaaat tataacagtg ccccattcac    360
   tttcggccct gggaccaaag tggatatcaa acgaactgtg gctgcaccct ctgtcttcat    420
   cttcccgcca tctgatgagc agttgaaatc tggaactgct agc                       463
<210> 17
   <211> 127
   <212> PRT
   <213> Homo sapiens
<400> 17
   Met Arg Val Pro Ala Gln Leu Leu Gly Leu Leu Leu Leu Trp Leu Pro
   1               5                   10                  15
   Asp Thr Arg Cys Asp Ile Gln Met Thr Gln Ser Pro Ser Ser Leu Ser
               20                  25                  30
   Ala Ser Val Gly Asp Arg Val Thr Ile Thr Cys Arg Ala Ser Gln Gly
           35                  40                  45
   Ile Ser Asn Tyr Leu Ala Trp Tyr Gln Gln Lys Thr Gly Lys Val Pro
       50                  55                  60
   Lys Phe Leu Ile Tyr Glu Ala Ser Thr Leu Gln Ser Gly Val Pro Ser
   65                  70                  75                  80
   Arg Phe Ser Gly Gly Gly Ser Gly Thr Asp Phe Thr Leu Thr Ile Ser
                   85                  90                  95
   Ser Leu Gln Pro Glu Asp Val Ala Thr Tyr Tyr Cys Gln Asn Tyr Asn
               100                 105                 110
   Ser Ala Pro Phe Thr Phe Gly Pro Gly Thr Lys Val Asp Ile Lys
           115                 120                 125
<210> 18
   <211> 508
   <212> DNA
   <213> Artificial Sequence
<220>
   <223> Includes BamHI/Bg1II cloning junction, signal peptide, v region, portion of C
   region and 3'XbaI/NheI (heavy) or NheI (light) cloning junction
<400> 18
   ggatctcacc atggggtcaa ccgccatcct caccatggag ttggggctgc gctgggttct 60
   cctcgttgct cttttaagag gtgtccagtg tcaggtgcag ctggtggagt ctgggggagg 120
   cgtggtccag cctgggaggt ccctgagact ctcctgtgca gcgtctggat tcaccttcag 180
   taactatgtc atgcactggg tccgccaggc tccaggcaag gggctggagt gggtggcaat 240
   tatatggtat gatggaagta ataaatacta tgcagactcc gtgaagggcc gattcaccat 300
   ctccagagac aattccaaga acacgctgta tctgcaaatg aacagcctga gagccgagga 360
   cacggctgtg tattactgtg cgggtggata taactggaac tacgagtacc actactacgg 420
   tatggacgtc tggggccaag ggaccacggt caccgtctcc tcagcctcca ccaagggccc 480
   atcggtcttc cccctggcac cctctagc 508
<210> 19
   <211> 143
   <212> PRT
   <213> Homo sapiens
<400> 19
   Met Glu Leu Gly Leu Arg Trp Val Leu Leu Val Ala Leu Leu Arg Gly
   1 5 10 15
   Val Gln Cys Gln Val Gln Leu Val Glu Ser Gly Gly Gly Val Val Gln
   20 25 30
   Pro Gly Arg Ser Leu Arg Leu Ser Cys Ala Ala Ser Gly Phe Thr Phe
   35 40 45
   Ser Asn Tyr Val Met His Trp Val Arg Gln Ala Pro Gly Lys Gly Leu
   50 55 60
   Glu Trp Val Ala Ile Ile Trp Tyr Asp Gly Ser Asn Lys Tyr Tyr Ala
   65 70 75 80
   Asp Ser Val Lys Gly Arg Phe Thr Ile Ser Arg Asp Asn Ser Lys Asn
   85 90 95
   Thr Leu Tyr Leu Gln Met Asn Ser Leu Arg Ala Glu Asp Thr Ala Val
   100 105 110
   Tyr Tyr Cys Ala Gly Gly Tyr Asn Trp Asn Tyr Glu Tyr His Tyr Tyr
   115 120 125
   Gly Met Asp Val Trp Gly Gln Gly Thr Thr Val Thr Val Ser Ser
   130 135 140
<210> 20
   <211> 463
   <212> DNA
   <213> Artificial Sequence
<220>
   <223> Includes BamHI/Bg1II cloning junction, signal peptide, v region, portion of C
   region and 3'XbaI/NheI (heavy) or NheI (light) cloning junction
<400> 20
   ggatctcacc atgagggtcc ccgctcagct cctggggctc ctgctgctct gtttcccagg     60
   tgccagatgt gacatccaga tgacccagtc tccatcctca ctgtctgcat ctgtaggaga    120
   cagagtcacc atcacttgtc gggcgagtca gggcattacc aattatttag cctggtttca    180
   gcagaaacca gggaaagccc ctaagtccct tatctatgct gcatccagtt tgcaaagtgg    240
   ggtcccatca aagttcagcg gcagtggatc tgggacagat ttcagtctca ccatcagcag    300
   cctgcagcct gaagattttg caacttatta ctgccaacag tataatagtt acccgatcac    360
   cttcggccaa gggacacgac tggagattaa acgaactgtg gctgcaccat ctgtcttcat    420
   cttcccgcca tctgatgagc agttgaaatc tggaactgct agc                      463
<210> 21
   <211> 127
   <212> PRT
   <213> Homo sapiens
<400> 21
   Met Arg Val Pro Ala Gln Leu Leu Gly Leu Leu Leu Leu Cys Phe Pro
   1               5                   10                  15
   Gly Ala Arg Cys Asp Ile Gln Met Thr Gln Ser Pro Ser Ser Leu Ser
               20                  25                  30
   Ala Ser Val Gly Asp Arg Val Thr Ile Thr Cys Arg Ala Ser Gln Gly
           35                  40                  45
   Ile Thr Asn Tyr Leu Ala Trp Phe Gln Gln Lys Pro Gly Lys Ala Pro
       50                  55                  60
   Lys Ser Leu Ile Tyr Ala Ala Ser Ser Leu Gln Ser Gly Val Pro Ser
   65                  70                  75                  80
   Lys Phe Ser Gly Ser Gly Ser Gly Thr Asp Phe Ser Leu Thr Ile Ser
                   85                  90                  95
   Ser Leu Gln Pro Glu Asp Phe Ala Thr Tyr Tyr Cys Gln Gln Tyr Asn
               100                 105                 110
   Ser Tyr Pro Ile Thr Phe Gly Gln Gly Thr Arg Leu Glu Ile Lys
           115                 120                 125
<210> 22
   <211> 490
   <212> DNA
   <213> Artificial Sequence
<220>
   <223> Includes BamHI/Bg1II cloning junction, signal peptide, v region, portion of C
   region and 3'XbaI/NheI (heavy) or NheI (light) cloning junction
<400> 22
   ggatctcacc atggagttgg gacttagctg ggttttcctc gttgctcttt taagaggtgt      60
   ccagtgtcag gtccagctgg tggagtctgg gggaggcgtg gtccagcctg ggaggtccct     120
   gagactctcc tgtgcagcgt ctggattcac cttcagtagc tatggcatgc actgggtccg     180
   ccaggctcca ggcaaggggc tggactgggt ggcaattatt tggcatgatg gaagtaataa     240
   atactatgca gactccgtga agggccgatt caccatctcc agagacaatt ccaagaagac     300
   gctgtacctg caaatgaaca gtttgagagc cgaggacacg gctgtgtatt actgtgcgag     360
   agcttgggcc tatgactacg gtgactatga atactacttc ggtatggacg tctggggcca     420
   agggaccacg gtcaccgtct cctcagcctc caccaagggc ccatcggtct tccccctggc      480
   accctctagc                                                            490
<210> 23
   <211> 145
   <212> PRT
   <213> Homo sapiens
<400> 23
   Met Glu Leu Gly Leu Ser Trp Val Phe Leu Val Ala Leu Leu Arg Gly
   1               5                   10                  15
   Val Gln Cys Gln Val Gln Leu Val Glu Ser Gly Gly Gly Val Val Gln
               20                  25                  30
   Pro Gly Arg Ser Leu Arg Leu Ser Cys Ala Ala Ser Gly Phe Thr Phe
           35                  40                  45
   Ser Ser Tyr Gly Met His Trp Val Arg Gln Ala Pro Gly Lys Gly Leu
       50                  55                  60
   Asp Trp Val Ala Ile Ile Trp His Asp Gly Ser Asn Lys Tyr Tyr Ala
   65                  70                  75                  80
   Asp Ser Val Lys Gly Arg Phe Thr Ile Ser Arg Asp Asn Ser Lys Lys
                   85                  90                  95
   Thr Leu Tyr Leu Gln Met Asn Ser Leu Arg Ala Glu Asp Thr Ala Val
               100                 105                 110
   Tyr Tyr Cys Ala Arg Ala Trp Ala Tyr Asp Tyr Gly Asp Tyr Glu Tyr
           115                 120                 125
   Tyr Phe Gly Met Asp Val Trp Gly Gln Gly Thr Thr Val Thr Val Ser
       130                 135                 140
   Ser
   145
<210> 24
   <211> 463
   <212> DNA
   <213> Artificial Sequence
<220>
   <223> Includes BamHI/Bg1II cloning junction, signal peptide, v region, portion of C
   region and 3'XbaI/NheI (heavy) or NheI (light) cloning junction
<400> 24
   ggatctcacc atgagggtcc ctgctcagct cctggggctc ctgctgctct gtttcccagg     60
   tgccagatgt gacatccaga tgacccagtc tccatcctca ctgtctgcat ctgtaggaga    120
   cagagtcacc atcacttgtc gggcgagtca gggcattagc cattatttag cctggtttca    180
   gcagaaacca gggaaagccc ctaagtccct gatctatgct gcatccagtt tgcaaagtgg    240
   ggtcccatca aagttcagcg gcagtggatc tgggacagat ttcactctca ccatcagcag    300
   cctacagcct gaagattttg caacttatta ctgccaacag tataatagtt tcccgctcac    360
   tttcggcgga gggaccaagg tggagatcaa acgaactgtg gctgcaccat ctgtcttcat    420
   cttcccgcca tctgatgagc agttgaaatc tggaactgct agc                      463
<210> 25
   <211> 127
   <212> PRT
   <213> Homo sapiens
<400> 25
   Met Arg Val Pro Ala Gln Leu Leu Gly Leu Leu Leu Leu Cys Phe Pro
   1               5                   10                  15
   Gly Ala Arg Cys Asp Ile Gln Met Thr Gln Ser Pro Ser Ser Leu Ser
               20                  25                  30
   Ala Ser Val Gly Asp Arg Val Thr Ile Thr Cys Arg Ala Ser Gln Gly
           35                  40                  45
   Ile Ser His Tyr Leu Ala Trp Phe Gln Gln Lys Pro Gly Lys Ala Pro
       50                  55                  60
   Lys Ser Leu Ile Tyr Ala Ala Ser Ser Leu Gln Ser Gly Val Pro Ser
   65                  70                  75                  80
   Lys Phe Ser Gly Ser Gly Ser Gly Thr Asp Phe Thr Leu Thr Ile Ser
                   85                  90                  95
   Ser Leu Gln Pro Glu Asp Phe Ala Thr Tyr Tyr Cys Gln Gln Tyr Asn
               100                 105                 110
   Ser Phe Pro Leu Thr Phe Gly Gly Gly Thr Lys Val Glu Ile Lys
           115                 120                 125
<210> 26
   <211> 469
   <212> DNA
   <213> Artificial Sequence
<220>
   <223> Includes BamHI/Bg1II cloning junction, signal peptide, v region, portion of C
   region and 3'XbaI/NheI (heavy) or NheI (light) cloning junction
<400> 26
   ggatcccacc atggggtcaa ccgtcatcct cgccctcctc ctggctgttc tccaaggagt     60
   ctgtgccgag gtgcagctgg tgcagtctgg agcagaggtg aaaaagcccg gggagtctct    120
   gaagatctcc tgtaagggtt ctggatacag ctttaccagt tactggatcg gctgggtgcg    180
   ccagatgccc gggaaaggcc tggagtggat ggggatcatc tatcctggtg actctgatac    240
   cagatacagc ccgtccttcc aaggccaggt caccatctca gccgacaagt ccatcagcac    300
   cgcctacctg cagtggagca gcctgaaggc ctcggacacc gccatgtatt actgtgcgag    360
   acggatggca gcagctggcc cctttgacta ctggggccag ggaaccctgg tcaccgtctc    420
   ctcagcctcc accaagggcc catcggtctt ccccctggca ccctctagc               469
<210> 27
   <211> 138
   <212> PRT
   <213> Homo sapiens
<400> 27
   Met Gly Ser Thr Val Ile Leu Ala Leu Leu Leu Ala Val Leu Gln Gly
   1               5                   10                  15
   Val Cys Ala Glu Val Gln Leu Val Gln Ser Gly Ala Glu Val Lys Lys
               20                  25                  30
   Pro Gly Glu Ser Leu Lys Ile Ser Cys Lys Gly Ser Gly Tyr Ser Phe
           35                  40                  45
   Thr Ser Tyr Trp Ile Gly Trp Val Arg Gln Met Pro Gly Lys Gly Leu
       50                  55                  60
   Glu Trp Met Gly Ile Ile Tyr Pro Gly Asp Ser Asp Thr Arg Tyr Ser
   65                  70                  75                  80
   Pro Ser Phe Gln Gly Gln Val Thr Ile Ser Ala Asp Lys Ser Ile Ser
                   85                  90                  95
   Thr Ala Tyr Leu Gln Trp Ser Ser Leu Lys Ala Ser Asp Thr Ala Met
               100                 105                 110
   Tyr Tyr Cys Ala Arg Arg Met Ala Ala Ala Gly Pro Phe Asp Tyr Trp
           115                 120                 125
   Gly Gln Gly Thr Leu Val Thr Val Ser Ser
       130                 135
<210> 28
   <211> 466
   <212> DNA
   <213> Artificial Sequence
<220>
   <223> Includes BamHI/Bg1II cloning junction, signal peptide, v region, portion of C
   region and 3'XbaI/NheI (heavy) or NheI (light) cloning junction
<400> 28
   ggatctcacc atgagggtcc ccgctcagct tctcttcctt ctgctactct ggctcccaga 60
   taccactgga ggaatagtga tgacgcagtc tccagccacc ctgtctgtgt ctccagggga 120
   aagagccacc ctctcctgca ggaccagtca gagtattggc tggaacttag cctggtacca 180
   acagaaacct ggccaggctc ccaggctcct catctatggt gcatcttcca ggaccactgg 240
   tatcccagcc aggttcagtg gcagtgggtc tgggacagag ttcactctca ccatcagcag 300
   cctgcagtct gaagattctg cagtttatta ctgtcagcat tatgataact ggcccatgtg 360
   cagttttggc caggggaccg agctggagat caaacgaact gtggctgcac catctgtctt 420
   catcttcccg ccatctgatg agcagttgaa atctggaact gctagc 466
<210> 29
   <211> 128
   <212> PRT
   <213> Homo sapiens
<400> 29
   Met Arg Val Pro Ala Gln Leu Leu Phe Leu Leu Leu Leu Trp Leu Pro
   1 5 10 15
   Asp Thr Thr Gly Gly Ile Val Met Thr Gln Ser Pro Ala Thr Leu Ser
   20 25 30
   Val Ser Pro Gly Glu Arg Ala Thr Leu Ser Cys Arg Thr Ser Gln Ser
   35 40 45
   Ile Gly Trp Asn Leu Ala Trp Tyr Gln Gln Lys Pro Gly Gln Ala Pro
   50 55 60
   Arg Leu Leu Ile Tyr Gly Ala Ser Ser Arg Thr Thr Gly Ile Pro Ala
   65 70 75 80
   Arg Phe Ser Gly Ser Gly Ser Gly Thr Glu Phe Thr Leu Thr Ile Ser
   85 90 95
   Ser Leu Gln Ser Glu Asp Ser Ala Val Tyr Tyr Cys Gln His Tyr Asp
   100 105 110
   Asn Trp Pro Met Cys Ser Phe Gly Gln Gly Thr Glu Leu Glu Ile Lys
   115 120 125
<210> 30
   <211> 487
   <212> DNA
   <213> Artificial Sequence
<220>
   <223> Includes BamHI/Bg1II cloning junction, signal peptide, v region, portion of C
   region and 3'XbaI/NheI (heavy) or NheI (light) cloning junction
<400> 30
   ggatctcacc atggagtttg ggctgtgctg gattttcctc gttgctcttt taagaggtgt    60
   ccagtgtcag gtgcagctgg tggagtctgg gggaggcgtg gtccagcctg ggaggtccct   120
   gagactctcc tgtgcagcct ctggattcac cttcattagc tatggcatgc actgggtccg   180
   ccaggctcca ggcaaggggc tggagtgggt ggcagttata tcatatgatg gaagtaataa   240
   atactatgca gactccgtga agggccgatt caccatctcc agagacaatt ccaagaacac   300
   gctgtatctg caaatgaaca gcctgagagc tgaggacacg gctgtgtatt actgtgcgag   360
   agtattagtg ggagctttat attattataa ctactacggg atggacgtct ggggccaagg   420
   gaccacggtc accgtctcct cagcctccac caagggccca tcggtcttcc ccctggcacc   480
   ctctagc                                                             487
<210> 31
   <211> 144
   <212> PRT
   <213> Homo sapiens
<400> 31
   Met Glu Phe Gly Leu Cys Trp Ile Phe Leu Val Ala Leu Leu Arg Gly
   1               5                   10                  15
   Val Gln Cys Gln Val Gln Leu Val Glu Ser Gly Gly Gly Val Val Gln
               20                  25                  30
   Pro Gly Arg Ser Leu Arg Leu Ser Cys Ala Ala Ser Gly Phe Thr Phe
           35                  40                  45
   Ile Ser Tyr Gly Met His Trp Val Arg Gln Ala Pro Gly Lys Gly Leu
       50                  55                  60
   Glu Trp Val Ala Val Ile Ser Tyr Asp Gly Ser Asn Lys Tyr Tyr Ala
   65                  70                  75                  80
   Asp Ser Val Lys Gly Arg Phe Thr Ile Ser Arg Asp Asn Ser Lys Asn
                   85                  90                  95
   Thr Leu Tyr Leu Gln Met Asn Ser Leu Arg Ala Glu Asp Thr Ala Val
               100                 105                 110
   Tyr Tyr Cys Ala Arg Val Leu Val Gly Ala Leu Tyr Tyr Tyr Asn Tyr
           115                 120                 125
   Tyr Gly Met Asp Val Trp Gly Gln Gly Thr Thr Val Thr Val Ser Ser
       130                 135                 140
<210> 32
   <211> 478
   <212> DNA
   <213> Artificial Sequence
<220>
   <223> Includes BamHI/Bg1II cloning junction, signal peptide, v region, portion of C
   region and 3'XbaI/NheI (heavy) or NheI (light) cloning junction
<400> 32
   ggatctcacc atgagggtcc ctgctcagct cctggggctg ctaatgctct ggatacctgg    60
   atccagtgca gatattgtga tgacccagac tccactctct ctgtccgtca cccctggaca   120
   gccggcctcc atctcctgca agtctagtca gagcctcctg catagtgatg gaaagacctt   180
   tttgtattgg tatctgcaga agccaggcca gcctccacag ctcctgatct atgaggtttc   240
   caaccggttc tctggagtgc cagataggtt cagtggcagc gggtcaggga cagatttcac   300
   actgaaaatc agccgggtgg aggctgagga tgttgggctt tattactgca tgcaaagtat   360
   acagcttccg ctcactttcg gcggagggac caaggtggag atcaaacgaa ctgtggctgc   420
   accatctgtc ttcatcttcc cgccatctga tgagcagttg aaatctggaa ctgctagc     478
<210> 33
   <211> 132
   <212> PRT
   <213> Homo sapiens
<400> 33
   Met Arg Val Pro Ala Gln Leu Leu Gly Leu Leu Met Leu Trp Ile Pro
   1               5                   10                  15
   Gly Ser Ser Ala Asp Ile Val Met Thr Gln Thr Pro Leu Ser Leu Ser
               20                  25                  30
   Val Thr Pro Gly Gln Pro Ala Ser Ile Ser Cys Lys Ser Ser Gln Ser
           35                  40                  45
   Leu Leu His Ser Asp Gly Lys Thr Phe Leu Tyr Trp Tyr Leu Gln Lys
       50                  55                  60
   Pro Gly Gln Pro Pro Gln Leu Leu Ile Tyr Glu Val Ser Asn Arg Phe
   65                  70                  75                  80
   Ser Gly Val Pro Asp Arg Phe Ser Gly Ser Gly Ser Gly Thr Asp Phe
                   85                  90                  95
   Thr Leu Lys Ile Ser Arg Val Glu Ala Glu Asp Val Gly Leu Tyr Tyr
               100                 105                 110
   Cys Met Gln Ser Ile Gln Leu Pro Leu Thr Phe Gly Gly Gly Thr Lys
           115                 120                 125
   Val Glu Ile Lys
       130

## Description

### BACKGROUND

Prostate cancer is the most common malignancy and the second leading cause of cancer death in men in the United States (Jemal A, et al., CA Cancer J Clin 2005;55:10-30). Localized prostate cancer typically is treated with surgery or radiation, and recurrent disease can be controlled temporarily with androgen ablation (Klein EA, et al., Urol Clin North Am 2003;30:315-30). However, almost all prostate carcinomas eventually become hormone refractory and then rapidly progress (Denmeade SR, et al., Nat Rev Cancer 2002;2:389-96). Hormone-refractory or androgen-independent prostate cancer has proven to be largely resistant to conventional chemotherapy. Therapies that have been shown to prolong life in subjects with metastatic, castration-resistant prostate cancer (mCRPC) include: Taxotere® (docetaxel), Jevtana® (cabazitaxel), Zytiga® (abiraterone acetate), Provenge® (sipuleucel-T), Xtandi® (enzalutamide) and Xofigo® (radium Ra-223 dichloride). The survival benefits may be modest as in the case of Jevtana®, which offer 2.4 month survival benefit (Gulley J, et al., Am J Ther. 2004;351:1513-20; Petrylak DP, et al., New Engl J Med 2004;351:1513-20). New therapies are needed to expand therapeutic options, such as for subjects with mCRPC.

WO 2010/005721 A1 described nanoparticles comprising GL2 and their use for treating prostate cancer. West Lafayette et al: "Endocyte to advance development of EC 1069, a PSMA-targeted small molecule drug conjugate therapy for prostate cancer company expects to file IND for therapeutic agent in 2013 human study of companion imaging agent EC0652 demonstrates safety and binding specificity", 2013-01-29, XP055359848, describes EC1069 and its use to treat prostate cancer.

### SUMMARY OF THE INVENTION

The present invention relates, at least in part, to the surprising discovery that antiandrogens and prostate-specific membrane antigen (PSMA) ligands bound to therapeutic agents (referred to herein as "PSMA ligand conjugates"), such as anticancer agents or cytotoxic agents (referred to herein as, when bound to a PSMA ligand, "PSMA ligand-anticancer agent conjugates" or "PSMA ligand-cytotoxic agent conjugates", respectively), can act synergistically to inhibit proliferation of PSMA-expressing cancer cells. Unexpectedly, this synergism occurs in both androgen-dependent and androgen-independent cells. It was also discovered that mTOR inhibitors and PSMA ligand conjugates also act synergistically to inhibit proliferation of PSMA-expressing cancer cells, such as androgen-independent cells in particular. It was also discovered that prednisone and PSMA ligand conjugates also acted synergistically in combination. Thus, provided herein are methods, compositions and kits for inhibiting proliferation of, or killing, PSMA-expressing cells (*e.g.,* cancer cells such as prostate cancer cells) as well as for sensitizing or resensitizing cells that can express PSMA, such as androgen-independent PSMA-expressing cells, to androgen therapy.

An aspect of the invention provides an *in vitro* method of inhibiting proliferation of prostate-specific membrane antigen (PSMA)-expressing cancer cells. The method comprises: contacting PSMA-expressing cancer cells with an antiandrogen, wherein the antiandrogen is abiraterone, enzalutamide, nilutamide, flutamide, bicalutamide, ARN 509, galeterone or orteronel; and contacting the PSMA-expressing cancer cells with a PSMA ligand-anticancer agent conjugate or a PSMA ligand-cytotoxic agent conjugate, wherein the PSMA ligand of the conjugate comprises a small molecule ligand that binds specifically PSMA and the small molecule ligand comprises a glutamate-urea-amino acid moiety, wherein the glutamate-urea-amino acid moiety is a glutamate-urea-lysine; wherein the PSMA-expressing cancer cells comprise PSMA-expressing cancer cells insensitive to antiandrogen therapy.

In some embodiments, the antiandrogen is enzalutamide or abiraterone.

As described herein, the antiandrogen may be in an amount effective to upregulate PSMA expression.

In some embodiments, the step of contacting the PSMA-expressing cancer cells with the antiandrogen and the step of contacting the PSMA-expressing cancer cells with the PSMA ligand-anticancer agent conjugate are concurrent. In other embodiments, the step of contacting the PSMA-expressing cancer cells with the antiandrogen and the step of contacting the PSMA-expressing cancer cells with the PSMA ligand-anticancer agent conjugate are sequential.

As described herein, the step of contacting the PSMA-expressing cancer cells with the antiandrogen may be prior to the step of contacting the PSMA-expressing cancer cells with the PSMA ligand-anticancer agent conjugate.

As described herein, the step of contacting the PSMA-expressing cancer cells with the PSMA ligand-anticancer agent conjugate may occur within one week of the step of contacting the PSMA-expressing cancer cells with the antiandrogen.

As described herein, the PSMA-expressing cancer cells may be in contact with the antiandrogen for at least 3 days. As described herein, the PSMA-expressing cancer cells may be in contact with the compound for at least 7 days. As described herein, the PSMA-expressing cancer cells may be in contact with the compound for at least 14 days. As described herein, the PSMA-expressing cancer cells may be in contact with the compound for at least 21 days. As described herein, the PSMA-expressing cancer cells may be in contact with the compound for at least 28 days.

In some embodiments, the PSMA-expressing cancer cells comprise androgen-independent PSMA-expressing cancer cells.

As described herein, the PSMA-expressing cancer cells insensitive to antiandrogen therapy may be re-sensitized to antiandrogen therapy after the step of contacting the PSMA-expressing cancer cells with the antiandrogen.

In some embodiments, the PSMA-expressing cancer cells are of a tumor.

In some embodiments, the PSMA-expressing cancer cells are PSMA-expressing prostate cancer cells. In other embodiments, the PSMA-expressing cancer cells are PSMA-expressing non-prostate cancer cells. As described herein, the PSMA-expressing cancer cells may be of the neovasculature of a non-prostate cancer or non-prostate tumor.

In some embodiments, the PSMA-expressing cancer cells are of a subject. In some embodiments, the subject has progressive metastatic castration-resistant prostate cancer. As described herein, the subject may have had prior chemotherapy with one or more taxanes. As described herein, the subject may have had prior treatment with one or more antiandrogens. In some embodiments, the subject has prostate cancer that has progressed despite prior treatment, such as with one or more antiandrogens. As described herein, the subject may have prostate cancer that is starting to progress despite treatment, such as with one or more antiandrogens. As described herein, the subject may have not had prior cytotoxic chemotherapy. As described herein, the subject may have not had prior treatment with one or more antiandrogens. As described herein, the subject may have not had prior treatment with enzalutamide or abiraterone. As described herein, the subject that has not had prior treatment with antiandrogens, such as enzalutamide or abiraterone, may be one with metastatic castration-resistant prostate cancer. In some embodiments, the subject is any one of the subjects described herein.

Further described herein are methods of inhibiting proliferation of prostate-specific membrane antigen (PSMA)-expressing cancer cells. The methods may comprise contacting PSMA-expressing cancer cells with an mTOR inhibitor, and contacting the PSMA-expressing cancer cells with a PSMA ligand-anticancer agent conjugate.

As described herein, the mTOR inhibitor may be rapamycin.

As described herein, the mTOR inhibitor may be in an amount effective to upregulate PSMA expression.

As described herein, the step of contacting the PSMA-expressing cancer cells with the mTOR inhibitor and the step of contacting the PSMA-expressing cancer cells with the PSMA ligand-anticancer agent conjugate may be concurrent.

As described herein, the step of contacting the PSMA-expressing cancer cells with the mTOR inhibitor and the step of contacting the PSMA-expressing cancer cells with the PSMA ligand-anticancer agent conjugate may be sequential.

As described herein, the step of contacting the PSMA-expressing cancer cells with the mTOR inhibitor may be prior to the step of contacting the PSMA-expressing cancer cells with the PSMA ligand-anticancer agent conjugate.

As described herein, the PSMA-expressing cancer cells may be in contact with the mTOR inhibitor for at least 7 days.

As described herein, the PSMA-expressing cancer cells may comprise androgen-independent PSMA-expressing cancer cells.

As described herein, the PSMA-expressing cancer cells may comprise PSMA-expressing cancer cells insensitive to antiandrogen therapy.

As described herein, the PSMA-expressing cancer cells insensitive to antiandrogen therapy may be re-sensitized to antiandrogen therapy after the step of contacting the PSMA-expressing cancer cells with the mTOR inhibitor.

As described herein, the PSMA-expressing cancer cells may be of a tumor.

As described herein, the PSMA-expressing cancer cells may be PSMA-expressing prostate cancer cells. As described herein, the PSMA-expressing cancer cells may be PSMA-expressing non-prostate cancer cells. As described herein, the PSMA-expressing cancer cells may be of the neovasculature of a non-prostate cancer or non-prostate tumor.

As described herein, the PSMA-expressing cancer cells may be of a subject. As described herein, the subject may have progressive metastatic castration-resistant prostate cancer. As described herein, the subject may have had prior chemotherapy with one or more taxanes. As described herein, the subject may have had prior treatment with one or more antiandrogens. As described herein, the subject may have prostate cancer that has progressed despite prior treatment, such as with one or more antiandrogens. As described herein, the subject may have prostate cancer that is starting to progress despite treatment, such as with one or more antiandrogens. As described herein, the subject may have not had prior cytotoxic chemotherapy. As described herein, the subject may have not had prior treatment with one or more antiandrogens. As described herein, the subject may have not had prior treatment with enzalutamide or abiraterone. As described herein, the subject that has not had prior treatment with antiandrogens, such as enzalutamide or abiraterone, may be one with metastatic castration-resistant prostate cancer. As described herein, the subject may be any one of the subjects described herein.

Additionally described herein are methods of inhibiting proliferation of prostate-specific membrane antigen (PSMA)-expressing cancer cells. The methods may comprise contacting PSMA-expressing cancer cells with prednisone, and contacting the PSMA-expressing cancer cells with a PSMA ligand conjugate. As described herein, the method may further comprise contacting the PSMA-expressing cancer cells with a compound that increases cell surface expression of PSMA, such as an antiandrogen.

As described herein, the antiandrogen may block enzyme cytochrome CYP17. As described herein, the antiandrogen may block enzyme cytochrome CYP17A1.

As described herein, the antiandrogen may be an androgen receptor antagonist.

As described herein, the antiandrogen may be abiraterone, enzalutamide, nilutamide, flutamide, bicalutamide, ARN 509, galeterone or orteronel. As described herein, the antiandrogen may be enzalutamide or abiraterone.

As described herein, the antiandrogen may be in an amount effective to upregulate PSMA expression.

As described herein, the step of contacting the PSMA-expressing cancer cells with prednisone and/or the compound that increases cell surface expression of PSMA, such as an antiandrogen, and the step of contacting the PSMA-expressing cancer cells with the PSMA ligand conjugate may be concurrent. As described herein, the step of contacting the PSMA-expressing cancer cells with prednisone and/or the compound, such as an antiandrogen, and the step of contacting the PSMA-expressing cancer cells with the PSMA ligand conjugate may be sequential.

As described herein, the step of contacting the PSMA-expressing cancer cells with prednisone and/or the compound, such as an antiandrogen, may be prior to the step of contacting the PSMA-expressing cancer cells with the PSMA ligand conjugate.

As described herein, the step of contacting the PSMA-expressing cancer cells with the PSMA ligand conjugate may occur within one week of the step of contacting the PSMA-expressing cancer cells with the compound, such as an antiandrogen.

As described herein, the PSMA-expressing cancer cells may be in contact with the compound, such as an antiandrogen, for at least 3 days. As described herein, the PSMA-expressing cancer cells may be in contact with the compound, such as an antiandrogen, for at least 7 days. As described herein, the PSMA-expressing cancer cells may be in contact with the compound, such as an antiandrogen, for at least 14 days. As described herein, the PSMA-expressing cancer cells may be in contact with the compound, such as an antiandrogen, for at least 21 days. As described herein, the PSMA-expressing cancer cells may be in contact with the compound, such as an antiandrogen, for at least 28 days.

As described herein, the PSMA-expressing cancer cells may comprise androgen-independent PSMA-expressing cancer cells.

As described herein, the PSMA-expressing cancer cells may comprise PSMA-expressing cancer cells insensitive to antiandrogen therapy.

As described herein, the PSMA-expressing cancer cells insensitive to antiandrogen therapy may be re-sensitized to antiandrogen therapy after the step of contacting the PSMA-expressing cancer cells with the compound, such as an antiandrogen.

As described herein, the PSMA-expressing cancer cells may be of a tumor.

As described herein, the PSMA-expressing cancer cells may be PSMA-expressing prostate cancer cells. As described herein, the PSMA-expressing cancer cells may be PSMA-expressing non-prostate cancer cells. As described herein, the PSMA-expressing cancer cells may be of the neovasculature of a non-prostate cancer or non-prostate tumor.

As described herein, the PSMA-expressing cancer cells may be of a subject. As described herein, the subject may have progressive metastatic castration-resistant prostate cancer. As described herein, the subject may have had prior chemotherapy with one or more taxanes. As described herein, the subject may have had prior treatment with one or more antiandrogens. As described herein, the subject may have prostate cancer that has progressed despite prior treatment, such as with one or more antiandrogens. As described herein, the subject may have prostate cancer that is starting to progress despite treatment, such as with one or more antiandrogens. As described herein, the subject may have not had prior cytotoxic chemotherapy. As described herein, the subject may have not had prior treatment with one or more antiandrogens. As described herein, the subject may have not had prior treatment with enzalutamide or abiraterone. As described herein, the subject that has not had prior treatment with antiandrogens, such as enzalutamide or abiraterone, may be one with metastatic castration-resistant prostate cancer. As described herein, the subject may be any one of the subjects described herein.

Further described herein are methods of specific delivery of a cytotoxic agent to PSMA-expressing cells in a subject. The methods may comprise administering to a subject a compound that increases cell surface expression of PSMA, and administering to the subject a PSMA ligand-cytotoxic agent conjugate.

As described herein, the compound that increases cell surface expression of PSMA may be an antiandrogen.

As described herein, the antiandrogen may block enzyme cytochrome CYP17. As described herein, the antiandrogen may block enzyme cytochrome CYP17A1.

As described herein, the antiandrogen may be an androgen receptor antagonist. As described herein, the antiandrogen may be abiraterone, enzalutamide, nilutamide, flutamide, bicalutamide, ARN 509, galeterone or orteronel. As described herein, the antiandrogen may be enzalutamide or abiraterone.

As described herein, the compound that increases cell surface expression of PSMA may be an mTOR inhibitor. As described herein, the mTOR inhibitor may be rapamycin.

As described herein, the step of administering the compound and the step of administering the PSMA ligand-cytotoxic agent conjugate may be concurrent. As described herein, the step of administering the compound and the step of administering the PSMA ligand-cytotoxic agent conjugate may be sequential. As described herein, the step of administering the compound may be prior to the step of administering the PSMA ligand-cytotoxic agent conjugate.

As described herein, the step of administering the PSMA ligand-cytotoxic agent conjugate may occur within one week of the step of administering the compound.

As described herein, the PSMA-expressing cells may be in contact with the compound for at least 3 days. As described herein, the PSMA-expressing cells may be in contact with the compound for at least 7 days. As described herein, the PSMA-expressing cells may be in contact with the compound for at least 14 days. As described herein, the PSMA-expressing cells may be in contact with the compound for at least 21 days. As described herein, the PSMA-expressing cells may be in contact with the compound for at least 28 days.

As described herein, the PSMA-expressing cells may comprise androgen-independent PSMA-expressing cancer cells.

As described herein, the PSMA-expressing cells may comprise PSMA-expressing cancer cells insensitive to antiandrogen therapy.

As described herein, the PSMA-expressing cells insensitive to antiandrogen therapy may be re-sensitized to antiandrogen therapy after the step of administering the compound.

As described herein, the PSMA-expressing cells may be of a tumor.

As described herein, the PSMA-expressing cells may be PSMA-expressing prostate cancer cells. As described herein, the PSMA-expressing cells may be PSMA-expressing non-prostate cancer cells. As described herein, the PSMA-expressing cells may be of the neovasculature of a non-prostate cancer or non-prostate tumor.

As described herein, the PSMA-expressing cancer cells may be of a subject. As described herein, the subject may have progressive metastatic castration-resistant prostate cancer. As described herein, the subject may have had prior chemotherapy with one or more taxanes. As described herein, the subject may have had prior treatment with one or more antiandrogens. As described herein, the subject may have prostate cancer that has progressed despite prior treatment, such as with one or more antiandrogens. As described herein, the subject may have prostate cancer that is starting to progress despite treatment, such as with one or more antiandrogens. As described herein, the subject may have not had prior cytotoxic chemotherapy. As described herein, the subject may have not had prior treatment with one or more antiandrogens. As described herein, the subject may have not had prior treatment with enzalutamide or abiraterone. As described herein, the subject that has not had prior treatment with antiandrogens, such as enzalutamide or abiraterone, may be one with metastatic castration-resistant prostate cancer. As described herein, the subject may be any one of the subjects described herein.

Further described herein are compounds that increase cell surface expression of PSMA and a PSMA ligand-anticancer agent conjugate or PSMA ligand-cytotoxic agent conjugate. Additionally described are compositions that comprise prednisone and a PSMA ligand conjugates. As described herein, the compositions may further comprise a compound that increases cell surface expression of PSMA.

As described herein, the compound that increases cell surface expression of PSMA may be an antiandrogen.

As described herein, the antiandrogen may block enzyme cytochrome CYP17. As described herein, the antiandrogen may block enzyme cytochrome CYP17A1.

As described herein, the antiandrogen may be an androgen receptor antagonist. As described herein, the antiandrogen may be abiraterone, enzalutamide, nilutamide, flutamide, bicalutamide, ARN 509, galeterone or orteronel. As described herein, the antiandrogen may be enzalutamide or abiraterone.

As described herein, the compound that increases cell surface expression of PSMA may be an mTOR inhibitor. As described herein, the mTOR inhibitor may be rapamycin.

As described herein, the compositions may further comprise a pharmaceutically acceptable carrier and/or excipient.

According to an aspect of the invention there is provided an antiandrogen selected from the group consisting of abiraterone, enzalutamide, nilutamide, flutamide, bicalutamide, ARN 509, galeterone or orteronel for use in an *in vivo* method of inhibiting proliferation of prostate-specific membrane antigen (PSMA)-expressing cancer cells in a subject, the method comprising:
contacting PSMA-expressing cancer cells with the antiandrogen; and
contacting the PSMA-expressing cancer cells with a PSMA ligand-anticancer agent conjugate or a PSMA ligand-cytotoxic agent conjugate, wherein the PSMA ligand of the conjugate comprises a small molecule ligand that binds specifically PSMA and the small molecule ligand comprises a glutamate-urea-amino acid moiety, wherein the glutamate-urea-amino acid moiety is a glutamate-urea-lysine;
wherein the PSMA-expressing cancer cells comprise PSMA-expressing cancer cells insensitive to antiandrogen therapy.

According to an aspect of the invention there is provided a PSMA ligand-anticancer agent conjugate or a PSMA ligand-cytotoxic agent conjugate for use in an *in vivo* method of inhibiting proliferation of prostate-specific membrane antigen (PSMA)-expressing cancer cells in a subject, the method comprising:
contacting PSMA-expressing cancer cells with an antiandrogen selected from the group consisting of abiraterone, enzalutamide, nilutamide, flutamide, bicalutamide, ARN 509, galeterone or orteronel; and
contacting the PSMA-expressing cancer cells with the PSMA ligand-anticancer agent conjugate or a PSMA ligand-cytotoxic agent conjugate, wherein the PSMA ligand of the conjugate comprises a small molecule ligand that binds specifically PSMA and the small molecule ligand comprises a glutamate-urea-amino acid moiety, wherein the glutamate-urea-amino acid moiety is a glutamate-urea-lysine;
wherein the PSMA-expressing cancer cells comprise PSMA-expressing cancer cells insensitive to antiandrogen therapy.

According to an aspect of the invention there is provided a composition comprising an antiandrogen and a PSMA ligand-anticancer agent conjugate or PSMA ligand-cytotoxic agent conjugate, for use in an *in vivo* method of inhibiting proliferation of prostate-specific membrane antigen (PSMA)-expressing cancer cells, wherein the antiandrogen is abiraterone, enzalutamide, nilutamide, flutamide, bicalutamide, ARN 509, galeterone or orteronel, and wherein the PSMA ligand of the conjugate comprises a small molecule ligand that binds specifically PSMA and the small molecule ligand comprises a glutamate-urea-amino acid moiety, wherein the glutamate-urea-amino acid moiety is a glutamate-urea-lysine;
wherein the PSMA-expressing cancer cells comprise PSMA-expressing cancer cells insensitive to antiandrogen therapy.

Further described herein are kits that may comprise a container containing a compound that increases cell surface expression of PSMA in a PSMA-expressing cell, and a container containing a PSMA ligand-anticancer agent conjugate or PSMA ligand-cytotoxic agent conjugate. Additionally described herein are kits that comprise a container containing prednisone, and a container containing a PSMA ligand conjugate. As described herein, the kit may further comprise a container that comprises a compound that increases cell surface expression of PSMA.

As described herein, the compound that increases cell surface expression of PSMA may be an antiandrogen.

As described herein, the antiandrogen may block enzyme cytochrome CYP17. As described herein, the antiandrogen may block enzyme cytochrome CYP17A1.

As described herein, the antiandrogen may be an androgen receptor antagonist. As described herein, the antiandrogen may be abiraterone, enzalutamide, nilutamide, flutamide, bicalutamide, ARN 509, galeterone or orteronel. As described herein, the antiandrogen may be enzalutamide or abiraterone.

As described herein, the compound that increases cell surface expression of PSMA may be an mTOR inhibitor. As described herein, the mTOR inhibitor may be rapamycin.

As described herein, the kits further may comprise a pharmaceutically acceptable carrier and/or excipient.

As described herein, the compound and/or the PSMA ligand conjugate may be in an aqueous medium. As described herein, the compound and/or the PSMA ligand conjugate may be lyophilized.

As described herein, the kits may further comprise a diluent.

As described herein, the kits may further comprise instructions for reconstituting the compound and/or the PSMA ligand conjugate. As described herein, the kits may further comprise instructions for reconstituting prednisone and/or the PSMA ligand conjugate and/or the compound (when such kits further comprise the compound).

As described herein, the kits may further comprise instructions for combining the compound and/or the PSMA ligand conjugate. As described herein, the kits may further comprise instructions for combining prednisone and/or the PSMA ligand conjugate. As described herein, the kits may further comprise instructions for combinding the compound with prednisone and/or the PSMA ligand conjugate (when such kits further comprise the compound).

According to an aspect of the invention there is provided a kit comprising:
a container containing antiandrogen, wherein the antiandrogen is abiraterone, enzalutamide, nilutamide, flutamide, bicalutamide, ARN 509, galeterone or orteronel; and
a container containing a PSMA ligand-anticancer agent conjugate or PSMA ligand-cytotoxic agent conjugate, wherein the PSMA ligand of the conjugate comprises a small molecule ligand that binds specifically PSMA and the small molecule ligand comprises a glutamate-urea-amino acid moiety, wherein the glutamate-urea-amino acid moiety is a glutamate-urea-lysine;
wherein the kit is for use in an *in vivo* method of inhibiting proliferation of prostate-specific membrane antigen (PSMA)-expressing cancer cells, wherein the PSMA-expressing cancer cells comprise PSMA-expressing cancer cells insensitive to antiandrogen therapy;
optionally wherein the compound and/or the PSMA ligand conjugate is in aqueous medium or is lyophilized; and
optionally further comprising a pharmaceutically acceptable carrier and/or an excipient and/or a diluent.

Further described herein is a method of inhibiting proliferation of prostate-specific membrane antigen (PSMA)-expressing cancer cells. As described herein, the method may comprise contacting PSMA-expressing cancer cells with prednisone; and contacting the PSMA-expressing cancer cells with a PSMA ligand conjugate. As described herein, the PSMA-expressing cancer cells may be any one of the cells provided herein. As described herein, the PSMA ligand conjugate may be any one of the PSMA ligand conjugates provided herein. Further described herein is a composition comprising prednisone and a PSMA ligand conjugate. Additionally described herein is a kit comprising a container containing prednisone, and a container containing a PSMA ligand conjugate.

In any one of these described methods, compositions or kits, the PSMA ligand of the conjugate may comprise an antibody, or antigen-binding fragment thereof, that binds specifically PSMA. As described herein, the PSMA ligand may be any one of the antibodies or antigen-binding fragments provided herein.

In any one of these described methods, compositions or kits, the PSMA ligand of the conjugate may comprises a small molecule ligand that binds specifically PSMA. As described herein, the PSMA ligand may be any one of the small molecule ligands provided herein.

In some embodiments of the aspects of the invention or in any one of these described methods, compositions or kits, the PSMA ligand conjugate comprises MIP-1095 or MIP-1072 conjugated to a cytotoxic radionuclide selected from the group consisting of I¹²³, I¹²⁵, I¹³¹, I¹²⁴ Br⁷⁵, Br⁷⁷ and F¹⁸.

In some embodiments of the aspects of the invention or in any one of these described methods, compositions or kits, the anticancer or cytotoxic agent of the PSMA ligand conjugate is any one of the anticancer or cytotoxic agents provided herein. In some embodiments, the agent comprises an auristatin, tubulysin, a pyrrolobenzodiazepine dimer, calicheamicin, colchicine, ispinesib, combrestatin A4, maytansinoid DM1, maytansinoid DM4, doxorubicin, or a cytotoxic radionuclide. As described herein, the auristatin may comprise monomethylauristatin norephedrine or monomethylauristatin phenylalanine.

In any one of these described methods, the method may further comprise contacting the PSMA expressing cells with a compound that increases cell surface expression of PSMA. In any one of these described compositions or kits, the compositions or kits may further comprise a compound that increases cell surface expression of PSMA. As described herein, the compound may be an antiandrogen or an mTOR inhibitor. As described herein, the antiandrogen can be any one of the antiandrogens provided herein. As described herein, the mTOR inhibitor may be any one of the mTOR inhibitors provided herein.

In any one of these described methods, compositions or kits, the antiandrogen may be abiraterone, enzalutamide, nilutamide, flutamide, bicalutamide, ARN 509, galeterone or orteronel. As described herein, the antiandrogen may be enzalutamide, abiraterone or ARN 509.

In any one of the described methods, the steps of contacting may be concurrent. As described herein, the steps of contacting may be sequential. As described herein, the step of contacting the PSMA-expressing cancer cells with the prednisone and/or compound may be prior to the step of contacting the PSMA-expressing cancer cells with the PSMA ligand conjugate.

In any one of these described methods, compositions or kits, the PSMA-expressing cancer cells may comprise androgen-independent PSMA-expressing cancer cells. As described herein, the PSMA-expressing cancer cells may comprise PSMA-expressing cancer cells insensitive to antiandrogen therapy.

In any one of these described methods, the PSMA-expressing cancer cells may be of a subject. As described herein, the subject can be any one of the subjects provided herein.

In any one of the foregoing described methods or compositions or kits, the antiandrogen may be enzalutamide, abiraterone or ARN 509, and the PSMA ligand-anticancer agent conjugate may be a PSMA ADC.

In any one of the foregoing described compositions or kits, the compositions or kits may further comprise a pharmaceutically acceptable carrier and/or excipient.

In any one of the foregoing described kits, any one or all of the components of the kits may be in an aqueous medium. In any one of the foregoing described kits, any one or all of the components of the kits may be lyophilized.

In any one of the foregoing described kits, the kits may further comprise instructions for reconstituting any one or all of the components of the kits. In any one of the foregoing described kits, the kits may further compise instructions for combining any one or all of the components of the kits.

In any one of the foregoing described compositions or kits, the compositions or kits may further comprise a diluent.

In any one of the foregoing described methods, kits or compositions, the PSMA ligand of the conjugate may comprise an antibody, or antigen-binding fragment thereof, that binds specifically PSMA. As described herein, the PSMA ligand may be an antibody or antigen-binding fragment thereof that binds the extracellular portion of PSMA. As described herein, the PSMA ligand may be an antibody or antigen-binding fragment thereof that binds a PSMA protein dimer. As described herein, the PSMA ligand may be an antibody or antigen-binding fragment thereof that binds preferentially a PSMA protein dimer not a PSMA protein monomer. As described herein, the antibody or antigen-binding fragment thereof may bind with at least a 2-fold, 3-fold, 4-fol, 5-fold, 10-fold or more greater affinity to a PSMA protein dimer as compared to a PSMA protein monomer. As described herein, the PSMA protein dimer and PSMA protein monomer may be in a non-denatured form, such as a native conformation.

As described herein, the antibody or antigen-binding fragment thereof may be an antibody selected from the group consisting of: PSMA 3.7, PSMA 3.8, PSMA 3.9, PSMA 3.11, PSMA 5.4, PSMA 7.1, PSMA 7.3, PSMA 10.3, PSMA 1.8.3, PSMA A3.1.3, PSMA A3.3.1, Abgenix 4.248.2, Abgenix 4.360.3, Abgenix 4.7.1, Abgenix 4.4.1, Abgenix 4.177.3, Abgenix 4.16.1, Abgenix 4.22.3, Abgenix 4.28.3, Abgenix 4.40.2, Abgenix 4.48.3, Abgenix 4.49.1, Abgenix 4.209.3, Abgenix 4.219.3, Abgenix 4.288.1, Abgenix 4.333.1, Abgenix 4.54.1, Abgenix 4.153.1, Abgenix 4.232.3, Abgenix 4.292.3, Abgenix 4.304.1, Abgenix 4.78.1 and Abgenix 4.152.1, or an antigen-binding fragment thereof.

As described herein, the antibody or antigen-binding fragment thereof may comprise (i) the three complementarity determining regions of a heavy chain variable region comprising an amino acid sequence set forth as SEQ ID NO:15 and (ii) the three complementarity determining regions of a light chain variable region comprising an amino acid sequence set forth as SEQ ID NO: 17.

As described herein, the antibody or antigen-binding fragment thereof may comprise (i) the three complementarity determining regions of a heavy chain variable region comprising an amino acid sequence set forth as SEQ ID NO:19 and (ii) the three complementarity determining regions of a light chain variable region comprising an amino acid sequence set forth as SEQ ID NO: 21.

As described herein, the antibody or antigen-binding fragment thereof may comprise (i) the three complementarity determining regions of a heavy chain variable region comprising an amino acid sequence set forth as SEQ ID NO:23 and (ii) the three complementarity determining regions of a light chain variable region comprising an amino acid sequence set forth as SEQ ID NO: 25.

As described herein, the antibody or antigen-binding fragment thereof may comprise (i) the three complementarity determining regions of a heavy chain variable region comprising an amino acid sequence set forth as SEQ ID NO:27 and (ii) the three complementarity determining regions of a light chain variable region comprising an amino acid sequence set forth as SEQ ID NO: 29.

As described herein, the antibody or antigen-binding fragment thereof may comprise (i) the three complementarity determining regions of a heavy chain variable region comprising an amino acid sequence set forth as SEQ ID NO: 31 and (ii) the three complementarity determining regions of a light chain variable region comprising an amino acid sequence set forth as SEQ ID NO: 33.

As described herein, the antibody or antigen-binding fragment thereof may be antibody PSMA 10.3, AB-PG1-XG1-006, AB-PG1-XG1-026, AB-PG1-XG1-051, AB-PG1-XG1-069, AB-PG1-XG1-077, or an antigen-binding fragment thereof.

As described herein, the antibody or antigen-binding fragment thereof may be antibody E99, J415, J533 or J591 or an antibody produced by a hybridoma having ATCC Accession Number HB-12101, HB-12109, HB-12127or HB-12126, or an antigen -binding fragment thereof.

As described herein, the antibody or antigen-binding fragment thereof may be an antibody produced by a hybidoma having ATCC Accession Number HB12060 (3F5.4G6), HB12309 (3D7-1.1), HB12310 (4E10-1.14), HB12489 (1G3), HB12495 (1G9), HB12490 (2C7), HB12494 (3C4), HB12491 (3C6), HB12484 (3C9), HB12486 (3E6), HB12488 (3E11), HB12485 (3G6), HB12493 (4D4), HB12487 (4D8), HB12492 (4C8B9), HB12664 (3F6), HB12678 (2E4), HB12665 (3C2), HB12672 (2D4), HB12660 (4C8G8), HB12675 (2C4), HB12663 (4C11), HB12661 (1D11), HB12667 (4E8), HB12674 (2G5), HB12620 (4E6), HB12677 (1F4), HB12666 (2E3), HB12662 (3D8), HB12668 (4F8), HB12673 (3D2), HB12676 (1G7), HB12669 (3D4), HB12679 (5G10) or HB12671 (5E9), or an antigen-binding fragment thereof.

According to the invention, the PSMA ligand of the conjugate comprises a small molecule ligand that binds specifically PSMA. In an embodiment, the small molecule ligand binds or inhibits an enzymatic site of PSMA. As described herein, the small molecule ligand may bind or inhibit the glutamate carboxypeptidase II (CCPII) site on PSMA.

In an embodiment, the small molecule ligand may comprise MIP-1095, MIP-1072, or DUPA.

As described herein, the PSMA ligand-anticancer agent conjugate may comprise EC1069 or EC1719.

As described herein, the PSMA ligand-anticancer agent conjugate may comprise BIND-014.

In an embodiment, the PSMA ligand conjugate comprises MIP-1095 or MIP-1072 conjugated to a cytotoxic radionuclide selected from the group consisting of I¹²³, I¹²⁵, I¹³¹, I¹²⁴ Br⁷⁵, Br⁷⁷ and F¹⁸.

In an embodiment, the PSMA ligand conjugate may comprise ¹²³I-MIP-1095 or ¹²³I-MIP-1072.

In an embodiment, the anticancer agent may comprise an auristatin, tubulysin, a pyrrolobenzodiazepine dimer, calicheamicin, colchicine, ispinesib, combrestatin A4, maytansinoid DM1, maytansinoid DM4, doxorubicin, or a cytotoxic radionuclide.

As described herein, the auristatin may comprise monomethylauristatin norephedrine or monomethylauristatin phenylalanine.

In any one of the methods provided, the subject may have received treatment with an antiandrogen and may have experienced no or minimal sensitivity to the antiandrogen therapy. In any one of the methods provided, the subject may have experienced a loss of sensitivity to an antiandrogen therapy. A subject that is sensitive to antiandrogen therapy is one that experiences an appreciable reduction in the subject's cancer, such as a decrease in the number of cancer cells, such as a decrease in the number of circulating cancer cells or tumor size, or symptoms associated with the cancer. Sensitivity to antiandrogen therapy may be measured by, for example, determining the level of proliferation of the subject's cancer cells after treatment and may be compared to the level of proliferation of the cancer cells prior to the treatment. Sensitivity to antiandrogen therapy may be determined during a course of a treatment with an antiandrogen, and when the level of reduction in the subject's cancer no longer appreciably changes or the subject experiences an undesired increase in the progression of the cancer, the subject may be considered to have experienced a loss of sensitivity to the antiandrogen therapy. An increase in a subject's cancer may refer to an increase in the number of cancer cells or tumor size or to an increase in the symptoms associated with the cancer. Sensitivity to antiandrogen therapy may be determined using routine methods by a clinician.

In any one of the methods provided herein the subject may be one that has no or minimal sensitivity to an antiandrogen therapy. Additionally, in any one of the methods provided herein the subject may be one that has experienced a loss of sensitivity to antiandrogen therapy. Any one of the methods and compositions provided herein can be used to sensitize or resensitize such subjects to treatment with an antiandrogen when administered concurrently or sequentially with the PSMA ligand conjugates provided herein.

As described herein, any one of the methods provided herein can include the step of identifying a subject that has no or minimal sensitivity to antiandrogen therapy or that has experienced a loss of sensitivity to antiandrogen therapy. As described herein, any one of the methods provided herein can include the step of assessing the level of sensitivity to antiandrogen therapy in a subject. As described herein, any one of the methods provided herein can include the step of administering an antiandrogen until there is a loss of sensitivity thereto as well as a step of administering an antiandrogen concurrently or sequentially with a PSMA ligand conjugate as provided herein.

As described herein, any one of the methods provided herein can include a step of assessing the progression of the cancer in the subject. Progression can be assessed in a number of ways. For example, progression may be assessed by any one or more of the following: determining the level of PSA, assessing bone metastases and assessing measurable disease. Progression of bone metastases may be the appearance of 2 or more new bone lesions on a bone scan (CT scan, MRI), or new radiographic lesions. Progression may also be determined by RECIST (Eisenhauer EA et al Eur J Cancer 2009:45(2):228-47). Progression of the cancer may be occurring when there is an increase in pain, such as bone pain. In any one of the methods provided herein, assessing progression can comprise assessing any one or more of the foregoing.

In some embodiments of any one of the methods provided herein, the PSMA-expressing cancer cells may be of a subject. In some embodiments, the subject may have progressive metastatic castration-resistant prostate cancer. As described herein, the subject may have had prior chemotherapy with one or more taxanes. In some embodiments, the subject may have had prior treatment with one or more antiandrogens. In some embodiments, the subject may have prostate cancer that has progressed despite prior treatment, such as with one or more antiandrogens. As described herein, the subject may have prostate cancer that is starting to progress despite treatment, such as with one or more antiandrogens. As described herein, the subject may have not had prior cytotoxic chemotherapy. As described herein, the subject may have not had prior treatment with one or more antiandrogens. As described herein, the subject may have not had prior treatment with enzalutamide or abiraterone. As described herein, the subject that has not had prior treatment with antiandrogens, such as enzalutamide or abiraterone, may be one with metastatic castration-resistant prostate cancer. The subject may be any one of the subjects described herein.

### BRIEF DESCRIPTION OF THE DRAWINGS

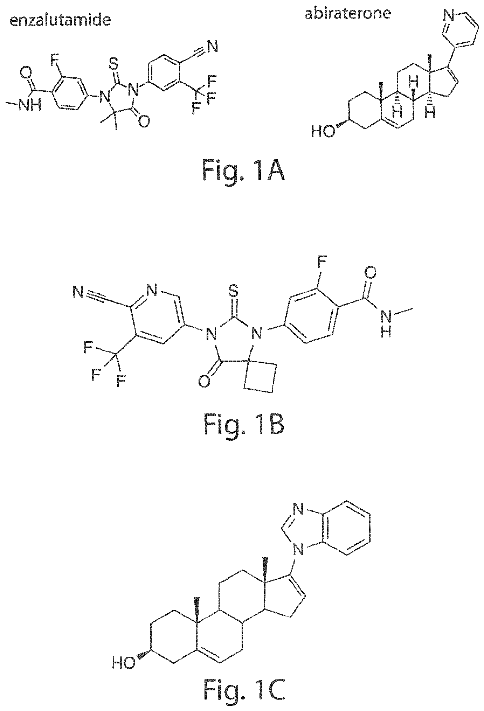

**FIG. 1A** presents a schematic showing the structure of two examples of antiandrogens that increase the expression of PSMA, enzalutamide and abiraterone; **FIG. 1B** presents a schematic showing the chemical structure of ARN-509 (MW=477.43); **FIG. 1C** presents a schematic showing the chemical structure of galeterone (TOK-001) (MW=388.55); **FIG. ID** presents a schematic showing the structure of a PSMA ligand conjugate that includes a PSMA antibody conjugated to monomethyl auristatin E (MMAE) (PSMA antibody-drug conjugate (PSMA ADC)), an average of four molecules of MMAE in this embodiment.

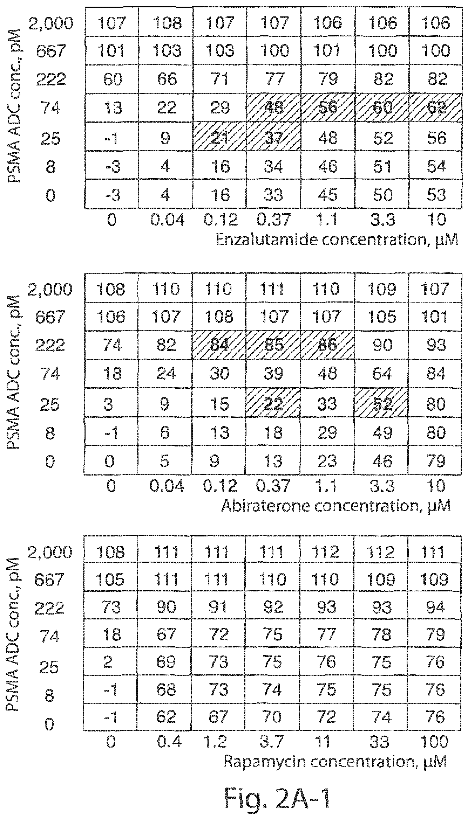

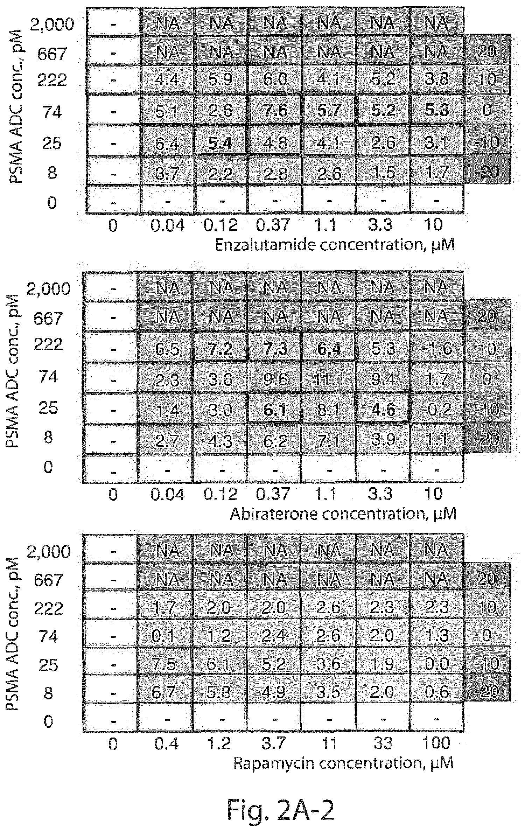

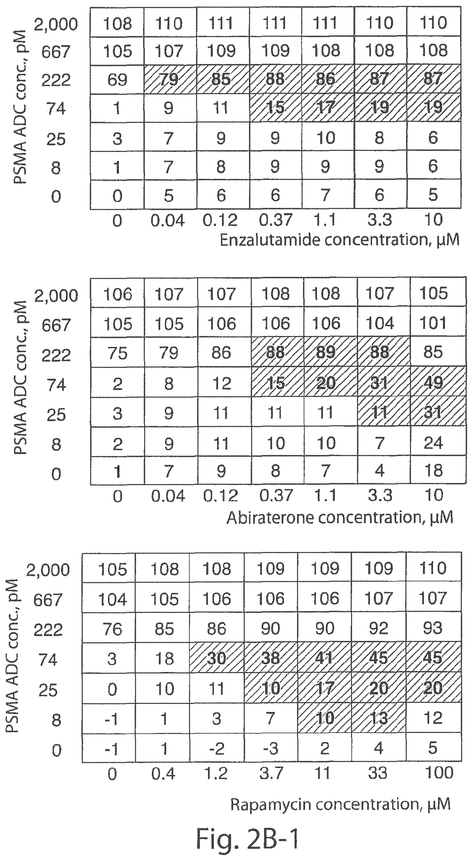

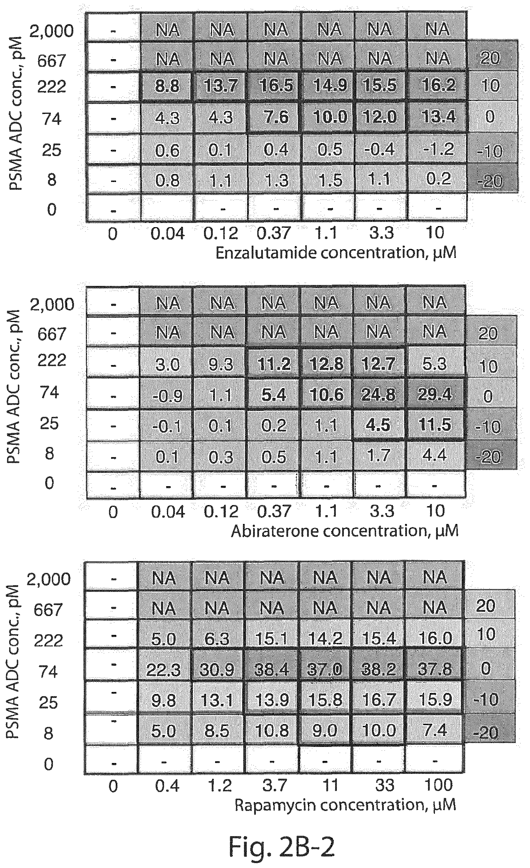

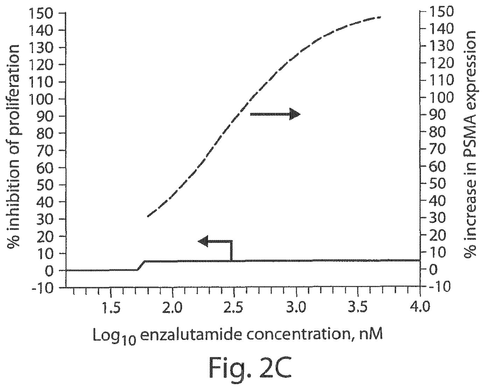

**FIG. 2** shows percent cell proliferation inhibition values and Bliss differences for LNCaP cells (A) and C4-2 cells (B) contacted with combinations of PSMA ligand conjugate and enzalutamide, abiraterone or rapamycin. Each data point represents the mean of three to seven independent assays. Bliss differences are depicted via heat maps of values that are positive (medium gray), negative (dark gray), or near zero (light gray). Bliss parameters that are significantly different from zero (P<0.05) are highlighted via bold text and dark gray borders, and the corresponding percent inhibition values are highlighted with shading and dark gray text. NA = not applicable. (C) Graph showing increase in PSMA expression and inhibition of cell proliferation as a function of increasing concentration of enzalutamide.

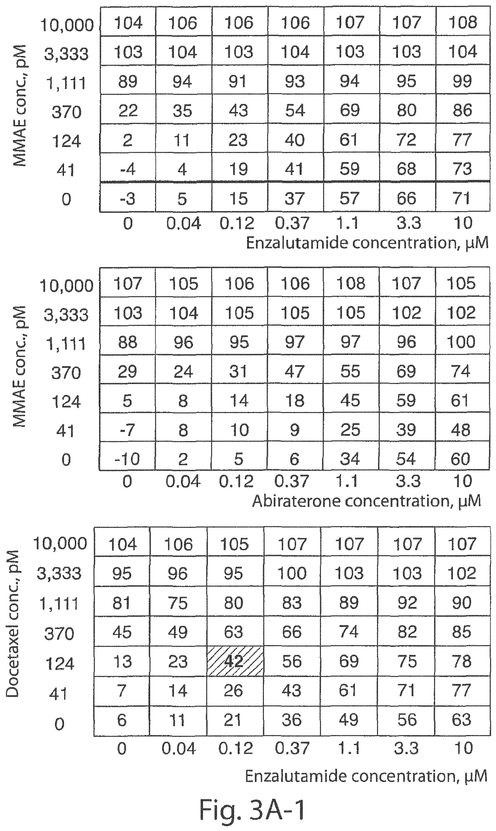

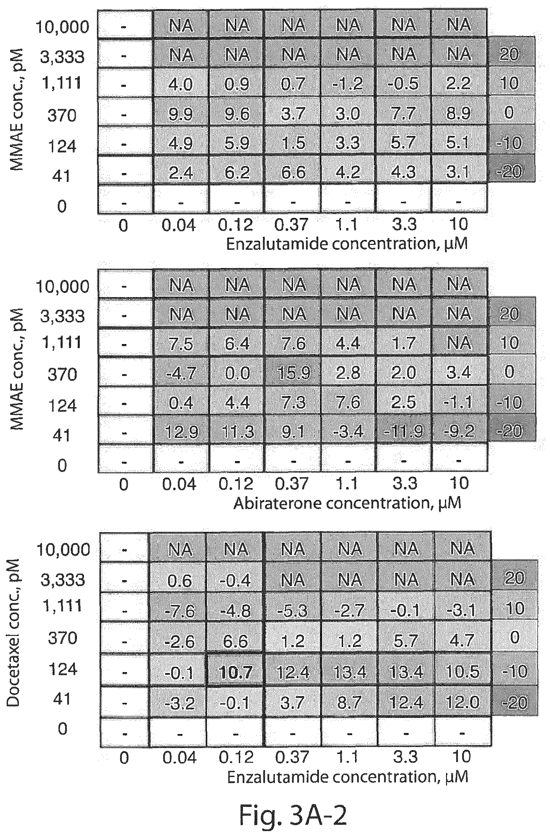

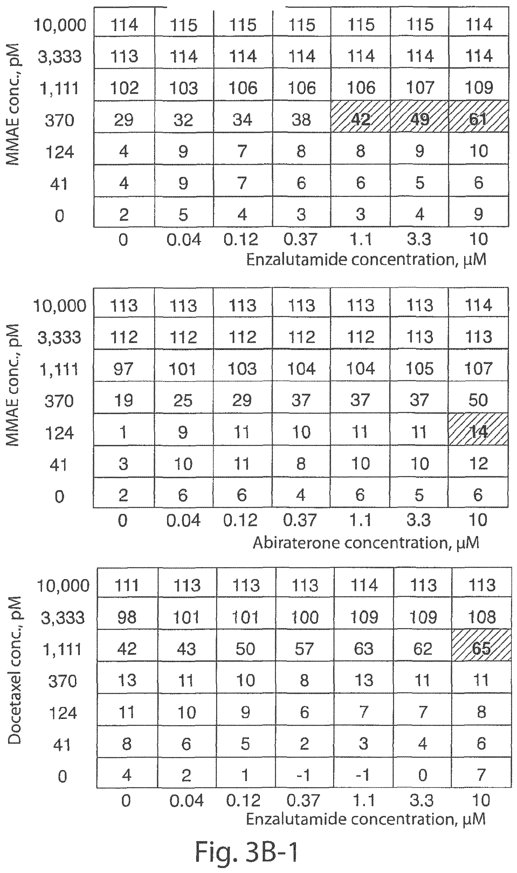

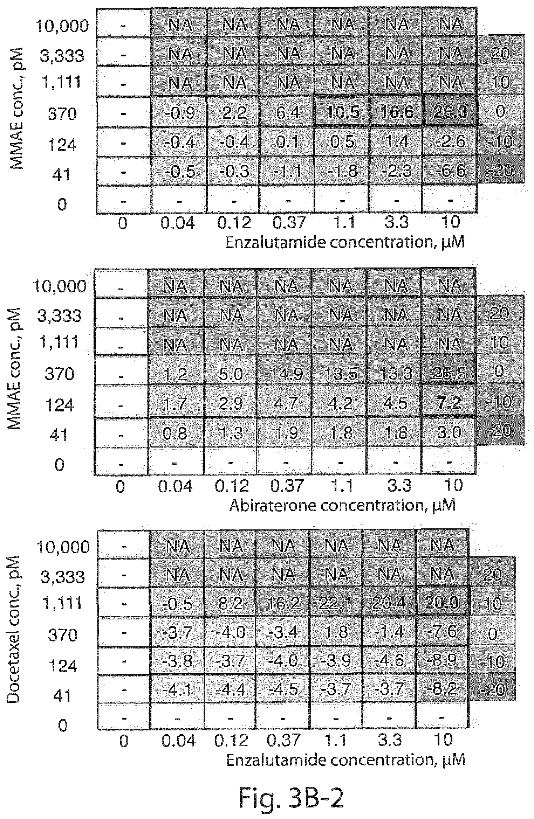

**FIG. 3** shows percent cell proliferation inhibition values and Bliss differences for LNCaP cells (A) and C4-2 cells (B) contacted with combinations of microtubule inhibitors and antiandrogens. Each data point represents the mean of three to five independent assays. Bliss differences are depicted via heat maps of values that are positive (medium gray), negative (dark gray), or near zero (light gray). Bliss parameters that are significantly different from zero (P<0.05) are highlighted via bold text and dark gray borders, and the corresponding percent inhibition values are highlighted with shading and dark gray text. NA = not applicable.

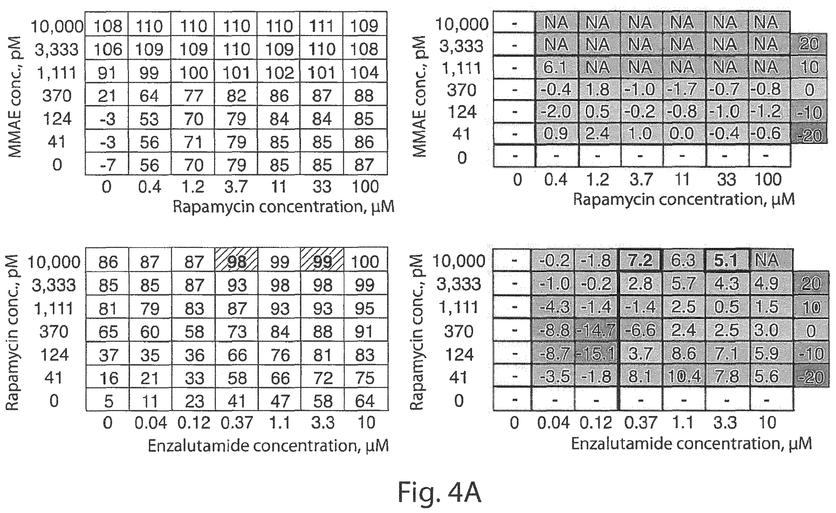

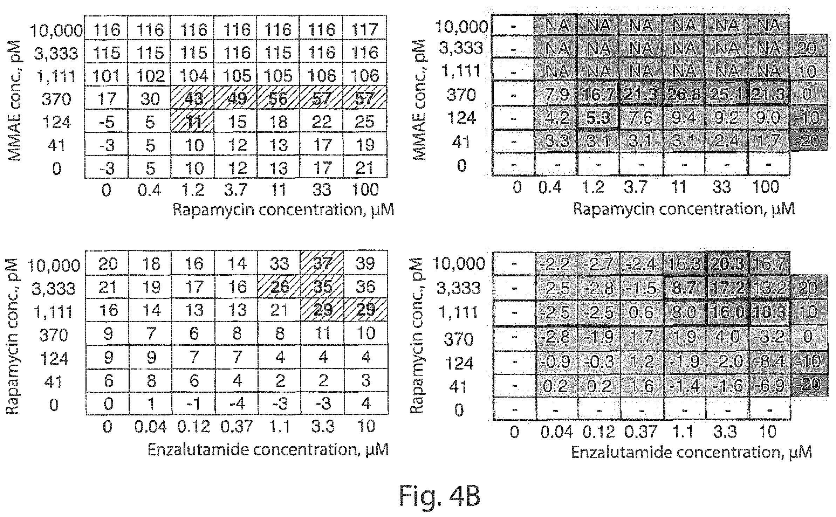

**FIG. 4** shows percent cell proliferation inhibition values and Bliss differences for LNCaP cells (A) and C4-2 cells (B) contacted with combinations of rapamycin with MMAE or enzalutamide. Each data point represents the mean of three independent assays. Bliss differences are depicted via heat maps of values that are positive (medium gray), negative (dark gray), or near zero (light gray). Bliss parameters that are significantly different from zero (P<0.05) are highlighted via bold text and dark gray borders, and the corresponding percent inhibition values are highlighted with shading and dark gray text. NA = not applicable.

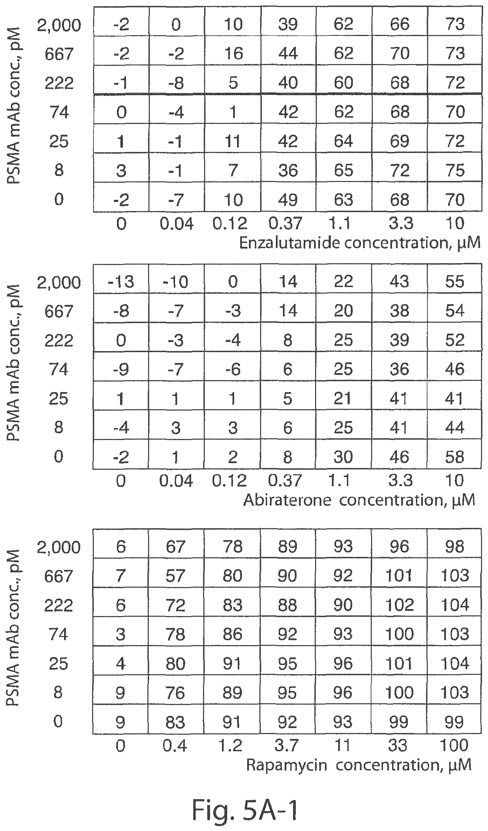

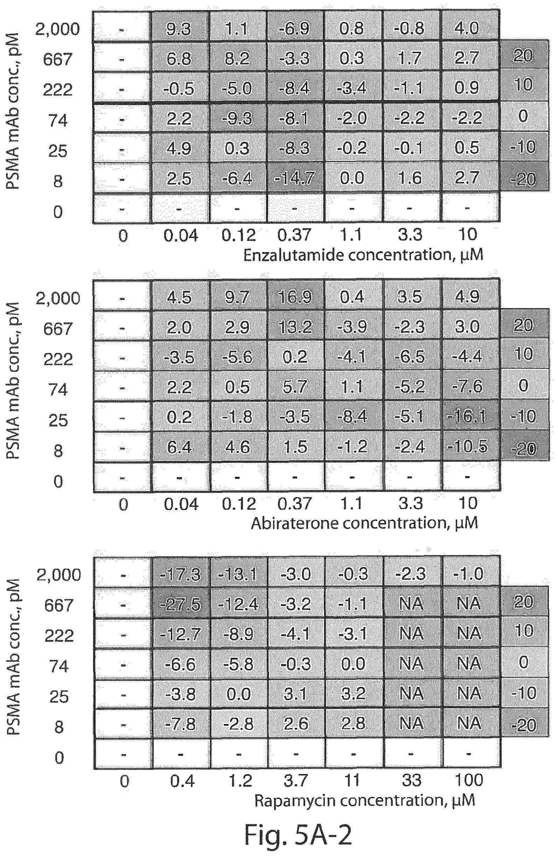

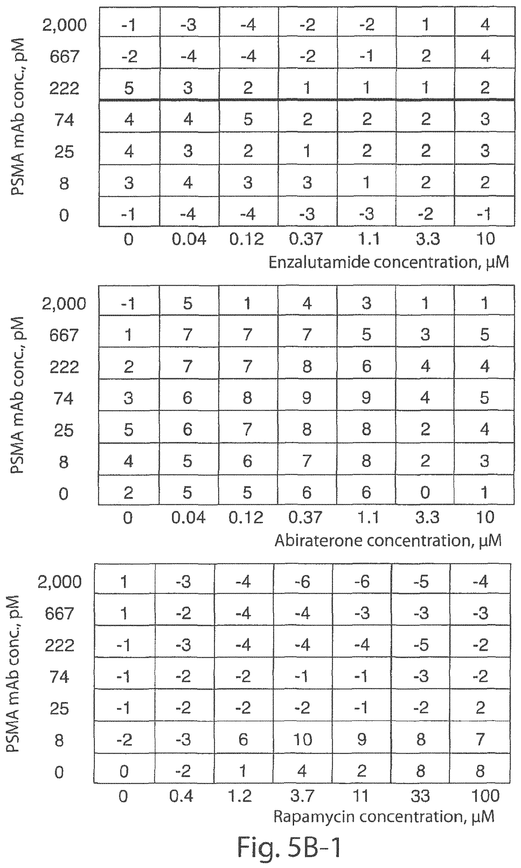

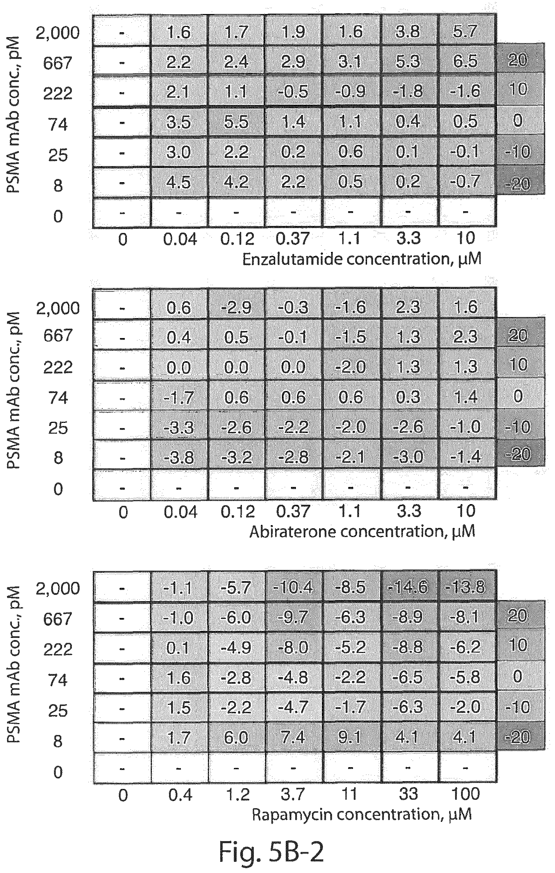

**FIG. 5** shows percent cell proliferation inhibition values and Bliss differences for LNCaP cells (A) and C4-2 cells (B) contacted with combinations of PSMA monoclonal antibody and enzalutamide, abiraterone or rapamycin. Each data point represents the mean of two or three independent assays. Bliss differences are depicted via heat maps of values that are positive (medium gray), negative (dark gray), or near zero (light gray). No Bliss parameters were significantly different from zero (P<0.05). NA = not applicable.

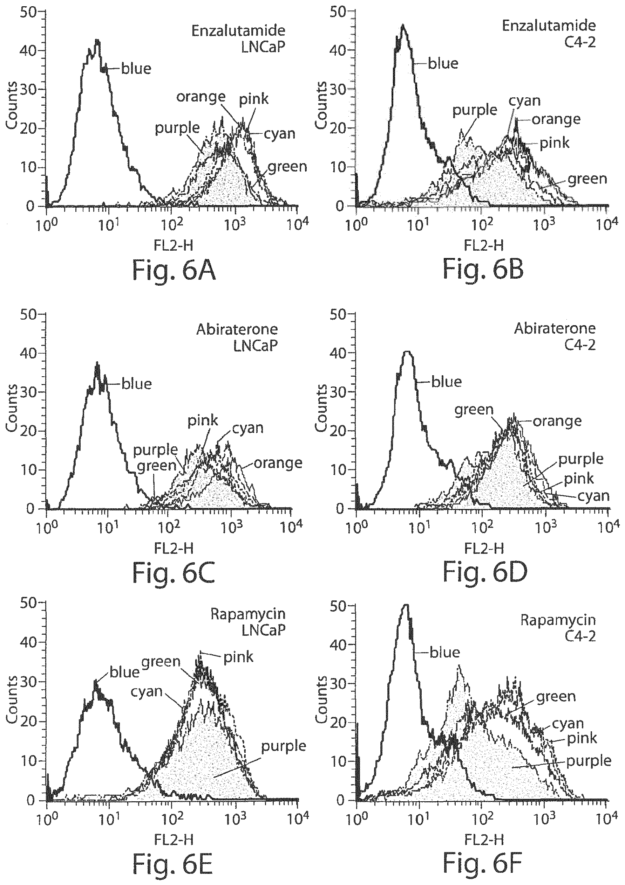

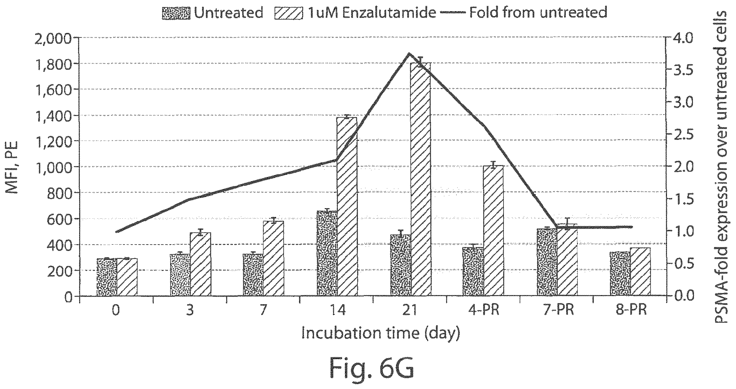

**FIG. 6** shows a flow cytometry analysis of PSMA expression. PSMA expression was measured by flow cytometry on cells treated for 7 days with varying concentrations of enzalutamide, abiraterone or rapamycin (A-F) or treated with 1 µM enzalutamide for varying times (G). In A-D, anti-PSMA staining is shown as a function of antiandrogen concentrations as follows: green (0.125 µM), pink (0.5 µM), cyan (1 µM) and orange (5 µM). In E-F, the green, pink and cyan histograms represent rapamycin concentrations of 1, 10 and 100 nM, respectively. Filled purple histograms denote untreated cells, and the blue line depicts background staining with isotype-control antibody. (G) Time-course of PSMA expression in enzalutamide-treated C4-2 cells. Vertical bars depict mean fluorescence intensity (MFI) values on the left axis, and the red line represents the fold increase in PSMA expression in treated cells relative to untreated cells on the right axis. (PR = days post enzalutamide removal.)

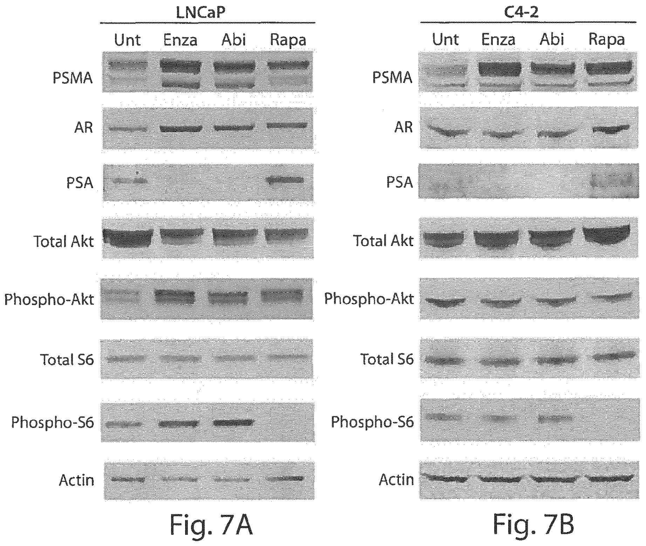

**FIG. 7** shows a Western blot protein expression analysis of whole-cell extracts of LNCaP (A) or C4-2 (B) cells left untreated (Unt) or treated for 7 days with enzalutamide (Enza), abiraterone (Abi) or rapamycin (Rapa) prior to the analysis.

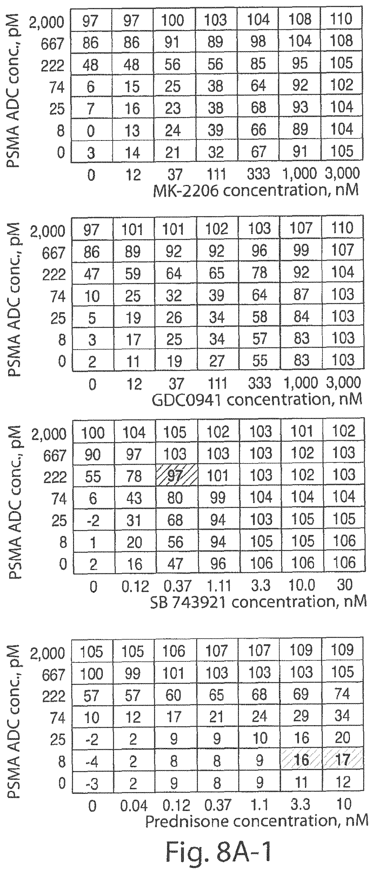

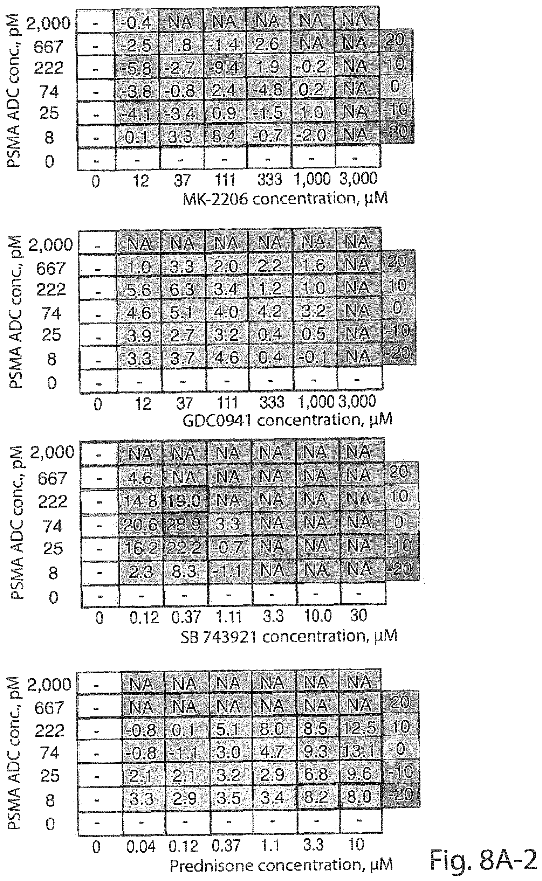

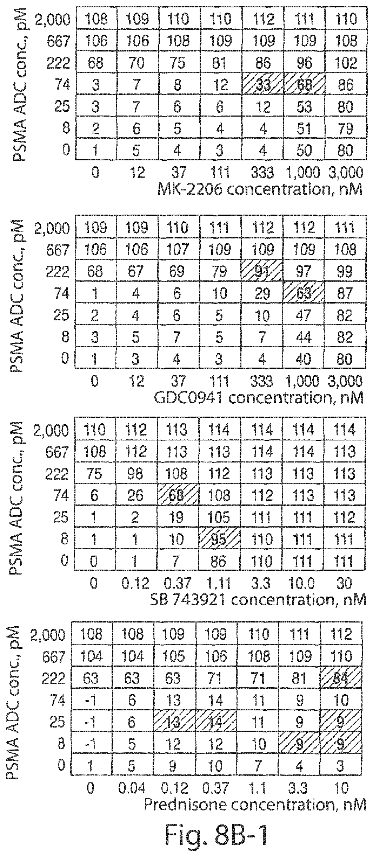

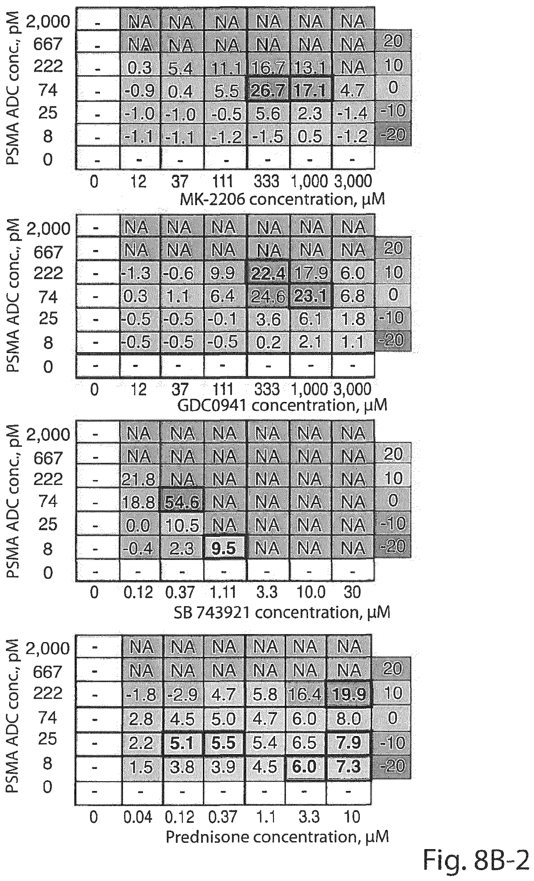

**FIG. 8** shows percent cell proliferation inhibition values and Bliss differences for LNCaP cells (A) and C4-2 cells (B) contacted with combinations of PSMA ligand conjugate and prednisone or inhibitors of Akt, PI3K or kinesin spindle protein (inhibitors MK-2206, GDC-0941 and SB743921, respectively). Each data point represents the mean of four or five independent assays. Bliss differences are depicted via heat maps of values that are positive (medium gray), negative (dark gray), or near zero (light gray). Bliss parameters that are significantly different from zero (P<0.05) are highlighted via bold text and dark gray borders, and the corresponding percent inhibition values are highlighted with shading and dark gray text. NA = not applicable.

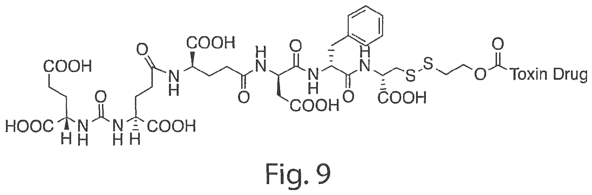

**FIG. 9** presents a schematic showing a chemical structure with a D-γ-Glu D-Asp-D-Phe-D-Cys linker.

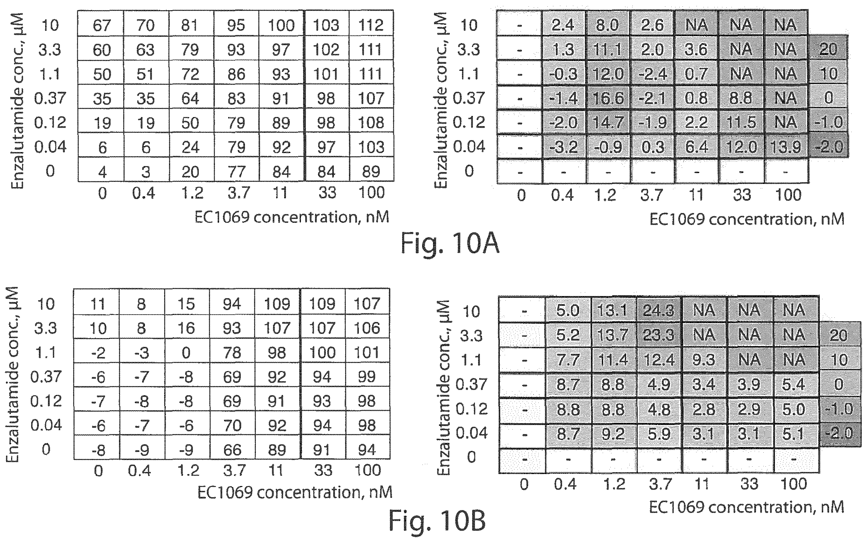

**FIG. 10** shows percent cell proliferation inhibition values and Bliss differences for LNCaP cells (A) and C4-2 cells (B) contacted with combinations of PSMAsmall molecule ligand conjugate, EC1069 and enzalutamide. Each data point represents one independent assay. Bliss differences are depicted via heat maps of values that are positive (medium gray), negative (dark gray), or near zero (light gray). NA = not applicable.

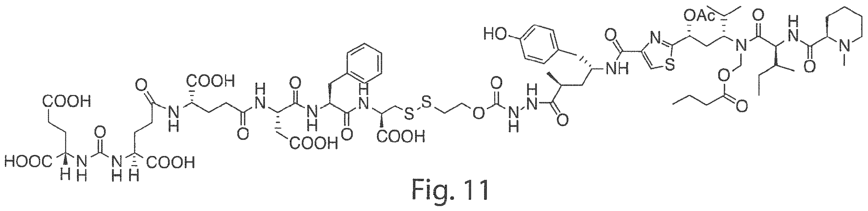

**FIG. 11** presents a schematic showing the structure of PSMA small molecule ligand conjugate that comprises an anticancer agent, tubulysin (also referred to herein as EC1069).

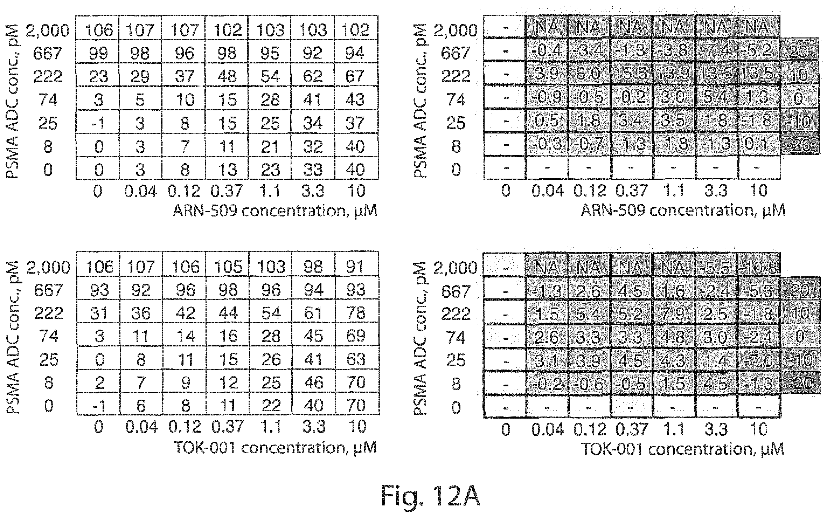

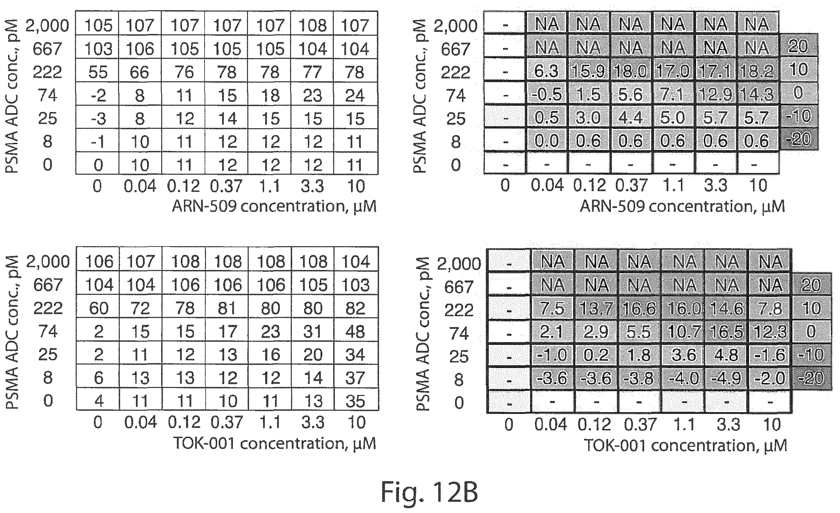

**FIG. 12** presents data from combinations of PSMA ADC with ARN-509 (Aragon Pharmaceuticals, androgen receptor inhibitor) and TOK-001 (Tokai Pharmaceuticals , CYP17 inhibitor and androgen receptor antagonist). Percent inhibition values and Bliss differences are shown for LNCaP cells (A) and C4-2 cells (B). Bliss differences are depicted via heat maps of values that are positive (medium gray), negative (dark gray), or near zero (light gray). NA = not applicable. ARN-509 and TOK-001 were purchased from Selleck Chemicals.

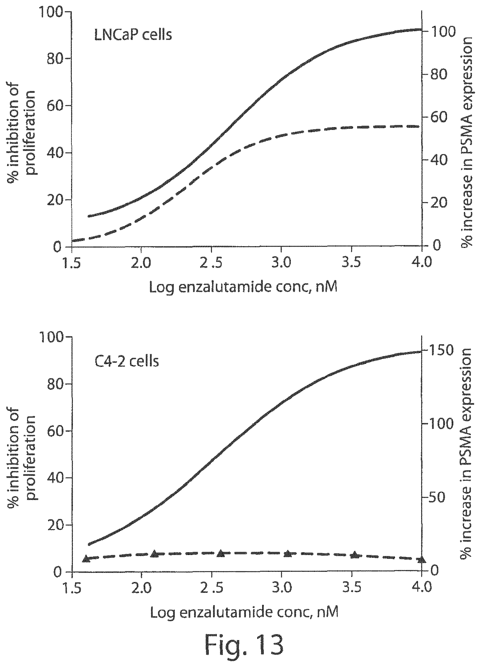

**FIG. 13** demonstrates the effects of enzalutamide on proliferation and PSMA expression. These data show that the effects on proliferation and PSMA expression occur at similar concentrations in LNCaP cells (but are uncoupled in C4-2 cells).

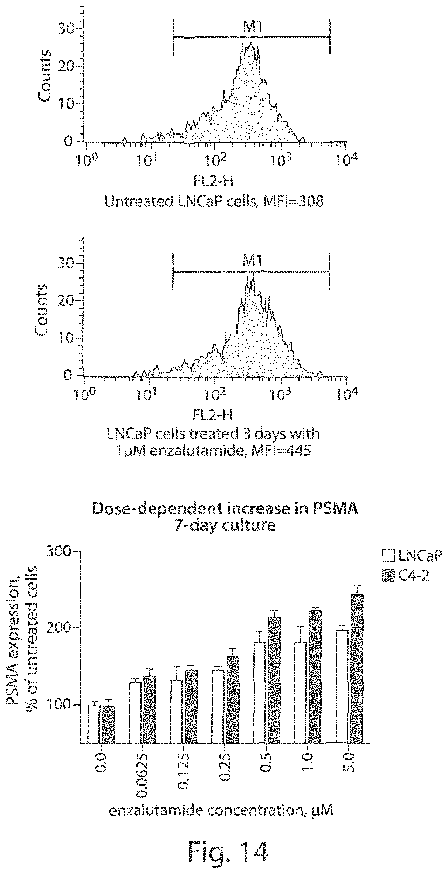

**FIG. 14** demonstrates the rapid, significant and dose-dependent increases in PSMA expression with enzalutamide. The effects were exhibited in a clinically meaningful dose range and were seen for androgen-dependent (LNCaP) and androgen-independent (C4-2) cells.

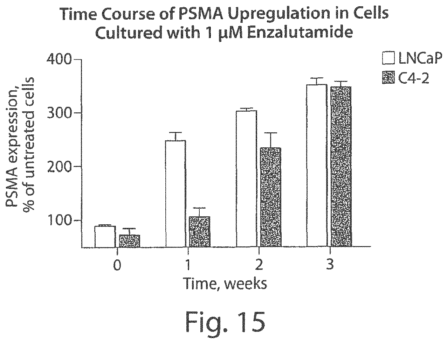

**FIG. 15** also demonstrates the increased expression of PSMA with enzalutamide. PSMA expression was maximal after 3 to 4 weeks of exposure but returned to basal levels one week after removal of enzalutamide.

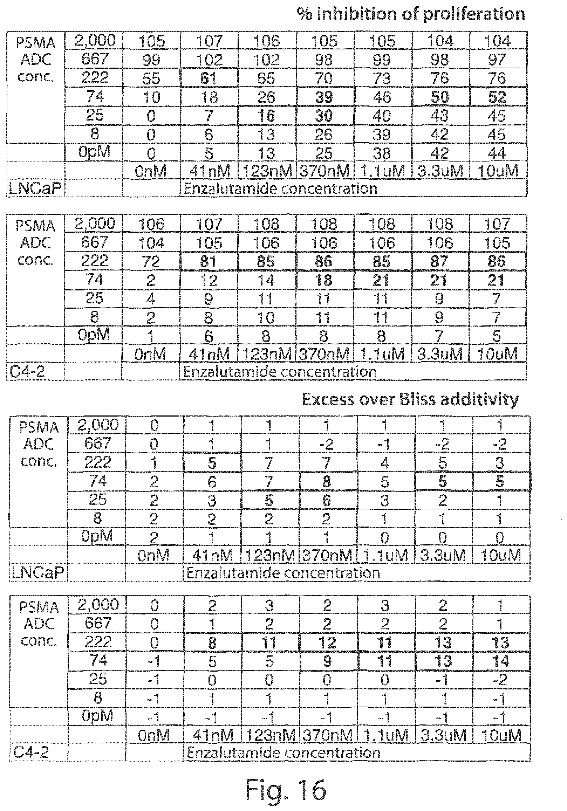

**FIG. 16** shows the synergistic anticancer activity of enzalutamide and PSMA ADC *in vitro.* Results are the average of four repetitions for each condition. Values in bold indicate statistically significant synergy.

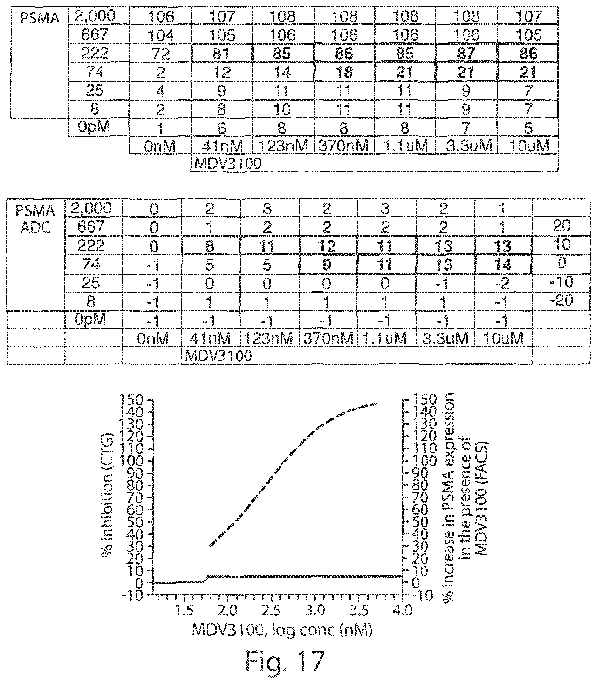

**FIG. 17** shows that PSMA ADC synergizes with enzalutamide *in vitro.* The synergy was shown in both androgen-dependent and androgen-independent cells and was associated with the increased expression of PSMA. Surprisingly, the synergy was observed even when enzalutamide alone had minimal antitumor activity.

### DETAILED DESCRIPTION OF THE INVENTION

The invention relates, at least in part, to combination treatments for PSMA-expressing cancer, such as prostate cancer and, in particular, progressive, metastatic prostate cancer. As described herein, the treatments can include administration of at least one agent that targets prostate specific membrane antigen (PSMA) in combination with an agent that targets androgen receptors (ARs). The invention is based, at least in part, on the surprising discovery that antiandrogens (*e.g.*, enzalutamide or abiraterone) significantly and reversibly augment PSMA expression and potentiate the activity of PSMA ligand conjugates. In androgen-independent cells, the effects on PSMA expression and conjugate activity were synergistic and uncoupled from any anti-proliferative effect of the antiandrogens. Synergistic inhibition of tumor cell growth was associated with an upregulation of PSMA expression by antiandrogens, even in cells that were refractory to treatment with antiandrogens alone. Co-treatment, in effect, can re-sensitize the cells to antiandrogen therapy.

Both enzalutamide and abiraterone potently synergized with PSMA ligand conjugates over a range of concentrations. Synergy was observed on cells that were both responsive and unresponsive to treatment with antiandrogens alone. In androgen-dependent LNCaP cells, the pharmacologic effects of antiandrogens (*e.g*., antiproliferative effects, effects on gene expression, and synergy with PSMA ligand conjugates) were observed over a similar range of clinically relevant concentrations. In androgen-independent C4-2 cells, effects on gene expression and synergy were uncoupled from any appreciable antiproliferative activity of either enzalutamide or abiraterone. Thus, these antiandrogens remain pharmacologically active for C4-2 cells; however, the cells have adopted compensatory survival mechanisms that allow them to proliferate in the presence of ongoing androgen receptor (AR) blockade.

Little to no synergy was observed between the antiandrogens and the components of the conjugates (*i.e*., free MMAE and unmodified PSMA mAb). Free MMAE showed additive to weakly synergistic effects when combined with the antiandrogens. There was modest synergy between the antiandrogens and docetaxel. The unmodified mAb showed no antiproliferative activity either alone or in combination with antiandrogens. Therefore, the strong synergy observed with PSMA ligand conjugates is specific to the conjugates and is neither a pass-through effect of its components nor generalizable to microtubule inhibitors as a class.

PSMA expression was doubled after 7 days treatment with enzalutamide, and 4-fold higher after 21 days. The magnitude of PSMA upregulation by enzalutamide or abiraterone approached that induced by charcoal-stripped serum, which is depleted in a range of hormones, cytokines, and growth factors (40). The findings suggest near-maximal androgen suppression in our system. The time course of expression was monitored in cells treated with enzalutamide. PSMA expression increased with continued treatment over three weeks and then rapidly returned to baseline upon removal of enzalutamide. The findings have implications for combining and sequencing potent antiandrogens and PSMA-targeted therapies in the clinic.

In addition, PSMA ligand conjugates synergized with a PI3K/mTOR pathway inhibitor via a multimodal mechanism involving increased PSMA expression and disruption of microtubule function. In C4-2 cells, rapamycin exhibited minimal single-agent activity but potentiated the activity of PSMA ligand conjugates, suggesting that PI3K pathway activation is an adaptive response to ADC treatment in C4-2 cells. Thus, there is also the surprising discovery that, in some instances mTOR inhibitor, such as rapamycin, activity synergized with the activity of PSMA ligand conjugates.

As used herein, a "PSMA ligand conjugate" comprises a molecule that binds specifically PSMA, such as an extracellular domain of PSMA, and is conjugated to a therapeutic agent. The therapeutic agent may be an anticancer agent or a cytotoxic agent. A "PSMA ligand," therefore, herein refers to a molecule that specifically binds PSMA, as described herein. When a PSMA ligand is conjugated to an anticancer agent, the PSMA ligand conjugate is also referred to herein as a "PSMA ligand-anticancer agent conjugate". When a PSMA ligand is conjugated to a cytotoxic agent, the PSMA ligand conjugate is also referred to herein as a "PSMA ligand-cytotoxic agent conjugate."

As used herein, "PSMA-expressing cells" refers to cells that express PSMA or that can express PSMA (*e.g.*, human PSMA). PSMA is a 100 kD Type II membrane glycoprotein expressed in prostate tissues (Horoszewicz et al., 1987, Anticancer Res. 7:927-935; U.S. Pat. No. 5,162,504). PSMA was characterized as a type II transmembrane protein having sequence homology with the transferrin receptor (Israeli et al., 1994, Cancer Res. 54:1807-1811) and with NAALADase activity (Carter et al., 1996, Proc. Natl. Acad. Sci. U.S.A. 93:749-753). PSMA is expressed in increased amounts in prostate cancer (Horoszewicz et al., 1987, Anticancer Res. 7:927-935; Rochon et al., 1994, Prostate 25:219-223; Murphy et al., 1995, Prostate 26:164-168; and Murphy et al., 1995, Anticancer Res. 15:1473-1479). PSMA expression in cancerous prostate is approximately 10-fold greater than that in normal prostate. Expression in normal prostate is approximately 10-fold greater than that in the brain and is 50-to 100-fold greater than that of the liver or kidney. In most normal tissues, no expression of PSMA is observed.

PSMA expression increases with disease progression, becoming highest in metastatic, hormone-refractory disease. In addition, PSMA is also abundantly expressed on the neovasculature of a variety of non-prostate tumors, including bladder, breast, colon, pancreas, sarcoma, melanoma, renal, liver, lung (*e.g*., non-small cell lung carcinoma), and kidney tumors, but not on normal vasculature. "PSMA-expressing cells," therefore, include PSMA-expressing cancer cells such as prostate cancer cells as well as PSMA-expressing cells (*e.g.,* endothelial cells) of the neovasculature of a number of non-prostate cancers or non-prostate tumors.

As used herein, an "androgen-dependent PSMA-expressing cell" refers to a PSMA-expressing cell, such as a cancer cell, that is responsive to antiandrogens. A cell is herein considered to be responsive to an antiandrogen if proliferation of the cell can be substantially inhibited by the antiandrogen.

As used herein, an "androgen-independent PSMA-expressing cell" refers to a PSMA-expressing cell, such as a cancer cell, that is non-responsive to antiandrogens, also referred to herein as a "PSMA-expressing cell insensitive to antiandrogen therapy." A cell is herein considered to be non-responsive to an antiandrogen if proliferation of the cell is not substantially inhibited by the antiandrogen.

PSMA-expressing cells insensitive to antiandrogen therapy may be sensitized to antiandrogen therapy after the step of contacting the PSMA-expressing cells with a compound that increases cell surface expression of PSMA (*e.g.*, antiandrogen or mTOR inhibitor). Such cells can then be further contacted with a PSMA ligand conjugate as provided herein, and such contact can occur concurrently or sequentially. Thus, androgen-independent PSMA-expressing cells (*e.g.,* cancer cells) that are otherwise non-responsive to antiandrogens may become responsive, and, therefore, sensitized to antiandrogens when contacted with an antiandrogen, or an mTOR inhibitor, and can be further contacted with a PSMA ligand conjugate to effect cell proliferation inhibition or cell killing. Proliferation of such "sensitized" PSMA-expressing cancer cells may be substantially inhibited as a result. Sensitization of androgen-independent PSMA-expressing cells to antiandrogens may occur within 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days or more of being contacted with a compound that increases cell surface expression of PSMA. Sensitization of androgen-independent PSMA-expressing cells to antiandrogens may occur in less than 2 days.

PSMA-expressing cells may be contacted with a compound that increases cell surface expression of PSMA for a period of time sufficient to increase (*e.g*., upregulate) expression of PSMA on the surface of the cells. A time sufficient to upregulate cell surface expression of PSMA can be determined by myriad protein expression assays, which are known in the art, including for example enzyme-linked immunosorbent assays (ELISAs) and Western blotting. A time sufficient to upregulate cell surface expression of PSMA may be 1 day, 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days or more. A time sufficient to upregulate cell surface expression of PSMA may be less than 1 day.

Examples of PSMA ligands for use as provided herein include, without limitation, antibodies or antigen binding fragments thereof as well as small molecule ligands that bind specifically PSMA and may act as substrate mimicks of enzymatic sites on PSMA. Antibodies that bind specifically to PSMA may be referred to herein as "PSMA antibodies." Likewise, small molecule ligands that bind specifically PSMA may be referred to herein as "PSMA small molecule ligands."

As used herein, "specific binding" refers to molecule (*e.g*., antibody) binding to a predetermined target (*e.g.,* antigen), in this case PSMA (*e.g.,* human PSMA). The sequence of PSMA may be as set forth as SEQ ID NO: 1. Typically, the molecule binds with an affinity that is at least two-fold greater than its affinity for binding to a non-specific target (*e.g*., BSA, casein), which is a target other than PSMA, an isoform or variant of PSMA, or a closely-related target.

An antibody or an antigen-binding fragment thereof of a PSMA ligand conjugate may be any antibody or antigen-binding fragment thereof that binds PSMA (*e.g*., binds specifically to an epitope of PSMA). Examples of PSMA antibodies for use as provided herein include, without limitation, those listed in **Table 1.** Antigen-binding fragments of these antibodies are also examples of antigen-binding fragments for use in the methods and compositions as provided herein.

The antibody may be produced by hybridomas referred to herein. These hybridomas were deposited pursuant to, and in satisfaction of, the requirements of the Budapest Treaty on the International Recognition of the Deposit of Microorganisms for the Purposes of Patent Procedure with the American Type Culture Collection ("ATCC"), having the address 10801 University Boulevard, Manassas, Va. 20110-2209, as an International Depository Authority and given the Patent Deposit Designations (**Table 1**):

**Table 1**

| Antibody | Hybridoma/Plasmid | Patent Deposit Designation | Date of Deposit |
|---|---|---|---|
| PSMA 3.7 | PSMA 3.7 | PTA-3257 | April 5, 2001 |
| PSMA 3.9 | PSMA 3.9 | PTA-3258 | April 5, 2001 |
| PSMA 3.11 | PSMA 3.11 | PTA-3269 | April 10, 2001 |
| PSMA 5.4 | PSMA 5.4 | PTA-3268 | April 10, 2001 |
| PSMA 7.1 | PSMA 7.1 | PTA-3292 | April 18, 2001 |
| PSMA 7.3 | PSMA 7.3 | PTA-3293 | April 18, 2001 |
| PSMA 10.3 | PSMA 10.3 | PTA-3347 | May 1, 2001 |
| | PSMA 10.3 HC in pcDNA (SEQ ID NO: 7) | PTA-4413 | May 29, 2002 |
| | PSMA 10.3 Kappa in pcDNA (SEQ ID NO: 13) | PTA-4414 | May 29, 2002 |
| PSMA 1.8.3 | PSMA 1.8.3 | PTA-3906 | Dec. 5, 2001 |
| PSMA A3.1.3 | PSMA A3.1.3 | PTA-3904 | Dec. 5, 2001 |
| PSMA A3.3.1 | PSMA A3.3.1 | PTA-3905 | Dec. 5, 2001 |
| Abgenix 4.248.2 | Abgenix 4.248.2 | PTA-4427 | June 4, 2002 |
| Abgenix 4.360.3 | Abgenix 4.360.3 | PTA-4428 | June 4, 2002 |
| Abgenix 4.7.1 | Abgenix 4.7.1 | PTA-4429 | June 4, 2002 |
| Abgenix 4.4.1 | Abgenix 4.4.1 | PTA-4556 | July 18, 2002 |
| Abgenix 4.177.3 | Abgenix 4.177.3 | PTA-4557 | July 18, 2002 |
| Abgenix 4.16.1 | Abgenix 4.16.1 | PTA-4357 | May 16, 2002 |
| Abgenix 4.22.3 | Abgenix 4.22.3 | PTA-4358 | May 16, 2002 |
| Abgenix 4.28.3 | Abgenix 4.28.3 | PTA-4359 | May 16, 2002 |
| Abgenix 4.40.2 | Abgenix 4.40.2 | PTA-4360 | May 16, 2002 |
| Abgenix 4.48.3 | Abgenix 4.48.3 | PTA-4361 | May 16, 2002 |
| Abgenix 4.49.1 | Abgenix 4.49.1 | PTA-4362 | May 16, 2002 |
| Abgenix 4.209.3 | Abgenix 4.209.3 | PTA-4365 | May 16, 2002 |
| Abgenix 4.219.3 | Abgenix 4.219.3 | PTA-4366 | May 16, 2002 |
| Abgenix 4.288.1 | Abgenix 4.288.1 | PTA-4367 | May 16, 2002 |
| Abgenix 4.333.1 | Abgenix 4.333.1 | PTA-4368 | May 16, 2002 |
| Abgenix 4.54.1 | Abgenix 4.54.1 | PTA-4363 | May 16, 2002 |
| Abgenix 4.153.1 | Abgenix 4.153.1 | PTA-4388 | May 23, 2002 |
| Abgenix 4.232.3 | Abgenix 4.232.3 | PTA-4389 | May 23, 2002 |
| Abgenix 4.292.3 | Abgenix 4.292.3 | PTA-4390 | May 23, 2002 |
| Abgenix 4.304.1 | Abgenix 4.304.1 | PTA-4391 | May 23, 2002 |
| AB-PG1-XG1-006 | AB-PG1-XG1-006 Heavy Chain (SEQ ID NO: 2) | PTA-4403 | May 29, 2002 |
| | AB-PG1-XG1-006 Light Chain (SEQ ID NO: 8) | PTA-4404 | |
| AB-PG1-XG1-026 | AB-PG1-XG1-026 Heavy Chain (SEQ ID NO: 3) | PTA-4405 | May 29, 2002 |
| | AB-PG1-XG1-026 Light Chain (SEQ ID NO: 9) | PTA-4406 | |
| AB-PG1-XG1-051 | AB-PG1-XG1-051 Heavy Chain (SEQ ID NO: 4) | PTA-4407 | May 29, 2002 |
| | AB-PG1-XG1-051 Light Chain (SEQ ID NO: 10) | PTA-4408 | |
| AB-PG1-XG1-069 | AB-PG1-XG1-069 Heavy Chain (SEQ ID NO: 5) | PTA-4409 | May 29, 2002 |
| | AB-PG1-XG1-069 Light Chain (SEQ ID NO: 11) | PTA-4410 | |
| AB-PG1-XG1-077 | AB-PG1-XG1-077 Heavy Chain (SEQ ID NO: 6) | PTA-4411 | May 29, 2002 |
| | AB-PG1-XG1-077 Light Chain (SEQ ID NO: 12) | PTA-4412 | |

An antibody or an antigen-binding fragment thereof of a PSMA ligand conjugate may comprise: (i) any one of the heavy chains provided herein and (ii) any one of the light chains provided herein. A PSMA antibody as provided herein may include (i) any one of the heavy chain variable regions provided herein and (ii) any one of the light chain variable regions provided herein. PSMA antibodies may include (i) the three complementarity determining regions of any one of the heavy chain variable regions provided herein and (ii) the three complementarity determining regions of any one of the light chain variable regions provided herein.

Plasmids encoding exemplary heavy and light chains of antibodies were also deposited with the ATCC and are shown in **Table 1** above.

Also provided are antigen-binding fragments of any one of the foregoing antibodies for use as the PSMA ligands of the PSMA ligand conjugates provided herein.

As used herein, the names of the deposited hybridomas or plasmids may be used interchangeably with the names of the antibodies. It would be clear to one of skill in the art when the name is intended to refer to the antibody or when it refers to the plasmids or hybridomas that encode or produce the antibodies, respectively. Additionally, antibody names may be an abbreviated form of the name shown in **Table 1.** For instance, antibody AB-PG1-XG1-006 may be referred to as "AB-PG1-XG1-006," "PG1-XG1-006," "XG1-006," or "006." In another example, the antibody PSMA 4.232.3 may be referred to as "PSMA 4.232.3," "4.232.3," "4.232.3," or "4.232." It is intended that all of the variations in the name of the antibody refer to the same antibody and not a different one.

As used herein, a "coding region" refers to a region of a nucleotide sequence that encodes a polypeptide sequence; the coding region can include a region coding for a portion of a protein that is later cleaved off, such as a signal peptide.

Those of skill in the art will appreciate that nucleic acids and polypeptides as provided herein include nucleotide sequences and amino acid sequences as provided herein. In some instances, the nucleotide sequences and amino acid sequences may include sequences that encode, or that are, signal peptides. Each of these sequences with, or without, the portion of the sequence that encodes or is a signal peptide is contemplated herein.

A PSMA antibody may be encoded by a nucleic acid molecule or amino acid molecule that is highly homologous to the nucleic acid molecules or amino acid molecules provided herein, respectively. For example, homologous nucleic acid molecule or amino acid molecule may comprise a nucleotide sequence or amino acid sequence that is at least about 90% identical to a nucleotide sequence or amino acid sequence provided herein. As another example, a nucleotide sequence or amino acid sequence is at least about 95% identical, at least about 97% identical, at least about 98% identical, or at least about 99% identical to a nucleotide sequence or amino acid sequence as provided herein. Homology can be calculated using various, publicly available software, which are well known to one of ordinary skill in the art. Exemplary tools include the BLAST system available from the website of the National Center for Biotechnology Information (NCBI) at the National Institutes of Health.

PSMA antibodies may include the antibodies provided in U.S. Patents 6,107,090, 6,649,163 and 6,962,981. PSMA antibodies, therefore, include E99, J415, J533, and J591 monoclonal antibodies; monoclonal antibodies produced by hybridomas having ATCC Accession Numbers HB-12101, HB-12109, HB-12127 and HB-12126; and monoclonal antibodies produced by hybidomas having ATCC Accession Numbers HB12060 (3F5.4G6), HB12309 (3D7-1.1), HB12310 (4E10-1.14), HB12489 (1G3), HB12495 (1G9), HB12490 (2C7), HB12494 (3C4), HB12491 (3C6), HB12484 (3C9), HB12486 (3E6), HB12488 (3E11), HB12485 (3G6), HB12493 (4D4), HB12487 (4D8), HB12492 (4C8B9), HB12664 (3F6), HB12678 (2E4), HB12665 (3C2), HB12672 (2D4), HB12660 (4C8G8), HB12675 (2C4), HB 12663 (4C11), HB12661 (1D11), HB 12667 (4E8), HB 12674 (2G5), HB 12620 (4E6), HB12677 (1F4), HB12666 (2E3), HB12662 (3D8), HB12668 (4F8), HB12673 (3D2), HB12676 (1G7), HB12669 (3D4), HB12679 (5G10), and HB12671 (5E9). Antigen-binding fragments of these antibodies are also contemplated for use as PSMA ligands of the PSMA ligand conjugates provided herein.

Also provided herein are nucleic acid molecules encoding PSMA antibodies and antigen-binding fragments thereof and vectors comprising the nucleic acid molecules as described herein. The vectors provided can be used to transform or transfect host cells for producing PSMA antibodies and antigen-binding fragments thereof with the specificity described herein.

As used herein, "antibody" refers to a glycoprotein comprising at least two heavy (H) chains and two light (L) chains inter-connected by disulfide bonds. Each heavy chain is comprised of a heavy chain variable region (abbreviated herein as HCVR or V_{H}) and a heavy chain constant region. The heavy chain constant region is comprised of three domains, C_{H}1, C_{H}2 and C_{H}3. Each light chain is comprised of a light chain variable region (abbreviated herein as LCVR or V_{L}) and a light chain constant region. The light chain constant region is comprised of one domain, CL. The V_{H} and V_{L} regions can be further subdivided into regions of hypervariability, termed complementarity determining regions (CDRs), interspersed with regions that are more conserved, termed framework regions (FRs). Each V_{H} and V_{L} is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (*e.g*., effector cells) and the first component (C1q) of the classical complement system.

As used herein, "antigen-binding fragment" of an antibody refers to one or more portions of an antibody that retain the ability to bind specifically to an antigen (*e.g*., PSMA). The antigen-binding function of an antibody can be performed by fragments of a full-length antibody. Examples of binding fragments encompassed within the term "antigen-binding fragment" of an antibody include (i) a Fab fragment, a monovalent fragment consisting of the V_{L}, V_{H}, C_{L} and C_{H}1 domains; (ii) a F(ab')₂ fragment, a bivalent fragment comprising two Fab fragments linked by a disulfide bridge at the hinge region; (iii) a Fd fragment consisting of the V_{H} and CH1 domains; (iv) a Fv fragment consisting of the V_{L} and V_{H} domains of a single arm of an antibody, (v) a dAb fragment (Ward et al., (1989) Nature 341:544-546), which consists of a V_{H} domain; and (vi) an isolated complementarity determining region (CDR). Furthermore, although the two domains of the Fv fragment, V and V_{H}, are coded for by separate genes, they can be joined, using recombinant methods, by a synthetic linker that enables them to be made as a single protein chain in which the V_{L} and V_{H} regions pair to form monovalent molecules (known as single chain Fv (scFv); see e.g., Bird et al. (1988) Science 242:423-426; and Huston et al. (1988) Proc. Natl. Acad. Sci. USA 85:5879-5883). Such single chain antibodies are also intended to be encompassed within the term "antigen-binding portion" of an antibody.

As used herein, "isolated antibody" refers to an antibody that is substantially free of other antibodies having different antigenic specificities (*e.g*., an isolated antibody that specifically binds to PSMA is substantially free of antibodies that specifically bind antigens other than PSMA). An isolated antibody that specifically binds to an epitope, isoform or variant of PSMA may, however, have cross-reactivity to other related antigens, *e.g*., from other species (*e.g*., PSMA species homo logs). Moreover, an isolated antibody may be substantially free of other cellular material and/or chemicals.

Isolated antibodies described herein encompass various antibody isotypes, such as IgG1, IgG2, IgG3, IgG4, IgM, IgA1, IgA2, IgAsec, IgD, IgE. As used herein, "isotype" refers to the antibody class (*e.g.,* IgM or IgG1) that is encoded by heavy chain constant region genes. Antibodies can be full length or can include only an antigen-binding fragment such as the antibody constant and/or variable domain of IgG1, IgG2, IgG3, IgG4, IgM, IgA1, IgA2, IgAsec, IgD or IgE or could consist of a Fab fragment, a F(ab')₂ fragment, and a Fv fragment.

Antibodies may be polyclonal, monoclonal, or a mixture of polyclonal and monoclonal antibodies. Antibodies can be produced by a variety of techniques well known in the art. Procedures for raising polyclonal antibodies are well known. Monoclonal antibody production may be effected by techniques that are also well known in the art.

As used herein, "monoclonal antibody" refers to a preparation of antibody molecules of single molecular composition. A monoclonal antibody displays a single binding specificity and affinity for a particular epitope.

Recombinant antibodies are contemplated for use as provided herein. As used herein, a "recombinant antibody" refers to an antibody that is prepared, expressed, created or isolated by recombinant means, such as an antibody isolated from an animal (*e.g*., a mouse) that is transgenic for another species' immunoglobulin genes, an antibody expressed using a recombinant expression vector transfected into a host cell, an antibody isolated from a recombinant, combinatorial antibody library, or an antibody prepared, expressed, created or isolated by any other means that involves splicing of immunoglobulin gene sequences to other DNA sequences.

An antibody may be a chimeric or humanized antibody. As used herein, a "chimeric antibody" refers to an antibody that combines the murine variable or hypervariable regions with the human constant region or constant and variable framework regions. As used herein, "humanized antibody" refers to an antibody that retains only the antigen-binding CDRs from the parent antibody in association with human framework regions (*see,* Waldmann, 1991, Science 252:1657). Such chimeric or humanized antibodies retaining binding specificity of the murine antibody are expected to have reduced immunogenicity when administered *in vivo* for diagnostic, prophylactic or therapeutic applications as provided herein.

Monoclonal antibodies provided here may be modified to be in the form of a bispecific antibody or a multispecific antibody. As used herein, a "bispecific antibody" includes any agent, *e.g.,* a protein, peptide, or protein or peptide complex, that has two different binding specificities which bind to, or interact with (a) a cell surface antigen and (b) an Fc receptor on the surface of an effector cell. As used herein, a "multispecific antibody" includes any agent, *e.g.,* a protein, peptide, or protein or peptide complex, that has more than two different binding specificities that bind to, or interact with, (a) a cell surface antigen, (b) an Fc receptor on the surface of an effector cell, and (c) at least one other component. Accordingly, antibodies provided herein may be bispecific, trispecific, tetraspecific, or other multispecific antibodies directed to cell surface antigens, such as PSMA, and to Fc receptors on effector cells. "Bispecific antibodies" also includes diabodies. Diabodies are bivalent, bispecific antibodies in which the V_{H} and V_{L} domains are expressed on a single polypeptide chain and use a linker that is too short to allow for pairing between the two domains on the same chain, thereby forcing the domains to pair with complementary domains of another chain and creating two antigen-binding sites (*see, e.g.,* Holliger, P., et al. (1993) Proc. Natl. Acad. Sci. USA 90:6444-6448; Poijak, R. J., et al. (1994) Structure 2:1121-1123).

A bispecific antibody can be formed of an antigen-binding region specific for the extracellular domain of PSMA and an antigen-binding region specific for an effector cell that has tumoricidal or tumor inhibitory activity. The two antigen-binding regions of the bispecific antibody are either chemically linked or can be expressed by a cell genetically engineered to produce the bispecific antibody. (*See* generally, Fanger et al., 1995 Drug News & Perspec. 8(3):133-137). Suitable effector cells having tumoricidal activity include, without limitation, cytotoxic T-cells (primarily CD8⁺ cells), and natural killer cells.

Antibodies provided herein may be human antibodies. As used herein, "human antibodies" include antibodies having variable and constant regions derived from human germline immunoglobulin sequences. Human antibodies may include amino acid residues not encoded by human germline immunoglobulin sequences (*e.g*., mutations introduced by random or site-specific mutagenesis *in vitro* or by somatic mutation *in vivo*). The term "human antibodies", as used herein, is not intended to include antibodies in which CDR sequences derived from the germline of another mammalian species, such as a mouse have been grafted onto human framework sequences (otherwise referred to herein as "humanized antibodies"). Human antibodies directed against PSMA can be generated using transgenic mice carrying parts of the human immune system rather than the mouse system. Human PSMA antibodies provided herein specifically bind cell-surface PSMA and/or rsPSMA (recombinant soluble PSMA, *i.e*., with the sequence of the extracellular portion of PSMA) with sub-nanomolar affinity. Human PSMA antibodies provided herein may have binding affinities of about 1×10⁻⁹M or less, about 1×10⁻¹⁰ M or less, or 1×10⁻¹¹ M or less. The binding affinity of human PSMA antibodies as provided herein may be less than about 5×10⁻¹⁰ M.

Antibodies or antigen-binding fragments thereof can be selected, for example, based on the following criteria, which are not intended to be exclusive:
∘ 1) binding to live cells expressing PSMA;
∘ 2) high affinity of binding to PSMA;
∘ 3) binding to a unique epitope on PSMA (to eliminate the possibility that antibodies with complimentary activities when used in combination would compete for binding to the same epitope);
∘ 4) opsonization of cells expressing PSMA;
∘ 5) mediation of growth inhibition, phagocytosis and/or killing of cells expressing PSMA in the presence of effector cells;
∘ 6) modulation (inhibition or enhancement) of NAALADase, folate hydrolase, dipeptidyl peptidase IV and/or γ-glutamyl hydrolase activities;
∘ 7) growth inhibition, cell cycle arrest and/or cytotoxicity in the absence of effector cells;
∘ 8) internalization of PSMA;
∘ 9) binding to a conformational epitope on PSMA;
∘ 10) minimal cross-reactivity with cells or tissues that do not express PSMA; and
∘ 11) preferential binding to dimeric forms of PSMA rather than monomeric forms of PSMA.

PSMA antibodies and antigen-binding fragments thereof provided herein typically meet one or more, and in some instances, all of the foregoing criteria.

An isolated antibody or antigen-binding fragment thereof may be selected for its ability to bind live cells expressing PSMA. In order to demonstrate binding of monoclonal antibodies to live cells expressing the PSMA, flow cytometry can be used. Binding of the antibody or antigen-binding fragment thereof to live cells expressing PSMA can inhibit the growth of the cells or mediate cytolysis of the cells. Cytolysis can be complement mediated or can be mediated by effector cells. The cytolysis may be carried out in a living organism, preferably a mammal, and the live cell is a tumor cell. Examples of tumors that can be targeted by PSMA antibodies (*e.g*., PSMA antibody conjugates) provided herein, include any tumor that expresses PSMA such as, *e.g*., prostate, bladder, pancreas, lung, colon, kidney, melanomas and sarcomas. A tumor that expresses PSMA includes tumors with neovasculature expressing PSMA.

A PSMA antibody, or antigen-binding fragment thereof, may bind to and be internalized with PSMA expressed on cells. Thus, a PSMA ligand conjugate comprising a PSMA antibody may be internalized with PSMA expressed on cells. The mechanism by which this internalization occurs is not critical to the practice of the present disclosure. For example, the antibody or antigen-binding fragment thereof can induce internalization of PSMA.

A PSMA antibody, or antigen-binding fragment thereof, may bind to a conformational epitope within the extracellular domain of the PSMA molecule. Antibodies that bind to native protein but not denatured protein are those antibodies that bind conformational epitopes, and may be preferred antibodies.

A PSMA antibody, or antigen-binding fragment thereof, may bind to a dimer-specific epitope on PSMA. Generally, antibodies or antigen-binding fragments thereof that bind to a dimer-specific epitope preferentially bind the PSMA dimer rather than the PSMA monomer. Antibodies that bind to the PSMA dimer but not to the monomeric PSMA protein may be preferred antibodies.

Other PSMA antibodies, or antigen-binding fragments thereof, provided herein include antibodies that bind specifically to an epitope on PSMA defined by a second antibody. To determine the epitope, one can use standard epitope mapping methods known in the art.

The PSMA antibody of a PSMA ligand conjugate may be a monoclonal antibody that binds prostate specific membrane antigen (PSMA) protein dimer, PSMA protein dimer being a homodimer of PSMA protein monomer having the sequence of SEQ ID NO: 1, or an antigen-binding fragment thereof, wherein the antibody, or the antigen-binding fragment, (i) binds live cells and (ii) binds with at least a two-fold greater affinity to PSMA protein dimer than to PSMA protein monomer, as described in U.S. Patent No. 8,114,965.

PSMA antibodies may be conjugated to radioactive molecules. An example of such a PSMA ligand conjugate, thus, includes ¹⁷⁷Lu-J591, which contains monoclonal PSMA antibody J591 conjugated through 1,4,7,10-tetraazacyclododecane-1,4,7,10-tetraacetic acid (DOTA) to ¹⁷⁷Lutetium (¹⁷⁷Lu).

The PSMA ligand of a PSMA ligand conjugate may be any small molecule ligand that binds specifically PSMA. Such small molecule ligands may bind to the enzymatic site of PSMA in its native conformation. Also, such small molecule ligands may possess any one or more of the characteristics described above for PSMA antibody ligands.

The small molecule ligand may be based on a glutamate-urea-lysine heterodimer (*e.g*., glutamate-urea-lysine analog), or a glutamate-urea-glutamate based dimer, that binds specifically to an enzymatic site on PSMA. According to the invention, the small molecule ligand comprises a glutamate-urea-amino acid moiety, wherein the glutamate-urea-amino acid moiety is a glutamate-urea-lysine. In some embodiments, such small molecule ligands are conjugated to a radionuclide as the anticancer, or cytotoxic, agent (*e.g*., cytotoxic radionuclide or radio therapeutic isotope). Examples of PSMA ligand conjugates, thus, include glutamate-urea-amino acid based small molecule ligands conjugated to a radionuclide through an intervening linker such as ¹²³I-MIP-1095 (also referred to as ¹²³I-MIP-1466) and ¹²³I-MIP-1072 (Molecular Insight Pharmaceuticals, Inc.). Other examples of PSMA small molecule ligands and PSMA ligand conjugates can be found in U.S. Patent 8,465,725 and U.S. 8,487,129. In some embodiments, I¹²³ may be substituted with other radiohalogens including those selected from the group consisting of I¹²⁵, I¹³¹, I¹²⁴, BR⁷⁵, BR⁷⁷ and F¹⁸.

The chemical structure of ¹²³I-MIP-1095 (*i.e.,* ¹²³I-(S)-2-(3-((S)-1-carboxy-5-(3-(4-iodophenyl)ureido)pentyl)ureido)pentanedioic acid) is :

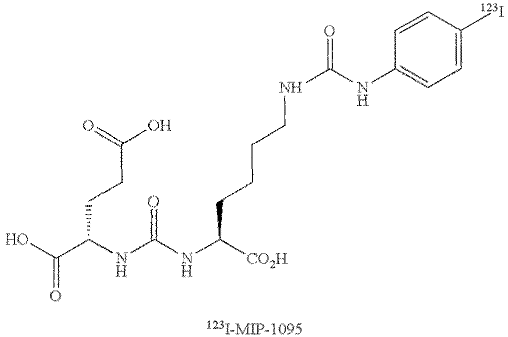

In another embodiment, the PSMA ligand conjugate is ¹²⁴I-MIP-1095. In another embodiment, the PSMA ligand conjugate is ¹³¹I-MIP-1095.

The chemical structure of ¹²³I- MIP-1072 (*i.e.,* ¹²³I-(S)-2-(3-((S)-1-carboxy-5-(4-iodobenzylamino)pentyl)ureido)pentanedioic acid) is:

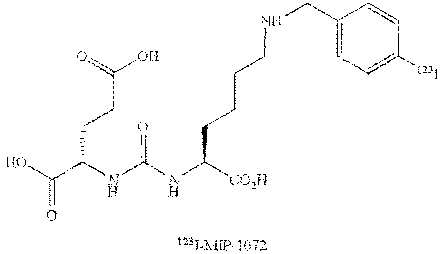

The small molecule ligand of a PSMA ligand conjugate may be a GL2 molecule as described in International Publication No. WO2010/005723. Any of the small molecules ligands provided herein, including a GL2 molecule, may be conjugated to a therapeutic agent by way of a nanoparticle (*e.g*., polymer-based, lipid-based and/or nucleic acid-based nanoparticles). The nanoparticle may contain the therapeutic agent. Thus, the PSMA ligand conjugate may comprise a small molecule ligand conjugated to a nanoparticle that contains an anticancer or cytotoxic agent. Examples of such PSMA ligand conjugates include, without limitation, BIND-014 (Bind Biosciences, Inc.) described in International Publication No. WO2010/005723.

PSMA small molecule ligands may be selected from the group consisting of compounds I, II, III and IV: 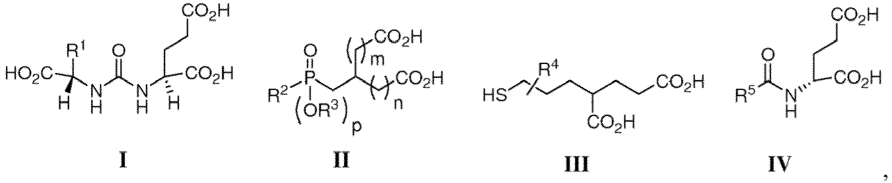 and enantiomers, stereoisomers, rotamers, tautomers, diastereomers, or racemates thereof; wherein m and n are each, independently, 0, 1, 2 or 3; p is 0 or 1; R1, R2, R4 and R5 are each, independently, selected from the group consisting of substituted or unsubstituted alkyl, substituted or unsubstituted aryl, and any combination thereof; and R3 is H or CH3; wherein R1, R2, R4 or R5 comprise a point of covalent attachment to the nanoparticle. For example, R1, R2, R4 and R5 may be each, independently, Ci₁₋₆-alkyl or phenyl, or any combination of Ci₁₋₆-alkyl or phenyl, which are independently substituted one or more times with OH, SH, NH2, or CO2H, and wherein the alkyl group may be interrupted by N(H), S or O. In some instances, for example, R1, R2, R4 and R5 are each, independently, CH₂-Ph, (CH₂)₂-SH, CH₂-SH, (CH₂)₂C(H)(NH₂)CO₂H, CH₂C(H)(NH₂)CO₂H, CH(NH₂)CH₂CO₂H, (CH₂)₂C(H)(SH)CO₂H, CH₂-N(H)-Ph, O-CH₂-Ph, or O-(CH₂)₂-Ph, wherein each Ph may be independently substituted one or more times with OH, NH₂, CO₂H or SH.

PSMA small molecule ligands may be selected from the group consisting of: 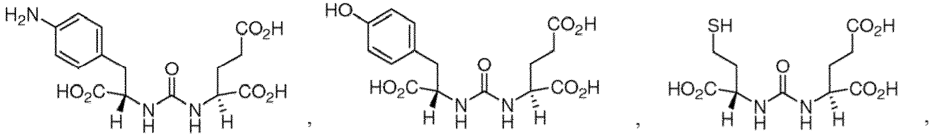 and 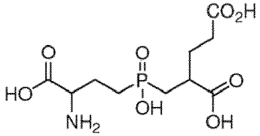 and enantiomers, stereoisomers, rotamers, tautomers, diastereomers, or racemates thereof; and wherein the NH₂, OH or SH groups serve as a point of covalent attachment, or may be selected from the group consisting of 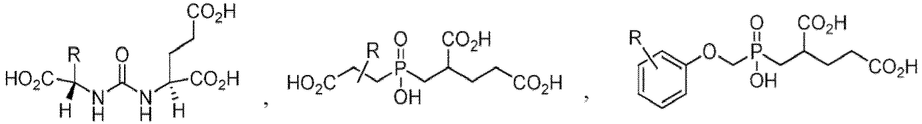 and 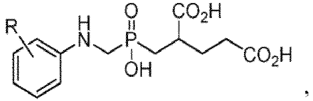 and enantiomers, stereoisomers, rotamers, tautomers, diastereomers, or racemates thereof; wherein R is independently selected from the group consisting of NH₂, SH, OH, CO₂H, Ci_6-alkyl that is substituted with NH₂, SH, OH or CO₂H, and phenyl that is substituted with NH₂, SH, OH or CO₂H, and wherein R serves as the point of covalent attachment.

PSMA small molecule ligands may be selected from the group consisting of: 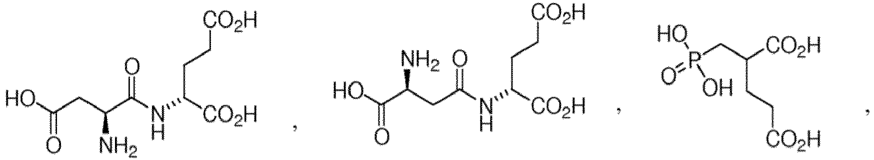 and 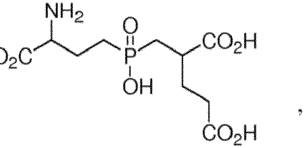 and enantiomers, stereoisomers, rotamers, tautomers, diastereomers, or racemates thereof; any of which may be further substituted with NH2, SH, OH, CO2H, Ci₁₋₆-alkyl that is substituted with NH₂, SH, OH or CO₂H, or phenyl that is substituted with NH₂, SH, OH or CO₂H, wherein these functional groups serve as the point of covalent attachment. For example, a low-molecular weight PSMA ligand may be 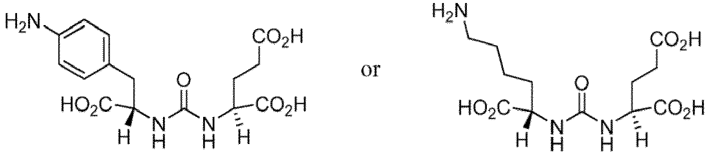 and enantiomers, stereoisomers, rotamers, tautomers, diastereomers, or racemates thereof; wherein the NH₂ groups serve as the point of covalent attachment. Attachment may be to a linker, polymer, particle, etc.

In some embodiments, the PSMA small molecule ligand that comprises a molecule that is or mimics a substrate that binds the enzymatic site on PSMA includes 2-[3-(1,3-dicarboxypropyl)ureido] pentanedioic acid (DUPA). In some embodiments, such small molecule ligands are conjugated to a chemotherapeutic agent, such as tubulysin hydrazide (TubH). The synthesis and uses of an example of such a PSMA ligand conjugate (EC1069) are described in Kularatne, SA et al J Med Chem 2010, 53, 7767-7777; Kularatne, SA et al Mol Phramaceutics Vol 6, no 3, 780-789, 2009. EC1719 is another example of a PSMA ligand conjugate that includes TubH. EC1069 and EC1719 can target the chemotherapy drug to PSMA receptors expressed on prostate cancer cells (Endocyte). Other EC1069 and EC1719 analogs linking DUPA and TubH can also target PSMA receptors expressed on prostate cancer cells. Thus, also contemplated herein are analogs of EC1069 and analogs of EC1719. The terms "EC 1069" and "EC1719," therefore, encompasses EC 1069, EC1719 and analogs thereof. The linkers of the analogs may be peptides with D-amino-acid(s), or peptides attached with sugar moieties, amides or esters. An example of a linker, therefore, is D-γ-Glu D-Asp-D-Phe-D-Cys (FIG. 9). Other linkers may be used as provided herein.

Conjugation of one or more therapeutic agents to a PSMA ligand can include many chemical mechanisms, for instance covalent binding, affinity binding, intercalation, coordinate binding, electrostatic binding and complexation. Conjugation may also include encapsulation and is intended to refer to any mechanism by which one component may be associated with another component. Conjugation may be direct conjugation of the therapeutic agent to the PSMA ligand or it may be indirect, such as via a linker, polymer, particle etc., and it is the linker, polymer, particle, etc. to which the therapeutic agent is bound.

Covalent binding can be achieved either by direct condensation of existing side chains or by the incorporation of external bridging molecules. Many bivalent or polyvalent agents are useful in coupling protein molecules to other proteins, peptides or amine functions, etc. For example, the literature is replete with coupling agents such as carbodiimides, diisocyanates, glutaraldehyde, diazobenzenes, and hexamethylene diamines. This list is not intended to be exhaustive of the various coupling agents known in the art but, rather, is exemplary of the more common coupling agents.

Where the PSMA ligand is an antibody, it is contemplated the antibody may be first derivatized, and then the therapeutic agent may be attached to the derivatized product. Suitable cross-linking agents for use in this manner include, for example, SPDP (N-succinimidyl-3-(2-pyridyldithio)propionate), and SMPT, 4-succinimidyl-oxycarbonyl-methyl-(2-pyridyldithio)toluene.

Where the agent is a protein toxin, it may be fused to the PSMA ligand by genetic methods to form a hybrid immunotoxin fusion protein. The fusion proteins can include additional peptide sequences, such as peptide spacers that operatively attach, for example, the PSMA ligand and toxin, as long as such additional sequences do not appreciably affect the targeting or toxin activities of the fusion protein. The proteins may be attached by a peptide linker or spacer, such as a glycine-serine spacer peptide, or a peptide hinge, as is well known in the art. Thus, for example, if the PSMA ligand is a PSMA antibody, the C-terminus of PSMA antibody can be fused to the N-terminus of the protein toxin molecule to form an immunotoxin that retains the binding properties of the PSMA antibody. Other fusion arrangements will be known to one of ordinary skill in the art. To express the fusion immunotoxin, the nucleic acid encoding the fusion protein is inserted into an expression vector in accordance with standard methods, for stable expression of the fusion protein, such as in mammalian cells, such as CHO cells. The fusion protein can be isolated and purified from the cells or culture supernatant using standard methodology, such as a PSMA affinity column.

Examples of anticancer agents for use as provided herein include, without limitation, cytotoxic agents, chemotherapeutic agents and agents that act on tumor neovasculature. Cytotoxic agents include, but are not limited to, cytotoxic radionuclides, chemical toxins and protein toxins. Cytotoxic radionuclides or radiotherapeutic isotopes include alpha-emitting isotopes such as, for example, ²²⁵Ac, ²¹¹At, ²¹²Bi, ²¹³Bi, ²¹²Pb, ²²⁴Ra, ²²³Ra. Cytotoxic radionuclides or radiotherapeutic isotopes include beta-emitting isotopes such as, for example, ¹⁸⁶Rh, ¹⁸⁸Rh, ¹⁷⁷Lu, ⁹⁰Y, ¹³¹I, ⁶⁷Cu, ⁶⁴Cu, ¹⁵³Sm, ¹⁶⁶Ho. In some instances, cytotoxic radionuclides may emit Auger and/or low energy electrons and include the isotopes ¹²³I, ¹²⁴I, ¹²⁵I, ¹³¹I, ⁷⁵Br, ⁷⁷Br and ¹⁸F.

Radionuclides typically are coupled to an antibody or antigen-binding fragment thereof by chelation. For example, in the case of metallic radionuclides, a bifunctional chelator is commonly used to link the isotope to the antibody or other protein of interest. Typically, the chelator is first attached to the antibody, and the chelator-antibody conjugate is contacted with the metallic radioisotope. A number of bifunctional chelators have been developed for this purpose, including the diethylenetriamine pentaacetic acid (DTPA) series of amino acids described in U.S. patents 5,124,471, 5,286,850 and 5,434,287. As another example, hydroxamic acid-based bifunctional chelating agents are described in U.S. patent 5,756,825. Another example is the chelating agent termed *p*-SCN-Bz-HEHA (1,4,7,10,13,16-hexaazacyclo-octadecane- N,N',N",N"',N"",N""'-hexaacetic acid) (Deal et al., J. Med. Chem. 42:2988, 1999), which is an effective chelator of radiometals such as ²²⁵Ac. Yet another example is DOTA (1,4,7,10-tetraazacyclododecane N,N',N",N"'-tetraacetic acid), which is a bifunctional chelating agent (*see* McDevitt et al., Science 294:1537-1540, 2001) that can be used in a two-step method for labeling followed by conjugation.

Chemical toxins or chemotherapeutic agents include, but are not limited to, members of the enediyne family of molecules, such as calicheamicin and esperamicin. Chemical toxins or chemotherapeutic agents can also include pyrrolobenzodiazepine (PBD) dimers (*e.g.*, SJG-136, SG2000, SG2202, SG2285 as described in Hartley JA et al., Cancer Res. 2010, 70(17):6849-58), calicheamicins, colchicine, ispinesib (a novel small molecule inhibitor of kinesin spindle protein), combrestatin (*e.g*., combrestatin A4), maytansine derivatives such as maytansinoid DM4 (*N*2'-deacetyl-*N*2'-(4-mercapto-4-methyl-1-oxopentyl)maytansine) and maytansinoid DM1 (mertansine), methotrexate, doxorubicin, melphalan, chlorambucil, ARA-C, vindesine, mitomycin C, cis-platinum, etoposide, bleomycin and/or 5-fluorouracil. Other antineoplastic agents include dolastatins (U.S. Patent Nos. 6,034,065 and 6,239,104) and derivatives thereof. Dolastatins and derivatives thereof include dolastatin 10 (dolavaline-valine-dolaisoleuine-dolaproine-dolaphenine) and the derivatives auristatin PHE (dolavaline-valine-dolaisoleuine-dolaproine-phenylalanine-methyl ester) (Pettit, G.R. et al., Anticancer Drug Des. 13(4):243-277, 1998; Woyke, T. et al., Antimicrob. Agents Chemother. 45(12):3580-3584, 2001), aurastatin E (*e.g*., monomethylauristatin norephedrine), aurastatin F (*e.g.,* monomethylauristatin phenylalanine) and the like. Toxins also include poisonous lectins, plant toxins such as ricin, abrin, modeccin, botulina and diphtheria toxins. Other chemotherapeutic agents are known to those skilled in the art and may be used as provided herein.

Agents that act on the tumor vasculature include, but are not limited to, tubulin-binding agents (*e.g.,* anti-tubulin agents) such as tubulysin and derivatives thereof (Kaur et al., Biochem J. 396(Pt 2):235-242, 2006), combrestatin A4 (Griggs et al., Lancet Oncol. 2:82, 2001), angiostatin and endostatin (reviewed in Rosen, Oncologist 5:20, 2000) and interferon inducible protein 10 (U.S. Patent No. 5,994,292). A number of other antiangiogenic agents are also contemplated and include: 2ME2, angiostatin, angiozyme, anti-VEGF RhuMAb, Apra (CT-2584), avicine, benefin, BMS275291, carboxyamidotriazole, CC4047, CC5013, CC7085, CDC801, CGP-41251 (PKC 412), CM101, combretastatin A-4 prodrug, EMD 121974, endostatin, flavopiridol, genistein (GCP), IM-862, ImmTher, interferon alpha, interleukin-12, gefitinib (ZD1839), marimastat, metastat (Col-3), neovastat, octreotide, paclitaxel, penicillamine, photofrin, photopoint, PI-88, prinomastat (AG-3340), PTK787 (ZK22584), RO317453, solimastat, squalamine, SU 101, SU 5416, SU-6668, suradista (FCE 26644), suramin (metaret), tetrathiomolybdate, thalidomide, TNP-470 and vitaxin. Additional antiangiogenic agents are described by Kerbel, J. Clin. Oncol. 19(18s):45s-51s, 2001. Such agents are contemplated for use in the PSMA ligand conjugates provided herein.

A PSMA ligand conjugate may be a PSMA antibody-drug conjugate. Non-limiting examples of PSMA antibody-drug conjugates are described in US-2007-0160617-A1 and US-2011-0250216-A. A PSMA antibody-drug conjugate may comprise an antibody or antigen-binding fragment thereof that specifically binds PSMA and is conjugated to a dolastatin 10 derivative, in particular auristatins such as, MMAE (also referred to herein as monomethylauristatin E or monomethylauristatin norephedrine) or MMAF (also referred to herein as monomethylauristatin F or monomethylauristatin phenylalanine).

The antibody or antigen-binding fragment thereof can be conjugated to MMAE or MMAF with a compound of the following formula (**Formula 1**): -Aₙ-Yₘ-Zₘ-Xₙ-Wₙ-, wherein A is a carboxylic acyl unit; Y is an amino acid; Z is an amino acid; X and W are each a self-immolative spacer; n is an integer of 0 or 1; and m is an integer of 0 or 1, 2, 3, 4, 5 or 6. The ADC may be represented by the formula **(Formula 2):** L-{Aₙ-Yₘ-Zₘ-Xₙ-Wₙ-D}ₚ wherein L is an antibody or antigen-binding fragment thereof that binds PSMA, D is MMAE or MMAF and p is an integer of 1, 2, 3, 4, 5, 6, 7 or 8. The other components are as described above. The carboxylic unit "Aₙ" may be linked to the antibody or antigen-binding fragment via a sulfur atom derived from the antibody or antigen-binding fragment: 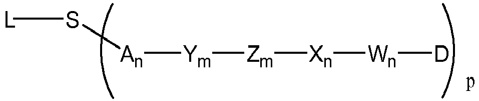 In one instance A may be 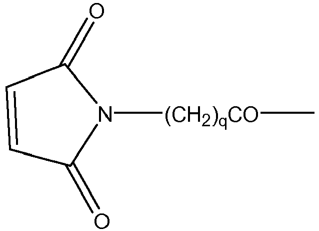 in which q is 1-10. Therefore, the conjugate may be: 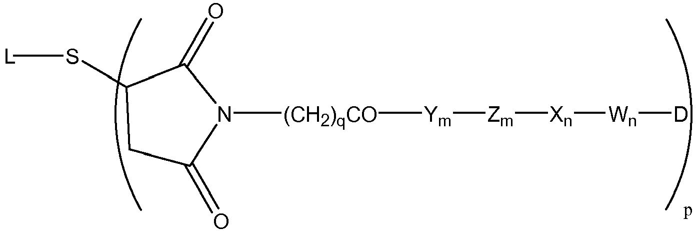 wherein L, Y, Z, X, W, D, n, m, q and p are as previously defined.

In another instance, A may be 4-(N-succinimidomethyl)cyclohexane-1-carbonyl, m-succinimidobenzoyl, 4-(p-succinimidophenyl) -butyryl, 4-(2-acetamido)benzoyl, 3-thiopropionyl, 4-(1-thioethyl)-benzoyl, 6-(3-thiopropionylamido)-hexanoyl or maleimide caproyl. In a further instance, A may be maleimide caproyl. Representative examples of various carboxylic acyl units and methods for their synthesis and attachment are described in US Pat. No. 6,214,345.

In another instance, Y may be alanine, valine, leucine, isoleucine, methionine, phenylalanine, tryptophan or proline. In yet another instance, Y may be valine. In a further instance, Z may be lysine, lysine protected with acetyl or formyl, arginine, arginine protected with tosyl or nitro groups, histidine, ornithine, ornithine protected with acetyl or formyl, or citrulline. In still a further instance, Z may be citrulline. In one instance Yₘ-Zₘ may be valine-citrulline. In another instance, Yₘ-Zₘ may be a protein sequence which is selectively cleavable by a protease.

In a further instance, X may be a compound having the formula 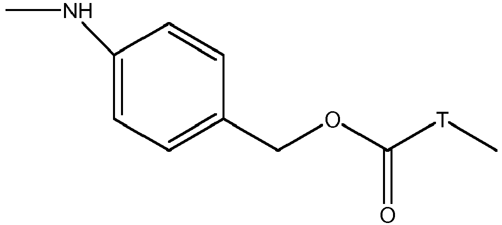 in which T is O, N, or S. In another instance, X may be a compound having the formula -HN-R¹ -COT in which R¹ is C₁-C₅ alkyl, T is O, N or S. In a further instance, X may be a compound having the formula 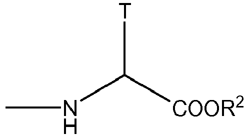 in which T is O, N, or S, R² is H or C₁-C₅ alkyl. In one instance, X may be p-aminobenzylcarbamoyloxy. In another instance, X may be p-aminobenzylalcohol. In a further instance, X may be p-aminobenzylcarbamate. In yet a further instance, X may be p-aminobenzyloxycarbonyl. In another instance, X may be γ-aminobutyric acid; α,α-dimethyl γ-aminobutyric acid or β,β-dimethyl γ-aminobutyric acid.

In some instances, W may be 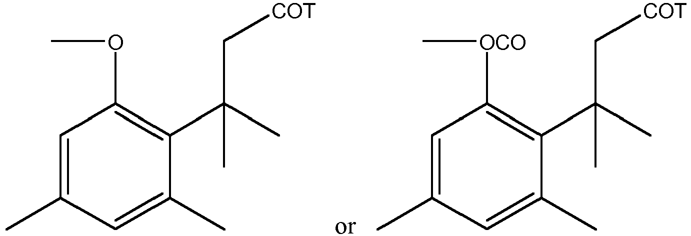 in which T is O, S or N.

In one instance, the compound of **Formula 1** may be maleimidocaproyl. Maleimidocaproyl has been used for conjugation of two specific auristatins to an anti-CD30 mAb (AC10) (Doronina, Svetlana et al. "Novel Linkers for Monoclonal Antibody-Mediated Delivery of Anticancer Agents", AACR, Anaheim, CA, Abstract No. 1421, April 16-20, 2005). Maleimidocaproyl reacts with thiol groups to form a thioether.

MMAE or MMAF can be conjugated to an antibody or antigen-binding fragment thereof using methods known to those of ordinary skill in the art (*e.g., See,* Niemeyer, CM, Bioconjugation Protocols, Strategies and Methods, Humana Press, 2004) or as described herein. More than one MMAE or MMAF molecule may be conjugated to the antibody or antigen-binding fragment thereof. 1, 2, 3, 4, 5, 6, 7 or 8 MMAE or MMAF molecules may be conjugated to the antibody or antigen-binding fragment thereof. At least 2, 3, 4 or 5 MMAE or MMAF molecules may be conjugated to the antibody or antigen-binding fragment thereof. 2, 3, 4 or 5 MMAE or MMAF molecules may be conjugated to the antibody or antigen-binding fragment thereof.

The PSMA ligand conjugate may be PSMA antibody (or antigen-binding fragment thereof)-maleimide caproyl-valine-citrulline-p-aminobenzyloxycarbonyl-monomethylauristatin norephedrine, PSMA antibody (or antigen-binding fragment thereof)-maleimide caproyl-valine-citrulline-p-aminobenzylcarbamate-monomethylauristatin norephedrine, PSMA antibody (or antigen-binding fragment thereof) -maleimide caproyl-monomethylauristatin norephedrine, PSMA antibody (or antigen-binding fragment thereof) -maleimide caproyl-valine-citrulline-p-aminobenzyloxycarbonyl-monomethylauristatin phenylalanine, PSMA antibody (or antigen-binding fragment thereof) -maleimide caproyl-valine-citrulline-p-aminobenzylcarbamate-monomethylauristatin phenylalanine or PSMA antibody (or antigen-binding fragment thereof) -maleimide caproyl-monomethylauristatin phenylalanine. In any of the foregoing, the PSMA antibody or antigen-binding fragment thereof may be any of the antibodies or antigen-binding fragments provided herein.

PSMA antibody-drug conjugates, for example, have been found to have particularly high levels of selectivity when killing of non-PSMA-expressing cells is compared to killing of PSMA-expressing cells. Therefore, PSMA antibody-MMAE or PSMA antibody-MMAF conjugates may have a PC-3™ cell to C4-2 cell or LNCaP™ cell selectivity of at least 250. The selectivity may be at least 300, 350, 400, 450, 500, 600, 700, 800, 900, 1000, 1100, 1200, 1300, 1400, 1500, 1600, 1700, 1800, 1900, 2000, 2250, 2500, 2750, 3000, 3500, 4000, 4500, 5000, 5500, 6000, 6500, 7000, 7500, 8000, 8500, 9000, 9500, 10000, 11000, 12000, 13000, 14000, 15000, 17500, 20000 or more. The selectivity may be between 250-500, 500-750, 750-1000, 1000-2000, 2000-5000, 5000-10000, 10000-15000 or 15000-20000. "Selectivity", as defined herein, refers to the ratio of IC₅₀ values of a PSMA antibody-MMAE conjugate or PSMA antibody-MMAF conjugate on PC-3™ cells (non-PSMA-expressing cells) to C4-2 cells or LNCaP™ cells (PSMA-expressing cells).

PSMA antibody-drug conjugates may mediate PSMA-expressing cell specific killing at very low concentrations, such as at or near picomolar concentrations. PSMA antibody-drug conjugates may exhibit IC₅₀s at concentrations of less than about 1 X 10⁻¹⁰M, less than about 1 X 10⁻¹¹ M, or less than about 1 X 10⁻¹²M. An IC₅₀ may be achieved at a concentration of less than about 1.5 X 10⁻¹¹M. PSMA antibody-drug conjugates may exhibit IC₅₀s of between 10-210, 40-210, 60-210 or 65-210 picomoles (pM). PSMA antibody-drug conjugates may exhibit IC₅₀s of about 10, 40, 60 or 80 pM. PSMA antibody-drug conjugates may exhibit IC₅₀s of about 11, 42, 60 or 83 pM.

The level of cell killing can be determined by any of the methods provided herein or otherwise known to those of ordinary skill in the art.

An "antiandrogen," as used herein, refers to an agent that blocks (*e.g*., inhibits) the action of androgen hormones and androgen-regulated molecules. Adrenergic receptor antagonists are herein considered to be antiandrogens. The term "antiandrogen" includes antiandrogens, antiandrogen analogs, and antiandrogen derivatives. In prostate cancer, antiandrogens block the activity of testosterone, which typically slows prostate cancer growth. In some embodiments, an antiandrogen blocks enzyme cytochrome P450 17A1, encoded by the *CYP17A* gene. Antiandrogens may be steroidal or non-steroidal (also referred to as "pure"). Examples of antiandrogens for use as provided herein include, without limitation, abiraterone (ZYTIGA®), enzalutamide (XTANDI®), nilutamide (NILANDRON®), flutamide (EULEXIN®), bicalutamide (CASODEX®), orteronel (TAK-700, Tokai Pharmaceuticals, Inc.) Potent antiandrogens such as, for example, enzalutamide, abiraterone, ARN 509 (Aragon Pharmaceuticals, Inc.) and galeterone (TOK-001 or VN/124-1, Tokai Pharmaceuticals, Inc.), which are typically used in progressive, metatstatic castration-resistant prostate cancer and which affect expression of a host of androgen-regulated molecules, such as PSMA expression.

"Potent" antiandrogens, or "second-generation" antiandrogens herein include antiandrogens that retain activity in a cellular environment of increased androgen receptor expression relative to wild-type expression. Potent antiandrogens include those that are active in subjects with castration-resistant prostate cancer. Potent antiandrogens also include antiandrogens that bind to the androgen receptor with greater relative affinity than common, clinically used antiandrgogens (*e.g*., bicalutamide, nilutamide, flutamide). Examples of potent antiandrogens include, without limitation, enzalutamide, abiraterone, ARN-509, galeterone, and diarylthiohydantoins RD162 and MDV310, 3beta-hydroxyandrosta-5,16-diene (HAD) and androsta-1,4-diene-3,17-dione-17-ethylene ketal (OAK) (Miyamoto H et al., Int J Cancer 2005, 117(5):866-72). Abiraterone, for example, inhibits the CYP17A1 enzyme. Enzalutamide, as another example, is an androgen receptor antagonist. As yet another example, galeterone is a selective CYP17 inhibitor and an androgen receptor antagonist. Methods that may be used to identify potent antiandrogens are described, for example, by Tran C et al., Science, 2009 324(5928):787-90. Any of the antiandrogens described herein may be used in any of the methods provided herein.

The compound that increases the cell surface expression of PSMA can also be an mTOR inhibitor. Examples of mTOR inhibitors for use as provided herein include, without limitation, rapamycin, everolimus (AFINITOR®, ZORTRESS®) and temsirolimus (TORISEL®). The term "mTOR inhibitor" includes mTOR inhibitors, mTOR inhibitor analogs, and mTOR inhibitor derivatives.

Also provided herein are compositions, for example, pharmaceutical compositions, which comprise a compound that increases cell surface expression of PSMA, a PSMA ligand conjugate, or a combination of the two. As described herein a composition may be administered to a subject (*e.g*.,a mammal, such as a human). As described herein, a composition comprising a PSMA ligand conjugate may be administered to a subject and another composition comprising a compound that increases cell surface expression of PSMA may be separately administered to the subject, either sequentially or concurrently. Alternatively, as described herein, a composition comprising both a PSMA ligand conjugate and a compound that increases cell surface expression of PSMA may be administered to a subject.

As described herein, a composition comprising a compound that increases cell surface expression of PSMA may be first administered to a subject, and then a composition comprising a PSMA ligand conjugate may be administered to the subject immediately after, within an hour, within a couple of hours, within a day, within 2 days, within 3 days, within 4 days, with 5 days, within 6 days, or within a week or more. Either composition may be administered once or more than once (*e.g.*, twice, thrice, etc.)

As used herein, "a compound that increases cell surface expression of PSMA," refers to a compound that increases the cell surface expression of PSMA by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 125%, at least 150%, at least 175%, at least 200% or more, relative to normal cell surface expression of a PSMA-expressing cell or relative to cell surface expression in the absence of such compound. For example, an antiandrogen may, in some embodiments, increase the cell surface expression of PSMA on cancer cells by at least 20%, or more.

A composition, in some embodiments, includes a physiologically or pharmaceutically acceptable carrier, excipient, or stabilizer combined with a compound that increases cell surface expression of PSMA and/or aPSMA ligand conjugate. As used herein, "pharmaceutically acceptable carrier" or "physiologically acceptable carrier" includes any and all salts, solvents, dispersion media, coatings, antibacterial and antifungal agents, isotonic and absorption delaying agents, and the like that are physiologically compatible. A "pharmaceutically-acceptable carrier," as used herein, refers to one or more compatible solid or liquid fillers, diluents or encapsulating substances that are suitable for administration into a human. The term "carrier" denotes an organic or inorganic ingredient, natural or synthetic, with which the active ingredient is combined to facilitate the application. A carrier may be suitable for intravenous, intramuscular, subcutaneous, parenteral, spinal or epidermal administration (*e.g*., by injection or infusion).

As described herein, a composition may be administered to a subject in pharmaceutically-acceptable amounts and in pharmaceutically-acceptable compositions. The term "pharmaceutically acceptable" means a non-toxic material that does not interfere with the effectiveness of the biological activity of the active ingredients (*e.g*., PSMA ligands, anticancer agents, cytotoxic agents, antiandrogens, mTOR inhibitors). Such compositions may contain salts, buffering agents, preservatives, compatible carriers, and optionally other therapeutic agents, such as supplementary immune potentiating agents including adjuvants, chemokines and cytokines. When used in medicine, the salts should be pharmaceutically acceptable, but non-pharmaceutically acceptable salts may conveniently be used to prepare pharmaceutically-acceptable salts thereof and are not excluded.

A salt retains the desired biological activity of the parent compound and does not impart any undesired toxicological effects (*see e.g.,* Berge, S. M., et al. (1977) J. Pharm. Sci. 66: 1-19). Examples of such salts include acid addition salts and base addition salts. Acid addition salts include those derived from nontoxic inorganic acids, such as hydrochloric, nitric, phosphoric, sulfuric, hydrobromic, hydroiodic, phosphorous and the like, as well as from nontoxic organic acids such as aliphatic mono- and dicarboxylic acids, phenyl substituted alkanoic acids, hydroxy alkanoic acids, aromatic acids, aliphatic and aromatic sulfonic acids and the like. Base addition salts include those derived from alkaline earth metals, such as sodium, potassium, magnesium, calcium and the like, as well as from nontoxic organic amines, such as N,N'-dibenzylethylenediamine, N-methylglucamine, chloroprocaine, choline, diethanolamine, ethylenediamine, procaine and the like.
The pharmaceutical compositions may contain suitable buffering agents, including: acetic acid in a salt; citric acid in a salt; boric acid in a salt; and phosphoric acid in a salt.

In some embodiments, a composition may contain suitable preservatives, such as: benzalkonium chloride; chlorobutanol; parabens and/or thimerosal.

In some embodiments, a composition may conveniently be presented in unit dosage form and may be prepared by any of the methods well-known in the art of pharmacy. All methods include the step of bringing the active compound(s) (*e.g*., PSMA ligand, anticancer agent, cytotoxic agent and/or compound that upregulates cell surface expression of PSMA) into association with a carrier that constitutes one or more accessory ingredients. In some embodiments, compositions are prepared by uniformly and intimately bringing the active compound into association with a liquid carrier, a finely divided solid carrier, or both, and then, if necessary, shaping the product.

Compositions suitable for parenteral administration conveniently comprise a sterile aqueous or non-aqueous preparation of PSMA ligand conjugates and/or compounds that increase cell surface expression of PSMA (*e.g*., antiandrogens, mTOR inhibitors), which is preferably isotonic with the blood of the recipient. This preparation may be formulated according to known methods using suitable dispersing or wetting agents and suspending agents. The sterile injectable preparation also may be a sterile injectable solution or suspension in a non-toxic parenterally-acceptable diluent or solvent, for example, as a solution in 1,3-butane diol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or di-glycerides. In addition, fatty acids such as oleic acid may be used in the preparation of injectables. Carrier formulations suitable for oral, subcutaneous, intravenous, intramuscular, etc. administration can be found in Remington's Pharmaceutical Sciences, Mack Publishing Co., Easton, Pa. Any of the compositions provided herein may be sterile.

Active compounds (*e.g*., PSMA ligand, anticancer agent, cytotoxic agent and/or compound that upregulates cell surface expression of PSMA) can be prepared with carriers that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art. *See, e.g.,* Sustained and Controlled Release Drug Delivery Systems, J. R. Robinson, ed., Marcel Dekker, Inc., New York, 1978.

As described herein, a composition can be administered by any conventional route, including injection or by gradual infusion over time. The administration may, for example, be oral, intravenous, intraperitoneal, intramuscular, intracavity, intratumor, or transdermal. When compositions are used therapeutically, preferred routes of administration include intravenous and by pulmonary aerosol. Techniques for preparing aerosol delivery systems containing antibodies are well known to those of skill in the art. Generally, such systems should utilize components that will not significantly impair the biological properties of the antibodies, such as the paratope binding capacity (see, for example, Sciarra and Cutie, "Aerosols," in Remington's Pharmaceutical Sciences, 18th edition, 1990, pp. 1694-1712). Those of skill in the art can readily determine the various parameters and conditions for producing antibody aerosols without resorting to undue experimentation.

As described herein, compositions as provided herein may be administered in effective amounts. An "effective amount" is that amount of an active compound (*e.g*., PSMA ligand conjugate and/or compound that increases cell surface expression of PSMA) that alone, or together with further doses, produces the desired response, *e.g*., inhibits cell proliferation of PSMA-expressing cells and/or kills PSMA-expressing cells. For cancer, this may involve only slowing the progression of a cancer, for example, temporarily, although more preferably, it involves halting the progression of the cancer permanently. This can be monitored by routine methods. The desired response to treatment of cancer or other disease or condition also can be delaying the onset or even preventing the onset of the cancer or other disease or condition.

Such effective amounts will depend, of course, on the particular condition being treated (*e.g*., PSMA-expressing cancer), the severity of the condition, the individual patient parameters including age, physical condition, size and weight, the duration of the treatment, the nature of concurrent therapy (if any), the specific route of administration and like factors within the knowledge and expertise of the health practitioner. These factors are well known to those of ordinary skill in the art and can be addressed with no more than routine experimentation. It is generally preferred that a maximum dose of the individual components or combinations thereof be used, that is, the highest safe dose according to sound medical judgment. It will be understood by those of ordinary skill in the art, however, that a patient/subject may insist upon a lower dose or tolerable dose for medical reasons, psychological reasons or for virtually any other reason.

Compositions as provided herein, in some embodiments, are sterile and contain an effective amount of PSMA ligand conjugates and/or compound that increases cell surface expression of PSMA, etc. for producing the desired response in a unit of weight or volume suitable for administration to a patient/subject. The response can, for example, be measured by determining the physiological effects of the composition, such as regression of a tumor or decrease of disease symptoms. Other assays will be known to one of ordinary skill in the art and can be employed for measuring the level of the response.

The doses of compositions administered to a subject can be chosen in accordance with different parameters, in particular in accordance with the mode of administration used and the state of the subject. Other factors include the desired period of treatment. In the event that a response in a subject is insufficient at the initial doses applied, higher doses (or effectively higher doses by a different, more localized delivery route) may be employed to the extent that patient tolerance permits.

The synergistic effect provided by various combinations of PSMA ligand conjugates with compounds provided herein, such as antiandrogens or mTOR inhibitors, may result in efficacious doses which are lower than the doses required for efficacy when either component of the combination is used singly. The added advantage of a dose lowering effect may have the added benefit of a wider safety profile, *i.e.,* fewer adverse side effects.

For example, doses of PSMA ligand conjugate and/or compound that increases cell surface expression of PSMA can range from about 10 µg/kg to about 100,000 µg/kg. Based on the composition, the dose can be delivered continuously, such as by continuous pump, or at periodic intervals. Desired time intervals of multiple doses of a particular composition can be determined without undue experimentation by one skilled in the art. Other protocols for the administration of compositions will be known to one of ordinary skill in the art, in which the dose amount, schedule of administration, sites of administration, mode of administration and the like vary from the foregoing.

As described herein, the dose of PSMA ligand conjugate may be administered intravenously. In such instances, the dose of PSMA ligand conjugate may be about 1.0 mg/kg to 2.5 mg/kg. For example, in some instances, the dose of PSMA ligand conjugate administered intravenously may be 1.0 mg/kg, 1.1 mg/kg, 1.2 mg/kg, 1.3 mg/kg, 1.4 mg/kg, 1.5 mg/kg, 1.6 mg/kg, 1.7 mg/kg, 1.8 mg/kg, 1.9 mg/kg, 2.0 mg/kg, 2.1 mg/kg, 2.2 mg/kg, 2.3 mg/kg, 2.4 mg/kg, or 2.5 mg/kg. In some instances, the dose of PSMA ligand administered intravenously conjugate may be about 1.0 mg/kg to 2.3 mg/kg. In one instance, the PSMA ligand conjugated is a PSMA ADC, and the PSMA ADC is provided in a dose of about 1.8 mg/kg to 2.3 mg/kg.

The length of time during which a PSMA ligand conjugate is administered administered intravenously may vary. In some instances, a PSMA ligand conjugate may be administered intravenously for 30 minutes, 35 minutes, 40 minutes, 45 minutes, 50 minutes, 55 minutes, 60 minutes, 65 minutes, 70 minutes, 75 minutes, 80 minutes, 85 minutes or 90 minutes.

In some instances, a PSMA ligand conjugate may be administered intravenously at repeated intervals such as, for example, once a week, once every two weeks, or once every three weeks for up to a total of four, six or eight doses. In some instances, the PSMA ligand conjugate may be administered intravenously twice a week, or more.

In some instances, a PSMA ligand conjugate may be administered intravenously for about 60 minutes once every three weeks at a dose of about 1.0 mg/kg to 2.3 mg/kg for up to a total of eight doses.

In some instances, a compound as provided herein, such as an antiandrogen, may be administered in the form of oral capsules. In such instances, the dose of such compound may be about 40 mg to about 160 mg (*e.g.,* 20 mg or 40 mg per capsule). For example, in some instances, the dose of such compound administered orally may be 40 mg, 60 mg, 80 mg, 100 mg, 120 mg, 140 mg or 160 mg. In some instances, the oral dose of such compound may be administered once daily, or twice daily, for about 21 to about 28 days. In some instances, the dose of such compound may be 80 mg (*e.g.,* 2 oral capsules, 40 mg each) to 160 mg (*e.g.,* 4 oral capsules, 40 mg each) and may be administered once daily (OD) for 28 days. In some instances, such compound may be enzulatamide, at a dose of 80 mg (*e.g.,* 2 oral capsules, 40 mg each) to 160 mg (*e.g.,* 4 oral capsules, 40 mg each) and may be administered once daily (OD) for 28 days.

In some instances, the oral dose of a compound as provided herein, such as an antiandrogen, may be about 500 mg to about 1000 mg. For example, in some instances, the dose of such compound administered orally may be 500 mg, 550 mg, 600 mg, 650 mg, 70 mg 0, 750 mg, 800 mg, 850 mg, 900 mg, 950 mg or 1000 mg. In some instances, the oral dose of such compound may be administered once daily, or twice daily, for about 21 to about 28 days. In some instances, the dose of such compound may be 500 mg to 1000 and may be administered once daily (OD) for 28 days. In some instances, such compound may be abiraterone or abiraterone acetate, at a dose of 500 mg to 1000 mg, and may be administered once daily (OD) for 28 days.

In some instances, an mTOR inhibitor may be administered in an I.V. dose of about 5 to 25 mg at weekly intervals. In another instance, the mTOR inhibitor may be administered in an I.V. dose of 5 to 10 mg at weekly inervals. In one instances, the mTOR inhibitor may be temsirolimus (Toresal®) dosed 25 mg I.V. over 30 to 60 minutes at weekly intervals.

In general, doses of radionuclides delivered by a PSMA ligand conjugate provided herein can range from about 0.01 mCi/Kg to about 10 mCi/kg. In some instances, the dose of radionuclide may range from about 0.1 mCi/Kg to about 1.0 mCi/kg. In one instance, the PSMA ligand conjugate may be ¹³¹I-MIP-1095 provided in an I.V. dose of about 1 to 10 GBq. In another instances, ¹³¹I-MIP-1095 may be provided in a dose range of 2 to 8 GBq. In yet another instances, the mean dose may be about 5 GBq. The optimal dose of a given isotope can be determined empirically by simple routine titration experiments well known to one of ordinary skill in the art.

Administration of compositions provided herein to mammals other than humans, *e.g.* for testing purposes or veterinary therapeutic purposes, is carried out under substantially the same conditions as described above.

Compositions (*e.g*., that comprise PSMA ligand conjugate and/or compound that increases cell surface expression of PSMA) as provided herein have *in vitro* and *in vivo* diagnostic and therapeutic utilities. For example, these compounds can be administered to cells in culture, *e.g., in vitro* or *ex vivo,* or in a subject, *e.g., in vivo,* to treat, prevent or diagnose cancer or other disease or condition. As used herein, the term "subject" is intended to include humans and non-human animals. Preferred subjects include a human patient having a disorder characterized by expression, typically aberrant expression (*e.g*., overexpression) of PSMA.

The compositions provided herein, in some embodiments, may be used in conjunction with other therapeutic treatment modalities. Such other treatments include surgery, radiation, cryosurgery, thermotherapy, hormone treatment, chemotherapy, vaccines, and other immunotherapies.

Subjects with prostate cancer may have undergone, or are undergoing, or will undergo hormone therapy. Thus, as described herein, the compositions provided herein may be administered to a subject subsequent to, together with, or prior to hormone therapy, such as for prostate cancer. Examples of hormone therapies for prostate cancer include, without limitation: luteinizing hormone-releasing hormone agonists (*e.g*., leuprolide, goserelin, and buserelin), which can stop the testicles from making testosterone; antiandrogens (*e.g*., flutamide, bicalutamide, enzalutamide and nilutamide), as discussed elsewhere herein, which can block the action of androgens such as testosterone; drugs that can prevent the adrenal glands from making androgens (*e.g*., ketoconazole and aminoglutethimide); orchiectomy, which is a surgical procedure to remove one or both testicles, the main source of male hormones such as testosterone, to decrease the amount of hormone being produced; and estrogens, which can prevent the testicles from making testosterone.

A myriad of subjects may benefit from the methods and compositions provided herein. Such a subject may have progressive metastatic castration-resistant prostate cancer despite a castrate level of serum testosterone (*e.g*., < 50 mg/dL) and having had prior chemotherapy with docetaxel. The subject may have metastatic castration resistant prostate cancer, have had prior treatment with taxane chemotherapy and have received and progressed on abiraterone and/or enzalutamide. The subject may have progressive metastatic castration-resistant prostate cancer despite a castrate level of serum testosterone, have had prior treatment with abiraterone and/or enzalutamide, and have had no prior treatment with cytotoxic chemotherapy. The subject may have progressive metastatic castration-resistant prostate cancer despite a castrate level of serum testosterone and having had one prior treatment with abiraterone and/or enzalutamide. The subject may have progressive metastatic castration-resistant prostate cancer despite a castrate level of serum testosterone and having had no prior treatment with abiraterone and or enzalutamide. The subject may have asymptomatic or minimally symptomatic metastatic castration-resistant prostate cancer despite a castrate level of serum testosterone and having had no prior treatment with abiraterone and/or enzalutamide. The subject may have stable metastatic castration-resistant prostate cancer and is receiving treatment with abiraterone and/or enzalutamide. The subject may have biochemically recurrent prostate cancer and may have previously undergone a primary therapy (*e.g.,* radical prostatectomy (*e.g.,* open, laparoscopic, or robot-assisted) or radiation therapy (*e.g*., dose-escalated three-dimensional conformal RT, intensity-modulated RT, brachytherapy, or a combination thereof)). The subject may have localized high-risk prostate cancer (*e.g*., prostate specific antigen (PSA) greater than 10 nanogram per milliliter (ng/ml); PSA velocity greater than 2 ng/ml per /year (defined as a rise in PSA of greater than 2 ng/ml in the preceding 12 month period); Gleason score greater than or equal to 7 (4+3); or Gleason score 6 if either PSA greater than or equal to 10 ng/ml or PSA velocity greater than or equal to 2 ng/ml/year) and is a candidate for prostatectomy.

Also provided herein are kits comprising the composition(s). The kits may comprise a container containing a compound that increases cell surface expression of PSMA, and a container containing a PSMA ligand conjugate (or the components thereof). The kits can further contain at least one additional reagent as provided herein. The kit may comprise a carrier being compartmentalized to receive in close confinement therein one or more containers or series of containers such as test tubes, vials, flasks, bottles, syringes, or the like. One container or series of containers may contain one or more compound that increases cell surface expression of PSMA. Another container, or series of containers, may contain a PSMA ligand conjugate (or the components thereof). The components of the kits can be packaged either in aqueous medium or in lyophilized form. The components of the conjugates can be supplied either in fully conjugated form, in the form of intermediates or as separate moieties to be conjugated by the user of the kit.

Kits may also comprise a diluent and/or instructions for reconstituting lyophilized forms of the PSMA ligand conjugates and/or compounds for increasing cell surface expression of PSMA, or instructions for diluting aqueous components of the kits. Kits may also comprise instructions for combining a compound that increases cell surface expression of PSMA and/or a PSMA ligand conjugate.

The present invention is further illustrated by the following **Examples,** which in no way should be construed as further limiting. The citation of any reference is not intended to be admission that said reference is prior art.

### EXAMPLES

### Example 1: PSMA ADC synergizes with antiandrogens and rapamycin.

Inhibitors were tested for anti-proliferative activity individually and in combination across a range of concentrations in a matrix fashion. Inhibition data were compared with Bliss predictions, and the differences between the experimental and predicted results were calculated for each point in the matrix. Positive differences indicate supra-additive or synergistic effects. Negative differences indicate antagonism. Results were assessed for statistical significance, as described below.

Mean inhibition data and Bliss differences are shown in **FIG. 2** for PSMA antibody-drug conjugate (PSMA ADC) combined with enzalutamide, abiraterone or rapamycin. Bliss differences are depicted via heat maps of values that are positive (medium gray), negative (dark gray), or near zero (light gray). Boxes are used to indicate statistically significant Bliss differences and the corresponding percent inhibitions. PSMA ADC exhibited similar single-agent activity against LNCaP and C4-2 cells, with IC₅₀ values of approximately 200 pM in each case. These results are in line with prior reports (20, 21). Enzalutamide, abiraterone, and rapamycin each inhibited proliferation of LNCaP cells **(****FIG. 2A****)** but had minimal antiproliferative effects on C4-2 cells when used as single agents **(****FIG. 2B****).**

Despite lack of direct antiproliferative activity in C4-2 cells, enzalutamide, abiraterone and rapamycin all significantly enhanced the activity of PSMA ADC. Bliss differences ranged to nearly 40% and were statistically significant across a range of inhibitor concentrations and levels of inhibition **(****FIG. 2B****).** No significant antagonism was observed for these combinations under any condition. Statistically significant synergy also was observed for PSMA ADC/enzalutamide and PSMA ADC/abiraterone combinations in LNCaP cells **(****FIG. 2A****);** however, the breadth and magnitude were more limited as compared to C4-2 cells. Similarly, Bliss differences were generally positive across the matrix of concentrations for the PSMA ADC/rapamycin combination in LNCaP cells; however, no value reached statistical significance.

Mean inhibition data and Bliss differences are shown in **FIG. 13** for PSMA ADC combined with the antiandrogens, ARN-509 or TOK-001. Bliss differences are depicted via heat maps of values that are positive (medium gray), negative (dark gray), or near zero (light gray). ARN-509 and TOK-001 each inhibited proliferation of LNCaP cells but had minimal antiproliferative effects on C4-2 cells when used as single agents. Despite lack of direct antiproliferative activity in C4-2 cells, ARN-509 and TOK-001 enhanced the activity of PSMA ADC.

To determine if the observed synergies were unique to PSMA ADC/antiandrogen combinations, a small molecule ligand was tested in combination with antiandrogens. Mean inhibition data and Bliss differences are shown in **FIG. 10** for the small molecule PSMA ligand-anticancer agent conjugate, EC1069, combined with enzalutamide. Heat maps illustrate Bliss differences that are positive for synergy (medium gray), negative (dark gray), or near zero (light gray).

As an initial step towards dissecting mechanisms of synergy, the antiandrogens and rapamycin were combined with free MMAE and with unmodified PSMA mAb. Free MMAE showed subnanomolar potency (IC₅₀ ∼ 0.5 nM) against both LNCaP and C4-2 cells, as expected (21). When paired with enzalutamide or abiraterone, free MMAE exhibited additive activity in LNCaP cells and additive to weakly synergistic activity on C4-2 **(****FIG. 3****).** Similar results were obtained when docetaxel, another microtubule inhibitor, was combined with enzalutamide. There were only sporadic instances of statistically significant synergy for the docetaxel/enzalutamide combination **(****FIG. 3****).**

The rapamycin/MMAE combination showed additive effects in LNCaP and moderate synergy in C4-2 cells **(****FIG. 4****).** Thus, rapamycin synergized less potently with MMAE as compared with PSMA ADC on both cell lines. Nonetheless, the rapamycin/MMAE combination appeared to be as or more potent than the enzalutamide/MMAE or abiraterone/MMAE combination in C4-2 cells. Weak to moderate synergy was observed between rapamycin and enzalutamide in both cell lines. In C4-2 cells, non-inhibitory concentrations of enzalutamide enhanced the weak antiproliferative activity of rapamycin **(****FIG. 4****).**

Unmodified PSMA mAb was inactive both alone and in combination with enzalutamide, abiraterone or rapamycin **(****FIG. 5****).** Due to the very limited activity of the individual agents or combinations, assays were performed only twice in some cases. Thus, the robust synergies observed upon combining PSMA ADC with antiandrogens are specific to the conjugate and are not reproduced with either of its individual components.

As a control, a mock combination was prepared and assessed for synergy by adding PSMA ADC to the assay plate via two separate additions. As expected, the mock combination did not show statistically significant synergy or antagonism in either cell line.

### Example 2: Antiandrogens reversibly increase PSMA expression in a time- and dose-dependent manner.

Additional studies examined treatment-induced changes in PSMA expression as a potential correlate of synergy. Flow cytometry and Western blotting were used to assess cell-surface and total PSMA, respectively.

Enzalutamide and abiraterone increased cell-surface levels of PSMA in a dose-dependent manner in both cell lines **(****FIG. 6A-D****).** PSMA expression was approximately doubled at antiandrogens concentrations above 1 mM at 7 days post-treatment. In contrast, rapamycin increased PSMA expression in C4-2 cells only **(****FIG. 6E-F****).** Low nanomolar concentrations of rapamycin were sufficient to induce a >2-fold increase in PSMA expression in C4-2 cells.

To probe the dynamics of expression, C4-2 cells were cultured in enzalutamide (1 mM) for 3 weeks prior to continued culture in the absence of drug for an additional 8 days. Cell-surface PSMA was measured by flow cytometry. PSMA expression increased steadily over time in the presence of enzalutamide. After 21 days, PSMA expression on treated cells was nearly 4-fold greater than expression on untreated cells passaged in parallel. PSMA expression returned to baseline levels within 7 days of culture in the absence of enzalutamide **(****FIG. 6G****).**

Treatment-induced increases in PSMA were also apparent by Western blotting **(****FIG. 7****).** The magnitude of increase was approximately 5-fold for enzalutamide-treated LNCaP cells as determined by serial dilution of lysates and semi-quantitative analysis of the Western blots (data not shown). Enzalutamide, abiraterone and rapamycin upregulated PSMA to similar extents in C4-2 cells. The magnitude of PSMA expression in the presence of the antiandrogens approached but seldom exceeded the expression seen in cells cultured in charcoal-stripped serum. Overall, the flow cytometry and Western blot data showed consistent patterns of treatment-induced changes in PSMA expression.

### Example 3: Treatment-induced changes in AR, PSA and PI3K pathway components.

Levels of AR, PSA, total Akt, phospho-Akt (S473), total S6, and phospho-S6 were also evaluated pre- and post-treatment by Western blotting. Actin was probed as a measure of total cell loading. As expected, antiandrogen-induced upregulation of PSMA was accompanied by downregulation of PSA **(****FIG. 7****).** In contrast, rapamycin increased PSA expression. This result is consistent with prior observations for rapamycin or its analogs (28, 29). The co-upregulation of PSMA and PSA by rapamycin contrasts with the opposing effects on PSMA and PSA exerted by antiandrogens. AR protein levels were affected modestly by treatment **(****FIG. 7****).**

Akt activation was a common response to antiandrogen or rapamycin treatment of LNCaP cells. However, treatment had limited effects on Akt activation in C4-2 cells. Similarly, the antiandrogens increased levels of phospho-S6 in LNCaP but not C4-2 cells. These findings are consistent with an mTOR-mediated adaptation to antiandrogen treatment in LNCaP cells. Rapamycin-mediated activation of Akt is known to occur via disruption of a negative feedback loop involving insulin receptor substrate 1 (30-33). As expected, rapamycin effectively ablated S6 phosphorylation in both LNCaP and C4-2, indicating that rapamycin is pharmacologically active in both cell lines **(****FIG. 7****).**

### Example 5: Additive to weakly synergistic effects for other drug combinations.

In exploratory studies, an additional group of compounds was screened for activity alone and in combination with PSMA ADC. These agents include the Akt inhibitor MK-2206 (34); the PI3K inhibitor GDC-0941 (35); prednisone; and SB-743921 (36), an inhibitor of the kinesin spindle protein (ksp) Eg5. MK-2206 and GDC-0941 are selective inhibitors of upstream components of the PI3K/mTOR pathway. Prednisone is a component of abiraterone- and docetaxel-based treatment regimens in prostate cancer. SB-743931 was included in our study based on gene expression profiling within the cBio cancer genomics database (37), which revealed coordinated expression (P=0.000004) of the genes for PSMA and Eg5 within primary and metastatic prostate cancer tumors. Each of these compounds showed additive to weakly synergistic activity when combined with PSMA ADC **(****FIG. 8****).** That is, Bliss differences trended towards synergy for most conditions and reached statistical significance in a few isolated cases. No significant antagonism was observed for any combination. Similar results were obtained when prednisone was replaced with its active metabolite, prednisolone.

**Table 2** provides an overview of the synergies observed in this study. For each drug:drug combination, the breadth of synergy is represented as the percentage of evaluable conditions that resulted in statistically significant synergy across the matrix of drug concentrations. Combinations are sorted, highest to lowest, according to the breadth of synergy observed in LNCaP cells. The PSMA ADC/enzalutamide and PSMA ADC/abiraterone combinations showed the broadest synergy in LNCaP cells. Next were PSMA ADC combinations with prednisone or SB-743921. For the PSMA ADC/SB-743921 combination, the data should be interpreted with caution, since several drug-drug combinations resulted in >100% inhibition and thus were not evaluable for Bliss differences. Further testing of this combination over a more focused range of concentrations is warranted. Most combinations did not show significant synergy in LNCaP cells.

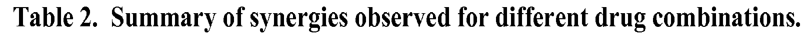

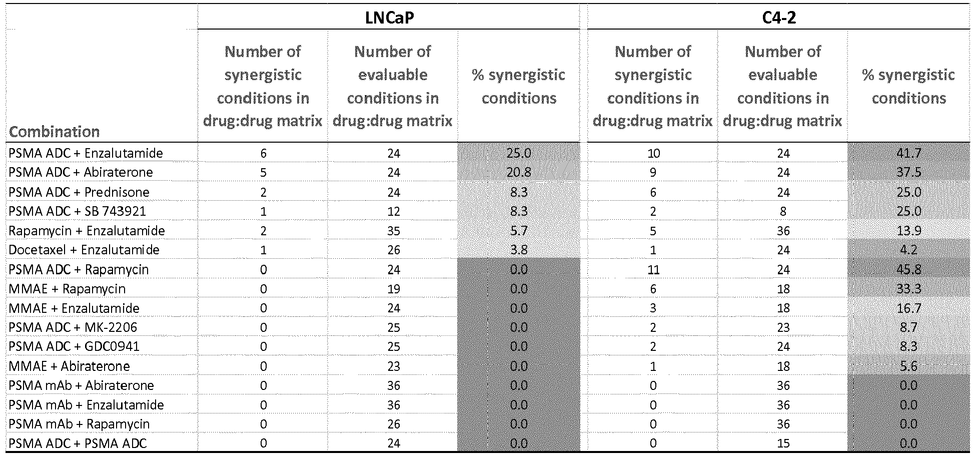

Synergy was more widespread in C4-2 cells. The PSMA ADC/enzalutamide and PSMA ADC/abiraterone combinations exhibited a high frequency of synergistic conditions in C4-2 cells, as did rapamycin in combinations with PSMA ADC or MMAE. Other combinations (*e.g.,* PSMA ADC in combination with MK-2206 or GDC0941) also exhibited significant synergy in C4-2 but not LNCaP cells **(Table 2).** Combinations that did not show significant synergy in either LNCaP or C4-2 cells were those that involved PSMA mAb and the PSMA ADC mock combination.

### Materials and Methods

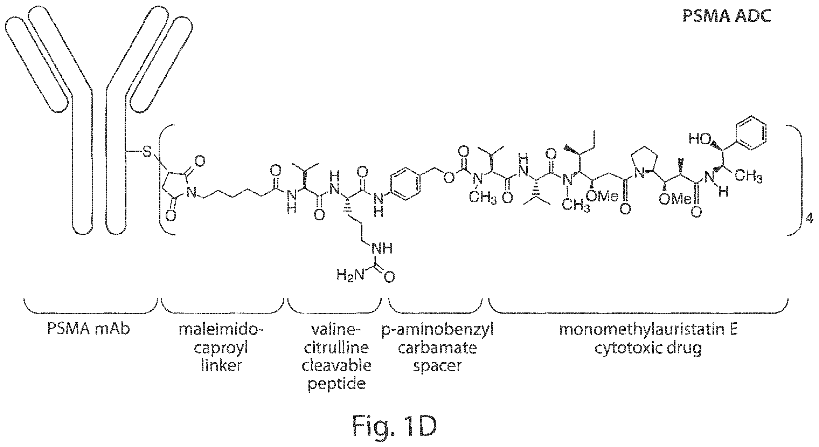

**Materials** LNCaP cells were obtained from American Type Culture Collection (ATCC). C4-2 is an androgen-independent subclone of LNCaP (22). LNCaP and C4-2 are characterized by a T877A mutation in AR and loss of PTEN expression (23, 24). The cell lines allow investigations of androgen dependence in an isogenic background. Both cell lines were passaged in RPMI1640 (Mediatech Inc.) supplemented with 2 mM L-glutamine, 10 mM HEPES, 1 mM sodium pyruvate, 100 µM non-essential amino acids, 1% penicillin/streptomycin and 10% fetal bovine serum (FBS, Life Technologies). Cells were drawn from a common bank and then used within 10 passages. PSMA ADC was prepared as described (20), and enzalutamide and abiraterone (parent drug) were obtained from MedKoo Biosciences **(****FIG. 1****).** Prednisone and prednisolone were purchased from Sigma; rapamycin was from EMD Millipore. GDC0941, MK-226 and SB-743921 were from Selleck Chemicals. The following antibodies were obtained from Santa Cruz Biotechnology: Anti-AR (sc-7305, 441) and anti-PSA (sc-7638, C-19). Two murine anti-PSMA mAbs were used, MAB544 (Maine Biotechnology) for Westerns and 3.9 (Progenics) for flow cytometry. The following antibodies were obtained from Cell Signaling: Total Akt (#2920S), phospho-Akt (S473, #4051L), total-S6 (#2317S), and phospho-S6 (#2211L). Anti-actin antibody (MAB1501) was from Millipore. Isotype-specific secondary IRDye antibodies were from LI-COR Biosciences.

**Cell viability assay** Cells were plated at a density of 1x10³ cells in 25 µL in 384-well white-colored microplates (Perkin Elmer) and cultured overnight. The next day, cells were treated with 1 or 2 drugs in a total volume of 50 µL. Cultures were incubated at 37°C for 7 days. Viability was assessed on days 0 and 7 using CellTiter-Glo (Promega) and an Analyst GT luminescence reader at 560nm emission (Molecular Devices). The percent inhibition of proliferation was calculated as (Vᵤ - Vₜ)/(Vᵤ - V₀) x 100, where Vᵤ, Vₜ and V₀ represent the viability values for untreated cells on day 7, treated cells on day 7, and cells prior to treatment on day 0, respectively. A value of 100% reflects complete inhibition of proliferation (Vₜ = V₀). Values of >100% reflect cytotoxic compounds or combinations, where Vₜ < V₀.

**Flow cytometry** For short-term treatments (≤7 days), cells were plated on 24-well plates (BD Falcon) overnight, at a density of 200,000 cells per 500 µL. The next day, wells were exchanged with 500 µL of fresh media that included inhibitor, and incubated at 37°C until the indicated time point. For the long-term treatments (>7 days), cells were plated on T-25 flasks (BD Falcon) overnight, at a density of 500,000 cells per flask in 5 mL of media. 5 mL of fresh media with or without treatment was exchanged the next day. On weekly splits thereafter, cells were detached with Cell Dissociation solution (Sigma), washed with fresh media, counted using Vi-CELL XR (Beckman Coulter), and split into two T-25 flasks for continued culture with or without inhibitor. Prior to analysis, cells were detached, counted and suspended in PBS (PBS(-), without Ca/Mg, Life Technologies) containing 0.3% bovine serum albumin (BSA, Sigma) and 0.1% sodium azide (VWR). Cells (100,000 in 100 µL) were placed in round-bottom 96-well plates (Falcon) and incubated for 30 minutes at room temperature with 1 µg/mL of anti-PSMA mAb 3.9 conjugated to phycoerythrin (PE). Cells then were washed twice with 200 µL PBS/BSA/azide buffer and read using a FACSCalibur instrument (BD Biosciences). A mouse IgG2b-PE conjugate of irrelevant specificity (Abcam) was included as an isotype control.

**Western blotting** Cells (300,000) were incubated overnight in 6-well plates (BD Falcon) in 4 mL of 10% FBS RPMI1640 media prior to addition of inhibitors. Plates were incubated at 37°C for an additional 7 days, washed once with 5 mL of cold PBS(-) and placed on ice. Cells were lysed with 130 µL of RIPA Lysis Buffer (Santa Cruz Biotechnology) and then centrifuged at 14,000 x g for 10 minutes at 4°C. The supernatant was quantitated for protein concentration using a BCA kit (Pierce/Thermo), resolved under reducing conditions using NuPAGE Novex 4-12% Bis-Tris gels (Life Technologies), and transferred to nitrocellulose using the iBlot 7-Minute Blotting System (Life Technologies). Membranes were blocked using Odyssey Blocking Reagent (LI-COR Biosciences), incubated with primary and secondary antibodies, and visualized using an Odyssey infrared imager (LI-COR Biosciences).

**Synergy calculations and statistical methods** Drug combinations were tested in 3 to 7 independent repeat cell viability assays unless otherwise indicated. Inhibition data were fit to a four-parameter logistic equation using GraphPad Prism. Potential non-additive effects were evaluated by the Bliss independence method (25-27). Briefly, the predicted Bliss value for the combination (F_{c}) was calculated as F_{c} = Fₐ + F_{b} - (Fₐ x F_{b}), where Fₐ and F_{b} represent the observed fractional growth inhibitions caused by compounds A and B used alone. Bliss differences were calculated by subtracting the predicted value (F_{c}) from the experimentally observed inhibition. Mean Bliss differences were calculated for each point in a matrix of drug-drug combinations, converted to percentages, and assessed for statistical significance from the null value of zero using two-sided t-tests with a significance level of 0.05. Statistical evaluations were not performed in cases where the fractional growth inhibition exceeded unity, since such values fall outside the Bliss framework. In addition, Bliss differences of less than 5% were considered to be of limited biological relevance and were excluded from synergy considerations.

### References

1. MacVicar GR, Hussain MH. Emerging therapies in metastatic castration-sensitive and castration-resistant prostate cancer. Curr Opin Oncol 2013;25:252-60.
2. Rathkopf D, Scher HI. Androgen receptor antagonists in castration-resistant prostate cancer. Cancer J 2013;19:43-9.
3. Ferraldeschi R, de Bono J. Agents that target androgen synthesis in castration-resistant prostate cancer. Cancer J 2013;19:34-42.
4. Richards J, Lim AC, Hay CW, Taylor AE, Wingate A, Nowakowska K, et al. Interactions of abiraterone, eplerenone, and prednisolone with wild-type and mutant androgen receptor: a rationale for increasing abiraterone exposure or combining with MDV3100. Cancer Res 2012;72:2176-82.
5. Soifer HS, Souleimanian N, Wu S, Voskresenskiy AM, Collak FK, Cinar B, et al. Direct regulation of androgen receptor activity by potent CYP17 inhibitors in prostate cancer cells. J Biol Chem 2012;287:3777-87.
6. Carver BS, Chapinski C, Wongvipat J, Hieronymus H, Chen Y, Chandarlapaty S, et al. Reciprocal feedback regulation of PI3K and androgen receptor signaling in PTEN-deficient prostate cancer. Cancer Cell 2011;19:575-86.
7. Thomas C, Lamoureux F, Crafter C, Davies BR, Beralidi E, Fazli L, et al. Synergistic targeting of PI3K/AKT-pathway and androgen-receptor axis significantly delays castration-resistant prostate cancer progression in vivo. Mol Cancer Ther 2013;Epub ahead of print.
8. Wright GL, Jr., Grob BM, Haley C, Grossman K, Newhall K, Petrylak D, et al. Upregulation of prostate-specific membrane antigen after androgen-deprivation therapy. Urology 1996;48:326-34.
9. Mostaghel EA, Page ST, Lin DW, Fazli L, Coleman IM, True LD, et al. Intraprostatic androgens and androgen-regulated gene expression persist after testosterone suppression: therapeutic implications for castration-resistant prostate cancer. Cancer Res 2007;67:5033-41.
10. Evans MJ, Smith-Jones PM, Wongvipat J, Navarro V, Kim S, Bander NH, et al. Noninvasive measurement of androgen receptor signaling with a positron-emitting radiopharmaceutical that targets prostate-specific membrane antigen. Proc Natl Acad Sci U S A 2011;108:9578-82.
11. Miyamoto DT, Lee RJ, Stott SL, Ting DT, Wittner BS, Ulman M, et al. Androgen receptor signaling in circulating tumor cells as a marker of hormonally responsive prostate cancer. Cancer Discov 2012;2:995-1003.
12. Foss CA, Mease RC, Cho SY, Kim HJ, Pomper MG. GCPII imaging and cancer. Curr Med Chem 2012;19:1346-59.
13. Olson WC, Heston WDW, Rajasekaran AK. Clinical trials of cancer therapies targeting prostate-specific membrane antigen. Reviews on Recent Clinical Trials 2007;2:182-90.
14. Perner S, Hofer MD, Kim R, Shah RB, Li H, Moller P, et al. Prostate-specific membrane antigen expression as a predictor of prostate cancer progression. Hum Pathol 2007;38:696-701.
15. Ross JS, Sheehan CE, Fisher HA, Kaufman RP, Jr., Kaur P, Gray K, et al. Correlation of primary tumor prostate-specific membrane antigen expression with disease recurrence in prostate cancer. Clin Cancer Res 2003;9:6357-62.
16. Silver DA, Pellicer I, Fair WR, Heston WD, Cordon-Cardo C. Prostate-specific membrane antigen expression in normal and malignant human tissues. Clin Cancer Res 1997;3:81-5.
17. Chang SS, Reuter VE, Heston WD, Bander NH, Grauer LS, Gaudin PB. Five different anti-prostate-specific membrane antigen (PSMA) antibodies confirm PSMA expression in tumor-associated neovasculature. Cancer Res 1999;59:3192-8.
18. Younes A, Bartlett NL, Leonard JP, Kennedy DA, Lynch CM, Sievers EL, et al. Brentuximab vedotin (SGN-35) for relapsed CD30-positive lymphomas. N Engl J Med 2010;363:1812-21.
19. Verma S, Miles D, Gianni L, Krop IE, Welslau M, Baselga J, et al. Trastuzumab emtansine for HER2-positive advanced breast cancer. N Engl J Med 2012;367:1783-91.
20. Ma D, Hopf C, Malewicz AD, Donovan GD, Senter PD, Goeckeler WF, et al. Potent antitumor activity of an auristatin-conjugated, fully human monoclonal antibody to prostate-specific membrane antigen. Clin Cancer Res 2006;12:2591-6.
21. Wang X, Ma D, Olson WC, Heston WD. In vitro and in vivo responses of advanced prostate tumors to PSMA ADC, an auristatin-conjugated antibody to prostate-specific membrane antigen. Mol Cancer Ther 2011;10:1728-39.
22. Wu HC, Hsieh JT, Gleave ME, Brown NM, Pathak S, Chung LW. Derivation of androgen-independent human LNCaP prostatic cancer cell sublines: role of bone stromal cells. Int J Cancer 1994;57:406-12.
23. Veldscholte J, Berrevoets CA, Ris-Stalpers C, Kuiper GG, Jenster G, Trapman J, et al. The androgen receptor in LNCaP cells contains a mutation in the ligand binding domain which affects steroid binding characteristics and response to antiandrogens. J Steroid Biochem Mol Biol 1992;41:665-9.
24. McMenamin ME, Soung P, Perera S, Kaplan I, Loda M, Sellers WR. Loss of PTEN expression in paraffin-embedded primary prostate cancer correlates with high Gleason score and advanced stage. Cancer Res 1999;59:4291-6.
25. Bliss CI. The toxicity of poisons applied jointly. Ann Appl Biol 1939;26:585-615.
26. Wallin JJ, Guan J, Prior WW, Lee LB, Berry L, Belmont LD, et al. GDC-0941, a novel class I selective PI3K inhibitor, enhances the efficacy of docetaxel in human breast cancer models by increasing cell death in vitro and in vivo. Clin Cancer Res 2012;18:3901-11.
27. Wong M, Tan N, Zha J, Peale FV, Yue P, Fairbrother WJ, et al. Navitoclax (ABT-263) reduces Bcl-x(L)-mediated chemoresistance in ovarian cancer models. Mol Cancer Ther 2012;11:1026-35.
28. Squillace RM, Miller D, Wardwell SD, Wang F, Clackson T, Rivera VM. Synergistic activity of the mTOR inhibitor ridaforolimus and the antiandrogen bicalutamide in prostate cancer models. Int J Oncol 2012;41:425-32.
29. Wang Y, Mikhailova M, Bose S, Pan CX, deVere White RW, Ghosh PM. Regulation of androgen receptor transcriptional activity by rapamycin in prostate cancer cell proliferation and survival. Oncogene 2008;27:7106-17.
30. Carracedo A, Ma L, Teruya-Feldstein J, Rojo F, Salmena L, Alimonti A, et al. Inhibition of mTORC1 leads to MAPK pathway activation through a PI3K-dependent feedback loop in human cancer. J Clin Invest 2008;118:3065-74.
31. O'Reilly KE, Rojo F, She QB, Solit D, Mills GB, Smith D, et al. mTOR inhibition induces upstream receptor tyrosine kinase signaling and activates Akt. Cancer Res 2006;66:1500-8.
32. Sarbassov DD, Guertin DA, Ali SM, Sabatini DM. Phosphorylation and regulation of Akt/PKB by the rictor-mTOR complex. Science 2005;307:1098-101.
33. Easton JB, Kurmasheva RT, Houghton PJ. IRS-1: auditing the effectiveness of mTOR inhibitors. Cancer Cell 2006;9:153-5.
34. Hirai H, Sootome H, Nakatsuru Y, Miyama K, Taguchi S, Tsujioka K, et al. MK-2206, an allosteric Akt inhibitor, enhances antitumor efficacy by standard chemotherapeutic agents or molecular targeted drugs in vitro and in vivo. Mol Cancer Ther 2010;9:1956-67.
35. Folkes AJ, Ahmadi K, Alderton WK, Alix S, Baker SJ, Box G, et al. The identification of 2-(1H-indazol-4-yl)-6-(4-methanesulfonyl-piperazin-1-ylmethyl)-4-morpholin-4-yl-t hieno[3,2-d]pyrimidine (GDC-0941) as a potent, selective, orally bioavailable inhibitor of class I PI3 kinase for the treatment of cancer. J Med Chem 2008;51:5522-32.
36. Good JA, Wang F, Rath O, Kaan HY, Talapatra SK, Podgorski D, et al. Optimized S-trityl-L-cysteine-based inhibitors of kinesin spindle protein with potent in vivo antitumor activity in lung cancer xenograft models. J Med Chem 2013;56:1878-93.
37. Cerami E, Gao J, Dogrusoz U, Gross BE, Sumer SO, Aksoy BA, et al. The cBio cancer genomics portal: an open platform for exploring multidimensional cancer genomics data. Cancer Discov 2012;2:401-4.
38. Zhu ML, Horbinski CM, Garzotto M, Qian DZ, Beer TM, Kyprianou N. Tubulin-targeting chemotherapy impairs androgen receptor activity in prostate cancer. Cancer Res 2010;70:7992-8002.
39. Darshan MS, Loftus MS, Thadani-Mulero M, Levy BP, Escuin D, Zhou XK, et al. Taxane-induced blockade to nuclear accumulation of the androgen receptor predicts clinical responses in metastatic prostate cancer. Cancer Res 2011;71:6019-29.
40. Dang ZC, Lowik CW. Removal of serum factors by charcoal treatment promotes adipogenesis via a MAPK-dependent pathway. Mol Cell Biochem 2005;268:159-67.
41. Tran C, Ouk S, Clegg NJ, Chen Y, Watson PA, Arora V, et al. Development of a second-generation antiandrogen for treatment of advanced prostate cancer. Science 2009;324:787-90.
42. Clegg NJ, Wongvipat J, Joseph JD, Tran C, Ouk S, Dilhas A, et al. ARN-509: a novel antiandrogen for prostate cancer treatment. Cancer Res 2012;72:1494-503.
43. Joseph JD, Lu N, Qian J, Sensintaffar J, Shao G, Brigham D, et al. A clinically relevant androgen receptor mutation confers resistance to second-generation antiandrogens enzalutamide and ARN-509. Cancer Discov 2013;3:1020-9.
44. Chen CD, Welsbie DS, Tran C, Baek SH, Chen R, Vessella R, et al. Molecular determinants of resistance to antiandrogen therapy. Nat Med 2004;10:33-9.
45. Pienta KJ, Abate-Shen C, Agus DB, Attar RM, Chung LW, Greenberg NM, et al. The current state of preclinical prostate cancer animal models. Prostate 2008;68:629-39.
46. Garcia JA, Danielpour D. Mammalian target of rapamycin inhibition as a therapeutic strategy in the management of urologic malignancies. Mol Cancer Ther 2008;7:1347-54.
47. Sarker D, Reid AH, Yap TA, de Bono JS. Targeting the PI3K/AKT pathway for the treatment of prostate cancer. Clin Cancer Res 2009;15:4799-805.
48. Bitting RL, Armstrong AJ. Targeting the PI3K/Akt/mTOR pathway in castration-resistant prostate cancer. Endocr Relat Cancer 2013;20:R83-R99.
49. Zhao L, Au JL, Wientjes MG. Comparison of methods for evaluating drug-drug interaction. Front Biosci (Elite Ed) 2010;2:241-9.

## Claims

1. An *in vitro* method of inhibiting proliferation of prostate-specific membrane antigen (PSMA)-expressing cancer cells, the method comprising:
contacting PSMA-expressing cancer cells with an antiandrogen, wherein the antiandrogen is abiraterone, enzalutamide, nilutamide, flutamide, bicalutamide, ARN 509, galeterone or orteronel; and
contacting the PSMA-expressing cancer cells with a PSMA ligand-anticancer agent conjugate or a PSMA ligand-cytotoxic agent conjugate, wherein the PSMA ligand of the conjugate comprises a small molecule ligand that binds specifically PSMA and the small molecule ligand comprises a glutamate-urea-amino acid moiety, wherein the glutamate-urea-amino acid moiety is a glutamate-urea-lysine;
wherein the PSMA-expressing cancer cells comprise PSMA-expressing cancer cells insensitive to antiandrogen therapy.

2. An antiandrogen selected from the group consisting of abiraterone, enzalutamide, nilutamide, flutamide, bicalutamide, ARN 509, galeterone or orteronel for use in an *in vivo* method of inhibiting proliferation of prostate-specific membrane antigen (PSMA)-expressing cancer cells in a subject, the method comprising:
contacting PSMA-expressing cancer cells with the antiandrogen; and
contacting the PSMA-expressing cancer cells with a PSMA ligand-anticancer agent conjugate or a PSMA ligand-cytotoxic agent conjugate, wherein the PSMA ligand of the conjugate comprises a small molecule ligand that binds specifically PSMA and the small molecule ligand comprises a glutamate-urea-amino acid moiety, wherein the glutamate-urea-amino acid moiety is a glutamate-urea-lysine;
wherein the PSMA-expressing cancer cells comprise PSMA-expressing cancer cells insensitive to antiandrogen therapy.

3. A PSMA ligand-anticancer agent conjugate or a PSMA ligand-cytotoxic agent conjugate for use in an *in vivo* method of inhibiting proliferation of prostate-specific membrane antigen (PSMA)-expressing cancer cells in a subject, the method comprising:
contacting PSMA-expressing cancer cells with an antiandrogen selected from the group consisting of abiraterone, enzalutamide, nilutamide, flutamide, bicalutamide, ARN 509, galeterone or orteronel; and
contacting the PSMA-expressing cancer cells with the PSMA ligand-anticancer agent conjugate or a PSMA ligand-cytotoxic agent conjugate, wherein the PSMA ligand of the conjugate comprises a small molecule ligand that binds specifically PSMA and the small molecule ligand comprises a glutamate-urea-amino acid moiety, wherein the glutamate-urea-amino acid moiety is a glutamate-urea-lysine;
wherein the PSMA-expressing cancer cells comprise PSMA-expressing cancer cells insensitive to antiandrogen therapy.

4. The antiandrogen for use according to claim 2, or the PSMA ligand-anticancer agent conjugate or PSMA ligand-cytotoxic agent conjugate for use according to claim 3, wherein the subject has prostate cancer that has progressed despite prior treatment with one or more antiandrogens.

5. The PSMA ligand-anticancer agent conjugate or PSMA ligand-cytotoxic agent conjugate for use according to claim 4, wherein the subject has metastatic castration resistant prostate cancer.

6. A composition comprising an antiandrogen and a PSMA ligand-anticancer agent conjugate or PSMA ligand-cytotoxic agent conjugate, for use in an *in vivo* method of inhibiting proliferation of prostate-specific membrane antigen (PSMA)-expressing cancer cells, wherein the antiandrogen is abiraterone, enzalutamide, nilutamide, flutamide, bicalutamide, ARN 509, galeterone or orteronel, and wherein the PSMA ligand of the conjugate comprises a small molecule ligand that binds specifically PSMA and the small molecule ligand comprises a glutamate-urea-amino acid moiety, wherein the glutamate-urea-amino acid moiety is a glutamate-urea-lysine;
wherein the PSMA-expressing cancer cells comprise PSMA-expressing cancer cells insensitive to antiandrogen therapy.

7. A kit comprising:
a container containing antiandrogen, wherein the antiandrogen is abiraterone, enzalutamide, nilutamide, flutamide, bicalutamide, ARN 509, galeterone or orteronel; and
a container containing a PSMA ligand-anticancer agent conjugate or PSMA ligand-cytotoxic agent conjugate, wherein the PSMA ligand of the conjugate comprises a small molecule ligand that binds specifically PSMA and the small molecule ligand comprises a glutamate-urea-amino acid moiety, wherein the glutamate-urea-amino acid moiety is a glutamate-urea-lysine;
wherein the kit is for use in an *in vivo* method of inhibiting proliferation of prostate-specific membrane antigen (PSMA)-expressing cancer cells, wherein the PSMA-expressing cancer cells comprise PSMA-expressing cancer cells insensitive to antiandrogen therapy;
optionally wherein the compound and/or the PSMA ligand conjugate is in aqueous medium or is lyophilized; and
optionally further comprising a pharmaceutically acceptable carrier and/or an excipient and/or a diluent.

8. The method of claim 1, the compounds for use of claims 2 to 5, the composition for use of claim 6, or the kit of claim 7, wherein the small molecule ligand binds an enzymatic site on PSMA.

9. The method of claim 1, the compounds for use of claims 2 to 5, the composition for use of claim 6, or the kit of claim 7, wherein the small molecule ligand comprises MIP-1095, MIP-1072 or DUPA.

10. The method, compounds for use, or composition for use or kit of claim 9, wherein the PSMA ligand-anticancer agent conjugate comprises MIP-1095 or MIP-1072 conjugated to a cytotoxic radionuclide selected from the group consisting of I¹²³, I¹²⁵, I¹³¹, I¹²⁴ Br⁷⁵, Br⁷⁷ and F¹⁸.

11. The method of any one of claims 1 or 8 to 10 when dependent on claim 1, the compounds for use of claims 2 to 5 or 8 to 10 when dependent on claims 2 to 5, the composition for use of claims 6 or 8 to 10 when dependent on claim 6, or the kit of claims 7 or 8 to 10 when dependent on claim 7, wherein the anticancer agent comprises an auristatin, tubulysin, a pyrrolobenzodiazepine dimer, calicheamicin, colchicine, ispinesib, combrestatin A4, maytansinoid DM1, maytansinoid DM4, doxorubicin, or a cytotoxic radionuclide.

12. The method of any one of claims 1 or 8 to 11 when dependent on claim 1, the compounds for use of claims 2 to 5 or 8 to 11 when dependent on claims 2 to 5, the composition for use of claims 6 or 8 to 11 when dependent on claim 6, or the kit of claims 7 or 8 to 11 when dependent on claim 7, wherein the antiandrogen increases cell surface expression of PSMA in PSMA-expressing cells.

13. The method of any one of claims 1 or 8 to 12 when dependent on claim 1, or the compounds for use of any one of claims 2 to 5 or 8 to 12 when dependent on claims 2 to 5, wherein the step of contacting the PSMA-expressing cancer cells with the antiandrogen and the step of contacting the PSMA-expressing cancer cells with the PSMA ligand-anticancer agent conjugate are concurrent, or wherein the step of contacting the PSMA-expressing cancer cells with the antiandrogen and the step of contacting the PSMA-expressing cancer cells with the PSMA ligand-anticancer agent conjugate are sequential.

14. The method of any one of claims 1 or 8 to 13 when dependent on claim 1, the compounds for use of any one of claims 2 to 5 or 8 to 13 when dependent on claims 2 to 5, or the composition for use of any one of claims 6 or 8 to 13 when dependent on claim 6, wherein the PSMA-expressing cancer cells:
(a) comprise androgen-independent PSMA-expressing cancer cells; and/or
(b) comprise PSMA-expressing cancer cells that have ceased to respond to an antiandrogen therapy; and/or
(c) are of a tumor; and/or
(d) are PSMA-expressing prostate cancer cells; and/or
(e) are PSMA-expressing non-prostate cancer cells.

15. The PSMA ligand-anticancer agent conjugate or PSMA ligand-cytotoxic agent conjugate for use according to claim 3 to 5, or the kit according to claim 7, wherein the PSMA ligand-cytotoxic agent comprises a cytotoxic radionuclide.

16. The PSMA ligand-anticancer agent conjugate or PSMA ligand-cytotoxic agent conjugate for use according to claim 15, or the kit according to claim 15, wherein the cytotoxic radionuclide is I¹²³, I¹²⁵, I¹³¹, I¹²⁴, Br⁷⁵, Br⁷⁷, F¹⁸, ²²⁵Ac, ²¹¹At, ²¹²Bi, ²¹³Bi, ²¹²Pb, ²²⁴Ra, ²²³Ra, ¹⁸⁶Rh, ¹⁸⁸Rh, ¹⁷⁷Lu, ⁹⁰Y, ⁶⁷Cu, ⁶⁴Cu, ¹⁵³Sm or ¹⁶⁶Ho.

## Patentansprüche

1. *In vitro*-Verfahren zum Inhibieren der Proliferation von prostataspezifisches Membranantigen (PSMA)-exprimierenden Krebszellen, wobei das Verfahren umfasst:
Kontaktieren von PSMA-exprimierenden Krebszellen mit einem Antiandrogen, wobei das Antiandrogen Abirateron, Enzalutamid, Nilutamid, Flutamid, Bicalutamid, ARN 509, Galeteron oder Orteronel ist; und
Kontaktieren der PSMA-exprimierenden Krebszellen mit einem Konjugat aus PSMA-Ligand und Antikrebsmittel oder einem Konjugat aus PSMA-Ligand und zytotoxischem Mittel, wobei der PSMA-Ligand des Konjugats einen kleinmolekularen Liganden umfasst, der PSMA spezifisch bindet, und wobei der kleinmolekulare Ligand einen Glutamat-Harnstoff-Aminosäureanteil umfasst, wobei der Glutamat-Harnstoff-Aminosäureanteil ein Glutamat-Harnstoff-Lysin ist;
wobei die PSMA-exprimierenden Krebszellen PSMA-exprimierende Krebszellen umfassen, die gegenüber Antiandrogentherapie unempfindlich sind.

2. Antiandrogen ausgewählt aus der Gruppe bestehend aus Abirateron, Enzalutamid, Nilutamid, Flutamid, Bicalutamid, ARN 509, Galeteron oder Orteronel zur Verwendung in einem *in vivo*-Verfahren zum Inhibieren der Proliferation von prostataspezifisches Membranantigen (PSMA)-exprimierenden Krebszellen bei einem Subjekt, wobei das Verfahren umfasst:
Kontaktieren von PSMA-exprimierenden Krebszellen mit dem Antiandrogen; und
Kontaktieren der PSMA-exprimierenden Krebszellen mit einem Konjugat aus PSMA-Ligand und Antikrebsmittel oder einem Konjugat aus PSMA-Ligand und zytotoxischem Mittel, wobei der PSMA-Ligand des Konjugats einen kleinmolekularen Liganden umfasst, der PSMA spezifisch bindet, und wobei der kleinmolekulare Ligand einen Glutamat-Harnstoff-Aminosäureanteil umfasst, wobei der Glutamat-Harnstoff-Aminosäureanteil ein Glutamat-Harnstoff-Lysin ist;
wobei die PSMA-exprimierenden Krebszellen PSMA-exprimierende Krebszellen umfassen, die gegenüber Antiandrogentherapie unempfindlich sind.

3. Konjugat aus PSMA-Ligand und Antikrebsmittel oder Konjugat aus PSMA-Ligand und zytotoxischem Mittel zur Verwendung in einem *in vivo*-Verfahren zum Inhibieren der Proliferation von prostataspezifisches Membranantigen (PSMA)-exprimierenden Krebszellen in einem Subjekt, wobei das Verfahren umfasst:
Kontaktieren von PSMA-exprimierenden Krebszellen mit einem Antiandrogen ausgewählt aus der Gruppe bestehend aus Abirateron, Enzalutamid, Nilutamid, Flutamid, Bicalutamid, ARN 509, Galeteron oder Orteronel; und
Kontaktieren der PSMA-exprimierenden Krebszellen mit dem Konjugat aus PSMA-Ligand und Antikrebsmittel oder einem Konjugat aus PSMA-Ligand und zytotoxischem Mittel, wobei der PSMA-Ligand des Konjugats einen kleinmolekularen Liganden umfasst, der PSMA spezifisch bindet, und wobei der kleinmolekulare Ligand einen Glutamat-Harnstoff-Aminosäureanteil umfasst, wobei der Glutamat-Harnstoff-Aminosäureanteil ein Glutamat-Harnstoff-Lysin ist;
wobei die PSMA-exprimierenden Krebszellen PSMA-exprimierende Krebszellen umfassen, die gegenüber Antiandrogentherapie unempfindlich sind.

4. Antiandrogen zur Verwendung nach Anspruch 2, oder Konjugat aus PSMA-Ligand und Antikrebsmittel oder Konjugat aus PSMA-Ligand und zytotoxischem Mittel zur Verwendung nach Anspruch 3, wobei das Subjekt Prostatakrebs hat, der trotz vorheriger Behandlung mit einem oder mehreren Antiandrogenen in die Progression gegangen ist.

5. Konjugat aus PSMA-Ligand und Antikrebsmittel oder Konjugat aus PSMA-Ligand und zytotoxischem Mittel zur Verwendung nach Anspruch 4, wobei das Subjekt metastasierten kastrationsresistenten Prostatakrebs hat.

6. Zusammensetzung, umfassend ein Antiandrogen und ein Konjugat aus PSMA-Ligand und Antikrebsmittel oder Konjugat aus PSMA-Ligand und zytotoxischem Mittel zur Verwendung in einem *In vivo*-Verfahren zum Inhibieren der Proliferation von prostataspezifisches Membranantigen (PSMA)-exprimierenden Krebszellen, wobei das Antiandrogen Abirateron, Enzalutamid, Nilutamid, Flutamid, Bicalutamid, ARN 509, Galeteron oder Orteronel ist, und wobei der PSMA-Ligand des Konjugats einen kleinmolekularen Liganden umfasst, der PSMA spezifisch bindet, und wobei der kleinmolekulare Ligand einen Glutamat-Harnstoff-Aminosäureanteil umfasst, wobei der Glutamat-Harnstoff-Aminosäureanteil ein Glutamat-Harnstoff-Lysin ist;
wobei die PSMA-exprimierenden Krebszellen PSMA-exprimierende Krebszellen umfassen, die gegenüber Antiandrogentherapie unempfindlich sind.

7. Kit, umfassend:
einen Behälter, der Antiandrogen enthält, wobei das Antiandrogen Abirateron, Enzalutamid, Nilutamid, Flutamid, Bicalutamid, ARN 509, Galeteron oder Orteronel ist; und
einen Behälter, der Konjugat aus PSMA-Ligand und Antikrebsmittel oder Konjugat aus PSMA-Ligand und zytotoxischem Mittel enthält, wobei der PSMA-Ligand des Konjugats einen kleinmolekularen Liganden umfasst, der PSMA spezifisch bindet, und wobei der kleinmolekulare Ligand einen Glutamat-Harnstoff-Aminosäureanteil umfasst, wobei der Glutamat-Harnstoff-Aminosäureanteil ein Glutamat-Harnstoff-Lysin ist;
wobei das Kit zur Verwendung in einem *In vivo*-Verfahren zum Inhibieren der Proliferation von prostataspezifisches Membranantigen (PSMA)-exprimierenden Krebszellen dient, wobei die PSMA-exprimierenden Krebszellen PSMA-exprimierende Krebszellen umfassen, die gegenüber Antiandrogentherapie unempfindlich sind;
wobei gegebenenfalls die Verbindung und/oder das PSMA-Ligand-Konjugat in wässrigem Medium vorliegt oder lyophilisiert ist; und
gegebenenfalls des Weiteren umfassend einen pharmazeutisch annehmbaren Träger und/oder einen Hilfsstoff und/oder ein Verdünnungsmittel.

8. Verfahren nach Anspruch 1, Verbindungen zur Verwendung nach den Ansprüchen 2 bis 5, Zusammensetzung zur Verwendung nach Anspruch 6 oder Kit nach Anspruch 7, wobei der kleinmolekulare Ligand an eine enzymatische Stelle auf PSMA bindet.

9. Verfahren nach Anspruch 1, Verbindungen zur Verwendung nach den Ansprüchen 2 bis 5, Zusammensetzung zur Verwendung nach Anspruch 6 oder Kit nach Anspruch 7, wobei der kleinmolekulare Ligand MIP-1095, MIP-1072 oder DUPA umfasst.

10. Verfahren, Verbindungen zur Verwendung oder Zusammensetzung zur Verwendung oder Kit nach Anspruch 9, wobei das Konjugat aus PSMA-Ligand und Antikrebsmittel MIP-1095 oder MIP-1072 konjugiert an ein zytotoxisches Radionuklid ausgewählt aus der Gruppe bestehend aus I¹²³, I¹²⁵, I¹³¹, I¹²⁴, Br⁷⁵, Br⁷⁷ und F¹⁸ umfasst.

11. Verfahren nach einem der Ansprüche 1 oder 8 bis 10 in Abhängigkeit von Anspruch 1, Verbindungen zur Verwendung nach den Ansprüchen 2 bis 5 oder 8 bis 10 in Abhängigkeit von den Ansprüchen 2 bis 5, Zusammensetzung zur Verwendung nach Anspruch 6 oder 8 bis 10 in Abhängigkeit von Anspruch 6, oder Kit nach den Ansprüchen 7 oder 8 bis 10 in Abhängigkeit von Anspruch 7, wobei das Antikrebsmittel ein Auristatin, Tubulysin, ein Pyrrolobenzodiazepindimer, Calicheamicin, Colchicin, Ispinesib, Combrestatin A4, Maytansinoid DM1, Maytansinoid DM4, Doxorubicin oder ein zytotoxisches Radionuklid umfasst.

12. Verfahren nach einem der Ansprüche 1 oder 8 bis 11 in Abhängigkeit von Anspruch 1, Verbindungen zur Verwendung nach den Ansprüchen 2 bis 5 oder 8 bis 11 in Abhängigkeit von den Ansprüchen 2 bis 5, Zusammensetzung zur Verwendung nach Anspruch 6 oder 8 bis 11 in Abhängigkeit von Anspruch 6, oder Kit nach den Ansprüchen 7 oder 8 bis 11 in Abhängigkeit von Anspruch 7, wobei das Antiandrogen die Zelloberflächenexpression von PSMA in PSMA exprimierenden Zellen erhöht.

13. Verfahren nach einem der Ansprüche 1 oder 8 bis 12 in Abhängigkeit von Anspruch 1, oder Verbindungen zur Verwendung nach einem der Ansprüche 2 bis 5 oder 8 bis 12 in Abhängigkeit von den Ansprüchen 2 bis 5, wobei der Schritt des Kontaktierens der PSMA-exprimierenden Krebszellen mit dem Antiandrogen und der Schritt des Kontaktierens der PSMA-exprimierenden Krebszellen mit dem Konjugat aus PSMA-Ligand und Antikrebsmittel gleichzeitig erfolgen, oder wobei der Schritt des Kontaktierens der PSMA-exprimierenden Krebszellen mit dem Antiandrogen und der Schritt des Kontaktierens der PSMA-exprimierenden Krebszellen mit dem Konjugat aus PSMA-Ligand und Antikrebsmittel nacheinander erfolgen.

14. Verfahren nach einem der Ansprüche 1 oder 8 bis 13 in Abhängigkeit von Anspruch 1, Verbindungen zur Verwendung nach einem der Ansprüche 2 bis 5 oder 8 bis 13 in Abhängigkeit von den Ansprüchen 2 bis 5, oder die Zusammensetzung zur Verwendung nach einem der Ansprüche 6 oder 8 bis 13 in Abhängigkeit von Anspruch 6, wobei die PSMA-exprimierenden Krebszellen:
(a) androgenunabhängige PSMA-exprimierende Krebszellen umfassen; und/oder
(b) PSMA-exprimierende Krebszellen umfassen, die aufgehört haben, auf eine Antiandrogentherapie anzusprechen; und/oder
(c) von einem Tumor sind; und/oder
(d) PSMA-exprimierende Prostatakrebszellen sind; und/oder
(e) PSMA-exprimierende Nicht-Prostatakrebszellen sind.

15. Konjugat aus PSMA-Ligand und Antikrebsmittel oder Konjugat aus PSMA-Ligand und zytotoxischem Mittel zur Verwendung nach Anspruch 3 bis 5 oder Kit nach Anspruch 7, wobei das PSMA-Ligand-zytotoxische Mittel ein zytotoxisches Radionuklid umfasst.

16. Konjugat aus PSMA-Ligand und Antikrebsmittel oder Konjugat aus PSMA-Ligand und zytotoxischem Mittel zur Verwendung nach Anspruch 15 oder Kit nach Anspruch 15, wobei das zytotoxische Radionuklid I¹²³, I¹²⁵, I¹³¹, I¹²⁴, Br⁷⁵, Br⁷⁷, F¹⁸, ²²⁵Ac, ²¹¹At, ²¹²Bi, ²¹³Bi, ²¹²Pb, ²²⁴Ra, ²²³Ra, ¹⁸⁶Rh, ¹⁸⁸Rh, ¹⁷⁷Lu, ⁹⁰Y, ⁶⁷Cu, ⁶⁴Cu, ¹⁵³Sm oder ¹⁶⁶Ho ist.

## Revendications

1. Procédé *in vitro* d'inhibition de prolifération de cellules cancéreuses exprimant l'antigène membranaire spécifique de la prostate (PSMA), le procédé comprenant :
la mise en contact de cellules cancéreuses exprimant le PSMA avec un antiandrogène, où l'antiandrogène est l'abiratérone, l'enzalutamide, le nilutamide, le flutamide, le bicalutamide, l'ARN 509, la galétérone ou l'ortéronel ; et
la mise en contact des cellules cancéreuses exprimant le PSMA avec un conjugué ligand de PSMA-agent anticancéreux ou un conjugué ligand de PSMA-agent cytotoxique, où le ligand de PSMA du conjugué comprend un ligand à petite molécule qui se lie spécifiquement au PSMA et le ligand à petite molécule comprend une fraction glutamate-urée-acide aminé, où la fraction glutamate-urée-acide aminé est un glutamate-urée-lysine ;
où les cellules cancéreuses exprimant le PSMA comprennent des cellules cancéreuses exprimant le PSMA insensibles à une thérapie antiandrogène.

2. Antiandrogène choisi dans le groupe constitué par l'abiratérone, l'enzalutamide, le nilutamide, le flutamide, le bicalutamide, l'ARN 509, la galétérone ou l'ortéronel, pour utilisation dans un procédé *in vivo* d'inhibition de prolifération de cellules cancéreuses exprimant l'antigène membranaire spécifique de la prostate (PSMA) chez un sujet, le procédé comprenant :
la mise en contact de cellules cancéreuses exprimant le PSMA avec l'antiandrogène ; et
la mise en contact des cellules cancéreuses exprimant le PSMA avec un conjugué ligand de PSMA-agent anticancéreux ou un conjugué ligand de PSMA-agent cytotoxique, où le ligand de PSMA du conjugué comprend un ligand à petite molécule qui se lie spécifiquement au PSMA et le ligand à petite molécule comprend une fraction glutamate-urée-acide aminé, où la fraction glutamate-urée-acide aminé est un glutamate-urée-lysine ;
où les cellules cancéreuses exprimant le PSMA comprennent des cellules cancéreuses exprimant le PSMA insensibles à une thérapie antiandrogène.

3. Conjugué ligand de PSMA-agent anticancéreux ou conjugué ligand de PSMA-agent cytotoxique pour utilisation dans un procédé *in vivo* d'inhibition de prolifération de cellules cancéreuses exprimant l'antigène membranaire spécifique de la prostate (PSMA) chez un sujet, le procédé comprenant :
la mise en contact de cellules cancéreuses exprimant le PSMA avec un antiandrogène choisi dans le groupe constitué par l'abiratérone, l'enzalutamide, le nilutamide, le flutamide, le bicalutamide, l'ARN 509, la galétérone ou l'ortéronel ; et
la mise en contact des cellules cancéreuses exprimant le PSMA avec le conjugué ligand de PSMA-agent anticancéreux ou un conjugué ligand de PSMA-agent cytotoxique, où le ligand de PSMA du conjugué comprend un ligand à petite molécule qui se lie spécifiquement au PSMA et le ligand à petite molécule comprend une fraction glutamate-urée-acide aminé, où la fraction glutamate-urée-acide aminé est un glutamate-urée-lysine ;
où les cellules cancéreuses exprimant le PSMA comprennent des cellules cancéreuses exprimant le PSMA insensibles à une thérapie antiandrogène.

4. Antiandrogène pour utilisation selon la revendication 2, ou conjugué ligand de PSMA-agent anticancéreux ou conjugué ligand de PSMA-agent cytotoxique pour utilisation selon la revendication 3, où le sujet a un cancer prostatique qui a progressé malgré un traitement préalable par un ou plusieurs antiandrogènes.

5. Conjugué ligand de PSMA-agent anticancéreux ou conjugué ligand de PSMA-agent cytotoxique pour utilisation selon la revendication 4, où le sujet a un cancer prostatique métastatique résistant à la castration.

6. Composition comprenant un antiandrogène et un conjugué ligand de PSMA-agent anticancéreux ou un conjugué ligand de PSMA-agent cytotoxique, pour utilisation dans un procédé *in vivo* d'inhibition de prolifération de cellules cancéreuses exprimant l'antigène membranaire spécifique de la prostate (PSMA), où l'antiandrogène est l'abiratérone, l'enzalutamide, le nilutamide, le flutamide, le bicalutamide, l'ARN 509, la galétérone ou l'ortéronel, et où le ligand de PSMA du conjugué comprend un ligand à petite molécule qui se lie spécifiquement au PSMA et le ligand à petite molécule comprend une fraction glutamate-urée-acide aminé, où la fraction glutamate-urée-acide aminé est un glutamate-urée-lysine ;
où les cellules cancéreuses exprimant le PSMA comprennent des cellules cancéreuses exprimant le PSMA insensibles à une thérapie antiandrogène.

7. Coffret comprenant :
un récipient contenant un antiandrogène, où l'antiandrogène est l'abiratérone, l'enzalutamide, le nilutamide, le flutamide, le bicalutamide, l'ARN 509, la galétérone ou l'ortéronel ; et
un récipient contenant un conjugué ligand de PSMA-agent anticancéreux ou un conjugué ligand de PSMA-agent cytotoxique, où le ligand de PSMA du conjugué comprend un ligand à petite molécule qui se lie spécifiquement au PSMA et le ligand à petite molécule comprend une fraction glutamate-urée-acide aminé, où la fraction glutamate-urée-acide aminé est un glutamate-urée-lysine ;
où le coffret est à utiliser dans un procédé *in vivo* d'inhibition de prolifération de cellules cancéreuses exprimant l'antigène membranaire spécifique de la prostate (PSMA), où les cellules cancéreuses exprimant le PSMA comprennent des cellules cancéreuses exprimant le PSMA insensibles à une thérapie antiandrogène ;
facultativement où le composé et/ou le conjugué de ligand de PSMA est en milieu aqueux ou est lyophilisé ; et
comprenant facultativement en outre un support et/ou un excipient et/ou un diluant pharmaceutiquement acceptable.

8. Procédé selon la revendication 1, composés pour utilisation selon les revendications 2 à 5, composition pour utilisation selon la revendication 6, ou coffret selon la revendication 7, où le ligand à petite molécule se lie à un site enzymatique sur le PSMA.

9. Procédé selon la revendication 1, composés pour utilisation selon les revendications 2 à 5, composition pour utilisation selon la revendication 6, ou coffret selon la revendication 7, où le ligand à petite molécule comprend du MIP-1095, du MIP-1072 ou du DUPA.

10. Procédé, composés à utiliser, ou composition à utiliser ou coffret selon la revendication 9, dans lequel le conjugué ligand de PSMA-agent anticancéreux comprend MIP-1095 ou MIP-1072 conjugué à un radionucléide cytotoxique choisi dans le groupe constitué de I¹²³, I¹²⁵, I¹³¹, I¹²⁴, Br⁷⁵, Br⁷⁷ et F¹⁸.

11. Procédé selon l'une quelconque des revendications 1 ou 8 à 10 lorsqu'elles dépendent de la revendication 1, composés pour utilisation selon les revendications 2 à 5 ou 8 à 10 lorsqu'elles dépendent des revendications 2 à 5, composition pour utilisation selon les revendications 6 ou 8 à 10 lorsqu'elles dépendent de la revendication 6, ou coffret selon les revendications 7 ou 8 à 10 lorsqu'elles dépendent de la revendication 7, où l'agent anticancéreux comprend une auristatine, de la tubulysine, un dimère de pyrrolobenzodiazépine, de la calichéamicine, de la colchicine, de l'ispinésib, de la combrestatine A4, du maytansinoïde DM1, du maytansinoïde DM4, de la doxorubicine, ou un radionucléide cytotoxique.

12. Procédé selon l'une quelconque des revendications 1 ou 8 à 11 lorsqu'elles dépendent de la revendication 1, composés pour utilisation selon les revendications 2 à 5 ou 8 à 11 lorsqu'elles dépendent des revendications 2 à 5, composition pour utilisation selon les revendications 6 ou 8 à 11 lorsqu'elles dépendent de la revendication 6, ou coffret selon les revendications 7 ou 8 à 11 lorsqu'elles dépendent de la revendication 7, où l'antiandrogène augmente l'expression de surface cellulaire du PSMA dans les cellules exprimant le PSMA.

13. Procédé selon l'une quelconque des revendications 1 ou 8 à 12 lorsqu'elles dépendent de la revendication 1, ou composés pour utilisation selon l'une quelconque des revendications 2 à 5 ou 8 à 12 lorsqu'elles dépendent des revendications 2 à 5, où l'étape de mise en contact des cellules cancéreuses exprimant le PSMA avec l'antiandrogène et l'étape de mise en contact des cellules cancéreuses exprimant le PSMA avec le conjugué ligand de PSMA-agent anticancéreux sont simultanées, ou où l'étape de mise en contact des cellules cancéreuses exprimant le PSMA avec l'antiandrogène et l'étape de mise en contact des cellules cancéreuses exprimant le PSMA avec le conjugué ligand de PSMA-agent anticancéreux sont séquentielles.

14. Procédé selon l'une quelconque des revendications 1 ou 8 à 13 lorsqu'elles dépendent de la revendication 1, composés pour utilisation selon l'une quelconque des revendications 2 à 5 ou 8 à 13 lorsqu'elles dépendent des revendications 2 à 5, ou composition pour utilisation selon l'une quelconque des revendications 6 ou 8 à 13 lorsqu'elles dépendent de la revendication 6, où les cellules cancéreuses exprimant le PSMA :
(a) comprennent des cellules cancéreuses exprimant le PSMA androgéno-indépendantes ; et/ou
(b) comprennent des cellules cancéreuses exprimant le PSMA qui ont cessé de répondre à une thérapie antiandrogène ; et/ou
(c) proviennent d'une tumeur ; et/ou
(d) sont des cellules de cancer prostatique exprimant le PSMA ; et/ou
(e) sont des cellules de cancer non prostatique exprimant le PSMA.

15. Conjugué ligand de PSMA-agent anticancéreux ou conjugué ligand de PSMA-agent cytotoxique pour utilisation selon les revendications 3 à 5, ou coffret selon la revendication 7, où le ligand de PSMA-agent cytotoxique comprend un radionucléide cytotoxique.

16. Conjugué ligand de PSMA-agent anticancéreux ou conjugué ligand de PSMA-agent cytotoxique pour utilisation selon la revendication 15, ou coffret selon la revendication 15, où le radionucléide cytotoxique est I¹²³ , I¹²⁵, I¹³¹, I¹²⁴, Br⁷⁵, Br⁷⁷, F¹⁸, ²²⁵Ac, ²¹¹At, ²¹²Bi, ²¹³Bi, ²¹²Pb, ²²⁴Ra, ²²³Ra, ¹⁸⁶Rh, ¹⁸⁸Rh, ¹⁷⁷Lu, ⁹⁰Y, ⁶⁷Cu, ⁶⁴Cu, ¹⁵³Sm ou ¹⁶⁶Ho.
